# EUROPEAN PATENT SPECIFICATION

(11) **EP 1 311 162 B1**
(45) Date of publication and mention of the grant of the patent: **01.06.2005**
(21) Application number: 01965209.8
(22) Date of filing: 23.08.2001
(51) Int. Cl.: A01N 63/00, C12N 15/62, C12N 15/32, C12N 15/82, C12N 5/10, C07K 14/325, C07K 19/00

(54) **BACILLUS THURIGIENSIS CRYSTAL PROTEIN HYBRIDS**
HYBRIDEN VON CRYSTAL PROTEINEN AUS BACILLUS THURIGIENSIS
HYBRIDES DE PROTEINES CRISTALLINES DERIVEES DE BACILLUS THURINGIENSIS

(30) Priority: 25.08.2000 US 227956 P
(43) Date of publication of application: 21.05.2003
(73) Proprietor: Syngenta Participations AG, 4058 Basel (CH)
(72) Inventor: CAROZZI, Nadine Barbara, Research Triangle Park, NC 27709 (US); RABE, Scott M., Research Triangle Park, NC 27709 (US); MILES, Paul J., Research Triangle Park, NC 27709 (US); WARREN, Gregory Wayne, Research Triangle Park, NC 27709 (US); DE HAAN, Petrus Theodorus, NL-2343 RR Oegstgeest (NL)
(74) Representative: Bastian, Werner Maria
(86) International application number: PCT/EP2001/009751
(87) International publication number: WO 2002/015701

(56) References cited:
- WO-A-95/06730
- DE MAAGD RUUD A ET AL: "Domain III substitution in Bacillus thuringiensis delta-endotoxin CryIA(b) results in superior toxicity for Spodoptera exigua and altered membrane protein recognition." APPLIED AND ENVIRONMENTAL MICROBIOLOGY, vol. 62, no. 5, 1996, pages 1537-1543, XP002209205 ISSN: 0099-2240
- BALLESTER V ET AL: "Role of Bacillus thuringiensis toxin domains in toxicity and receptor binding in the diamondback moth." APPLIED AND ENVIRONMENTAL MICROBIOLOGY, vol. 65, no. 5, May 1999 (1999-05), pages 1900-1903, XP002209206 ISSN: 0099-2240
- RANG CECILE ET AL: "Interaction between functional domains of Bacillus thuringiensis insecticidal crystal proteins." APPLIED AND ENVIRONMENTAL MICROBIOLOGY, vol. 65, no. 7, July 1999 (1999-07), pages 2918-2925, XP002209207 ISSN: 0099-2240

## Description

The invention relates to novel insecticidal toxins derived from *Bacillus thuringiensis* insecticidal crystal proteins, nucleic acid sequences whose expression results in said toxins, and methods of making and methods of using the toxins and corresponding nucleic acid sequences to control insects.

Insect pests are a major cause of crop losses. Solely in the US, billions of dollars are lost every year due to infestation by various genera of insects. In addition to losses in field crops, insect pests are also a burden to vegetable and fruit growers, to producers of ornamental flowers, and they are a nuisance to gardeners and homeowners.

Insect pests are mainly controlled by intensive applications of chemical insecticides, which are active through inhibition of insect growth, prevention of insect feeding or reproduction, or death of the insects. Good insect control can thus be reached, but these chemicals can sometimes also affect other, beneficial insects. Another problem resulting from the wide use of chemical pesticides is the appearance of resistant insect varieties. This has been partially alleviated by various resistance management strategies, but there is an increasing need for alternative pest control agents.

Biological insect control agents, such as *Bacillus thuringiensis* strains expressing insecticidal toxins have also been applied with satisfactory results, offering an alternative or a complement to chemical insecticides. *Bacillus thuringiensis* belongs to the large group of gram-positive, aerobic, endospore-forming bacteria. Unlike other very closely related species of *Bacillus* such as *B. cereus* or *B. anthracis,* the majority of the hitherto known *Bacillus thuringiensis* species produce in the course of their sporulation a parasporal inclusion body which, on account of its crystalline structure, is generally referred to also as a crystalline body. This crystalline body is composed of insecticidally active crystalline protoxin proteins, the so-called δ-endotoxins. These protein crystals are responsible for the toxicity to insects of *Bacillus thuringiensis.* The δ-endotoxin does not exhibit its insecticidal activity until after oral intake of the crystalline body, when the latter is dissolved in the intestinal juice of the target insects. In most cases the actual toxic component is released from the protoxin as a result of proteolytic cleavage caused by the action of proteases from the digestive tract of the insects. The δ-endotoxins of the various *Bacillus thuringiensis* strains are characterized by high specificity with respect to certain target insects, especially with respect to various Lepidoptera, Coleoptera and Diptera larvae, and by a high degree of activity against these larvae. A further advantage in using δ-endotoxins of *Bacillus thuringiensis* resides in the fact that the toxins are harmless to humans, other mammals, birds and fish.

Based on sequence homology and insecticidal specificity, *Bacillus thuringiensis* crystal proteins have been categorized into different classes. Best studied are the Cry1 class of proteins, which are produced as 140 kDa pro-toxins and are active towards lepidopterans. To some extent the mode of action of crystal proteins has been elucidated. After oral uptake the crystals dissolve in the alkaline environment of the larval midgut. The solubilized proteins are subsequently processed by midgut proteinases (e.g. trypsin) to a proteinase-resistant toxic fragment of about 65kDa that binds to receptors on epithelial cells of the insect midgut and penetrates the cell membrane. This eventually leads to bursting of the cells and death of the larvae.

The activity spectrum of a particular crystal protein is to a large extent determined by the occurrence of receptors on the midgut epithelial cells of susceptible insects. The spectrum is co-determined by the efficiency of solubilization of the crystal protein and its proteolytic activation *in vivo.* The importance of the binding of the crystal protein to midgut epithelial receptors is further demonstrated where insects have developed resistance to one of the crystal proteins in that the binding of crystal proteins to midgut epithelial cells in resistant insects is significantly reduced.

In the past several years, the genes coding for some of these crystal proteins have been isolated and their expression in heterologous hosts have been shown to provide another tool for the control of economically important insect pests. In particular, the expression of insecticidal toxins in transgenic plants, such as *Bacillus thuringiensis* crystal proteins, has provided efficient protection against selected insect pests, and transgenic plants expressing such toxins have been commercialized, allowing farmers to reduce applications of chemical insect control agents. Furthermore, it is also possible to express recombinant toxins that have a chosen combination of functions designed to enhance the degree of insecticidal activity against a particular insect or insect class, or to expand the spectrum of insects against which the toxin protein is active. For example, chimeric insecticidal proteins can be constructed having novel sequences not found in nature by combining the toxin portion from one δ-endotoxin with the protoxin (tail) portion of a different δ-endotoxin. *See,* for example, WO 98/15170, incorporated herein by reference.

Toxic fragments of crystal proteins are thought to be composed of three distinct structural domains. Domain I, the most N-terminal domain, consists of 7 a-helices and probably is partially or entirely inserted in the target cell membrane. Domain II comprises 3 β-sheets in a so-called Greek key-conformation. Domain II is believed by most researchers to interact with receptors and to thereby determine toxin specificity. Indeed, there is much evidence implicating domain II residues in specific toxicity and in high affinity binding. Domain III, the most C-terminal domain, consists of two β-sheets in a so-called jellyroll conformation and has also been implicated in determining specificity. Swapping domain III between toxins, such as by *in vivo* recombination between the coding regions, can result in changes in specific activity. Binding experiments using such hybrids have shown that domain III is involved in binding to putative receptors of target insects, suggesting that domain III may exert its role in specificity through a role in receptor recognition. If projected on Cry1 sequences, domain I runs from about amino acid residue 28 to 260, domain II from about 260 to 460 and domain III from about 460 to 600. *See*, Nakamura *et al., Agric. Biol. Chem*. 54(3): 715-724 (1990); Li *et al., Nature* 353: 815-821 (1991); Ge *et al., J. Biol. Chem*. 266(27): 17954-17958 (1991); and Honee *et al., Mol. Microbiol*. 5(11): 2799-2806 (1991); U.S. Pat. No. 5,736,131 (corresponding to International Publication Number WO95/06730), describes *Bacillus thuringiensis* hybrid toxin fragments comprising at their C-terminus domain III of a first Cry protein and at its N-terminus domains I and II of a second Cry protein. Such hybrid crystal proteins have altered insecticidal specificity. For example, the H04 hybrid toxin, which is also described in De Maagd *et al., Appl. Environ. Microbiol*. 62(5): 1537-1543 (1996), comprises at its N-terminus domains I and II of Cry1Ab and at its C-terminus domain III of Cry1C. H04 is reportedly highly toxic to *Spodoptera exigua* (beet anmyworm) compared with the parental Cry1Ab toxin and significantly more toxic than the Cry1C parental toxin. *See also*, Bosch *et al., FEMS Microbiology Letters* 118: 129-134 (1994); Bosch *et al., Bio*/*Technology* 12: 915-918 (1994); De Maagd *et al., Appl. Environ. Microbiol*. 62(8): 2753-2757 (1996); and De Maagd *et al., Mol. Microbiol*. 31(2): 463-471 (1999). Ballester *et al*., Appl. Environ. Microbiol. 65(5): 1900-1903 (1999) describes *Bacillus thuringiensis* hybrid toxins with domain III substitutions among Cry1E, Cry1C and Cry1Ab. Rang et al., Appl. Environ. Microbiol. 65(7): 2918-2925 (1999) describes hybrid genes prepared by exchanging the regions coding for either domain I or domain III among Cry1Ab, Cry1Ac, Cry1C and Cry1E.

Despite the previous successes realized by incorporation of insect resistant genes through breeding programs and genetic engineering, there remains a long-felt but unfulfilled need to discover new and effective insect control agents. Particularly needed are control agents that are targeted to economically important insect pests such as European Corn Borer and Fall Army Worm and that efficiently control insect species resistant to existing insect control agents. Furthermore, agents whose application minimizes the burden on the environment are desirable.

The present invention addresses the aforementioned needs by providing novel gene sequences that encode hybrid *Bacillus thuringiensis* toxins, including synthetic nucleotide sequences optimized for expression in plants. In preferred embodiments, the novel gene sequences encode varying forms of the hybrid *Bacillus thuringiensis* delta-endotoxin H04, the toxin portion of which contains domains I and II of Cry1Ab and domain III of Cry1C. The hybrid *Bacillus thuringiensis* toxins encoded by the novel gene sequences are highly active against economically important insect pests such as fall armyworm, pink bollworm, tobacco budworm, European comborer, and diamondback moth. The hybrid *Bucillus thuringiensis* toxins can be used in multiple insect control strategies, resulting in maximal efficiency with minimal impact on the environment.

The invention is further drawn to the hybrid insecticidal toxins resulting from the expression of the nucleotide sequences of the invention, and to compositions and formulations containing the hybrid insecticidal toxins, which are capable of inhibiting the ability of insect pests to survive, grow or reproduce, or of limiting insect-related damage or loss in crop plants. The invention is further drawn to a method of making the hybrid toxins and to methods of using the nucleotide sequences, for example in transgenic plants to confer insect resistance, and to methods of using the toxins, and compositions and formulations comprising the toxins, for example applying the toxins, composition, or formulation to insect infested areas, or to prophylactically treat insect susceptible areas or plants to confer protection or resistance against harmful insects. The hybrid toxins can be used in multiple insect control strategies, resulting in maximal efficiency with minimal impact on the environment.

According to one aspect, the present invention provides a method for controlling an insect selected from the group consisting of fall armyworm, pink bollworm, tobacco budworm, European comborer and diamondback moth, comprising delivering to the insect an effective amount of a hybrid *Bacillus thuringiensis* toxin comprising domains I and II from a Cry1Ab toxin joined in the amino to carboxy direction to domain III from a Cry1C toxin. In a preferred embodiment, the hybrid *Bacillus thuringiensis* toxin comprises an amino acid sequence at least 90% identical to SEQ ID NO:2, 4, 6, 8, or 10. In a more preferred embodiment, the hybrid *Bacillus thuringiensis* toxin comprises SEQ ID NO:2, 4, 6, 8, or 10.

In another embodiment of the above-described method of the invention, the hybrid *Bacillus thuringiensis* toxin further comprises a C-terminal tail region, such as a Cry1C tail region or a Cry1Ab tail region. The C-terminal tail region may be full-length or may be truncated, such as to approximately 40 amino acids in length.

In a preferred embodiment of the above-described method of the invention, delivering an effective amount of the hybrid *Bacillus thuringiensis* toxin to the insect comprises feeding or contacting the insect with transgenic plant tissue transformed with recombinant DNA comprising a nucleotide sequence that encodes the hybrid *Bacillus thuringiensis* toxin, wherein expression of the hybrid *Bacillus thuringiensis* toxin in said transgenic plant tissue confers resistance to the insect. Preferably, said nucleotide sequence is substantially identical to SEQ ID NO:1, 3, 5, 7, or 9.

According to another aspect, the present invention provides an isolated nucleic acid molecule comprising a nucleotide sequence that encodes a hybrid *Bacillus thuringiensis* toxin comprising: (a) an N-terminal toxin portion comprising domains I and II from a Cry1Ab toxin joined in the amino to carboxy direction to domain III from a Cry1C toxin; and (b) a C-terminal tail region from a Cry1Ab toxin. Preferably, the hybrid *Bacillus thuringiensis* toxin comprises an amino acid sequence at least 90% identical to SEQ ID NO:6, 8, or 10. More preferably, the hybrid *Bacillus thuringiensis* toxin comprises SEQ ID NO: 6, 8, or 10. Even more preferably, said nucleotide sequence is at least 90% identical to SEQ ID NO:5, 7, or 9. Most preferably, said nucleotide sequence comprises SEQ ID NO: 5, 7, or 9.

The present invention further provides a chimeric gene comprising a heterologous promoter sequence operatively linked to a nucleic acid molecule of the invention, as described above; a recombinant vector comprising such a chimeric gene; a transgenic host cell (e.g., a plant cell) comprising such a chimeric gene; a transgenic plant (e.g., a maize, cotton, rice, or cabbage plant) comprising such a transgenic plant cell; and seed from such a transgenic plant.

According to yet another aspect, the present invention provides a method of protecting a plant against insects, comprising expressing a hybrid *Bacillus thuringiensis* toxin in a plant transformed with a chimeric gene comprising: (a) a nucleic acid promoter sequence that promotes in a plant the transcription of an associated coding sequence at elevated levels, and (b) a nucleic acid molecule according to the invention operatively linked to said promoter sequence, wherein expression of the hybrid *Bacillus thuringiensis* toxin in said plant protects said plant against insects.

According to still another aspect, the present invention provides a method of producing a hybrid *Bacillus thuringiensis* toxin that is active against insects, comprising: (a) obtaining a transgenic host cell according to the invention; and (b) expressing the nucleic acid molecule of the invention in said transgenic host cell, which results in a hybrid *Bacillus thuringiensis* toxin that is active against insects.

According to still another aspect, the present invention provides a method of producing a plant resistant to insects, comprising introducing a nucleic acid molecule according to the present invention into said plant, wherein said nucleic acid molecule is expressible in said plant in an amount effective to control insects.

According to another aspect, the present invention provides an isolated nucleic acid molecule comprising SEQ ID NO:3, 5, 7, 9, 11, 12, 13, 14, 15, 16 or 17; a chimeric gene comprising a heterologous promoter sequence operatively linked to such a nucleic acid molecules; a recombinant vector comprising such a chimeric gene; a transgenic host cell (e.g., a plant cell) comprising such a chimeric gene; a transgenic plant (e.g., a maize, cotton, rice, or cabbage plant) comprising such a transgenic plant cell; and seed from such a transgenic plant.

According to a still further aspect, the present invention provides a hybrid *Bacillus thuringiensis* toxin, comprising: (a) an N-terminal toxin portion comprising domains I and II from a Cry1Ab toxin joined in the amino to carboxy direction to domain III from a Cry1C toxin; and (b) a C-terminal tail region from a Cry1Ab toxin. Preferably, the hybrid *Bacillus thuringiensis* toxin of the invention comprises an amino acid sequence at least 90% identical to SEQ ID NO:6, 8, or 10. More preferably, the hybrid *Bacillus thuringiensis* toxin of the invention comprises SEQ ID NO:6, 8, or 10.

According to a further aspect, the presesnt invention provides a composition comprising the hybrid *Bacillus thuringiensis* toxin of the invention in an amount effective to control insects.

Other aspects and advantages of the present invention will become apparent to those skilled in the art from a study of the following description of the invention and non-limiting examples.

### BRIEF DESCRIPTION OF THE SEQUENCES IN THE SEQUENCE LISTING

SEQ ID NO:1 shows the nucleotide sequence encoding the H04 hybrid toxin described in De Maagd *et al., Appl. Environ. Microbiol*. 62(5): 1537-1543 (1996), the toxin portion of which comprises at its N-terminus domains I and II of Cry1Ab and at its C-terminus domain III of Cry1C, plus afull-length Cry1C tail portion.

SEQ ID NO:2 shows the amino acid sequence of the H04 hybrid toxin encoded by the nucleotide sequence depicted in SEQ ID NO:1, comprising toxin domains I and II of Cry1Ab and toxin domain III of Cry1C, plus a full-length Cry1C tail portion.

SEQ ID NO:3 shows a synthetic nucleotide sequence encoding the toxin portion of H04 without a tail, as if the trypsin site had been cleaved.

SEQ ID NO:4 shows the amino acid sequence of the H04 toxin portion encoded by the synthetic nucleotide sequence depicted in SEQ ID NO:3.

SEQ ID NO:5 shows a synthetic nucleotide sequence encoding the toxin portion of H04 plus a full-length Cry1Ab tail portion.

SEQ ID NO:6 shows the amino acid sequence of the H04 + Cry1Ab tail encoded by the synthetic nucleotide sequence depicted in SEQ ID NO:5.

SEQ ID NO:7 shows another synthetic nucleotide sequence encoding the toxin portion of H04 plus a full-length Cry1Ab tail portion.

SEQ ID NO:8 shows the amino acid sequence of the H04 + Cry1Ab tail encoded by the synthetic nucleotide sequence depicted in SEQ ID NO:7.

SEQ ID NO:9 shows a synthetic nucleotide sequence encoding the toxin portion of H04 plus the first 40 amino acids of the Cry1Ab tail.

SEQ ID NO:10 shows the amino acid sequence of the H04 + 40-amino acid truncated Cry1Ab tail encoded by the synthetic nucleotide sequence depicted in SEQ ID NO:9.

SEQ ID NO:11 shows the nucleotide sequence of construct pNOV1308, which contains the constitutive maize ubiquitin promoter operatively linked to the synthetic nucleotide sequence encoding the toxin portion of H04 without a tail, as set forth in SEQ ID NO:3.

SEQ ID NO:12 shows the nucleotide sequence of construct pNOV1436, which contains the root-preferred maize MTL promoter operatively linked to the synthetic nucleotide sequence encoding the toxin portion of H04 plus a full-length Cry1Ab tail portion, as set forth in SEQ ID NO:5.

SEQ ID NO:13 shows the nucleotide sequence of construct pNOV1441, which contains the constitutive maize ubiquitin promoter operatively linked to the synthetic nucleotide sequence encoding the toxin portion of H04 plus a full-length Cry1Ab tail portion, as set forth in SEQ ID NO:5.

SEQ ID NO:14 shows the nucleotide sequence of construct pNOV1305, which contains the constitutive maize ubiquitin promoter operatively linked to the synthetic nucleotide sequence encoding the toxin portion of H04 plus a full-length Cry1Ab tail portion, as set forth in SEQ ID NO:7.

SEQ ID NO:15 shows the nucleotide sequence of construct pNOV1313, which contains the constitutive maize ubiquitin promoter operatively linked to the synthetic nucleotide sequence encoding the toxin portion of H04 plus a full-length Cry1Ab tail portion, as set forth in SEQ ID NO:7.

SEQ ID NO:16 shows the nucleotide sequence of construct pNOV1435, which contains the root-preferred maize MTL promoter operatively linked to the synthetic nucleotide sequence encoding the toxin portion of H04 plus the first 40 amino acids of the Cry1Ab tail, as set forth in SEQ ID NO:9.

SEQ ID NO:17 shows the nucleotide sequence of construct pZU578, which contains the Arabidopsis actin-2 promoter operatively linked to the synthetic nucleotide sequence encoding the toxin portion of H04 plus the first 40 amino acids of the Cry1Ab tail, as set forth in SEQ ID NO:9.

### DEFINITIONS

"Activity" of the toxins of the invention is meant that the toxins function as orally active insect control agents, have a toxic effect, or are able to disrupt or deter insect feeding, which may or may not cause death of the insect. When a toxin of the invention is delivered to the insect, the result is typically death of the insect, or the insect does not feed upon the source that makes the toxin available to the insect.

"Associated with / operatively linked" refer to two nucleic acid sequences that are related physically or functionally. For example, a promoter or regulatory DNA sequence is said to be "associated with" a DNA sequence that codes for an RNA or a protein if the two sequences are operatively linked, or situated such that the regulator DNA sequence will affect the expression level of the coding or structural DNA sequence.

"Binding site" means a site on a molecule wherein the binding between site and toxin is reversible such that the *Ka* between site and toxin is on the order of at least 10⁴dm³mole⁻¹.

A "chimeric gene" is a recombinant nucleic acid sequence in which a promoter or regulatory nucleic acid sequence is operatively linked to, or associated with, a nucleic acid sequence that codes for an mRNA or which is expressed as a protein, such that the regulator nucleic acid sequence is able to regulate transcription or expression of the associated nucleic acid sequence. The regulator nucleic acid sequence of the chimeric gene is not normally operatively linked to the associated nucleic acid sequence as found in nature.

A "coding sequence" is a nucleic acid sequence that is transcribed into RNA such as mRNA, rRNA, tRNA, snRNA, sense RNA or antisense RNA. Preferably the RNA is then translated in an organism to produce a protein.

Complementary: "complementary" refers to two nucleotide sequences that comprise antiparallel nucleotide sequences capable of pairing with one another upon formation of hydrogen bonds between the complementary base residues in the antiparallel nucleotide sequences.

"Conservatively modified variations" of a particular nucleic acid sequence refers to those nucleic acid sequences that encode identical or essentially identical amino acid sequences, or where the nucleic acid sequence does not encode an amino acid sequence, to essentially identical sequences. Because of the degeneracy of the genetic code, a large number of functionally identical nucleic acids encode any given polypeptide. For instance the codons CGT, CGC, CGA, CGG, AGA, and AGG all encode the amino acid arginine. Thus, at every position where an arginine is specified by a codon, the codon can be altered to any of the corresponding codons described without altering the encoded protein. Such nucleic acid variations are "silent variations" which are one species of "conservatively modified variations." Every nucleic acid sequence described herein which encodes a protein also describes every possible silent variation, except where otherwise noted. One of skill will recognize that each codon in a nucleic acid (except ATG, which is ordinarily the only codon for methionine) can be modified to yield a functionally identical molecule by standard techniques. Accordingly, each "silent variation" of a nucleic acid which encodes a protein is implicit in each described sequence.

Furthermore, one of skill will recognize that individual substitutions deletions or additions that alter, add or delete a single amino acid or a small percentage of amino acids (typically less than 5%, more typically less than 1 %) in an encoded sequence are "conservatively modified variations," where the alterations result in the substitution of an amino acid with a chemically similar amino acid. Conservative substitution tables providing functionally similar amino acids are well known in the art. The following five groups each contain amino acids that are conservative substitutions for one another: Aliphatic: Glycine (G), Alanine (A), Valine (V), Leucine (L), Isoleucine (I); Aromatic: Phenylalanine (F), Tyrosine (Y), Tryptophan (W); Sulfur-containing: Methionine (M), Cysteine (C); Basic: Arginine (R), Lysine (K), Histidine (H); Acidic: Aspartic acid (D), Glutamic acid (E), Asparagine (N), Glutamine (Q). *See also,* Creighton (1984) *Proteins, W.H*. Freeman and Company. In addition, individual substitutions, deletions or additions which alter, add or delete a single amino acid or a small percentage of amino acids in an encoded sequence are also "conservatively modified variations."

To "control" insects means to inhibit, through a toxic effect, the ability of insect pests to survive, grow, feed, and/or reproduce, or to limit insect-related damage or loss in crop plants. To "control" insects may or may not mean killing the insects, although it preferably means killing the insects.

Corresponding to: in the context of the present invention, "corresponding to" or "corresponds to" means that when the nucleic acid coding sequences or amino acid sequences of different δ-endotoxins of *Bacillus thuringiensis* are aligned with each other, the nucleic or amino acids that "correspond to" certain enumerated positions are those that align with these positions but that are not necessarily in these exact numerical positions relative to the particular δ-endotoxin's respective nucleic acid coding sequence or amino acid sequence. Likewise, when the coding or amino acid sequence of a particular δ-endotoxin (for example, CrylB) is aligned with the coding or amino acid sequence of a reference δ-endotoxin (for example, Cry1Ab), the nucleic acids or amino acids in the Cry1B sequence that "correspond to" certain enumerated positions of the Cry1Ab sequence are those that align with these positions of the Cry1Ab sequence, but are not necessarily in these exact numerical positions of the Cry1B toxin's respective nucleic acid coding sequence or amino acid sequence.

To "deliver" a toxin means that the toxin comes in contact with an insect, resulting in toxic effect and control of the insect. The toxin can be delivered in many recognized ways, e.g., orally by ingestion by the insect or by contact with the insect via transgenic plant expression, formulated protein composition(s), sprayable protein composition(s), a bait matrix, or any other art-recognized toxin delivery system.

"Expression cassette" as used herein means a nucleic acid sequence capable of directing expression of a particular nucleotide sequence in an appropriate host cell, comprising a promoter operably linked to the nucleotide sequence of interest which is operably linked to termination signals. It also typically comprises sequences required for proper translation of the nucleotide sequence. The expression cassette comprising the nucleotide sequence of interest may be chimeric, meaning that at least one of its components is heterologous with respect to at least one of its other components. The expression cassette may also be one which is naturally occurring but has been obtained in a recombinant form useful for heterologous expression. Typically, however, the expression cassette is heterologous with respect to the host, i.e., the particular nucleic acid sequence of the expression cassette does not occur naturally in the host cell and must have been introduced into the host cell or an ancestor of the host cell by a transformation event. The expression of the nucleotide sequence in the expression cassette may be under the control of a constitutive promoter or of an inducible promoter which initiates transcription only when the host cell is exposed to some particular external stimulus. In the case of a multicellular organism, such as a plant, the promoter can also be specific to a particular tissue, or organ, or stage of development.

Gene: the term "gene" is used broadly to refer to any segment of DNA associated with a biological function. Thus, genes include coding sequences and/or the regulatory sequences required for their expression. Genes also include nonexpressed DNA segments that, for example, form recognition sequences for other proteins. Genes can be obtained from a variety of sources, including cloning from a source of interest or synthesizing from known or predicted sequence information, and may include sequences designed to have desired parameters.

"Gene of interest" refers to any gene which, when transferred to a plant, confers upon the plant a desired characteristic such as antibiotic resistance, virus resistance, insect resistance, disease resistance, or resistance to other pests, herbicide tolerance, improved nutritional value, improved performance in an industrial process or altered reproductive capability. The "gene of interest" may also be one that is transferred to plants for the production of commercially valuable enzymes or metabolites in the plant.

As used herein, "H04" refers to the hybrid Bt toxin described in De Maagd *et al., Appl. Environ. MicrobioL* 62(5): 1537-1543 (1996), the toxin fragment of which comprises at its N-terminus domains I and II of Cry1Ab and at its C-terminus domain III of Cry1C.

Heterologous nucleic acid sequence: The terms "heterologous nucleic acid [or DNA] sequence", "exogenous nucleic acid [or DNA] segment" or "heterologous gene," as used herein, each refer to a sequence that originates from a source foreign to the particular host cell or, if from the same source, is modified from its original form. Thus, a heterologous gene in a host cell includes a gene that is endogenous to the particular host cell but has been modified through, for example, the use of codon optimization. The terms also includes non-naturally occurring multiple copies of a naturally occurring sequence. Thus, the terms refer to a nucleic acid segment that is foreign or heterologous to the cell, or homologous to the cell but in a position within the host cell nucleic acid in which the element is not ordinarily found. Exogenous nucleic acid segments are expressed to yield exogenous polypeptides.

A "homologous" nucleic acid [or DNA] sequence is a nucleic acid [or DNA] sequence naturally associated with a host cell into which it is introduced.

"Homologous recombination" is the reciprocal exchange of nucleic acid fragments between homologous nucleic acid molecules.

"Homoplastidic" refers to a plant, plant tissue or plant cell wherein all of the plastids are genetically identical. This is the normal state in a plant when the plastids have not been transformed, mutated, or otherwise genetically altered. In different tissues or stages of development, the plastids may take different forms, e.g., chloroplasts, proplastids, etioplasts, amyloplasts, chromoplasts, and so forth.

The terms "identical" or percent "identity" in the context of two or more nucleic acid or protein sequences, refer to two or more sequences or subsequences that are the same or have a specified percentage of amino_acid residues or nucleotides that are the same, when compared and aligned for maximum correspondence, as measured using one of the sequence comparison algorithms described below or by visual inspection.

"Insecticidal" is defined as a toxic biological activity capable of controlling insects, preferably by killing them.

A nucleic acid sequence is "isocoding with" a reference nucleic acid sequence when the nucleic acid sequence encodes a polypeptide having the same amino acid sequence as the polypeptide encoded by the reference nucleic acid sequence.

An "isolated" nucleic acid molecule or an isolated enzyme is a nucleic acid molecule or enzyme that, by the hand of man, exists apart from its native environment and is therefore not a product of nature. An isolated nucleic acid molecule or enzyme may exist in a purified form or may exist in a non-native environment such as, for example, a recombinant host cell.

A "juncture" between toxin domains in a hybrid toxin, i.e., between domains II and III of a hybrid insecticidal toxin according to the invention, is the homologous crossover region or site in the hybrid. Amino acids to the left of the crossover site are from one parental toxin, whereas amino acids to the right of the crossover site are from the other parental toxin.

Mature Protein: protein that is normally targeted to a cellular organelle and from which the transit peptide has been removed.

Minimal Promoter: promoter elements, particularly a TATA element, that are inactive or that have greatly reduced promoter activity in the absence of upstream activation. In the presence of a suitable transcription factor, the minimal promoter functions to permit transcription.

Native: refers to a gene that is present in the genome of an untransformed cell.

Naturally occurring: the term "naturally occurring" is used to describe an object that can be found in nature as distinct from being artificially produced by man. For example, a protein or nucleotide sequence present in an organism (including a virus), which can be isolated from a source in nature and which has not been intentionally modified by man in the laboratory, is naturally occurring.

Nucleic acid: the term "nucleic acid" refers to deoxynbonucleotides or nbonucleotides and polymers thereof in either single- or double-stranded form. Unless specifically limited, the term encompasses nucleic acids containing known analogues of natural nucleotides which have similar binding properties as the reference nucleic acid and are metabolized in a manner similar to naturally occurring nucleotides. Unless otherwise indicated, a particular nucleic acid sequence also implicitly encompasses conservatively modified variants thereof *(e.g.* degenerate codon substitutions) and complementary sequences and as well as the sequence explicitly indicated. Specifically, degenerate codon substitutions may be achieved by generating sequences in which the third position of one or more selected (or all) codons is substituted with mixed-base and/or deoxyinosine residues (Batzer *et al., Nucleic Acid Res.* 19: 5081 (1991); Ohtsuka *et al., J. Biol. Chem*. 260: 2605-2608 (1985); Rossolini *et al., Mol. Cell. Probes* 8: 91-98 (1994)). The terms "nucleic acid" or "nucleic acid sequence" may also be used interchangeably with gene, cDNA, and mRNA encoded by a gene.

"ORF" means Open Reading Frame.

By "part" of a protein is meant a peptide comprised by said protein and having at least 80% of the consecutive sequence thereof.

A "plant" is any plant at any stage of development, particularly a seed plant.

A "plant cell" is a structural and physiological unit of a plant, comprising a protoplast and a cell wall. The plant cell may be in form of an isolated single cell or a cultured cell, or as a part of higher organized unit such as, for example, plant tissue, a plant organ, or a whole plant.

"Plant cell culture" means cultures of plant units such as, for example, protoplasts, cell culture cells, cells in plant tissues, pollen, pollen tubes, ovules, embryo sacs, zygotes and embryos at various stages of development.

"Plant material" refers to leaves, stems, roots, flowers or flower parts, fruits, pollen, egg cells, zygotes, seeds, cuttings, cell or tissue cultures, or any other part or product of a plant.

A "plant organ" is a distinct and visibly structured and differentiated part of a plant such as a root, stem, leaf, flower bud, or embryo.

"Plant tissue" as used herein means a group of plant cells organized into a structural and functional unit. Any tissue of a plant *in planta* or in culture is included. This term includes, but is not limited to, whole plants, plant organs, plant seeds, tissue culture and any groups of plant cells organized into structural and/or functional units. The use of this term in conjunction with, or in the absence of, any specific type of plant tissue as listed above or otherwise embraced by this definition is not intended to be exclusive of any other type of plant tissue.

A "promoter" is an untranslated DNA sequence upstream of the coding region that contains the binding site for RNA polymerase II and initiates transcription of the DNA. The promoter region may also include other elements that act as regulators of gene expression.

A "protoplast" is an isolated plant cell without a cell wall or with only parts of the cell wall.

Purified: the term "purified," when applied to a nucleic acid or protein, denotes that the nucleic acid or protein is essentially free of other cellular components with which it is associated in the natural state. It is preferably in a homogeneous state although it can be in either a dry or aqueous solution. Purity and homogeneity are typically determined using analytical chemistry techniques such as polyacrylamide gel electrophoresis or high performance liquid chromatography. A protein which is the predominant species present in a preparation is substantially purified. The term "purified" denotes that a nucleic acid or protein gives rise to essentially one band in an electrophoretic gel. Particularly, it means that the nucleic acid or protein is at least about 50% pure, more preferably at least about 85% pure, and most preferably at least about 99% pure.

Two nucleic acids are "recombined" when sequences from each of the two nucleic acids are combined in a progeny nucleic acid. Two sequences are "directly" recombined when both of the nucleic acids are substrates for recombination. Two sequences are "indirectly recombined" when the sequences are recombined using an intermediate such as a cross-over oligonucleotide. For indirect recombination, no more than one of the sequences is an actual substrate for recombination, and in some cases, neither sequence is a substrate for recombination.

"Regulatory elements" refer to sequences involved in controlling the expression of a nucleotide sequence. Regulatory elements comprise a promoter operably linked to the nucleotide sequence of interest and termination signals. They also typically encompass sequences required for proper translation of the nucleotide sequence.

Substantially identical: the phrase "substantially identical," in the context of two nucleic acid or protein sequences, refers to two or more sequences or subsequences that have at least 60%, preferably 80%, more preferably 90, even more preferably 95%, and most preferably at least 99% nucleotide or amino acid residue identity, when compared and aligned for maximum correspondence, as measured using one of the following sequence comparison algorithms or by visual inspection. Preferably, the substantial identity exists over a region of the sequences that is at least about 50 residues in length, more preferably over a region of at least about 100 residues, and most preferably the sequences are substantially identical over at least about 150 residues. In a most preferred embodiment, the sequences are substantially identical over the entire length of the coding regions. Furthermore, substantially identical nucleic acid or protein sequences perform substantially the same function.

For sequence comparison, typically one sequence acts as a reference sequence to which test sequences are compared. When using a sequence comparison algorithm, test and reference sequences are input into a computer, subsequence coordinates are designated if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, e.g., by the local homology algorithm of Smith & Waterman, *Adv. Appl. Math*. 2: 482 (1981), by the homology alignment algorithm of Needleman & Wunsch, *J. Mol. Biol.* 48: 443 (1970), by the search for similarity method of Pearson & Lipman, *Proc. Nat'l. Acad. Sci. USA* 85: 2444 (1988), by computerized implementations of these algorithms (GAP, BESTFIT, PASTA, and TFASTA in the Wisconsin Genetics Software Package, Genetics Computer Group, 575 Science Dr., Madison, WI), or by visual inspection *(see generally,* Ausubel *et al., infra*).

One example of an algorithm that is suitable for determining percent sequence identity and sequence similarity is the BLAST algorithm, which is described in Altschul *et al., J. Mol. Biol*. 215: 403-410 (1990). Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al*., 1990). These initial neighborhood word hits act as seeds for initiating searches to find longer HSPs containing them The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when the cumulative alignment score falls off by the quantity X from its maximum achieved value, the cumulative score goes to zero or below due to the accumulation of one or more negative-scoring residue alignments, or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix *(see* Henikoff & Henikoff, *Proc. Natl. Acad. Sci. USA* 89: 10915 (1989)).

In addition to calculating percent sequence identity, the BLAST algorithm also performs a statistical analysis of the similarity between two sequences *(see, e.g.,* Karlin & Altschul, *Proc. Nat'l. Acad. Sci. USA* 90: 5873-5787 (1993)). One measure of similarity provided by the BLAST algorithm is the smallest sum probability (P(N)), which provides an indication of the probability by which a match between two nucleotide or amino acid sequences would occur by chance. For example, a test nucleic acid sequence is considered similar to a reference sequence if the smallest sum probability in a comparison of the test nucleic acid sequence to the reference nucleic acid sequence is less than about 0.1, more preferably less than about 0.01, and most preferably less than about 0.001.

Another indication that two nucleic acid sequences are substantially identical is that the two molecules hybridize to each other under stringent conditions. The phrase "hybridizing specifically to" refers to the binding, duplexing, or hybridizing of a molecule only to a particular nucleotide sequence under stringent conditions when that sequence is present in a complex mixture *(e.g.,* total cellular) DNA or RNA. "Bind(s) substantially" refers to complementary hybridization between a probe nucleic acid and a target nucleic acid and embraces minor mismatches that can be accommodated by reducing the stringency of the hybridization media to achieve the desired detection of the target nucleic acid sequence.

"Stringent hybridization conditions" and "stringent hybridization wash conditions" in the context of nucleic acid hybridization experiments such as Southern and Northern hybridizations are sequence dependent, and are different under different environmental parameters. Longer sequences hybridize specifically at higher temperatures. An extensive guide to the hybridization of nucleic acids is found in Tijssen (1993) *Laboratory Techniques in Biochemistry and Molecular Biology-Hybridization with Nucleic* Acid Probes part I chapter 2 "Overview of principles of hybridization and the strategy of nucleic acid probe assays" Elsevier, New York. Generally, highly stringent hybridization and wash conditions are selected to be about 5°C lower than the thermal melting point (Tₘ) for the specific sequence at a defined ionic strength and pH. Typically, under "stringent conditions" a probe will hybridize to its target subsequence, but to no other sequences.

The Tₘ is the temperature (under defined ionic strength and pH) at which 50% of the target sequence hybridizes to a perfectly matched probe. Very stringent conditions are selected to be equal to the Tₘ for a particular probe. An example of stringent hybridization conditions for hybridization of complementary nucleic acids which have more than 100 complementary residues on a filter in a Southern or northern blot is 50% formamide with 1 mg of heparin at 42°C, with the hybridization being carried out overnight. An example of highly stringent wash conditions is 0.1 5M NaCl at 72°C for about 15 minutes. An example of stringent wash conditions is a 0.2x SSC wash at 65°C for 15 minutes *(see,* Sambrook, *infra,* for a description of SSC buffer). Often, a high stringency wash is preceded by a low stringency wash to remove background probe signal. An example medium stringency wash for a duplex of, e.g., more than 100 nucleotides, is 1x SSC at 45°C for 15 minutes. An example low stringency wash for a duplex of, *e.g.,* more than 100 nucleotides, is 4-6x SSC at 40°C for 15 minutes. For short probes *(e.g.,* about 10 to 50 nucleotides), stringent conditions typically involve salt concentrations of less than about 1.0M Na ion, typically about 0.01 to 1.0 M Na ion concentration (or other salts) at pH 7.0 to 8.3, and the temperature is typically at least about 30°C. Stringent conditions can also be achieved with the addition of destabilizing agents such as formamide. In general, a signal to noise ratio of 2x (or higher) than that observed for an unrelated probe in the particular hybridization assay indicates detection of a specific hybridization. Nucleic acids that do not hybridize to each other under stringent conditions are still substantially identical if the proteins that they encode are substantially identical. This occurs, e.g., when a copy of a nucleic acid is created using the maximum codon degeneracy permitted by the genetic code.

The following are examples of sets of hybridization/wash conditions that may be used to clone homologous nucleotide sequences that are substantially identical to reference nucleotide sequences of the present invention: a reference nucleotide sequence preferably hybridizes to the reference nucleotide sequence in 7% sodium dodecyl sulfate (SDS), 0.5 M NaPO₄, 1 mM EDTA at 50°C with washing in 2X SSC, 0.1% SDS at 50°C, more desirably in 7% sodium dodecyl sulfate (SDS), 0.5 M NaPO₄, 1 mM EDTA at 50°C with washing in 1X SSC, 0.1% SDS at 50°C, more desirably still in 7% sodium dodecyl sulfate (SDS), 0.5 M NaPO₄, 1 mM EDTA at 50°C with washing in 0.5X SSC, 0.1 % SDS at 50°C, preferably in 7% sodium dodecyl sulfate (SDS), 0.5 M NaPO₄, 1 mM EDTA at 50°C with washing in 0.1X SSC, 0.1% SDS at 50°C, more preferably in 7% sodium dodecyl sulfate (SDS), 0.5 M NaPO₄, 1 mM EDTA at 50°C with washing in 0.1X SSC, 0.1% SDS at 65°C.

A further indication that two nucleic acid sequences or proteins are substantially identical is that the protein encoded by the first nucleic acid is immunologically cross reactive with, or specifically binds to, the protein encoded by the second nucleic acid. Thus, a protein is typically substantially identical to a second protein, for example, where the two proteins differ only by conservative substitutions.

The phrase "specifically (or selectively) binds to an antibody," or "specifically (or selectively) immunoreactive with," when referring to a protein or peptide, refers to a binding reaction which is determinative of the presence of the protein in the presence of a heterogeneous population of proteins and other biologies. Thus, under designated immunoassay conditions, the specified antibodies bind to a particular protein and do not bind in a significant amount to other proteins present in the sample. Specific binding to an antibody under such conditions may require an antibody that is selected for its specificity for a particular protein. For example, antibodies raised to the protein with the amino acid sequence encoded by any of the nucleic acid sequences of the invention can be selected to obtain antibodies specifically immunoreactive with that protein and not with other proteins except for polymorphic variants. A variety of immunoassay formats may be used to select antibodies specifically immunoreactive with a particular protein. For example, solid-phase EUSA immunoassays, Western blots, or immunohistochemistry are routinely used to select monoclonal antibodies specifically immunoreactive with a protein. See Harlow and Lane (1988) *Antibodies, A Laboratory Manual,* Cold Spring Harbor Publications, New York "Harlow and Lane"), for a description of immunoassay formats and conditions that can be used to determine specific immunoreactivity. Typically a specific or selective reaction will be at least twice background signal or noise and more typically more than 10 to 100 times background.

A "subsequence" refers to a sequence of nucleic acids or amino acids that comprise a part of a longer sequence of nucleic acids or amino acids (e.g., protein) respectively.

"Synthetic" refers to a nucleotide sequence comprising structural characters that are not present in the natural sequence. For example, an artificial sequence that resembles more closely the G+C content and the normal codon distribution of dicot and/or monocot genes is said to be synthetic.

"Transformation" is a process for introducing heterologous nucleic acid into a host cell or organism. In particular, "transformation" means the stable integration of a DNA molecule into the genome of an organism of interest. Transformed cells, tissues, or insects are understood to encompass not only the end product of a transformation process, but also transgenic progeny thereof.

"Transformed / transgenic / recombinant" refer to a host organism such as a bacterium or a plant into which a heterologous nucleic acid molecule has been introduced. The nucleic acid molecule can be stably integrated into the genome of the host or the nucleic acid molecule can also be present as an extrachromosomal molecule. Such an extrachromosomal molecule can be auto-replicating. Transformed cells, tissues, or plants are understood to encompass not only the end product of a transformation process, but also transgenic progeny thereof. A "non-transformed", "non-transgenic", or "non-recombinant" host refers to a wild-type organism, e.g., a bacterium or plant, which does not contain the heterologous nucleic acid molecule.

Nucleotides are indicated by their bases by the following standard abbreviations: adenine (A), cytosine (C), thymine (T), and guanine (G). Amino acids are likewise indicated by the following standard abbreviations: alanine (Ala; A), arginine (Arg; R), asparagine (Asn; N), aspartic acid (Asp; D), cysteine (Cys; C), glutamine (Gln; Q), glutamic acid (Glu; E), glycine (Gly; G), histidine (His; H), isoleucine (Ile; I), leucine (Leu; L), lysine (Lys; K), methionine (Met; M), phenylalanine (Phe; F), proline (Pro; P), serine (Ser; S), threonine (Thr; T), tryptophan (Trp; W), tyrosine (Tyr; Y), and valine (Val; V). Furthermore, (Xaa; X) represents any amino acid.

This invention relates to novel nucleic acid sequences whose expression results in novel toxins, and to the making and using of the toxins to control insect pests. In particular, the present invention concerns synthetic gene sequences optimized for expression in plants that encode varying forms of the hybrid *Bacillus thuringiensis* delta-endotoxin H04, the toxin portion of which contains domains I and II of Cry1Ab and domain III of Cry1C. The hybrid gene encoding the H04 hybrid toxin, as constructed from the native cry1Ab and Cry1C genes is described in U.S. Pat. No. 5,736,131 and De Maagd *et aL, Appl. Environ. Microbiol*. 62(5): 1537-1543 (1996). The preferred method for constructing the synthetic H04 genes of the invention is set forth in WO 93/07278. The hybrid *Bacillus thuringiensis* toxins encoded by the novel gene sequences are highly active against economically important insect pests such as fall armyworm, pink bollworm, tobacco budworm, European comborer, and diamondback moth. The hybrid *Bacillus thuringiensis* toxins can be used in multiple insect control strategies, resulting in maximal efficiency with minimal impact on the environment.

The present invention encompasses DNA molecules comprising nucleotide sequences that encode the insecticidal toxins of the invention. The present invention further encompasses recombinant vectors comprising the nucleic acid sequences of this invention. In such vectors, the nucleic acid sequences are preferably comprised in expression cassettes comprising regulatory elements for expression of the nucleotide sequences in a host cell capable of expressing the nucleotide sequences. Such regulatory elements usually comprise promoter and termination signals and preferably also comprise elements allowing efficient translation of proteins encoded by the nucleic acid sequences of the present invention. Vectors comprising the nucleic acid sequences are usually capable of replication in particular host cells, preferably as extrachromosomal molecules, and are therefore used to amplify the nucleic acid sequences of this invention in the host cells. In one embodiment, host cells for such vectors are microorganisms, such as bacteria, in particular *Bacillus thuringiensis* or *E. coli.* In another embodiment, host cells for such recombinant vectors are endophytes or epiphytes. A preferred host cell for such vectors is a eukaryotic cell, such as a plant cell. Plant cells such as maize cells are most preferred host cells.

In a particularly preferred embodiment, an insecticidal toxin of the invention is expressed in a plant. In this case, transgenic plants expressing effective amounts of the toxins protect themselves from insect pests. When the insect starts feeding on such a transgenic plant, it also ingests the expressed toxins. This will deter the insect from further biting into the plant tissue or may even harm or kill the insect.

The nucleic acid sequences described in this application can be incorporated into plant cells using conventional recombinant DNA technology. Generally, this involves inserting a coding sequence of the invention into an expression system to which the coding sequence is heterologous (i.e., not normally present) using standard cloning procedures known in the art. The vector contains the necessary elements for the transcription and translation of the inserted protein-coding sequences. A large number of vector systems known in the art can be used, such as plasmids, bacteriophage viruses and other modified viruses. Suitable vectors include, but are not limited to, viral vectors such as lambda vector systems λgtl1, λgtl0 and Charon 4; plasmid vectors such as pBI121, pBR322, pACYC177, pACYC184, pAR series, pKK223-3, pUC8, pUC9, pUC18, pUC19, pLG339, pRK290, pKC37, pKC101, pCDNAII; and other similar systems. Transformed cells can be regenerated into whole plants such that the nucleotide sequences of the invention confer insect resistance to the transgenic plants.

Plants transformed in accordance with the present invention may be monocots or dicots and include, but are not limited to, maize, wheat, barley, rye, sweet potato, bean, pea, chicory, lettuce, cabbage, cauliflower, broccoli, turnip, radish, spinach, asparagus, onion, garlic, pepper, celery, squash, pumpkin, hemp, zucchini, apple, pear, quince, melon (e.g., watermelon), plum, cherry, peach, nectarine, apricot, strawberry, grape, raspberry, blackberry, pineapple, avocado, papaya, mango, banana, soybean, tomato, sorghum, sugarcane, sugarbeet, sunflower, rapeseed, clover, tobacco, carrot, cotton, alfalfa, rice, potato, eggplant, cucumber, *Arabidopsis,* and woody plants such as coniferous and deciduous trees. Once a desired nucleotide sequence has been transformed into a particular plant species, it may be propagated in that species or moved into other varieties of the same species, particularly including commercial varieties, using traditional breeding techniques.

For their expression in transgenic plants, the nucleotide sequences of the invention may require modification and optimization. Although in many cases genes from microbial organisms can be expressed in plants at high levels without modification, low expression in transgenic plants may result from microbial nucleotide sequences having codons that are not preferred in plants. It is known in the art that all organisms have specific preferences for codon usage, and the codons of the nucleotide sequences described in this invention can be changed to conform with plant preferences, while maintaining the amino acids encoded thereby. Furthermore, high expression in plants is best achieved from coding sequences that have at least 35% about GC content, preferably more than about 45%, more preferably more than about 50%, and most preferably more than about 60%. Microbial nucleotide sequences which have low GC contents may express poorly in plants due to the existence of ATTTA motifs which may destabilize messages, and AATAAA motifs which may cause inappropriate polyadenylation. Although preferred gene sequences may be adequately expressed in both monocotyledonous and dicotyledonous plant species, sequences can be modified to account for the specific codon preferences and GC content preferences of monocotyledons or dicotyledons as these preferences have been shown to differ (Murray *et al. Nucl. Acids Res.* 17: 477-498 (1989)). In addition, the nucleotide sequences are screened for the existence of illegitimate splice sites that may cause message truncation. All changes required to be made within the nucleotide sequences such as those described above are made using well known techniques of site directed mutagenesis, PCR, and synthetic gene construction using the methods described in the published patent applications EP 0 385 962, EP 0 359 472, and WO 93/07278.

For efficient initiation of translation, sequences adjacent to the initiating methionine may require modification. For example, they can be modified by the inclusion of sequences known to be effective in plants. Joshi has suggested an appropriate consensus for plants (NAR 15: 6643-6653 (1987)) and Clontech suggests a further consensus translation initiator (1993/1994 catalog, page 210). These consensuses are suitable for use with the nucleotide sequences of this invention. The sequences are incorporated into constructions comprising the nucleotide sequences, up to and including the ATG (whilst leaving the second amino acid unmodified), or alternatively up to and including the GTC subsequent to the ATG (with the possibility of modifying the second amino acid of the transgene).

Expression of the nucleotide sequences in transgenic plants is driven by promoters shown to be functional in plants. The choice of promoter will vary depending on the temporal and spatial requirements for expression, and also depending on the target species. Thus, expression of the nucleotide sequences of this invention in leaves, in ears, in inflorescences (*e.g.* spikes, panicles, cobs, *etc.*), in roots, and/or seedlings is preferred. In many cases, however, protection against more than one type of insect pest is sought, and thus expression in multiple tissues is desirable. Although many promoters from dicotyledons have been shown to be operational in monocotyledons and *vice versa,* ideally dicotyledonous promoters are selected for expression in dicotyledons, and monocotyledonous promoters for expression in monocotyledons. However, there is no restriction to the provenance of selected promoters; it is sufficient that they are operational in driving the expression of the nucleotide sequences in the desired cell.

Preferred promoters that are expressed constitutively include promoters from genes encoding actin or ubiquitin and the CaMV 35S and 19S promoters. The nucleotide sequences of this invention can also be expressed under the regulation of promoters that are chemically regulated. This enables the insecticidal toxins to be synthesized only when the crop plants are treated with the inducing chemicals. Preferred technology for chemical induction of gene expression is detailed in the published application EP 0 332 104 and US patent 5,614,395. A preferred promoter for chemical induction is the tobacco PR-1a promoter.

A preferred category of promoters is that which is wound inducible. Numerous promoters have been described which are expressed at wound sites and also at the sites of phytopathogen infection. Ideally, such a promoter should only be active locally at the sites of infection, and in this way the insecticidal toxins only accumulate in cells which need to synthesize the insecticidal toxins to kill the invading insect pest. Preferred promoters of this kind include those described by Stanford *et al., Mol. Gen. Genet.* 215: 200-208 (1989), Xu *et al., Plant Molec. Biol*. 22: 573-588 (1993), Logemann *et al., Plant Cell* 1: 151-158 (1989), Rohrmeier & Lehle, *Plant Molec. Biol.* 22: 783-792 (1993), Firek *et al., Plant Molec. Biol.* 22: 129-142 (1993), and Warner *et al., Plant J.* 3: 191-201 (1993).

Preferred tissue specific expression patterns include green tissue specific, root specific, stem specific, and flower specific. Promoters suitable for expression in green tissue include many which regulate genes involved in photosynthesis and many of these have been cloned from both monocotyledons and dicotyledons. A preferred promoter is the maize PEPC promoter from the phosphoenol carboxylase gene (Hudspeth & Grula, *Plant Molec. Biol. 12:* 579-589 (1989)). A preferred promoter for root specific expression is the maize metallothionein-like (MTL) promoter described by de Framond *(FEBS* 290: 103-106 (1991); EP 0 452 269. A preferred stem specific promoter is that described in US patent 5,625,136 sand which drives expression of the maize *trpA* gene.

Especially preferred embodiments of the invention are transgenic plants expressing at least one of the nucleotide sequences of the invention in a root-preferred or root-specific fashion. Further preferred embodiments are transgenic plants expressing the nucleotide sequences in a wound-inducible or pathogen infection-inducible manner.

In addition to the selection of a suitable promoter, constructions for expression of an insecticidal toxin in plants require an appropriate transcription terminator to be attached downstream of the heterologous nucleotide sequence. Several such terminators are available and known in the art *(e.g. tml* from CaMV, E9 from *rbcS*). Any available terminator known to function in plants can be used in the context of this invention.

Numerous other sequences can be incorporated into expression cassettes described in this invention. These include sequences which have been shown to enhance expression such as intron sequences *(e.g.* from *Adh1* and *bronze1*) and viral leader sequences *(e.g.* from TMV, MCMV and AMV).

It may be preferable to target expression of the nucleotide sequences of the present invention to different cellular localizations in the plant. In some cases, localization in the cytosol may be desirable, whereas in other cases, localization in some subcellular organelle may be preferred. Subcellular localization of transgene encoded enzymes is undertaken using techniques well known in the art. Typically, the DNA encoding the target peptide from a known organelle-targeted gene product is manipulated and fused upstream of the nucleotide sequence. Many such target sequences are known for the chloroplast and their functioning in heterologous constructions has been shown. The expression of the nucleotide sequences of the present invention is also targeted to the endoplasmic reticulum or to the vacuoles of the host cells. Techniques to achieve this are well-known in the art.

Vectors suitable for plant transformation are described elsewhere in this specification. For Agrobacterium-mediated transformation, binary vectors or vectors carrying at least one T-DNA border sequence are suitable, whereas for direct gene transfer any vector is suitable and linear DNA containing only the construction of interest may be preferred. In the case of direct gene transfer, transformation with a single DNA species or co-transformation can be used (Schocher *et al*. Biotechnology 4: 1093-1096 (1986)). For both direct gene transfer and *Agrobacterium*-mediated transfer, transformation is usually (but not necessarily) undertaken with a selectable marker which may provide resistance to an antibiotic (kanamycin, hygromycin or methotrexate) or a herbicide (basta). Examples of such markers are neomycin phosphotransferase, hygromycin phosphotransferase, dihydrofolate reductase, phosphinothricin acetyltransferase, 2, 2-dichloroproprionic acid dehalogenase, acetohydroxyacid synthase, 5-enolpyruvyl-shikimate-phosphate synthase, haloarylnitrilase, protoporhyrinogen oxidase, acetyl-coenzyme A carboxylase, dihydropteroate synthase, chloramphenicol acetyl transferase, and β-glucuronidase. Another type of marker providing for positive selection is the mannose-6-phosphate isomerase (MPI/PMI) gene, which provides the ability to metabolize mannose mannose-6-phosphate isomerase. The choice of selectable or screenable marker for plant transformation is not, however, critical to the invention.

The recombinant DNA described above can be introduced into the plant cell in a number of art-recognized ways. Those skilled in the art will appreciate that the choice of method might depend on the type of plant targeted for transformation. Suitable methods of transforming plant cells include microinjection (Crossway et al., *BioTechniques 4*:320-334 (1986)), electroporation (Riggs *et al., Proc. Natl. Acad. Sci. USA 83*:5602-5606 (1986), *Agrobacterium-mediated* transformation (Hinchee et al., Biotechnology 6:915-921 (1988); *See also,* Ishida *et al., Nature Biotechnology 14*:745-750 (June 1996) for maize transformation), direct gene transfer (Paszkowski *et al., EMBO J. 3*:2717-2722 (1984); Hayashimoto *et al., Plant Physiol. 93*:857-863 (1990)(rice)), and ballistic particle acceleration using devices available from Agracetus, Inc., Madison, Wisconsin and Dupont, Inc., Wilmington, Delaware (see, for example, Sanford et al., U.S. Patent 4,945,050; and McCabe et al., *Biotechnology 6*:923-926 (1988)). *See also,* Weissinger *et al., Annual Rev. Genet. 22*:421-477 (1988); Sanford *et al., Particulate Science and Technology 5*:27-37 91987)(onion); Svab *et al., Proc. Natl. Acad*. *Sci. USA 87:* 8526-8530 (1990) (tobacco chloroplast); Christou *et al., Plant Physiol. 87*:671-674 (1988)(soybean); McCabe *et al., Bio*/*Technology 6*:923-926 (1988)(soybean); Klein *et al., Proc. Natl. Acad. Sci. USA, 85*:4305-4309 (1988)(maize); Klein *et al., Bio*/*Technology 6*:559-563 (1988) (maize); Klein *et al., Plant Physiol. 91*:440-444 (1988) (maize); Fromm *et al., Bio*/*Technology 8*:833-839 (1990); and Gordon-Kamm *et al., Plant Cell 2:* 603-618 (1990) (maize); Koziel *et al., Biotechnology 11:* 194-200 (1993) (maize); Shimamoto *et al., Nature* 338: 274-277 (1989) (rice); Christou *et al., Biotechnology 9:* 957-962 (1991) (rice); Datta *et al., Bio*/*Technology 8*:736-740 (1990) (rice); European Patent Application EP 0 332 581 (orchardgrass and other *Pooideae);* Vasil *et al., Biotechnology 11:* 1553-1558 (1993) (wheat); Weeks *et al., Plant Physiol. 102:* 1077-1084 (1993) (wheat); Wan *et al., Plant Physiol. 104:* 37-48 (1994) (barley); Jahne *et al., Theor. Appl. Genet. 89*:525-533 (1994)(barley); Umbeck *et al., Bio*/*Technology 5*: 263-266 (1987) (cotton); Casas *et al., Proc. Natl. Acad. Sci. USA 90:*11212-11216 (Dec. 1993) (sorghum); Somers *et al., Bio*/*Technology 10:*1589-1594 (Dec. 1992) (oat); Torbert *et al., Plant Cell Reports 14:*635-640 (1995) (oat); Weeks *et al., Plant Physiol*. *102:*1077-1084 (1993) (wheat); Chang *et al*., WO 94/13822 (wheat) and Nehra *et al., The Plant Journal 5*:285-297 (1994) (wheat). A particularly preferred set of embodiments for the introduction of recombinant DNA molecules into maize by microprojectile bombardment can be found in Koziel *et al., Biotechnology 11:* 194-200 (1993), Hill *et al., Euphytica 85*:119-123 (1995) and Koziel *et al., Annals of the New York Academy of Sciences 792:*164-171 (1996). An additional preferred embodiment is the protoplast transformation method for maize as disclosed in EP 0 292 435. Transformation of plants can be undertaken with a single DNA species or multiple DNA species (*i*.*e*. co-transformation) and both these techniques are suitable for use with a coding sequence of the invention.

In another preferred embodiment, a nucleotide sequence of the present invention is directly transformed into the plastid genome. A major advantage of plastid transformation is that plastids are generally capable of expressing bacterial genes without substantial modification, and plastids are capable of expressing multiple open reading frames under control of a single promoter. Plastid transformation technology is extensively described in U.S. Patent Nos. 5,451,513, 5,545,817, and 5,545,818, in PCT application no. WO 95/16783, and in McBride *et al*. (1994) *Proc. Natl. Acad. Sci. USA* 91, 7301-7305. The basic technique for chloroplast transformation involves introducing regions of cloned plastid DNA flanking a selectable marker together with the gene of interest into a suitable target tissue, e.g., using biolistics or protoplast transformation (e.g., calcium chloride or PEG mediated transformation). The 1 to 1.5 kb flanking regions, termed targeting sequences, facilitate homologous recombination with the plastid genome and thus allow the replacement or modification of specific regions of the plastome. Initially, point mutations in the chloroplast 16S rRNA and rps12 genes conferring resistance to spectinomycin and/or streptomycin are utilized as selectable markers for transformation (Svab, Z., Hajdukiewicz, P., and Maliga, P. (1990) Proc. Natl. Acad. Sci. USA 87, 8526-8530; Staub, J. M., and Maliga, P. (1992) Plant Cell 4, 39-45). This resulted in stable homoplasmic transformants at a frequency of approximately one per 100 bombardments of target leaves. The presence of cloning sites between these markers allowed creation of a plastid targeting vector for introduction of foreign genes (Staub, J.M., and Maliga, P. (1993) *EMBO J.* 12, 601-606). Substantial increases in transformation frequency are obtained by replacement of the recessive rRNA or r-protein antibiotic resistance genes with a dominant selectable marker, the bacterial *aadA* gene encoding the spectinomycin-detoxifying enzyme aminoglycoside-3'-adenyltransferase (Svab, Z., and Maliga, P. (1993) *Proc. Natl. Acad. Sci. USA* 90, 913-917). Previously, this marker had been used successfully for high-frequency transformation of the plastid genome of the green alga *Chlamydomonas reinhardtii* (Goldschmidt-Clermont, M. (1991) *Nucl. Acids Res*. 19: 4083-4089). Other selectable markers useful for plastid transformation are known in the art and encompassed within the scope of the invention. Typically, approximately 15-20 cell division cycles following transformation are required to reach a homoplastidic state. Plastid expression, in which genes are inserted by homologous recombination into all of the several thousand copies of the circular plastid genome present in each plant cell, takes advantage of the enormous copy number advantage over nuclear-expressed genes to permit expression levels that can readily exceed 10% of the total soluble plant protein. In a preferred embodiment, a nucleotide sequence of the present invention is inserted into a plastid targeting vector and transformed into the plastid genome of a desired plant host. Plants homoplastic for plastid genomes containing a nucleotide sequence of the present invention are obtained, and are preferentially capable of high expression of the nucleotide sequence.

### EXAMPLES

The invention will be further described by reference to the following detailed examples. These examples are provided for purposes of illustration only, and are not intended to be limiting unless otherwise specified. Standard recombinant DNA and molecular cloning techniques used here are well known in the art and are described by Ausubel (ed.), Current Protocols in Molecular Biology, John Wiley and Sons, Inc. (1994); T. Maniatis, E. F. Fritsch and J. Sambrook, Molecular Cloning: A Laboratory Manual, Cold Spring Harbor laboratory, Cold Spring Harbor, NY (1989); and by T.J. Silhavy, M.L. Berman, and L.W. Enquist, Experiments with Gene Fusions, Cold Spring Harbor Laboratory, Cold Spring Harbor, NY (1984).

### Example 1: Expression and Purification of an H04 Toxin Fragment

A truncated form of the H04 hybrid toxin gene (described in De Maagd *et al., Appl. Environ. Microbiol*. 62(5): 1537-1543 (1996), which encodes a Bt toxin consisting essentially of domains I and II of Cry1Ab and domain III of Cry1C, is cloned into an expression vector such as pBluescript SK-, Bacillus shuttle vector, or pET 21b(+) for overexpression in *E. coli.* Cells are grown in LB media containing 50 micrograms/ml ampicillin for 24 to 48 h at 37°C shaker (250 rpm). Cells are harvested by centrifugation for 10 min at 7,000 rpm. The pellet is sonicated with a Bronson sonifier for 2 min 30 sec with 2 sec pulse. Complete sonication is checked under microscope. Soluble fractions are removed by centrifugation at 10,000 rpm for 10 min. The resulting pellet containing crystal proteins is washed 4-5 times with 2% Triton X-100 containing 0.5 M NaCl. Continuous washing is done with 0.5 M NaCl (4-5 times) and the final pellet is washed with distilled water (2 times). The resulting pellet is solubilized in 50 mM Na₂CO₃ buffer containing 10 mM dithiothreitol at 37°C for 2 h. Solubilized protein is separated from insoluble materials by centrifugation at 12,000 rpm for 10 min. Protein samples are dialyzed with 50 mM Na₂CO₃, pH 9.0 buffer for bioassays.

### Example 2: Bioassays

LC50's are performed on fall armyworm, pink bollworm, tobacco budworm, and European comborer using purified truncated H04 protein that is produced, for example, as described above in Example 1. Results are as follows:
LC50 fall armyworm 133 ng/cm²
LC50 pink bollworm 691 ng/cm²
LC50 tobacco budworm 299 ng/cm²
LC50 European cornborer 31 ng/cm²

### Example 3: Synthetic H04 Gene Construction

A synthetic nucleotide sequence encoding the toxin portion of H04 is designed by backtranslating the amino acid sequence of the H04 hybrid toxin fragment described in De Maagd *et al., Appl. Environ. Microbiol*. 62(5): 1537-1543 (1996) (domains I and II of Cry1Ab and domain III of Cry1C) using the "Backtranslation" program found in the University of Wisconsin GCG group of programs using a maize preference codon table (Murray *et al*., Nucl Acids Res. 17:477-498, 1989, incorporated herein by reference). Preferably, the most frequently used maize codon is used for each amino acid, as described in WO 93/07278.

The synthetic nucleotide sequence encoding the toxin portion of H04 may be constructed in several fragments. Each fragment is constructed by hybridization of ten pairs of oligomers 60-75 nucleotides in length representing both strands of the gene. An approximately 15 nucleotide overlap is designed between sequential oligonucleotide pairs for correct orientation and assembly. Oligonucleotides may be synthesized by, for example, Genosys Biotechnologies Inc., TX. Each pair of oligomers is hybridized and phosphorylated using the enzyme polynucleotide kinase from, e.g., New England Biolabs, Inc., MA using conditions specified by the vendor. Kinased fragment pairs are then hybridized and ligated into a high copy plasmid vector containing, e.g., an ampicillin resistance gene and transformed into, e.g., competent DH5α cells. The cells are plated onto ampicillin containing media and incubated overnight at 37°C. Colonies are screened for inserted DNA. The DNA is sequenced and clones containing the correct sequence are selected. The fragments are then joined by restriction digestion, ligation and transformation using unique restriction sites between the fragments.

SEQ ID NO:3 shows the synthetic nucleotide sequence encoding the 631-amino acid toxin portion of H04 (without a protoxin tail region), and SEQ ID NO:4 shows the amino acid sequence of the H04 toxin encoded by the synthetic nucleotide sequence depicted in SEQ ID NO:3. SEQ ID NO:11 shows the nucleotide sequence of construct pNOV1308, which contains the constitutive maize ubiquitin promoter operatively linked to the synthetic H04 gene sequence set forth in SEQ ID NO:3.

In addition to the above-described synthetic gene (SEQ ID NO:3) that encodes only the toxin portion of the H04 hybrid (domains I and II of Cry1Ab and domain III of Cry1C), additional synthetic H04 genes are constructed with all or a portion of the synthetic *cry1Ab* tail region described in U.S. Patent No. 5,625,136 (herein incorporated by reference) fused to the 3' end of the H04 toxin portion. These synthetic H04 gene sequences with *cry1Ab* tails are described below:

SEQ ID NO:5 shows a synthetic nucleotide sequence encoding the toxin portion of H04 plus a full-length Cry1Ab tail portion, and SEQ ID NO:6 shows the amino acid sequence of the H04 + Cry1Ab tail encoded by the synthetic nucleotide sequence depicted in SEQ ID NO:5. SEQ ID NO:12 shows the nucleotide sequence of construct pNOV1436, which contains the root-preferred maize MTL promoter operatively linked to the synthetic H04 gene sequence set forth in SEQ ID NO:5. SEQ ID NO:13 shows the nucleotide sequence of construct pNOV1441, which contains the constitutive maize ubiquitin promoter operatively linked to the synthetic H04 gene sequence set forth in SEQ ID NO:5.

SEQ ID NO:7 shows another synthetic nucleotide sequence encoding the toxin portion of H04 plus a full-length Cry1Ab tail portion, and SEQ ID NO:8 shows the amino acid sequence of the H04 + Cry1Ab tail encoded by the synthetic nucleotide sequence depicted in SEQ ID NO:7. SEQ ID NO:14 shows the nucleotide sequence of construct pNOV1305, which contains the constitutive maize ubiquitin promoter operatively linked to the synthetic H04 gene sequence set forth in SEQ ID NO:7. SEQ ID NO:15 shows the nucleotide sequence of construct pNOV1313, which contains the constitutive maize ubiquitin promoter operatively linked to the synthetic H04 gene sequence set forth in SEQ ID NO:7.

SEQ ID NO:9 shows a synthetic nucleotide sequence encoding the toxin portion of H04 plus only the first 40 amino acids of the Cry1Ab tail, and SEQ ID NO:10 shows the amino acid sequence of the H04 + 40-amino acid truncated Cry1Ab tail encoded by the synthetic nucleotide sequence depicted in SEQ ID NO:9. SEQ ID NO:16 shows the nucleotide sequence of construct pNOV1435, which contains the root-preferred maize MTL promoter operatively linked to the synthetic H04 gene sequence set forth in SEQ ID NO:9. SEQ ID NO:17 shows the nucleotide sequence of construct pZU578, which contains the Arabidopsis actin-2 promoter operatively linked to the synthetic H04 gene sequence set forth in SEQ ID NO:9.

### Example 4: Modification of Coding Sequences and Adjacent Sequences

The nucleotide sequences described in this application can be modified for expression in transgenic plant hosts. A host plant expressing the nucleotide sequences and which produces the insecticidal toxins in its cells has enhanced resistance to insect attack and is thus better equipped to withstand crop losses associated with such attack.

The transgenic expression in plants of genes derived from microbial sources may require the modification of those genes to achieve and optimize their expression in plants. In particular, bacterial ORFs that encode separate enzymes but that are encoded by the same transcript in the native microbe are best expressed in plants on separate transcripts. To achieve this, each microbial ORF is isolated individually and cloned within a cassette which provides a plant promoter sequence at the 5' end of the ORF and a plant transcriptional terminator at the 3' end of the ORF. The isolated ORF sequence preferably includes the initiating ATG codon and the terminating STOP codon but may include additional sequence beyond the initiating ATG and the STOP codon. In addition, the ORF may be truncated, but still retain the required activity; for particularly long ORFs, truncated versions which retain activity may be preferable for expression in transgenic organisms. By "plant promoter" and "plant transcriptional terminator" it is intended to mean promoters and transcriptional terminators which operate within plant cells. This includes promoters and transcription terminators which may be derived from non-plant sources such as viruses (an example is the Cauliflower Mosaic Virus).

In some cases, modification to the ORF coding sequences and adjacent sequence is not required. It is sufficient to isolate a fragment containing the ORF of interest and to insert it downstream of a plant promoter. For example, Gaffney *et al.* (Science 261: 754-756 (1993)) have expressed the *Pseudomonas nahG* gene in transgenic plants under the control of the CaMV 35S promoter and the CaMV *tml* terminator successfully without modification of the coding sequence and with x bp of the *Pseudomonas* gene upstream of the ATG still attached, and y bp downstream of the STOP codon still attached to the *nahG* ORF. Preferably as little adjacent microbial sequence should be left attached upstream of the ATG and downstream of the STOP codon. In practice, such construction may depend on the availability of restriction sites.

In other cases, the expression of genes derived from microbial sources may provide problems in expression. These problems have been well characterized in the art and are particularly common with genes derived from certain sources such as *Bacillus.* These problems may apply to the nucleotide sequence of this invention and the modification of these genes can be undertaken using techniques now well known in the art. The following problems may be encountered:

### 1. Codon Usage.

The preferred codon usage in plants differs from the preferred codon usage in certain microorganisms. Comparison of the usage of codons within a cloned microbial ORF to usage in plant genes (and in particular genes from the target plant) will enable an identification of the codons within the ORF which should preferably be changed. Typically plant evolution has tended towards a strong preference of the nucleotides C and G in the third base position of monocotyledons, whereas dicotyledons often use the nucleotides A or T at this position. By modifying a gene to incorporate preferred codon usage for a particular target transgenic species, many of the problems described below for GC/AT content and illegitimate splicing will be overcome.

### 2. GC/AT Content.

Plant genes typically have a GC content of more than 35%. ORF sequences which are rich in A and T nucleotides can cause several problems in plants. Firstly, motifs of ATTTA are believed to cause destabilization of messages and are found at the 3' end of many short-lived mRNAs. Secondly, the occurrence of polyadenylation signals such as AATAAA at inappropriate positions within the message is believed to cause premature truncation of transcription. In addition, monocotyledons may recognize AT-rich sequences as splice sites (see below).

### 3. Sequences Adjacent to the Initiating Methionine.

Plants differ from microorganisms in that their messages do not possess a defined ribosome binding site. Rather, it is believed that ribosomes attach to the 5' end of the message and scan for the first available ATG at which to start translation. Nevertheless, it is believed that there is a preference for certain nucleotides adjacent to the ATG and that expression of microbial genes can be enhanced by the inclusion of a eukaryotic consensus translation initiator at the ATG. Clontech (1993/1994 catalog, page 210, incorporated herein by reference) have suggested one sequence as a consensus translation initiator for the expression of the *E. coli uidA* gene in plants. Further, Joshi (NAR 15: 6643-6653 (1987), incorporated herein by reference) has compared many plant sequences adjacent to the ATG and suggests another consensus sequence. In situations where difficulties are encountered in the expression of microbial ORFs in plants, inclusion of one of these sequences at the initiating ATG may improve translation. In such cases the last three nucleotides of the consensus may not be appropriate for inclusion in the modified sequence due to their modification of the second AA residue. Preferred sequences adjacent to the initiating methionine may differ between different plant species. A survey of 14 maize genes located in the GenBank database provided the following results:

| Position Before the Initiating ATG in 14 Maize Genes: | | | | | | | | | | |
|---|---|---|---|---|---|---|---|---|---|---|
| | -10 | -9 | -8 | -7 | -6 | -5 | -4 | -3 | -2 | -1 |
| C | 3 | 8 | 4 | 6 | 2 | 5 | 6 | 0 | 10 | 7 |
| T | 3 | 0 | 3 | 4 | 3 | 2 | 1 | 1 | 1 | 0 |
| A | 2 | 3 | 1 | 4 | 3 | 2 | 3 | 7 | 2 | 3 |
| G | 6 | 3 | 6 | 0 | 6 | 5 | 4 | 6 | 1 | 5 |

This analysis can be done for the desired plant species into which the nucleotide sequence is being incorporated, and the sequence adjacent to the ATG modified to incorporate the preferred nucleotides.

### 4. Removal of Illegitimate Splice Sites.

Genes cloned from non-plant sources and not optimized for expression in plants may also contain motifs which may be recognized in plants as 5' or 3' splice sites, and be cleaved, thus generating truncated or deleted messages. These sites can be removed using the techniques well known in the art.

Techniques for the modification of coding sequences and adjacent sequences are well known in the art. In cases where the initial expression of a microbial ORF is low and it is deemed appropriate to make alterations to the sequence as described above, then the construction of synthetic genes can be accomplished according to methods well known in the art. These are, for example, described in the published patent disclosures EP 0 385 962, EP 0 359 472 and WO 93/07278, all of which are incorporated herein by reference. In most cases it is preferable to assay the expression of gene constructions using transient assay protocols (which are well known in the art) prior to their transfer to transgenic plants.

### Example 5: Construction of Plant Expression Cassettes

Coding sequences intended for expression in transgenic plants are first assembled in expression cassettes behind a suitable promoter expressible in plants. The expression cassettes may also comprise any further sequences required or selected for the expression of the transgene. Such sequences include, but are not restricted to, transcription terminators, extraneous sequences to enhance expression such as introns, vital sequences, and sequences intended for the targeting of the gene product to specific organelles and cell compartments. These expression cassettes can then be easily transferred to the plant transformation vectors described below. The following is a description of various components of typical expression cassettes.

### 1. Promoters

The selection of the promoter used in expression cassettes will determine the spatial and temporal expression pattern of the transgene in the transgenic plant. Selected promoters will express transgenes in specific cell types (such as leaf epidermal cells, mesophyll cells, root cortex cells) or in specific tissues or organs (roots, leaves or flowers, for example) and the selection will reflect the desired location of accumulation of the gene product. Alternatively, the selected promoter may drive expression of the gene under various inducing conditions. Promoters vary in their strength, i.e., ability to promote transcription. Depending upon the host cell system utilized, any one of a number of suitable promoters can be used, including the gene's native promoter. The following are non-limiting examples of promoters that may be used in expression cassettes.

### a. Constitutive Expression, the Ubiquitin Promoter:

Ubiquitin is a gene product known to accumulate in many cell types and its promoter has been cloned from several species for use in transgenic plants *(e.g.* sunflower - Binet *et al.* Plant Science 79: 87-94 (1991); maize - Christensen *et al*. Plant Molec. Biol. 12: 619-632 (1989); *and Arabidopsis -* Norris *et al., Plant Mol. Biol*. 21:895-906 (1993)). The maize ubiquitin promoter has been developed in transgenic monocot systems and its sequence and vectors constructed for monocot transformation are disclosed in the patent publication EP 0 342 926 which is herein incorporated by reference. Taylor *et al*. (Plant Cel6 Rep. 12: 491-495 (1993)) describe a vector (pAHC25) that comprises the maize ubiquitin promoter and first intron and its high activity in cell suspensions of numerous monocotyledons when introduced via microprojectile bombardment. The *Arabidopsis* ubiquitin promoter is ideal for use with the nucleotide sequences of the present invention. The ubiquitin promoter is suitable for gene expression in transgenic plants, both monocotyledons and dicotyledons. Suitable vectors are derivatives of pAHC25 or any of the transformation vectors described in this application, modified by the introduction of the appropriate ubiquitin promoter and/or intron sequences.

### b. Constitutive Expression, the CaMV 35S Promoter:

Construction of the plasmid pCGN1761 is described in the published patent application EP 0 392 225 (Example 23), which is hereby incorporated by reference. pCGN1761 contains the "double" CaMV 35S promoter and the *tml* transcriptional terminator with a unique *EcoRI* site between the promoter and the terminator and has a pUC-type backbone. A derivative of pCGN1761 is constructed which has a modified polylinker which includes *NotI* and *XhoI* sites in addition to the existing *EcoRI* site. This derivative is designated pCGN1761ENX. pCGN1761ENX is useful for the cloning of cDNA sequences or coding sequences (including microbial ORF sequences) within its polylinker for the purpose of their expression under the control of the 35S promoter in transgenic plants. The entire 35S promoter-coding sequence-*tml* terminator cassette of such a construction can be excised by *HindIII, SphI, SalI*, and *XbaI* sites 5' to the promoter and *XbaI, BamHI* and *BglI* sites 3' to the terminator for transfer to transformation vectors such as those described below. Furthermore, the double 35S promoter fragment can be removed by 5' excision with *HindIII, SphI, SaII, XbaI,* or *Pstl,* and 3' excision with any of the polylinker restriction sites (*EcoRI, NotI* or *XhoI*) for replacement with another promoter. If desired, modifications around the cloning sites can be made by the introduction of sequences that may enhance translation. This is particularly useful when overexpression is desired. For example, pCGN1761ENX may be modified by optimization of the translational initiation site as described in Example 37 of U.S. Patent No. 5,639,949, incorporated herein by reference.

### c. Constitutive Expression, the Actin Promoter:

Several isoforms of actin are known to be expressed in most cell types and consequently the actin promoter is a good choice for a constitutive promoter. In particular, the promoter from the rice *ActI* gene has been cloned and characterized (McElroy *et al*. Plant Cell 2: 163-171 (1990)). A 1.3kb fragment of the promoter was found to contain all the regulatory elements required for expression in rice protoplasts. Furthermore, numerous expression vectors based on the *ActI* promoter have been constructed specifically for use in monocotyledons (McElroy *et al*. Mol. Gen. Genet. 231: 150-160 (1991)). These incorporate the *ActI*-intron 1, *AdhI* 5' flanking sequence and *AdhI*-intron 1 (from the maize alcohol dehydrogenase gene) and sequence from the CaMV 35S promoter. Vectors showing highest expression were fusions of 35S and *ActI* intron or the *ActI* 5' flanking sequence and the *ActI* intron. Optimization of sequences around the initiating ATG (of the GUS reporter gene) also enhanced expression. The promoter expression cassettes described by McElroy *et al*. (Mol. Gen. Genet. 231: 150-160 (1991)) can be easily modified for gene expression and are particularly suitable for use in monocotyledonous hosts. For example, promoter-containing fragments is removed from the McElroy constructions and used to replace the double 35S promoter in pCGN1761ENX, which is then available for the insertion of specific gene sequences. The fusion genes thus constructed can then be transferred to appropriate transformation vectors. In a separate report, the rice *ActI* promoter with its first intron has also been found to direct high expression in cultured barley cells (Chibbar *et al.* Plant Cell Rep. 12:506-509 (1993)).

### d. Inducible Expression, the PR-1 Promoter:

The double 35S promoter in pCGN1761ENX may be replaced with any other promoter of choice that will result in suitably high expression levels. By way of example, one of the chemically regulatable promoters described in U.S. Patent No. 5,614,395, such as the tobacco PR-1a promoter, may replace the double 35S promoter. Alternately, the *Arabidopsis* PR-1 promoter described in Lebel *et al., Plant J.* 16:223-233 (1998) may be used. The promoter of choice is preferably excised from its source by restriction enzymes, but can alternatively be PCR-amplified using primers that carry appropriate terminal restriction sites. Should PCR-amplification be undertaken, then the promoter should be re-sequenced to check for amplification errors after the cloning of the amplified promoter in the target vector. The chemically/pathogen regulatable tobacco PR-1a promoter is cleaved from plasmid pCIB1004 (for construction, see example 21 of EP 0 332 104, which is hereby incorporated by reference) and transferred to plasmid pCGN1761ENX (Uknes *et al., Plant Cell* 4: 645-656 (1992)). pCIB1004 is cleaved with *NcoI* and the resultant 3' overhang of the linearized fragment is rendered blunt by treatment with T4 DNA polymerase. The fragment is then cleaved with *HindIII* and the resultant PR-1a promoter-containing fragment is gel purified and cloned into pCGN1761ENX from which the double 35S promoter has been removed. This is done by cleavage with *XhoI* and blunting with T4 polymerase, followed by cleavage with *HindIII* and isolation of the larger vector-terminator containing fragment into which the pCIB1004 promoter fragment is cloned. This generates a pCGN1761ENX derivative with the PR-1a promoter and the *tml* terminator and an intervening polylinker with unique *EcoRI* and *Notl* sites. The selected coding sequence can be inserted into this vector, and the fusion products (*i*.*e*. promoter-gene-terminator) can subsequently be transferred to any selected transformation vector, including those described *infra.* Various chemical regulators may be employed to induce expression of the selected coding sequence in the plants transformed according to the present invention, including the benzothiadiazole, isonicotinic acid, and salicylic acid compounds disclosed in U.S. Patent Nos. 5,523,311 and 5,614,395.

### e. Inducible Expression, an Ethanol-Inducible Promoter:

A promoter inducible by certain alcohols or ketones, such as ethanol, may also be used to confer inducible expression of a coding sequence of the present invention. Such a promoter is for example the *alcA* gene promoter from *Aspergillus nidulans* (Caddick et al. (1998) *Nat. Biotechnol* 16:177-180). In *A. nidulans,* the *alcA* gene encodes alcohol dehydrogenase I, the expression of which is regulated by the AlcR transcription factors in presence of the chemical inducer. For the purposes of the present invention, the CAT coding sequences in plasmid palcA:CAT comprising a *alcA* gene promoter sequence fused to a minimal 35S promoter (Caddick et al. (1998) *Nat. Biotechnol* 16:177-180) are replaced by a coding sequence of the present invention to form an expression cassette having the coding sequence under the control of the *alcA* gene promoter. This is carried out using methods well known in the art.

### f. Inducible Expression, a Glucocorticoid-Inducible Promoter:

Induction of expression of a nucleic acid sequence of the present invention using systems based on steroid hormones is also contemplated. For example, a glucocorticoid-mediated induction system is used (Aoyama and Chua (1997) *The Plant Journal* 11: 605-612) and gene expression is induced by application of a glucocorticoid, for example a synthetic glucocorticoid, preferably dexamethasone, preferably at a concentration ranging from 0.1mM to 1mM, more preferably from 10mM to 100mM. For the purposes of the present invention, the luciferase gene sequences are replaced by a nucleic acid sequence of the invention to form an expression cassette having a nucleic acid sequence of the invention under the control of six copies of the GAL4 upstream activating sequences fused to the 35S minimal promoter. This is carried out using methods well known in the art. The trans-acting factor comprises the GAL4 DNA-binding domain (Keegan et al. (1986) *Science* 231: 699-704) fused to the transactivating domain of the herpes viral protein VP16 (Triezenberg et al. (1988) *Genes Devel.* 2: 718-729) fused to the hormone-binding domain of the rat glucocorticoid receptor (Picard et al. (1988) *Cell 54:* 1073-1080). The expression of the fusion protein is controlled by any promoter suitable for expression in plants known in the art or described here. This expression cassette is also comprised in the plant comprising a nucleic acid sequence of the invention fused to the 6xGAL4/minimal promoter. Thus, tissue- or organ-specificity of the fusion protein is achieved leading to inducible tissue- or organ-specificity of the insecticidal toxin.

### g. Root Specific Expression:

Another pattern of gene expression is root expression. A suitable root promoter is the promoter of the maize metallothionein-like (MTL) gene described by de Framond (FEBS 290: 103-106 (1991)) and also in U.S. Patent No. 5,466,785, incorporated herein by reference. This "MTL" promoter is transferred to a suitable vector such as pCGN1761ENX for the insertion of a selected gene and subsequent transfer of the entire promoter-gene-terminator cassette to a transformation vector of interest.

### h. Wound-Inducible Promoters:

Wound-inducible promoters may also be suitable for gene expression. Numerous such promoters have been described *(e.g.* Xu *et al.* Plant Molec. Biol. 22: 573-588 (1993), Logemann *et al*. Plant Cell 1: 151-158 (1989), Rohrmeier & Lehle, Plant Molec. Biol. 22: 783-792 (1993), Firek *et al.* Plant Molec. Biol. 22: 129-142 (1993), Warner *et al.* Plant J. 3: 191-201 (1993)) and all are suitable for use with the instant invention. Logemann *et al*. describe the 5' upstream sequences of the dicotyledonous potato *wunI* gene. Xu *et al*. show that a wound-inducible promoter from the dicotyledon potato (*pin2*) is active in the monocotyledon rice. Further, Rohrmeier & Lehle describe the cloning of the maize *WipI* cDNA which is wound induced and which can be used to isolate the cognate promoter using standard techniques. Similar, Firek *et al*. and Warner *et al*. have described a wound-induced gene from the monocotyledon *Asparagus officinalis,* which is expressed at local wound and pathogen invasion sites. Using cloning techniques well known in the art, these promoters can be transferred to suitable vectors, fused to the genes pertaining to this invention, and used to express these genes at the sites of plant wounding.

### i. Pith-Preferred Expression:

Patent Application WO 93/07278, which is herein incorporated by reference, describes the isolation of the maize *trpA* gene, which is preferentially expressed in pith cells. The gene sequence and promoter extending up to -1726 bp from the start of transcription are presented. Using standard molecular biological techniques, this promoter, or parts thereof, can be transferred to a vector such as pCGN1761 where it can replace the 35S promoter and be used to drive the expression of a foreign gene in a pith-preferred manner. In fact, fragments containing the pith-preferred promoter or parts thereof can be transferred to any vector and modified for utility in transgenic plants.

### j. Leaf-Specific Expression:

A maize gene encoding phosphoenol carboxylase (PEPC) has been described by Hudspeth & Grula (Plant Molec Biol 12: 579-589 (1989)). Using standard molecular biological techniques the promoter for this gene can be used to drive the expression of any gene in a leaf-specific manner in transgenic plants.

### k. Pollen-Specific Expression:

WO 93/07278 describes the isolation of the maize calcium-dependent protein kinase (CDPK) gene which is expressed in pollen cells. The gene sequence and promoter extend up to 1400 bp from the start of transcription. Using standard molecular biological techniques, this promoter or parts thereof, can be transferred to a vector such as pCGN1761 where it can replace the 35S promoter and be used to drive the expression of a nucleic acid sequence of the invention in a pollen-specific manner.

### 1. Receptor Mediated Transactivation In The Presence Of A Chemical Ligand:

U.S. Patent No. 5,880,333, incorporated herein by reference, describes a system whereby class II hormone receptors such as Ecdysone Receptor (EcR) and Ultraspiracle (USP), which function together as a heterodimer, regulate the expression of a target polypeptide in a plant cell in the presence of an appropriate chemical ligand, e.g. tebufenozide.

### 2. Transcriptional Terminators

A variety of transcriptional terminators are available for use in expression cassettes. These are responsible for the termination of transcription beyond the transgene and its correct polyadenylation. Appropriate transcriptional terminators are those that are known to function in plants and include the CaMV 35S terminator, the *tml* terminator, the nopaline synthase terminator and the pea *rbcS* E9 terminator. These can be used in both monocotyledons and dicotyledons. In addition, a gene's native transcription terminator may be used.

### 3. Sequences for the Enhancement or Regulation of Expression

Numerous sequences have been found to enhance gene expression from within the transcriptional unit and these sequences can be used in conjunction with the genes of this invention to increase their expression in transgenic plants.

Various intron sequences have been shown to enhance expression, particularly in monocotyledonous cells. For example, the introns of the maize *AdhI* gene have been found to significantly enhance the expression of the wild-type gene under its cognate promoter when introduced into maize cells. Intron 1 was found to be particularly effective and enhanced expression in fusion constructs with the chloramphenicol acetyltransferase gene (Callis *et al.,* Genes Develop. 1: 1183-1200 (1987)). In the same experimental system, the intron from the maize *bronzel* gene had a similar effect in enhancing expression. Intron sequences have been routinely incorporated into plant transformation vectors, typically within the non-translated leader.

A number of non-translated leader sequences derived from viruses are also known to enhance expression, and these are particularly effective in dicotyledonous cells. Specifically, leader sequences from Tobacco Mosaic Virus (TMV, the "W-sequence"), Maize Chlorotic Mottle Virus (MCMV), and Alfalfa Mosaic Virus (AMV) have been shown to be effective in enhancing expression (*e*.*g*. Gallie *et al*. Nucl. Acids Res. 15: 8693-8711 (1987); Skuzeski *et al*. Plant Molec. Biol. 15: 65-79 (1990)).

### 4. Targeting of the Gene Product Within the Cell

Various mechanisms for targeting gene products are known to exist in plants and the sequences controlling the functioning of these mechanisms have been characterized in some detail. For example, the targeting of gene products to the chloroplast is controlled by a signal sequence found at the amino terminal end of various proteins which is cleaved during chloroplast import to yield the mature protein *(e.g.* Comai *et al*. J. Biol. Chem. 263: 15104-15109 (1988)). These signal sequences can be fused to heterologous gene products to effect the import of heterologous products into the chloroplast (van den Broeck, et al. Nature 313: 358-363 (1985)). DNA encoding for appropriate signal sequences can be isolated from the 5' end of the cDNAs encoding the RUBISCO protein, the CAB protein, the EPSP synthase enzyme, the GS2 protein and many other proteins which are known to be chloroplast localized. *See also,* the section entitled "Expression With Chloroplast Targeting" in Example 37 of U.S. Patent No. 5,639,949.

Other gene products are localized to other organelles such as the mitochondrion and the peroxisome *(e.g.* Unger *et al.* Plant Molec. Biol. 13: 411-418 (1989)). The cDNAs encoding these products can also be manipulated to effect the targeting of heterologous gene products to these organelles. Examples of such sequences are the nuclear-encoded ATPases and specific aspartate amino transferase isoforms for mitochondria. Targeting cellular protein bodies has been described by Rogers *et al*. (Proc. Natl. Acad. Sci. USA 82: 6512-6516 (1985)).

In addition, sequences have been characterized which cause the targeting of gene products to other cell compartments. Amino terminal sequences are responsible for targeting to the ER, the apoplast, and extracellular secretion from aleurone cells (Koehler & Ho, Plant Cell 2: 769-783 (1990)). Additionally, amino terminal sequences in conjunction with carboxy terminal sequences are responsible for vacuolar targeting of gene products (Shinshi *et al.* Plant Molec. Biol. 14: 357-368 (1990)).

By the fusion of the appropriate targeting sequences described above to transgene sequences of interest it is possible to direct the transgene product to any organelle or cell compartment. For chloroplast targeting, for example, the chloroplast signal sequence from the RUBISCO gene, the CAB gene, the EPSP synthase gene, or the GS2 gene is fused in frame to the amino terminal ATG of the transgene. The signal sequence selected should include the known cleavage site, and the fusion constructed should take into account any amino acids after the cleavage site which are required for cleavage. In some cases this requirement may be fulfilled by the addition of a small number of amino acids between the cleavage site and the transgene ATG or, alternatively, replacement of some amino acids within the transgene sequence. Fusions constructed for chloroplast import can be tested for efficacy of chloroplast uptake by *in vitro* translation of *in vitro* transcribed constructions followed by *in vitro* chloroplast uptake using techniques described by Bartlett *et al.* In: Edehnann *et al.* (Eds.) Methods in Chloroplast Molecular Biology, Elsevier pp 1081-1091 (1982) and Wasmann *et al.* Mol. Gen. Genet. 205: 446-453 (1986). These construction techniques are well known in the art and are equally applicable to mitochondria and peroxisomes.

The above-described mechanisms for cellular targeting can be utilized not only in conjunction with their cognate promoters, but also in conjunction with heterologous promoters so as to effect a specific cell-targeting goal under the transcriptional regulation of a promoter that has an expression pattern different to that of the promoter from which the targeting signal derives.

### Example 6: Construction of Plant Transformation Vectors

Numerous transformation vectors available for plant transformation are known to those of ordinary skill in the plant transformation arts, and the genes pertinent to this invention can be used in conjunction with any such vectors. The selection of vector will depend upon the preferred transformation technique and the target species for transformation. For certain target species, different antibiotic or herbicide selection markers may be preferred. Selection markers used routinely in transformation include the *nptII* gene, which confers resistance to kanamycin and related antibiotics (Messing & Vierra. Gene 19: 259-268 (1982); Bevan et al., Nature 304:184-187 (1983)), the *bar* gene, which confers resistance to the herbicide phosphinothricin (White et al., Nucl. Acids Res 18: 1062 (1990), Spencer et al. Theor. Appl. Genet 79: 625-631 (1990)), the *hph* gene, which confers resistance to the antibiotic hygromycin (Blochinger & Diggelmann, Mol Cell Biol 4: 2929-2931), and the *dhfr* gene, which confers resistance to methatrexate (Bourouis et al., EMBO J. 2(7): 1099-1104 (1983)), the EPSPS gene, which confers resistance to glyphosate (U.S. Patent Nos. 4,940,935 and 5,188,642), and the mannose-6-phosphate isomerase gene, which provides the ability to metabolize mannose (U.S. Patent Nos. 5,767,378 and 5,994,629).

### 1. Vectors Suitable for Agrobacterium Transformation

Many vectors are available for transformation using *Agrobacterium tumefaciens*. These typically carry at least one T-DNA border sequence and include vectors such as pBIN19 (Bevan, Nucl. Acids Res. (1984)) and pXYZ. Below, the construction of two typical vectors suitable for *Agrobacterium* transformation is described.

### a. pCIB200 and pCTB2001:

The binary vectors pCIB200 and pCIB2001 are used for the construction of recombinant vectors for use with *Agrobacterium* and are constructed in the following manner. pTJS75kan is created by *NarI* digestion of pTJS75 (Schmidhauser & Helinski, J. Bacteriol. 164: 446-455 (1985)) allowing excision of the tetracycline-resistance gene, followed by insertion of an *Accl* fragment from pUC4K carrying an NPTII (Vieira & Messing, Gene 19: 259-268 (1982): Bevan et al., Nature 304: 184-187 (1983): McBride et al., Plant Molecular Biology 14: 266-276 (1990)). *XhoI* linkers are ligated to the *EcoRV* fragment of PCIB7 which contains the left and right T-DNA borders, a plant selectable *nos*/*nptII* chimeric gene and the pUC polylinker (Rothstein et al., Gene 53: 153-161 (1987)), and the *Xhol*-digested fragment are cloned into *SalI*-digested pTJS75kan to create pCIB200 (see also EP 0 332 104, example 19). pCIB200 contains the following unique polylinker restriction sites: *EcoRI, SstI, KpnI, BglII, XbaI*, and *SaU.* pCIB2001 is a derivative of pCIB200 created by the insertion into the polylinker of additional restriction sites. Unique restriction sites in the polylinker of pCIB2001 are *EcoRI, SstI, Kpnl, BglII, XbaI, SalI, MluI, BclI, AvrlI, Apal, Hpal,* and *StuI*. pCIB2001, in addition to containing these unique restriction sites also has plant and bacterial kanamycin selection, left and right T-DNA borders for *Agrobacterium*-mediated transformation, the RK2-derived *trfA* function for mobilization between *E. coli* and other hosts, and the *OriT* and *OriV* functions also from RK2. The pCIB2001 polylinker is suitable for the cloning of plant expression cassettes containing their own regulatory signals.

### b. pCIB10 and Hygromycin Selection Derivatives thereof:

The binary vector pCIB10 contains a gene encoding kanamycin resistance for selection in plants and T-DNA right and left border sequences and incorporates sequences from the wide host-range plasmid pRK252 allowing it to replicate in both *E. coli* and *Agrobacterium*. Its construction is described by Rothstein *et al.* (Gene 53: 153-161 (1987)). Various derivatives of pCIB10 are constructed which incorporate the gene for hygromycin B phosphotransferase described by Gritz *et al*. (Gene 25: 179-188 (1983)). These derivatives enable selection of transgenic plant cells on hygromycin only (pCIB743), or hygromycin and kanamycin (pCIB715, pCIB717).

### 2. Vectors Suitable for non-Agrobacterium Transformation

Transformation without the use of *Agrobacrerium tumefaciens* circumvents the requirement for T-DNA sequences in the chosen transformation vector and consequently vectors lacking these sequences can be utilized in addition to vectors such as the ones described above which contain T-DNA sequences. Transformation techniques that do not rely on *Agrobacterium* include transformation via particle bombardment, protoplast uptake (*e*.*g*. PEG and electroporation) and microinjection. The choice of vector depends largely on the preferred selection for the species being transformed. Below, the construction of typical vectors suitable for non-*Agrobacterium* transformation is described.

### a. pCIB3064:

pCIB3064 is a pUC-derived vector suitable for direct gene transfer techniques in combination with selection by the herbicide basta (or phosphinothricin). The plasmid pCIB246 comprises the CaMV 35S promoter in operational fusion to the *E. coli* GUS gene and the CaMV 35S transcriptional terminator and is described in the PCT published application WO 93/07278. The 35S promoter of this vector contains two ATG sequences 5' of the start site. These sites are mutated using standard PCR techniques in such a way as to remove the ATGs and generate the restriction sites *SspI* and *PvuII*. The new restriction sites are 96 and 37 bp away from the unique *SalI* site and 101 and 42 bp away from the actual start site. The resultant derivative of pCIB246 is designated pCIB3025. The GUS gene is then excised from pCIB3025 by digestion with *SalI* and *SacI*, the termini rendered blunt and religated to generate plasmid pCIB3060. The plasmid pJIT82 is obtained from the John Innes Centre, Norwich and the a 400 bp *SmaI* fragment containing the *bar* gene from *Streptomyces viridochromogenes* is excised and inserted into the *HpaI* site of pCIB3060 (Thompson *et al*. EMBO J 6: 2519-2523 (1987)). This generated pCIB3064, which comprises the *bar* gene under the control of the CaMV 35S promoter and terminator for herbicide selection, a gene for ampicillin resistance (for selection in *E. coli*) and a polylinker with the unique sites *Sphl, PstI, HindIII,* and *BamHI*. This vector is suitable for the cloning of plant expression cassettes containing their own regulatory signals.

### b. pSOG19 and pSOG35:

pSOG35 is a transformation vector that utilizes the *E. coli* gene dihydrofolate reductase (DFR) as a selectable marker conferring resistance to methotrexate. PCR is used to amplify the 35S promoter (-800 bp), intron 6 from the maize Adh1 gene (-550 bp) and 18 bp of the GUS untranslated leader sequence from pSOG10. A 250-bp fragment encoding the *E. coli* driydrofolate reductase type II gene is also amplified by PCR and these two PCR fragments are assembled with a *SacI-PstI* fragment from pB1221 (Clontech) which comprises the pUC19 vector backbone and the nopaline synthase terminator. Assembly of these fragments generates pSOG19 which contains the 35S promoter in fusion with the intron 6 sequence, the GUS leader, the DHFR gene and the nopaline synthase terminator. Replacement of the GUS leader in pSOG19 with the leader sequence from Maize Chlorotic Mottle Virus (MCMV) generates the vector pSOG35. pSOG19 and pSOG35 carry the pUC gene for ampicillin resistance and have *HindIII, SphI, PstI* and *EcoRI* sites available for the cloning of foreign substances.

### 3. Vector Suitable for Chloroplast Transformation

For expression of a nucleotide sequence of the present invention in plant plastids, plastid transformation vector pPH143 (WO 97/32011, example 36) is used. The nucleotide sequence is inserted into pPH143 thereby replacing the PROTOX coding sequence. This vector is then used for plastid transformation and selection of transformants for spectinomycin resistance. Alternatively, the nucleotide sequence is inserted in pPH143 so that it replaces the aadH gene. In this case, transformants are selected for resistance to PROTOX inhibitors.

### Example 7: Transformation

Once a nucleic acid sequence of the invention has been cloned into an expression system, it is transformed into a plant cell. Methods for transformation and regeneration of plants are well known in the art. For example, Ti plasmid vectors have been utilized for the delivery of foreign DNA, as well as direct DNA uptake, liposomes, electroporation, micro-injection, and microprojectiles. In addition, bacteria from the genus *Agrobacrerium* can be utilized to transform plant cells. Below are descriptions of representative techniques for transforming both dicotyledonous and monocotyledonous plants, as well as a representative plastid transformation technique.

### 1. Transformation of Dicotyledons

Transformation techniques for dicotyledons are well known in the art and include *Agrobacterium*-based techniques and techniques that do not require *Agrobacterium.* Non-*Agrobacterium* techniques involve the uptake of exogenous genetic material directly by protoplasts or cells. This can be accomplished by PEG or electroporation mediated uptake, particle bombardment-mediated delivery, or microinjection. Examples of these techniques are described by Paszkowski *et al*., EMBO J 3: 2717-2722 (1984), Potrykus *et al*.*,* Mol. Gen. Genet. 199: 169-177 (1985), Reich *et al*., Biotechnology 4: 1001-1004 (1986), and Klein *et al*., Nature 327: 70-73 (1987). In each case the transformed cells are regenerated to whole plants using standard techniques known in the art.

*Agrobacterium*-mediated transformation is a preferred technique for transformation of dicotyledons because of its high efficiency of transformation and its broad utility with many different species. *Agrobacterium* transformation typically involves the transfer of the binary vector carrying the foreign DNA of interest *(e.g.* pCIB200 or pCIB2001) to an appropriate *Agrobacterium* strain which may depend of the complement of vir genes carried by the host *Agrobacterium* strain either on a co-resident Ti plasmid or chromosomally (*e*.*g*. strain CIB542 for pCIB200 and pCIB2001 (Uknes *et al*. Plant Cell 5: 159-169 (1993)). The transfer of the recombinant binary vector to *Agrobacterium* is accomplished by a triparental mating procedure using *E. coli* carrying the recombinant binary vector, a helper *E. coli* strain which carries a plasmid such as pRK2013 and which is able to mobilize the recombinant binary vector to the target *Agrobacterium* strain. Alternatively, the recombinant binary vector can be transferred to *Agrobacterium* by DNA transformation (Höfgen & Willmitzer, Nucl. Acids Res. 16: 9877 (1988)).

Transformation of the target plant species by recombinant *Agrobacterium* usually involves co-cultivation of the *Agrobacterium* with explants from the plant and follows protocols well known in the art. Transformed tissue is regenerated on selectable medium carrying the antibiotic or herbicide resistance marker present between the binary plasmid T-DNA borders.

Another approach to transforming plant cells with a gene involves propelling inert or biologically active particles at plant tissues and cells. This technique is disclosed in U.S. Patent Nos. 4,945,050, 5,036,006, and 5,100,792. Generally, this procedure involves propelling inert or biologically active particles at the cells under conditions effective to penetrate the outer surface of the cell and afford incorporation within the interior thereof. When inert particles are utilized, the vector can be introduced into the cell by coating the particles with the vector containing the desired gene. Alternatively, the target cell can be surrounded by the vector so that the vector is carried into the cell by the wake of the particle. Biologically active particles (e.g., dried yeast cells, dried bacterium or a bacteriophage, each containing DNA sought to be introduced) can also be propelled into plant cell tissue.

### 2. Transformation of Monocotyledons

Transformation of most monocotyledon species has now also become routine. Preferred techniques include direct gene transfer into protoplasts using PEG or electroporation techniques, and particle bombardment into callus tissue. Transformations can be undertaken with a single DNA species or multiple DNA species (*i*.*e*. co-transformation) and both these techniques are suitable for use with this invention. Co-transformation may have the advantage of avoiding complete vector construction and of generating transgenic plants with unlinked loci for the gene of interest and the selectable marker, enabling the removal of the selectable marker in subsequent generations, should this be regarded desirable. However, a disadvantage of the use of co-transformation is the less than 100% frequency with which separate DNA species are integrated into the genome (Schocher *et al*. Biotechnology 4: 1093-1096 (1986)).

Patent Applications EP 0 292 435, EP 0 392 225, and WO 93/07278 describe techniques for the preparation of callus and protoplasts from an elite inbred line of maize, transformation of protoplasts using PEG or electroporation, and the regeneration of maize plants from transformed protoplasts. Gordon-Kamm *et al*. (Plant Cell 2: 603-618 (1990)) and Fromm *et al*. (Biotechnology 8: 833-839 (1990)) have published techniques for transformation of A188-derived maize line using particle bombardment. Furthermore, WO 93/07278 and Koziel *et al*. (Biotechnology 11: 194-200 (1993)) describe techniques for the transformation of elite inbred lines of maize by particle bombardment. This technique utilizes immature maize embryos of 1.5-2.5 mm length excised from a maize ear 14-15 days after pollination and a PDS-1000He Biolistics device for bombardment.

Transformation of rice can also be undertaken by direct gene transfer techniques utilizing protoplasts or particle bombardment. Protoplast-mediated transformation has been described for *Japonica*-types and *Indica*-types (Zhang *et al*. Plant Cell Rep 7: 379-384 (1988); Shimamoto *et al.* Nature 338: 274-277 (1989); Datta *et al*. Biotechnology 8: 736-740 (1990)). Both types are also routinely transformable using particle bombardment (Christou *et al*. Biotechnology 9: 957-962 (1991)). Furthermore, WO 93/21335 describes techniques for the transformation of rice via electroporation.

Patent Application EP 0 332 581 describes techniques for the generation, transformation and regeneration of Pooideae protoplasts. These techniques allow the transformation of *Dactylis* and wheat. Furthermore, wheat transformation has been described by Vasil *et al.* (Biotechnology 10: 667-674 (1992)) using particle bombardment into cells of type C long-term regenerable callus, and also by Vasil *et al*. (Biotechnology 11: 1553-1558 (1993)) and Weeks *et al*. (Plant Physiol. 102: 1077-1084 (1993)) using particle bombardment of immature embryos and immature embryo-derived callus. A preferred technique for wheat transformation, however, involves the transformation of wheat by particle bombardment of immature embryos and includes either a high sucrose or a high maltose step prior to gene delivery. Prior to bombardment, any number of embryos (0.75-1 mm in length) are plated onto MS medium with 3% sucrose (Murashiga & Skoog, Physiologia Plantarum 15: 473-497 (1962)) and 3 mg/l 2,4-D for induction of somatic embryos, which is allowed to proceed in the dark. On the chosen day of bombardment, embryos are removed from the induction medium and placed onto the osmoticum (*i*.*e*. induction medium with sucrose or maltose added at the desired concentration, typically 15%). The embryos are allowed to plasmolyze for 2-3 h and are then bombarded. Twenty embryos per target plate is typical, although not critical. An appropriate gene-carrying plasmid (such as pCIB3064 or pSG35) is precipitated onto micrometer size gold particles using standard procedures. Each plate of embryos is shot with the DuPont Biolistics® helium device using a burst pressure of ∼1000 psi using a standard 80 mesh screen. After bombardment, the embryos are placed back into the dark to recover for about 24 h (still on osmoticum). After 24 hrs, the embryos are removed from the osmoticum and placed back onto induction medium where they stay for about a month before regeneration. Approximately one month later the embryo explants with developing embryogenic callus are transferred to regeneration medium (MS + 1 mg/liter NAA, 5 mg/liter GA), further containing the appropriate selection agent (10 mg/l basta in the case of pCIB3064 and 2 mg/l methotrexate in the case of pSOG35). After approximately one month, developed shoots are transferred to larger sterile containers known as "GA7s" which contain half-strength MS, 2% sucrose, and the same concentration of selection agent.

Tranformation of monocotyledons using *Agrobacterium* has also been described. See, WO 94/00977 and U.S. Patent No. 5,591,616, both incorporated herein by reference.

### 3. Transformation of Plastids

Seeds of *Nicotiana tabacum* c.v. 'Xanthi nc' are germinated seven per plate in a 1" circular array on T agar medium and bombarded 12-14 days after sowing with 1 µm tungsten particles (M10, Biorad, Hercules, CA) coated with DNA from plasmids pPH143 and pPH145 essentially as described (Svab, Z. and Maliga, P. (1993) *PNAS* 90, 913-917). Bombarded seedlings are incubated on T medium for two days after which leaves are excised and placed abaxial side up in bright light (350-500 µmol photons/m²/s) on plates of RMOP medium (Svab, Z., Hajdukiewicz, P. and Maliga, P. (1990) *PNAS* 87, 8526-8530) containing 500 µg/ml spectinomycin dihydrochloride (Sigma, St. Louis, MO). Resistant shoots appearing underneath the bleached leaves three to eight weeks after bombardment are subcloned onto the same selective medium, allowed to form callus, and secondary shoots isolated and subcloned. Complete segregation of transformed plastid genome copies (bomoplasmicity) in independent subclones is assessed by standard techniques of Southern blotting (Sambrook et al., (1989) Molecular Cloning: A Laboratory Manual, Cold Spring Harbor Laboratory, Cold Spring Harbor). BamHI/EcoRI-digested total cellular DNA (Mettler, I. J. (1987) *Plant Mol Biol Reporter* 5, 346-349) is separated on 1% Tris-borate (TBE) agarose gels, transferred to nylon membranes (Amersham) and probed with ³²P-labeled random primed DNA sequences corresponding to a 0.7 kb BamHI/HindIII DNA fragment from pC8 containing a portion of the *rps7*/*12* plastid targeting sequence. Homoplasmic shoots are rooted aseptically on spectinomycin-containing MS/IBA medium (McBride, K. E. et al. (1994) *PNAS* 91, 7301-7305) and transferred to the greenhouse.

### Example 8: Breeding

The plants obtained via tranformation with a nucleic acid sequence of the present invention can be any of a wide variety of plant species, including those of monocots and dicots; however, the plants used in the method of the invention are preferably selected from the list of agronomically important target crops set forth *supra.* The expression of a gene of the present invention in combination with other characteristics important for production and quality can be incorporated into plant lines through breeding. Breeding approaches and techniques are known in the art. See, for example, Welsh J. R., *Fundamentals of Plant Genetics and Breeding,* John Wiley & Sons, NY (1981); *Crop Breeding,* Wood D. R. (Ed.) American Society of Agronomy Madison, Wisconsin (1983); Mayo O., *The Theory of Plant Breeding,* 2nd Edition, Clarendon Press, Oxford (1987); Singh, D.P., *Breeding for Resistance to Diseases and Insect Pests,* Springer-Verlag, NY (1986); Wricke and Weber, *Quantitative Genetics and Selection Plant Breeding,* Walter de Gruyter and Co., Berlin (1986).

The genetic properties engineered into the transgenic seeds and plants described above are passed on by sexual reproduction or vegetative growth and can thus be maintained and propagated in progeny plants. Generally said maintenance and propagation make use of known agricultural methods developed to fit specific purposes such as tilling, sowing or harvesting. Specialized processes such as hydroponics or greenhouse technologies can also be applied. As the growing crop is vulnerable to attack and damages caused by insects or infections as well as to competition by weed plants, measures are undertaken to control weeds, plant diseases, insects, nematodes, and other adverse conditions to improve yield. These include mechanical measures such a tillage of the soil or removal of weeds and infected plants, as well as the application of agrochemicals such as herbicides, fungicides, gametocides, nematicides, growth regulants, ripening agents and insecticides.

Use of the advantageous genetic properties of the transgenic plants and seeds according to the invention can further be made in plant breeding, which aims at the development of plants with improved properties such as tolerance of pests, herbicides, or stress, improved nutritional value, increased yield, or improved structure causing less loss from lodging or shattering. The various breeding steps are characterized by well-defined human intervention such as selecting the lines to be crossed, directing pollination of the parental lines, or selecting appropriate progeny plants. Depending on the desired properties, different breeding measures are taken. The relevant techniques are well known in the art and include but are not limited to hybridization, inbreeding, backcross breeding, multiline breeding, variety blend, interspecific hybridization, aneuploid techniques, etc. Hybridization techniques also include the sterilization of plants to yield male or female sterile plants by mechanical, chemical, or biochemical means. Cross pollination of a male sterile plant with pollen of a different line assures that the genome of the male sterile but female fertile plant will uniformly obtain properties of both parental lines. Thus, the transgenic seeds and plants according to the invention can be used for the breeding of improved plant lines, that for example, increase the effectiveness of conventional methods such as herbicide or pestidice treatment or allow one to dispense with said methods due to their modified genetic properties. Alternatively new crops with improved stress tolerance can be obtained, which, due to their optimized genetic "equipment", yield harvested product of better quality than products that were not able to tolerate comparable adverse developmental conditions.

### Example 9: Seed Production

In seed production, germination quality and uniformity of seeds are essential product characteristics, whereas germination quality and uniformity of seeds harvested and sold by the farmer is not important. As it is difficult to keep a crop free from other crop and weed seeds, to control seedborne diseases, and to produce seed with good germination, fairly extensive and well-defined seed production practices have been developed by seed producers, who are experienced in the art of growing, conditioning and marketing of pure seed. Thus, it is common practice for the farmer to buy certified seed meeting specific quality standards instead of using seed harvested from his own crop. Propagation material to be used as seeds is customarily treated with a protectant coating comprising herbicides, insecticides, fungicides, bactericides, nematicides, molluscicides, or mixtures thereof. Customarily used protectant coatings comprise compounds such as captan, carboxin, thiram (TMTD®), methalaxyl (Apron®), and pirimiphos-methyl (Actellic®). If desired, these compounds are formulated together with carriers, surfactants or application-promoting adjuvants customarily employed in formulation art to protect against damage caused by bacterial, fungal or animal pests. The protectant coatings may be applied by impregnating propagation material with a liquid formulation or by coating with a combined wet or dry formulation. Other methods of application are also possible such as treatment directed at the buds or the fruit.

### Example 10: Maize Plant Analysis

Maize plants transformed with plasmids pNOV1436, pNOV1441, and pNOV1313 via Agrobacterium-mediated transformation give 100% mortality against European cornborer and fall armyworm. ELISA data is set forth below:

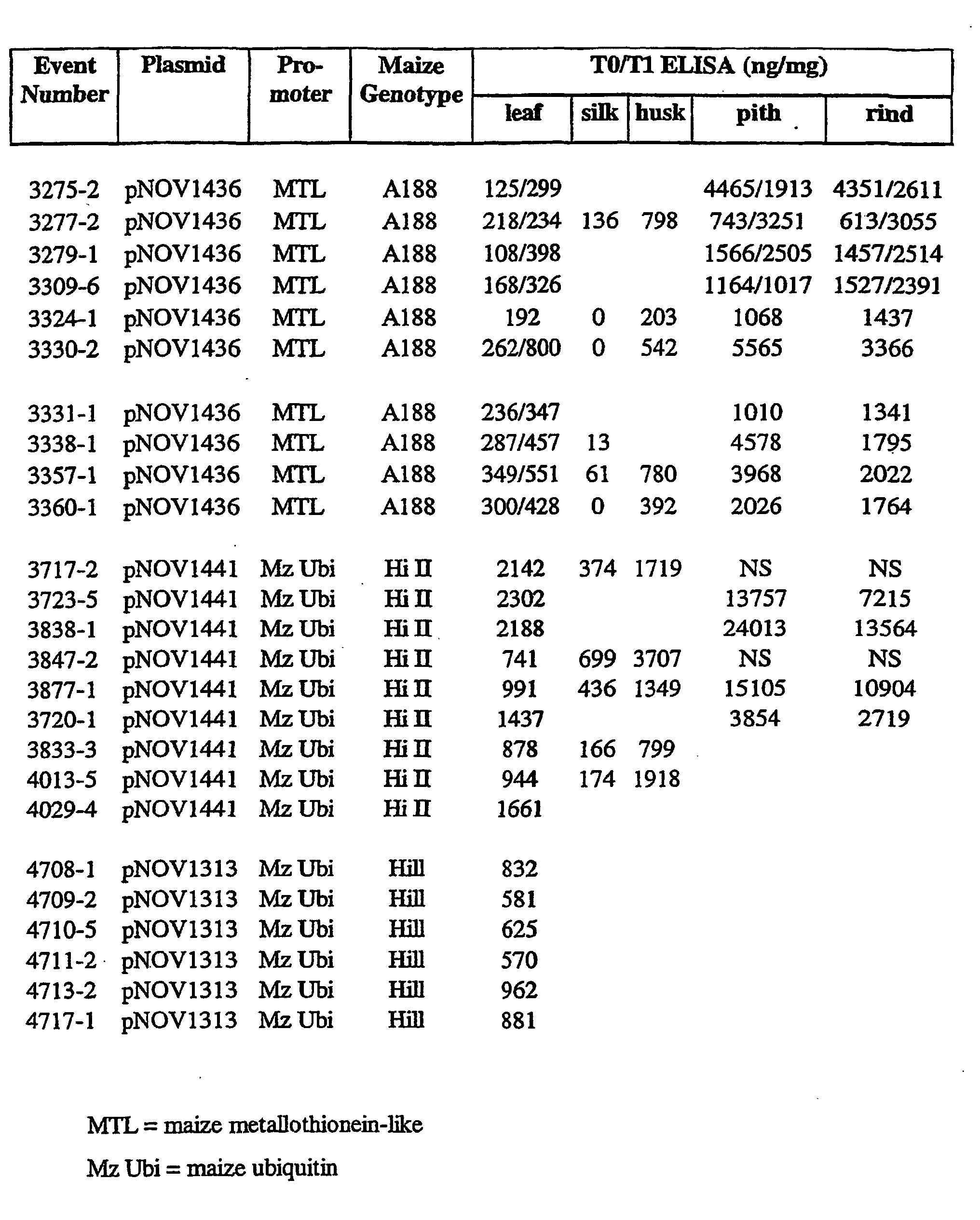

### Example 11. Rice Plant Analysis

Rice plants transformed with plasmid pNOV1305 via Agrobacterium-mediated transformation give 100% mortality against European cornborer and fall armyworm. ELISA data is set forth below:

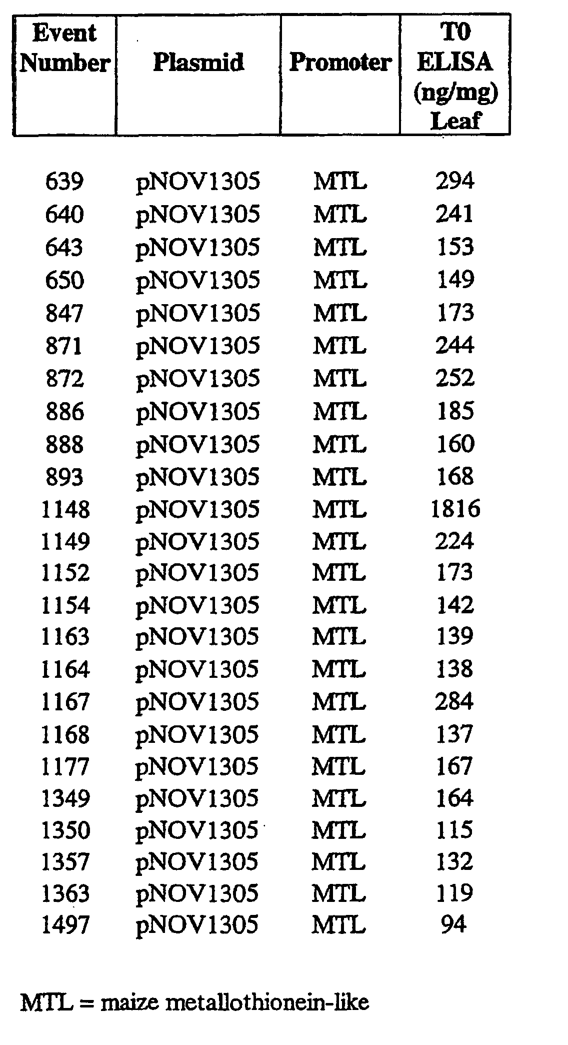

### Example 12. Cabbage Plant Analysis

Cabbage plants transformed with plasmid pZU578 (SEQ ID NO:17) via Agrobacterium-mediated transformation were tested against *Plutella xylostella* (Diamondback moth). Transgenic and control plants were infested with 16 larvae (1-3 instar), 4 on each of 4 leaves transferred with a paint brush from a caged *Plutella* culture (with cabbage plants). Infested plants were transferred to 1x1x1m cages for the duration of the test. Control plants included non-transformed cabbage plants (susceptible control) and non-transformed cabbage plants sprayed with the commercial Bt pesticide Dipel (resistant control). Scoring (after 2 weeks) was: - = no damage (or only tiny holes = resistant); + = large holes on plant (= susc.); ++ many large holes, plant heavily damaged (= susc.). Dipel plants always scored -, susceptible controls scored ++. Insect damage ratings for transgenic and control plants and ELISA data is set forth below.

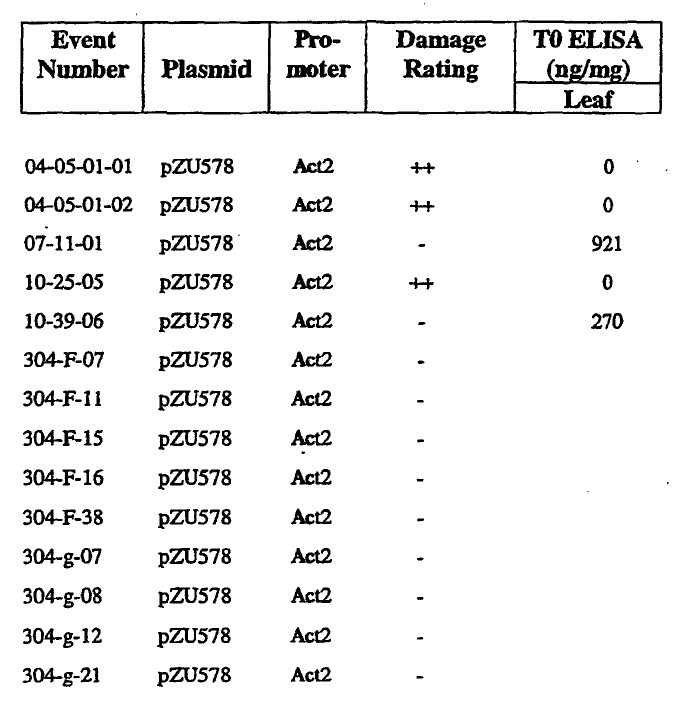

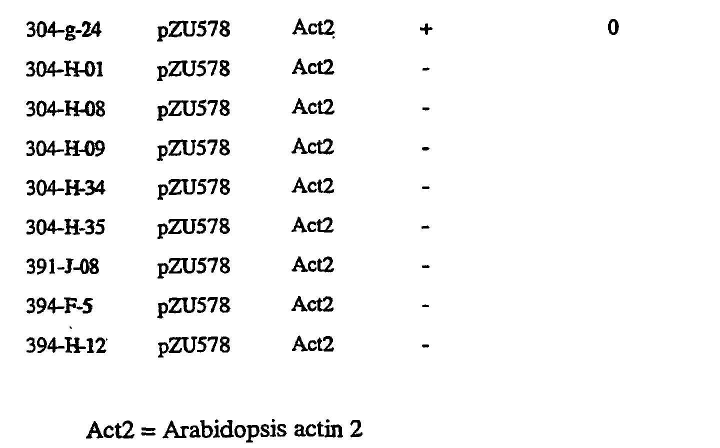

## Claims

1. A synthetic nucleotide sequence that encodes a hybrid *Bacillus thuringiensis* toxin comprising domains I and II from a CrylAb toxin joined in the amino to carboxy direction to domain III from a Cry1C toxin and a C-terminal tail region from a CrylAb toxin, wherein the synthetic nucleotide sequence has been modified to account for the specific GC content preferences of plants.

2. The synthetic nucleotide sequence according to claim 1, wherein said hybrid toxin comprises SEQ ID NO: 6, 8, or 10.

3. The synthetic nucleotide sequence according to claim 1 comprising SEQ ID NO: 5, 7, or 9.

4. An isolated nucleic acid molecule comprising SEQ ID NO: 5, 7, 9, 12, 13, 14, 15, 16, or 17.

5. The nucleic acid molecule of claim 4, comprising SEQ ID NO: 5, 7, or 9.

6. The nucleic acid molecule of claim 4, comprising SEQ ID NO:12, 13, 14, 15, 16, or 17.

7. A chimeric gene comprising a heterologous promoter sequence operatively linked to the synthetic DNA sequence of any one of claims 1 to 3.

8. A recombinant vector comprising the chimeric gene of claim 7.

9. A transgenic plant cell comprising the chimeric gene of claim 7.

10. A transgenic plant comprising the transgenic plant cell of claim 9.

11. The transgenic plant according to claim 10, which is a maize, cotton, rice, or cabbage plant.

12. Transgenic seed from the transgenic plant of claim 10, wherein said transgenic seed comprises the synthetic nucleotide sequence that encodes the *Bacillus thuringiensis* hybrid toxin of claim 1.

13. A method of controlling an insect pest selected from the group consisting of fall armyworm, pink bollworm, tobacco budworm, European corn borer, and diamondback moth, comprising contacting the insect pest with the transgenic plant according to claim 10.

14. The method according to claim 13, wherein said transgenic plant is maize, cotton, rice, or cabbage.

15. A method of producing and insect-resistant plant, comprising introducing a synthetic nucleotide sequence according to claim 1 into said plant, wherein said synthetic nucleotide sequence is expressible in said plant in an effective amount to control insect pests.

16. The method of claim 15, wherein said insect pests are lepidopteran insect pests.

17. The method of claim 16, wherein the insect pests are fall armyworm, pink bollworm, tobacco budworm, European corn borer, and diamondback moth.

18. The method of claim 15, wherein said plant is maize, cotton, rice, or cabbage.

## Patentansprüche

1. Synthetische Nukleotidsequenz, die ein Hybridtoxin aus Bacillus thuringiensis codiert, das Domänen I und II aus einem Cry1Ab-Toxin, verbunden in der Amino-zu-Carboxy-Richtung mit Domäne III aus einem Cry1C-Toxin, und eine C-terminale Schwanzregion aus einem CrylAb-Toxin umfasst, worin die synthetische Nukleotidsequenz modifiziert wurde, um die spezifischen GC-Gehaltvorzüge von Pflanzen zu berücksichtigen.

2. Synthetische Nukleotidsequenz gemäss Anspruch 1, worin das Hybridtoxin SEQ ID NO: 6, 8 oder 10 umfasst.

3. Synthetische Nukleotidsequenz gemäss Anspruch 1, die SEQ ID NO: 5, 7 oder 9 umfasst.

4. Isoliertes Nukleinsäuremolekül, das SEQ ID NO: 5, 7, 9, 12, 13, 14, 15, 16 oder 17 umfasst.

5. Nukleinsäuremolekül gemäss Anspruch 4, das SEQ ID NO: 5, 7 oder 9 umfasst.

6. Nukleinsäuremolekül gemäss Anspruch 4, das SEQ ID NO: 12, 13, 14, 15, 16 oder 17 umfasst.

7. Chimäres Gen, das eine heterologe Promotorsequenz umfasst, die funktionsfähig mit der synthetischen DNA-Sequenz gemäss einem der Ansprüche 1 bis 3 verbunden ist.

8. Rekombinanter Vektor, der das chimäre Gen gemäss Anspruch 7 umfasst.

9. Transgene Pflanzenzelle, die das chimäre Gen gemäss Anspruch 7 umfasst.

10. Transgene Pflanze, die die transgene Pflanzenzelle gemäss Anspruch 9 umfasst.

11. Transgene Pflanze gemäss Anspruch 10, die eine Mais-, Baumwoll-, Reis- oder Kohlpflanze ist.

12. Transgenes Saatgut aus der transgenen Pflanze gemäss Anspruch 10, worin das transgene Saatgut die synthetische Nukleotidsequenz umfasst, die das Hybridtoxin aus Bacillus thuringiensis gemäss Anspruch 1 codiert.

13. Verfahren zur Bekämpfung eines Insektenschädlings, der aus der Gruppe ausgewählt ist, die aus Spodoptera frugiperda, Roter Baumwollkapselwurm, Tabakknospenwurm, Maiszünsler und Kohlmotte besteht, umfassend das In-Kontakt-Bringen des Insektenschädlings mit der transgenen Pflanze gemäss Anspruch 10.

14. Verfahren gemäss Anspruch 13, worin die transgene Pflanze Mais, Baumwolle, Reis oder Kohl ist.

15. Verfahren zur Herstellung einer insektenresistenten Pflanze, umfassend das Einführen einer synthetischen Nukleotidsequenz gemäss Anspruch 1 in die Pflanze, worin die synthetische Nukleotidsequenz in der Pflanze in einer effektiven Menge zur Bekämpfung von Insektenschädlingen exprimierbar ist.

16. Verfahren gemäss Anspruch 15, worin die Insektenschädlinge Lepidoptera-Insektenschädlinge sind.

17. Verfahren gemäss Anspruch 16, worin die Insektenschädlinge Spodoptera frugiperda, Roter Baumwollkapselwurm, Tabakknospenwurm, Maiszünsler und Kohlmotte sind.

18. Verfahren gemäss Anspruch 15, worin die Pflanze Mais, Baumwolle, Reis oder Kohl ist.

## Revendications

1. Séquence nucléotidique synthétique qui code une toxine hybride de *Bacillus thuringiensis* comprenant les domaines I et II d'une toxine Cry1Ab reliés dans le sens amino-carboxy au domaine III d'une toxine Cry1C et une zone de queue C-terminale d'une toxine Cry1Ab, dans laquelle la séquence nucléotidique synthétique a été modifiée pour représenter les préférences de végétaux en matière de rapport GC spécifique.

2. Séquence nucléotidique synthétique selon la revendication 1, dans laquelle ladite toxine hybride comporte la séquence SEQ ID n° 6, 8 ou 10.

3. Séquence nucléotidique synthétique selon la revendication 1 comportant la séquence SEQ ID n° 5, 7 ou 9.

4. Molécule d'acide nucléique isolée comportant la séquence SEQ ID n° 5, 7, 9, 12, 13, 14, 15, 16 ou 17.

5. Molécule d'acide nucléique selon la revendication 4 comportant la séquence SEQ ID n° 5, 7 ou 9.

6. Molécule d'acide nucléique selon la revendication 4 comportant la séquence SEQ ID n° 12, 13, 14, 15, 16 ou 17.

7. Gène chimère comprenant une séquence de promoteur hétérologue liée de façon opérationnelle à la séquence d'ADN synthétique selon l'une quelconque des revendications 1 à 3.

8. Vecteur recombiné comprenant le gène chimère selon la revendication 7.

9. Cellule végétale transgénique comprenant le gène chimère selon la revendication 7.

10. Plante transgénique comprenant la cellule végétale transgénique selon la revendication 9.

11. Plante transgénique selon la revendication 10, qui est le mais, le coton, le riz ou le chou.

12. Graine transgénique issue de la plante transgénique selon la revendication 10, dans laquelle ladite graine transgénique comprend la séquence nucléotidique synthétique qui code la toxine hybride de *Bacillus thuringiensis* selon la revendication 1.

13. Procédé de lutte contre un insecte nuisible choisi dans le groupe constitué par le légionnaire d'automne, le ver rose du cotonnier, la noctuelle verdoyante, la pyrale du mais et la fausse-teigne des crucifères, comprenant l'étape consistant à mettre en contact l'insecte nuisible avec la plante transgénique selon la revendication 10.

14. Procédé selon la revendication 13, dans lequel ladite plante transgénique est le mais, le coton, le riz ou le chou.

15. Procédé de production d'une plante résistante aux insectes, comprenant l'étape consistant à introduire une séquence nucléotidique synthétique selon la revendication 1 dans ladite plante, dans lequel ladite séquence nucléotidique synthétique peut être exprimée dans ladite plante en une quantité efficace pour lutter contre des insectes nuisibles.

16. Procédé selon la revendication 15, dans lequel lesdits insectes nuisibles sont des Lépidoptères.

17. Procédé selon la revendication 16, dans lequel les insectes nuisibles sont le légionnaire d'automne, le ver rose du cotonnier, la noctuelle verdoyante, la pyrale du mais et la fausse-teigne des crucifères.

18. Procédé selon la revendication 15, dans lequel ladite plante est le mais, le coton, le riz ou le chou.


### SEQUENCE LISTING

<110> Syngenta Participations AG
<120> Novel insecticidal toxins derived from *Bacillus thuringiensis* insecticidal crystal proteins
<130> Case S-31282A
<140>
   <141>
<150> US 60/227956
   <151> 2000-08-25
<160> 17
<170> PatentIn Ver. 2.1
<210> 1
   <211> 3579
   <212> DNA
   <213> Artificial Sequences
<220>
   <223> Description of Artificial Sequence: H04 with Cry1C tail
<220>
   <221> CDS
   <222> (1)..(3579)
   <223> H04 with Cry1C tail
<300>
   <303> Appl. Environ. Microbiol.
   <304> 62
   <305> 5
   <306> 1537-1543
   <307> 1996
<300>
   <310> 5,736,131
<400> 1
   atg gat aac aat ccg aac atc aat gaa tgc att cct tat aat tgt tta     48
   Met Asp Asn Asn Pro Asn Ile Asn Glu Cys Ile Pro Tyr Asn Cys Leu
     1               5                  10                  15
   agt aac cct gaa gta gaa gta tta ggt gga gaa aga ata gaa act ggt     96
   Ser Asn Pro Glu Val Glu Val Leu Gly Gly Glu Arg Ile Glu Thr Gly
                20                  25                  30
   tac acc cca atc gat att tcc ttg tcg cta acg caa ttt ctt ttg agt    144
   Tyr Thr Pro Ile Asp Ile Ser Leu Ser Leu Thr Gln Phe Leu Leu Ser
            35                  40                  45
   gaa ttt gtt ccc ggt gct gga ttt gtg tta gga cta gtt gat ata ata    192
   Glu Phe Val Pro Gly Ala Gly Phe Val Leu Gly Leu Val Asp Ile Ile
        50                  55                  60
   tgg gga att ttt ggt ccc tct caa tgg gac gca ttt ctt gta caa att    240
   Trp Gly Ile Phe Gly Pro Ser Gln Trp Asp Ala Phe Leu Val Gln Ile
    65                  70                  75                  80
   gaa cag tta att aac caa aga ata gaa gaa ttc gct agg aac caa gcc    288
   Glu Gln Leu Ile Asn Gln Arg Ile Glu Glu Phe Ala Arg Asn Gln Ala
                    85                  90                  95
   att tct aga tta gaa gga cta agc aat ctt tat caa att tac gca gaa    336
   Ile Ser Arg Leu Glu Gly Leu Ser Asn Leu Tyr Gln Ile Tyr Ala Glu
               100                 105                 110
   tct ttt aga gag tgg gaa gca gat cct act aat cca gca tta aga gaa    384
   Ser Phe Arg Glu Trp Glu Ala Asp Pro Thr Asn Pro Ala Leu Arg Glu
           115                 120                 125
   gag atg cgt att caa ttc aat gac atg aac agt gcc ctt aca acc gct    432
   Glu Met Arg Ile Gln Phe Asn Asp Met Asn Ser Ala Leu Thr Thr Ala
       130                 135                 140
   att cct ctt ttt gca gtt caa aat tat caa gtt cct ctt tta tca gta    480
   Ile Pro Leu Phe Ala Val Gln Asn Tyr Gln Val Pro Leu Leu Ser Val
   145                 150                 155                 160
   tat gtt caa gct gca aat tta cat tta tca gtt ttg aga gat gtt tca    528
   Tyr Val Gln Ala Ala Asn Leu His Leu Ser Val Leu Arg Asp Val Ser
                   165                 170                 175
   gtg ttt gga caa agg tgg gga ttt gat gcc gcg act atc aat agt cgt    576
   Val Phe Gly Gln Arg Trp Gly Phe Asp Ala Ala Thr Ile Asn Ser Arg
               180                 185                 190
   tat aat gat tta act agg ctt att ggc aac tat aca gat cat gct gta    624
   Tyr Asn Asp Leu Thr Arg Leu Ile Gly Asn Tyr Thr Asp His Ala Val
           195                 200                 205
   cgc tgg tac aat acg gga tta gag cgt gta tgg gga ccg gat tct aga    672
   Arg Trp Tyr Asn Thr Gly Leu Glu Arg Val Trp Gly Pro Asp Ser Arg
       210                 215                 220
   gat tgg ata aga tat aat caa ttt aga aga gaa tta aca cta act gta    720
   Asp Trp Ile Arg Tyr Asn Gln Phe Arg Arg Glu Leu Thr Leu Thr Val
   225                 230                 235                 240
   tta gat atc gtt tct cta ttt ccg aac tat gat agt aga acg tat cca    768
   Leu Asp Ile Val Ser Leu Phe Pro Asn Tyr Asp Ser Arg Thr Tyr Pro
                   245                 250                 255
   att cga aca gtt tcc caa tta aca aga gaa att tat aca aac cca gta    816
   Ile Arg Thr Val Ser Gln Leu Thr Arg Glu Ile Tyr Thr Asn Pro Val
               260                 265                 270
   tta gaa aat ttt gat ggt agt ttt cga ggc tcg gct cag ggc ata gaa    864
   Leu Glu Asn Phe Asp Gly Ser Phe Arg Gly Ser Ala Gln Gly Ile Glu
           275                 280                 285
   gga agt att agg agt cca cat ttg atg gat ata ctt aac agt ata acc    912
   Gly Ser Ile Arg Ser Pro His Leu Met Asp Ile Leu Asn Ser Ile Thr
       290                 295                 300
   atc tat acg gat gct cat aga gga gaa tat tat tgg tca ggg cat caa     960
   Ile Tyr Thr Asp Ala His Arg Gly Glu Tyr Tyr Trp Ser Gly His Gln
   305                 310                 315                 320
   ata atg gct tct cct gta ggg ttt tcg ggg cca gaa ttc act ttt ccg    1008
   Ile Met Ala Ser Pro Val Gly Phe Ser Gly Pro Glu Phe Thr Phe Pro
                   325                 330                 335
   cta tat gga act atg gga aat gca gct cca caa caa cgt att gtt gct    1056
   Leu Tyr Gly Thr Met Gly Asn Ala Ala Pro Gln Gln Arg Ile Val Ala
               340                 345                 350
   caa cta ggt cag ggc gtg tat aga aca tta tcg tcc act tta tat aga    1104
   Gln Leu Gly Gln Gly Val Tyr Arg Thr Leu Ser Ser Thr Leu Tyr Arg
           355                 360                 365
   aga cct ttt aat ata ggg ata aat aat caa caa cta tct gtt ctt gac    1152
   Arg Pro Phe Asn Ile Gly Ile Asn Asn Gln Gln Leu Ser Val Leu Asp
       370                 375                 380
   ggg aca gaa ttt gct tat gga acc tcc tca aat ttg cca tcc gct gta    1200
   Gly Thr Glu Phe Ala Tyr Gly Thr Ser Ser Asn Leu Pro Ser Ala Val
   385                 390                 395                 400
   tac aga aaa agc gga acg gta gat tcg ctg gat gaa ata ccg cca cag    1248
   Tyr Arg Lys Ser Gly Thr Val Asp Ser Leu Asp Glu Ile Pro Pro Gln
                   405                 410                 415
   aat aac aac gtg cca cct agg caa gga ttt agt cat cga tta agc cat    1296
   Asn Asn Asn Val Pro Pro Arg Gln Gly Phe Ser His Arg Leu Ser His
               420                 425                 430
   gtt tca atg ttt cgt tca ggc ttt agt aat agt agt gta agt ata ata    1344
   Val Ser Met Phe Arg Ser Gly Phe Ser Asn Ser Ser Val Ser Ile Ile
           435                 440                 445
   aga gct cct atg ttc tct tgg ata cat cgt agt gca act ctt aca aat    1392
   Arg Ala Pro Met Phe Ser Trp Ile His Arg Ser Ala Thr Leu Thr Asn
       450                 455                 460
   aca att gat cca gag aga att aat caa ata cct tta gtg aaa gga ttt    1440
   Thr Ile Asp Pro Glu Arg Ile Asn Gln Ile Pro Leu Val Lys Gly Phe
   465                 470                 475                 480
   aga gtt tgg ggg ggc acc tct gtc att aca gga cca gga ttt aca gga    1488
   Arg Val Trp Gly Gly Thr Ser Val Ile Thr Gly Pro Gly Phe Thr Gly
                   485                 490                 495
   ggg gat atc ctt cga aga aat acc ttt ggt gat ttt gta tct cta caa    1536
   Gly Asp Ile Leu Arg Arg Asn Thr Phe Gly Asp Phe Val Ser Leu Gln
               500                 505                 510
   gtc aat att aat tca cca att acc caa aga tac cgt tta aga ttt cgt    1584
   Val Asn Ile Asn Ser Pro Ile Thr Gln Arg Tyr Arg Leu Arg Phe Arg
           515                 520                 525
   tac gct tcc agt agg gat gca cga gtt ata gta tta aca gga gcg gca    1632
   Tyr Ala Ser Ser Arg Asp Ala Arg Val Ile Val Leu Thr Gly Ala Ala
       530                 535                 540
   tcc aca gga gtg gga ggc caa gtt agt gta aat atg cct ctt cag aaa    1680
   Ser Thr Gly Val Gly Gly Gln Val Ser Val Asn Met Pro Leu Gln Lys
   545                 550                 555                 560
   act atg gaa ata ggg gag aac tta aca tct aga aca ttt aga tat acc    1728
   Thr Met Glu Ile Gly Glu Asn Leu Thr Ser Arg Thr Phe Arg Tyr Thr
                   565                 570                 575
   gat ttt agt aat cct ttt tca ttt aga gct aat cca gat ata att ggg    1776
   Asp Phe Ser Asn Pro Phe Ser Phe Arg Ala Asn Pro Asp Ile Ile Gly
               580                 585                 590
   ata agt gaa caa cct cta ttt ggt gca ggt tct att agt agc ggt gaa    1824
   Ile Ser Glu Gln Pro Leu Phe Gly Ala Gly Ser Ile Ser Ser Gly Glu
           595                 600                 605
   ctt tat ata gat aaa att gaa att att cta gca gat gca aca ttt gaa    1872
   Leu Tyr Ile Asp Lys Ile Glu Ile Ile Leu Ala Asp Ala Thr Phe Glu
       610                 615                 620
   gca gaa tct gat tta gaa aga gca caa aag gcg gtg aat gcc ctg ttt    1920
   Ala Glu Ser Asp Leu Glu Arg Ala Gln Lys Ala Val Asn Ala Leu Phe
   625                 630                 635                 640
   act tct tcc aat caa atc ggg tta aaa acc gat gtg acg gat tat cat    1968
   Thr Ser Ser Asn Gln Ile Gly Leu Lys Thr Asp Val Thr Asp Tyr His
                   645                 650                 655
   att gat caa gta tcc aat tta gtg gat tgt tta tca gat gaa ttt tgt    2016
   Ile Asp Gln Val Ser Asn Leu Val Asp Cys Leu Ser Asp Glu Phe Cys
               660                 665                 670
   ctg gat gaa aag cga gaa ttg tcc gag aaa gtc aaa cat gcg aag cga    2064
   Leu Asp Glu Lys Arg Glu Leu Ser Glu Lys Val Lys His Ala Lys Arg
           675                 680                 685
   ctc agt gat gag cgg aat tta ctt caa gat cca aac ttc aga ggg atc    2112
   Leu Ser Asp Glu Arg Asn Leu Leu Gln Asp Pro Asn Phe Arg Gly Ile
       690                 695                 700
   aat aga caa cca gac cgt ggc tgg aga gga agt aca gat att acc atc    2160
   Asn Arg Gln Pro Asp Arg Gly Trp Arg Gly Ser Thr Asp Ile Thr Ile
   705                 710                 715                 720
   caa gga gga gat gac gta ttc aaa gag aat tac gtc aca cta ccg ggt    2208
   Gln Gly Gly Asp Asp Val Phe Lys Glu Asn Tyr Val Thr Leu Pro Gly
                   725                 730                 735
   acc gtt gat gag tgc tat cca acg tat tta tat cag aaa ata gat gag    2256
   Thr Val Asp Glu Cys Tyr Pro Thr Tyr Leu Tyr Gln Lys Ile Asp Glu
               740                 745                 750
   tcg aaa tta aaa gct tat acc cgt tat gaa tta aga ggg tat atc gaa    2304
   Ser Lys Leu Lys Ala Tyr Thr Arg Tyr Glu Leu Arg Gly Tyr Ile Glu
           755                 760                 765
   gat agt caa gac tta gaa atc tat ttg atc cgt tac aat gca aaa cac    2352
   Asp Ser Gln Asp Leu Glu Ile Tyr Leu Ile Arg Tyr Asn Ala Lys His
       770                 775                 780
   gaa ata gta aat gtg cca ggc acg ggt tcc tta tgg ccg ctt tca gcc    2400
   Glu Ile Val Asn Val Pro Gly Thr Gly Ser Leu Trp Pro Leu Ser Ala
   785                 790                 795                 800
   caa agt cca atc gga aag tgt gga gaa ccg aat cga tgc gcg cca cac    2448
   Gln Ser Pro Ile Gly Lys Cys Gly Glu Pro Asn Arg Cys Ala Pro His
                   805                 810                 815
   ctt gaa tgg aat cct gat cta gat tgt tcc tgc aga gac ggg gaa aaa    2496
   Leu Glu Trp Asn Pro Asp Leu Asp Cys Ser Cys Arg Asp Gly Glu Lys
               820                 825                 830
   tgt gca cat cat tcc cat cat ttc acc ttg gat att gat gtt gga tgt    2544
   Cys Ala His His Ser His His Phe Thr Leu Asp Ile Asp Val Gly Cys
           835                 840                 845
   aca gac tta aat gag gac tta ggt gta tgg gtg ata ttc aag att aag    2592
   Thr Asp Leu Asn Glu Asp Leu Gly Val Trp Val Ile Phe Lys Ile Lys
       850                 855                 860
   acg caa gat ggc cat gca aga cta ggg aat cta gag ttt ctc gaa gag    2640
   Thr Gln Asp Gly His Ala Arg Leu Gly Asn Leu Glu Phe Leu Glu Glu
   865                 870                 875                 880
   aaa cca tta tta ggg gaa gca cta gct cgt gtg aaa aga gcg gag aag    2688
   Lys Pro Leu Leu Gly Glu Ala Leu Ala Arg Val Lys Arg Ala Glu Lys
                   885                 890                 895
   aag tgg aga gac aaa cga gag aaa ctg cag ttg gaa aca aat att gtt    2736
   Lys Trp Arg Asp Lys Arg Glu Lys Leu Gln Leu Glu Thr Asn Ile Val
               900                 905                 910
   tat aaa gag gca aaa gaa tct gta gat gct tta ttt gta aac tct caa    2784
   Tyr Lys Glu Ala Lys Glu Ser Val Asp Ala Leu Phe Val Asn Ser Gln
           915                 920                 925
   tat gat aga tta caa gtg gat acg aac atc gcg atg att cat gcg gca    2832
   Tyr Asp Arg Leu Gln Val Asp Thr Asn Ile Ala Met Ile His Ala Ala
       930                 935                 940
   gat aaa cgc gtt cat aga atc cgg gaa gcg tat ctg cca gag ttg tct    2880
   Asp Lys Arg Val His Arg Ile Arg Glu Ala Tyr Leu Pro Glu Leu Ser
   945                 950                 955                 960
   gtg att cca ggt gtc aat gcg gcc att ttc gaa gaa tta gag gga cgt    2928
   Val Ile Pro Gly Val Asn Ala Ala Ile Phe Glu Glu Leu Glu Gly Arg
                   965                 970                 975
   att ttt aca gcg tat tcc tta tat gat gcg aga aat gtc att aaa aat    2976
   Ile Phe Thr Ala Tyr Ser Leu Tyr Asp Ala Arg Asn Val Ile Lys Asn
               980                 985                 990
   ggc gat ttc aat aat ggc tta tta tgc tgg aac gtg aaa ggt cat gta    3024
   Gly Asp Phe Asn Asn Gly Leu Leu Cys Trp Asn Val Lys Gly His Val
           995                 1000                1005
   gat gta gaa gag caa aac aac cac cgt tcg gtc ctt gtt atc cca gaa    3072
   Asp Val Glu Glu Gln Asn Asn His Arg Ser Val Leu Val Ile Pro Glu
       1010                1015                1020
   tgg gag gca gaa gtg tca caa gag gtt cgt gtc tgt cca ggt cgt ggc    3120
   Trp Glu Ala Glu Val Ser Gln Glu Val Arg Val Cys Pro Gly Arg Gly
   1025                1030                1035                1040
   tat atc ctt cgt gtc aca gca tat aaa gag gga tat gga gag ggc tgc   3168
   Tyr Ile Leu Arg Val Thr Ala Tyr Lys Glu Gly Tyr Gly Glu Gly Cys
                   1045                1050                1055
   gta acg atc cat gag atc gaa gac aat aca gac gaa ctg aaa ttc agc   3216
   Val Thr Ile His Glu Ile Glu Asp Asn Thr Asp Glu Leu Lys Phe Ser
               1060                1065                1070
   aac tgt gta gaa gag gaa gta tat cca aac aac aca gta acg tgt aat   3264
   Asn Cys Val Glu Glu Glu Val Tyr Pro Asn Asn Thr Val Thr Cys Asn
           1075                1080                1085
   aat tat act ggg act caa gaa gaa tat gag ggt acg tac act tct cgt   3312
   Asn Tyr Thr Gly Thr Gln Glu Glu Tyr Glu Gly Thr Tyr Thr Ser Arg
       1090                1095                1100
   aat caa gga tat gac gaa gcc tat ggt aat aac cct tcc gta cca gct   3360
   Asn Gln Gly Tyr Asp Glu Ala Tyr Gly Asn Asn Pro Ser Val Pro Ala
   1105                1110                1115                1120
   gat tac gct tca gtc tat gaa gaa aaa tcg tat aca gat gga cga aga   3408
   Asp Tyr Ala Ser Val Tyr Glu Glu Lys Ser Tyr Thr Asp Gly Arg Arg
                   1125                1130                1135
   gag aat cct tgt gaa tct aac aga ggc tat ggg gat tac aca cca cta   3456
   Glu Asn Pro Cys Glu Ser Asn Arg Gly Tyr Gly Asp Tyr Thr Pro Leu
               1140                1145                1150
   ccg gct ggt tat gta aca aag gat tta gag tac ttc cca gag acc gat   3504
   Pro Ala Gly Tyr Val Thr Lys Asp Leu Glu Tyr Phe Pro Glu Thr Asp
           1155                1160                1165
   aag gta tgg att gag atc gga gaa aca gaa gga aca ttc atc gtg gat   3552
   Lys Val Trp Ile Glu Ile Gly Glu Thr Glu Gly Thr Phe Ile Val Asp
       1170                1175                1180
   agc gtg gaa tta ctc ctt atg gag gaa                               3579
   Ser Val Glu Leu Leu Leu Met Glu Glu
   1185                1190
<210> 2
   <211> 1193
   <212> PRT
   <213> Artificial Sequence
   <223> Description of Artificial Sequence: H04 with Cry1C tail
<400> 2
   Met Asp Asn Asn Pro Asn Ile Asn Glu Cys Ile Pro Tyr Asn Cys Leu
   1               5                   10                  15
   Ser Asn Pro Glu Val Glu Val Leu Gly Gly Glu Arg Ile Glu Thr Gly
               20                  25                  30
   Tyr Thr Pro Ile Asp Ile Ser Leu Ser Leu Thr Gln Phe Leu Leu Ser
           35                  40                  45
   Glu Phe Val Pro Gly Ala Gly Phe Val Leu Gly Leu Val Asp Ile Ile
       50                  55                  60
   Trp Gly Ile Phe Gly Pro Ser Gln Trp Asp Ala Phe Leu Val Gln Ile
    65                  70                  75                  80
   Glu Gln Leu Ile Asn Gln Arg Ile Glu Glu Phe Ala Arg Asn Gln Ala
                   85                  90                  95
   Ile Ser Arg Leu Glu Gly Leu Ser Asn Leu Tyr Gln Ile Tyr Ala Glu
               100                 105                 110
   Ser Phe Arg Glu Trp Glu Ala Asp Pro Thr Asn Pro Ala Leu Arg Glu
           115                 120                 125
   Glu Met Arg Ile Gln Phe Asn Asp Met Asn Ser Ala Leu Thr Thr Ala
       130                 135                 140
   Ile Pro Leu Phe Ala Val Gln Asn Tyr Gln Val Pro Leu Leu Ser Val
   145                 150                 155                 160
   Tyr Val Gln Ala Ala Asn Leu His Leu Ser Val Leu Arg Asp Val Ser
                   165                 170                 175
   Val Phe Gly Gln Arg Trp Gly Phe Asp Ala Ala Thr Ile Asn Ser Arg
               180                 185                 190
   Tyr Asn Asp Leu Thr Arg Leu Ile Gly Asn Tyr Thr Asp His Ala Val
           195                 200                 205
   Arg Trp Tyr Asn Thr Gly Leu Glu Arg Val Trp Gly Pro Asp Ser Arg
       210                 215                 220
   Asp Trp Ile Arg Tyr Asn Gln Phe Arg Arg Glu Leu Thr Leu Thr Val
   225                 230                 235                 240
   Leu Asp Ile Val Ser Leu Phe Pro Asn Tyr Asp Ser Arg Thr Tyr Pro
                   245                 250                 255
   Ile Arg Thr Val Ser Gln Leu Thr Arg Glu Ile Tyr Thr Asn Pro Val
               260                 265                 270
   Leu Glu Asn Phe Asp Gly Ser Phe Arg Gly Ser Ala Gln Gly Ile Glu
           275                 280                 285
   Gly Ser Ile Arg Ser Pro His Leu Met Asp Ile Leu Asn Ser Ile Thr
       290                 295                 300
   Ile Tyr Thr Asp Ala His Arg Gly Glu Tyr Tyr Trp Ser Gly His Gln
   305                 310                 315                 320
   Ile Met Ala Ser Pro Val Gly Phe Ser Gly Pro Glu Phe Thr Phe Pro
                   325                 330                 335
   Leu Tyr Gly Thr Met Gly Asn Ala Ala Pro Gln Gln Arg Ile Val Ala
               340                 345                 350
   Gln Leu Gly Gln Gly Val Tyr Arg Thr Leu Ser Ser Thr Leu Tyr Arg
           355                 360                 365
   Arg Pro Phe Asn Ile Gly Ile Asn Asn Gln Gln Leu Ser Val Leu Asp
       370                 375                 380
   Gly Thr Glu Phe Ala Tyr Gly Thr Ser Ser Asn Leu Pro Ser Ala Val
   385 390 395 400
   Tyr Arg Lys Ser Gly Thr Val Asp Ser Leu Asp Glu Ile Pro Pro Gln
   405 410 415
   Asn Asn Asn Val Pro Pro Arg Gln Gly Phe Ser His Arg Leu Ser His
   420 425 430
   Val Ser Met Phe Arg Ser Gly Phe Ser Asn Ser Ser Val Ser Ile Ile
   435 440 445
   Arg Ala Pro Met Phe Ser Trp Ile His Arg Ser Ala Thr Leu Thr Asn
   450 455 460
   Thr Ile Asp Pro Glu Arg Ile Asn Gln Ile Pro Leu Val Lys Gly Phe
   465 470 475 480
   Arg Val Trp Gly Gly Thr Ser Val Ile Thr Gly Pro Gly Phe Thr Gly
   485 490 495
   Gly Asp Ile Leu Arg Arg Asn Thr Phe Gly Asp Phe Val Ser Leu Gln
   500 505 510
   Val Asn Ile Asn Ser Pro Ile Thr Gln Arg Tyr Arg Leu Arg Phe Arg
   515 520 525
   Tyr Ala Ser Ser Arg Asp Ala Arg Val Ile Val Leu Thr Gly Ala Ala
   530 535 540
   Ser Thr Gly Val Gly Gly Gln Val Ser Val Asn Met Pro Leu Gln Lys
   545 550 555 560
   Thr Met Glu Ile Gly Glu Asn Leu Thr Ser Arg Thr Phe Arg Tyr Thr
   565 570 575
   Asp Phe Ser Asn Pro Phe Ser Phe Arg Ala Asn Pro Asp Ile Ile Gly
   580 585 590
   Ile Ser Glu Gln Pro Leu Phe Gly Ala Gly Ser Ile Ser Ser Gly Glu
   595 600 605
   Leu Tyr Ile Asp Lys Ile Glu Ile Ile Leu Ala Asp Ala Thr Phe Glu
   610 615 620
   Ala Glu Ser Asp Leu Glu Arg Ala Gln Lys Ala Val Asn Ala Leu Phe
   625 630 635 640
   Thr Ser Ser Asn Gln Ile Gly Leu Lys Thr Asp Val Thr Asp Tyr His
   645 650 655
   Ile Asp Gln Val Ser Asn Leu Val Asp Cys Leu Ser Asp Glu Phe Cys
   660 665 670
   Leu Asp Glu Lys Arg Glu Leu Ser Glu Lys Val Lys His Ala Lys Arg
   675 680 685
   Leu Ser Asp Glu Arg Asn Leu Leu Gln Asp Pro Asn Phe Arg Gly Ile
   690 695 700
   Asn Arg Gln Pro Asp Arg Gly Trp Arg Gly Ser Thr Asp Ile Thr Ile
   705 710 715 720
   Gln Gly Gly Asp Asp Val Phe Lys Glu Asn Tyr Val Thr Leu Pro Gly
   725 730 735
   Thr Val Asp Glu Cys Tyr Pro Thr Tyr Leu Tyr Gln Lys Ile Asp Glu
   740 745 750
   Ser Lys Leu Lys Ala Tyr Thr Arg Tyr Glu Leu Arg Gly Tyr Ile Glu
   755 760 765
   Asp Ser Gln Asp Leu Glu Ile Tyr Leu Ile Arg Tyr Asn Ala Lys His
   770 775 780
   Glu Ile Val Asn Val Pro Gly Thr Gly Ser Leu Trp Pro Leu Ser Ala
   785 790 795 800
   Gln Ser Pro Ile Gly Lys Cys Gly Glu Pro Asn Arg Cys Ala Pro His
   805 810 815
   Leu Glu Trp Asn Pro Asp Leu Asp Cys Ser Cys Arg Asp Gly Glu Lys
   820 825 830
   Cys Ala His His Ser His His Phe Thr Leu Asp Ile Asp Val Gly Cys
   835 840 845
   Thr Asp Leu Asn Glu Asp Leu Gly Val Trp Val Ile Phe Lys Ile Lys
   850 855 860
   Thr Gln Asp Gly His Ala Arg Leu Gly Asn Leu Glu Phe Leu Glu Glu
   865 870 875 880
   Lys Pro Leu Leu Gly Glu Ala Leu Ala Arg Val Lys Arg Ala Glu Lys
   885 890 895
   Lys Trp Arg Asp Lys Arg Glu Lys Leu Gln Leu Glu Thr Asn Ile Val
   900 905 910
   Tyr Lys Glu Ala Lys Glu Ser Val Asp Ala Leu Phe Val Asn Ser Gln
   915 920 925
   Tyr Asp Arg Leu Gln Val Asp Thr Asn Ile Ala Met Ile His Ala Ala
   930 935 940
   Asp Lys Arg Val His Arg Ile Arg Glu Ala Tyr Leu Pro Glu Leu Ser
   945 950 955 960
   Val Ile Pro Gly Val Asn Ala Ala Ile Phe Glu Glu Leu Glu Gly Arg
   965 970 975
   Ile Phe Thr Ala Tyr Ser Leu Tyr Asp Ala Arg Asn Val Ile Lys Asn
   980 985 990
   Gly Asp Phe Asn Asn Gly Leu Leu Cys Trp Asn Val Lys Gly His Val
   995 1000 1005
   Asp Val Glu Glu Gln Asn Asn His Arg Ser Val Leu Val Ile Pro Glu
   1010 1015 1020
   Trp Glu Ala Glu Val Ser Gln Glu Val Arg Val Cys Pro Gly Arg Gly
   025 1030 1035 1040
   Tyr Ile Leu Arg Val Thr Ala Tyr Lys Glu Gly Tyr Gly Glu Gly Cys
   1045 1050 1055
   Val Thr Ile His Glu Ile Glu Asp Asn Thr Asp Glu Leu Lys Phe Ser
   1060 1065 1070
   Asn Cys Val Glu Glu Glu Val Tyr Pro Asn Asn Thr Val Thr Cys Asn
   1075 1080 1085
   Asn Tyr Thr Gly Thr Gln Glu Glu Tyr Glu Gly Thr Tyr Thr Ser Arg
   1090 1095 1100
   Asn Gln Gly Tyr Asp Glu Ala Tyr Gly Asn Asn Pro Ser Val Pro Ala
   105 1110 1115 1120
   Asp Tyr Ala Ser Val Tyr Glu Glu Lys Ser Tyr Thr Asp Gly Arg Arg
   1125 1130 1135
   Glu Asn Pro Cys Glu Ser Asn Arg Gly Tyr Gly Asp Tyr Thr Pro Leu
   1140 1145 1150
   Pro Ala Gly Tyr Val Thr Lys Asp Leu Glu Tyr Phe Pro Glu Thr Asp
   1155 1160 1165
   Lys Val Trp Ile Glu Ile Gly Glu Thr Glu Gly Thr Phe Ile Val Asp
   1170 1175 1180
   Ser Val Glu Leu Leu Leu Met Glu Glu
   185 1190
<210> 3
   <211> 1896
   <212> DNA
   <213> Artificial Sequence
<220>
   <223> Description of Artificial Sequence: synthetic gene encoding the toxin portion of H04 without a tail
<220>
   <221> CDS
   <222> (1)..(1896)
   <223> H04 toxin portion without a tail
<400> 3
   atg gac aac aac ccc aac atc aac gag tgc atc ccc tac aac tgc ctg     48
   Met Asp Asn Asn Pro Asn Ile Asn Glu Cys Ile Pro Tyr Asn Cys Leu
    1               5                  10                  15
   agc aac ccc gag gtg gag gtg ctg ggc ggc gag cgc atc gag acc ggc     96
   Ser Asn Pro Glu Val Glu Val Leu Gly Gly Glu Arg Ile Glu Thr Gly
               20                  25                  30
   tac acc ccc atc gac atc agc ctg agc ctg acc cag ttc ctg ctg agc    144
   Tyr Thr Pro Ile Asp Ile Ser Leu Ser Leu Thr Gln Phe Leu Leu Ser
           35                  40                  45
   gag ttc gtg ccc ggc gcc ggc ttc gtg ctg ggc ctg gtg gac atc atc    192
   Glu Phe Val Pro Gly Ala Gly Phe Val Leu Gly Leu Val Asp Ile Ile
        50                  55                  60
   tgg ggc atc ttc ggc ccc agc cag tgg gac gcc ttc ctg gtg cag atc     240
   Trp Gly Ile Phe Gly Pro Ser Gln Trp Asp Ala Phe Leu Val Gln Ile
    65                  70                  75                  80
   gag cag ttg ata aac caa cgc ata gag gaa ttc gcc cgc aac cag gcc     288
   Glu Gln Leu Ile Asn Gln Arg Ile Glu Glu Phe Ala Arg Asn Gln Ala
                    85                  90                  95
   atc agc cgc ctg gag ggc ctg agc aac ctg tac caa atc tac gcc gag     336
   Ile Ser Arg Leu Glu Gly Leu Ser Asn Leu Tyr Gln Ile Tyr Ala Glu
               100                 105                 110
   agc ttc cgc gag tgg gag gcc gac ccc acc aac ccc gcc ctg cgc gag     384
   Ser Phe Arg Glu Trp Glu Ala Asp Pro Thr Asn Pro Ala Leu Arg Glu
           115                 120                 125
   gag atg cgc atc cag ttc aac gac atg aac agc gcc ctg acc acc gcc     432
   Glu Met Arg Ile Gln Phe Asn Asp Met Asn Ser Ala Leu Thr Thr Ala
       130                 135                 140
   atc ccc ctg ttc gcc gtg cag aac tac cag gtg ccc ctg ctg agc gtg     480
   Ile Pro Leu Phe Ala Val Gln Asn Tyr Gln Val Pro Leu Leu Ser Val
   145                 150                 155                 160
   tac gtg cag gcc gcc aac ctg cac ctg agc gtg ctg cgc gac gtc agc     528
   Tyr Val Gln Ala Ala Asn Leu His Leu Ser Val Leu Arg Asp Val Ser
                   165                 170                 175
   gtg ttc ggc cag cgc tgg ggc ttc gac gcc gcc acc atc aac agc cgc     576
   Val Phe Gly Gln Arg Trp Gly Phe Asp Ala Ala Thr Ile Asn Ser Arg
               180                 185                 190
   tac aac gac ctg acc cgc ctg atc ggc aac tac acc gac cac gcc gtg     624
   Tyr Asn Asp Leu Thr Arg Leu Ile Gly Asn Tyr Thr Asp His Ala Val
           195                 200                 205
   cgc tgg tac aac acc ggc ctg gag cgc gtg tgg ggt ccc gac agc cgc     672
   Arg Trp Tyr Asn Thr Gly Leu Glu Arg Val Trp Gly Pro Asp Ser Arg
       210                 215                 220
   gac tgg atc agg tac aac cag ttc cgc cgc gag ctg acc ctg acc gtg     720
   Asp Trp Ile Arg Tyr Asn Gln Phe Arg Arg Glu Leu Thr Leu Thr Val
   225                 230                 235                 240
   ctg gac atc gtg agc ctg ttc ccc aac tac gac agc cgc acc tac ccc     768
   Leu Asp Ile Val Ser Leu Phe Pro Asn Tyr Asp Ser Arg Thr Tyr Pro
                   245                 250                 255
   atc cgc acc gtg agc cag ctg acc cgc gag att tac acc aac ccc gtg     816
   Ile Arg Thr Val Ser Gln Leu Thr Arg Glu Ile Tyr Thr Asn Pro Val
               260                 265                 270
   ctg gag aac ttc gac ggc agc ttc cgc ggc agc gcc cag ggc atc gag     864
   Leu Glu Asn Phe Asp Gly Ser Phe Arg Gly Ser Ala Gln Gly Ile Glu
           275                 280                 285
   ggc agc atc cgc agc ccc cac ctg atg gac atc ctg aac agc atc acc     912
   Gly Ser Ile Arg Ser Pro His Leu Met Asp Ile Leu Asn Ser Ile Thr
   290                 295                 300
   atc tac acc gac gcc cac cgc ggc gag tac tac tgg agc ggc cac cag    960
   Ile Tyr Thr Asp Ala His Arg Gly Glu Tyr Tyr Trp Ser Gly His Gln
   305                 310                 315                 320
   atc atg gcc agc ccc gtc ggc ttc agc ggc ccc gag ttc acc ttc ccc   1008
   Ile Met Ala Ser Pro Val Gly Phe Ser Gly Pro Glu Phe Thr Phe Pro
                   325                 330                 335
   ctg tac ggc acc atg ggc aac gct gca cct cag cag cgc atc gtg gca   1056
   Leu Tyr Gly Thr Met Gly Asn Ala Ala Pro Gln Gln Arg Ile Val Ala
               340                 345                 350
   cag ctg ggc cag gga gtg tac cgc acc ctg agc agc acc ctg tac cgt   1104
   Gln Leu Gly Gln Gly Val Tyr Arg Thr Leu Ser Ser Thr Leu Tyr Arg
           355                 360                 365
   cga cct ttc aac atc ggc atc aac aac cag cag ctg agc gtg ctg gac   1152
   Arg Pro Phe Asn Ile Gly Ile Asn Asn Gln Gln Leu Ser Val Leu Asp
       370                 375                 380
   ggc acc gag ttc gcc tac ggc acc agc agc aac ctg ccc agc gcc gtg   1200
   Gly Thr Glu Phe Ala Tyr Gly Thr Ser Ser Asn Leu Pro Ser Ala Val
   385                 390                 395                 400
   tac cgc aag agc ggc acc gtg gac agc ctg gac gag atc ccc cct cag   1248
   Tyr Arg Lys Ser Gly Thr Val Asp Ser Leu Asp Glu Ile Pro Pro Gln
                   405                 410                 415
   aac aac aac gtg cca cct cga cag ggc ttc agc cac cgt ctg agc cac   1296
   Asn Asn Asn Val Pro Pro Arg Gln Gly Phe Ser His Arg Leu Ser His
               420                 425                 430
   gtg agc atg ttc cgc agt ggc ttc agc aac agc agc gtg agc atc atc   1344
   Val Ser Met Phe Arg Ser Gly Phe Ser Asn Ser Ser Val Ser Ile Ile
           435                 440                 445
   cgt gca ccc atg ttc agc tgg att cac cgc agc gcc acc ctg acc aac   1392
   Arg Ala Pro Met Phe Ser Trp Ile His Arg Ser Ala Thr Leu Thr Asn
       450                 455                 460
   acc atc gac ccc gag cgc atc aac cag atc ccc ctg gtg aag ggc ttc   1440
   Thr Ile Asp Pro Glu Arg Ile Asn Gln Ile Pro Leu Val Lys Gly Phe
   465                 470                 475                 480
   cgg gtg tgg ggc ggc acc agc gtg atc acc ggc ccc ggc ttc acc gga   1488
   Arg Val Trp Gly Gly Thr Ser Val Ile Thr Gly Pro Gly Phe Thr Gly
                   485                 490                 495
   ggc gac atc ctg cgc aga aac acc ttc ggc gac ttc gtg agc ctg cag   1536
   Gly Asp Ile Leu Arg Arg Asn Thr Phe Gly Asp Phe Val Ser Leu Gln
               500                 505                 510
   gtg aac atc aac agc ccc atc acc cag cgt tac cgc ctg cgc ttc cgc   1584
   Val Asn Ile Asn Ser Pro Ile Thr Gln Arg Tyr Arg Leu Arg Phe Arg
           515                 520                 525
   tac gcc agc agc cgc gac gcc cgt gtg atc gtg ctg act ggc gcc gct   1632
   Tyr Ala Ser Ser Arg Asp Ala Arg Val Ile Val Leu Thr Gly Ala Ala
       530                 535                 540
   agc acc ggt gtg ggc ggt cag gtg agc gtg aac atg ccc ctg cag aag     1680
   Ser Thr Gly Val Gly Gly Gln Val Ser Val Asn Met Pro Leu Gln Lys
   545                 550                 555                 560
   act atg gag atc ggc gag aac ctg act agt cgc acc ttc cgc tac acc     1728
   Thr Met Glu Ile Gly Glu Asn Leu Thr Ser Arg Thr Phe Arg Tyr Thr
                   565                 570                 575
   gac ttc agc aac ccc ttc agc ttc cgc gcc aac ccc gac atc atc ggc     1776
   Asp Phe Ser Asn Pro Phe Ser Phe Arg Ala Asn Pro Asp Ile Ile Gly
               580                 585                 590
   atc agc gag cag ccc ctg ttc ggt gcc ggc agc atc agc agc ggc gag     1824
   Ile Ser Glu Gln Pro Leu Phe Gly Ala Gly Ser Ile Ser Ser Gly Glu
           595                 600                 605
   ctg tac atc gac aag atc gag atc atc ctg gcc gac gcc acc ttc gag     1872
   Leu Tyr Ile Asp Lys Ile Glu Ile Ile Leu Ala Asp Ala Thr Phe Glu
       610                 615                 620
   gcc gag agc gac ctg gag cgc taa                                     1896
   Ala Glu Ser Asp Leu Glu Arg
   625                 630
<210> 4
   <211> 631
   <212> PRT
   <213> Artificial Sequence
   <223> Description of Artificial Sequence: synthetic gene encoding the toxin portion of H04 without a tail
<400> 4
   Met Asp Asn Asn Pro Asn Ile Asn Glu Cys Ile Pro Tyr Asn Cys Leu
   1               5                   10                  15
   Ser Asn Pro Glu Val Glu Val Leu Gly Gly Glu Arg Ile Glu Thr Gly
               20                  25                  30
   Tyr Thr Pro Ile Asp Ile Ser Leu Ser Leu Thr Gln Phe Leu Leu Ser
           35                  40                  45
   Glu Phe Val Pro Gly Ala Gly Phe Val Leu Gly Leu Val Asp Ile Ile
       50                  55                  60
   Trp Gly Ile Phe Gly Pro Ser Gln Trp Asp Ala Phe Leu Val Gln Ile
   65                  70                  75                  80
   Glu Gln Leu Ile Asn Gln Arg Ile Glu Glu Phe Ala Arg Asn Gln Ala
                   85                  90                  95
   Ile Ser Arg Leu Glu Gly Leu Ser Asn Leu Tyr Gln Ile Tyr Ala Glu
               100                 105                 110
   Ser Phe Arg Glu Trp Glu Ala Asp Pro Thr Asn Pro Ala Leu Arg Glu
           115                 120                 125
   Glu Met Arg Ile Gln Phe Asn Asp Met Asn Ser Ala Leu Thr Thr Ala
       130                 135                 140
   Ile Pro Leu Phe Ala Val Gln Asn Tyr Gln Val Pro Leu Leu Ser Val
   145                 150                 155                 160
   Tyr Val Gln Ala Ala Asn Leu His Leu Ser Val Leu Arg Asp Val Ser
                   165                 170                 175
   Val Phe Gly Gln Arg Trp Gly Phe Asp Ala Ala Thr Ile Asn Ser Arg
               180                 185                 190
   Tyr Asn Asp Leu Thr Arg Leu Ile Gly Asn Tyr Thr Asp His Ala Val
           195                 200                 205
   Arg Trp Tyr Asn Thr Gly Leu Glu Arg Val Trp Gly Pro Asp Ser Arg
       210                 215                 220
   Asp Trp Ile Arg Tyr Asn Gln Phe Arg Arg Glu Leu Thr Leu Thr Val
   225                 230                 235                 240
   Leu Asp Ile Val Ser Leu Phe Pro Asn Tyr Asp Ser Arg Thr Tyr Pro
                   245                 250                 255
   Ile Arg Thr Val Ser Gln Leu Thr Arg Glu Ile Tyr Thr Asn Pro Val
               260                 265                 270
   Leu Glu Asn Phe Asp Gly Ser Phe Arg Gly Ser Ala Gln Gly Ile Glu
           275                 280                 285
   Gly Ser Ile Arg Ser Pro His Leu Met Asp Ile Leu Asn Ser Ile Thr
       290                 295                 300
   Ile Tyr Thr Asp Ala His Arg Gly Glu Tyr Tyr Trp Ser Gly His Gln
   305                 310                 315                 320
   Ile Met Ala Ser Pro Val Gly Phe Ser Gly Pro Glu Phe Thr Phe Pro
                   325                 330                 335
   Leu Tyr Gly Thr Met Gly Asn Ala Ala Pro Gln Gln Arg Ile Val Ala
               340                 345                 350
   Gln Leu Gly Gln Gly Val Tyr Arg Thr Leu Ser Ser Thr Leu Tyr Arg
           355                 360                 365
   Arg Pro Phe Asn Ile Gly Ile Asn Asn Gln Gln Leu Ser Val Leu Asp
       370                 375                 380
   Gly Thr Glu Phe Ala Tyr Gly Thr Ser Ser Asn Leu Pro Ser Ala Val
   385                 390                 395                 400
   Tyr Arg Lys Ser Gly Thr Val Asp Ser Leu Asp Glu Ile Pro Pro Gln
                   405                 410                 415
   Asn Asn Asn Val Pro Pro Arg Gln Gly Phe Ser His Arg Leu Ser His
               420                 425                 430
   Val Ser Met Phe Arg Ser Gly Phe Ser Asn Ser Ser Val Ser Ile Ile
           435                 440                 445
   Arg Ala Pro Met Phe Ser Trp Ile His Arg Ser Ala Thr Leu Thr Asn
       450                 455                 460
   Thr Ile Asp Pro Glu Arg Ile Asn Gln Ile Pro Leu Val Lys Gly Phe
   465                 470                 475                 480
   Arg Val Trp Gly Gly Thr Ser Val Ile Thr Gly Pro Gly Phe Thr Gly
                   485                 490                 495
   Gly Asp Ile Leu Arg Arg Asn Thr Phe Gly Asp Phe Val Ser Leu Gln
               500                 505                 510
   Val Asn Ile Asn Ser Pro Ile Thr Gln Arg Tyr Arg Leu Arg Phe Arg
           515                 520                 525
   Tyr Ala Ser Ser Arg Asp Ala Arg Val Ile Val Leu Thr Gly Ala Ala
       530                 535                 540
   Ser Thr Gly Val Gly Gly Gln Val Ser Val Asn Met Pro Leu Gln Lys
   545                 550                 555                 560
   Thr Met Glu Ile Gly Glu Asn Leu Thr Ser Arg Thr Phe Arg Tyr Thr
                   565                 570                 575
   Asp Phe Ser Asn Pro Phe Ser Phe Arg Ala Asn Pro Asp Ile Ile Gly
               580                 585                 590
   Ile Ser Glu Gln Pro Leu Phe Gly Ala Gly Ser Ile Ser Ser Gly Glu
           595                 600                 605
   Leu Tyr Ile Asp Lys Ile Glu Ile Ile Leu Ala Asp Ala Thr Phe Glu
       610                 615                 620
   Ala Glu Ser Asp Leu Glu Arg
   625                 630
<210> 5
   <211> 3582
   <212> DNA
   <213> Artificial Sequence
<220>
   <223> Description of Artificial Sequence: synthetic gene encoding H04 with full-length Cry1Ab tail
<220>
   <221> CDS
   <222> (1)..(3582)
   <223> H04 with full-length Cry1Ab tail
<400> 5
   atg gac aac aac ccc aac atc aac gag tgc atc ccc tac aac tgc ctg      48
   Met Asp Asn Asn Pro Asn Ile Asn Glu Cys Ile Pro Tyr Asn Cys Leu
    1               5                  10                  15
   agc aac ccc gag gtg gag gtg ctg ggc ggc gag cgc atc gag acc ggc      96
   Ser Asn Pro Glu Val Glu Val Leu Gly Gly Glu Arg Ile Glu Thr Gly
               20                  25                  30
   tac acc ccc atc gac atc agc ctg agc ctg acc cag ttc ctg ctg agc     144
   Tyr Thr Pro Ile Asp Ile Ser Leu Ser Leu Thr Gln Phe Leu Leu Ser
           35                  40                  45
   gag ttc gtg ccc ggc gcc ggc ttc gtg ctg ggc ctg gtg gac atc atc     192
   Glu Phe Val Pro Gly Ala Gly Phe Val Leu Gly Leu Val Asp Ile Ile
       50                  55                  60
   tgg ggc atc ttc ggc ccc agc cag tgg gac gcc ttc ctg gtg cag atc     240
   Trp Gly Ile Phe Gly Pro Ser Gln Trp Asp Ala Phe Leu Val Gln Ile
   65                  70                  75                  80
   gag cag ttg ata aac caa cgc ata gag gaa ttc gcc cgc aac cag gcc     288
   Glu Gln Leu Ile Asn Gln Arg Ile Glu Glu Phe Ala Arg Asn Gln Ala
                   85                  90                  95
   atc agc cgc ctg gag ggc ctg agc aac ctg tac caa atc tac gcc gag     336
   Ile Ser Arg Leu Glu Gly Leu Ser Asn Leu Tyr Gln Ile Tyr Ala Glu
               100                 105                 110
   agc ttc cgc gag tgg gag gcc gac ccc acc aac ccc gcc ctg cgc gag     384
   Ser Phe Arg Glu Trp Glu Ala Asp Pro Thr Asn Pro Ala Leu Arg Glu
           115                 120                 125
   gag atg cgc atc cag ttc aac gac atg aac agc gcc ctg acc acc gcc     432
   Glu Met Arg Ile Gln Phe Asn Asp Met Asn Ser Ala Leu Thr Thr Ala
       130                 135                 140
   atc ccc ctg ttc gcc gtg cag aac tac cag gtg ccc ctg ctg agc gtg     480
   Ile Pro Leu Phe Ala Val Gln Asn Tyr Gln Val Pro Leu Leu Ser Val
   145                 150                 155                 160
   tac gtg cag gcc gcc aac ctg cac ctg agc gtg ctg cgc gac gtc agc     528
   Tyr Val Gln Ala Ala Asn Leu His Leu Ser Val Leu Arg Asp Val Ser
                   165                 170                 175
   gtg ttc ggc cag cgc tgg ggc ttc gac gcc gcc acc atc aac agc cgc     576
   Val Phe Gly Gln Arg Trp Gly Phe Asp Ala Ala Thr Ile Asn Ser Arg
               180                 185                 190
   tac aac gac ctg acc cgc ctg atc ggc aac tac acc gac cac gcc gtg     624
   Tyr Asn Asp Leu Thr Arg Leu Ile Gly Asn Tyr Thr Asp His Ala Val
           195                 200                 205
   cgc tgg tac aac acc ggc ctg gag cgc gtg tgg ggt ccc gac agc cgc     672
   Arg Trp Tyr Asn Thr Gly Leu Glu Arg Val Trp Gly Pro Asp Ser Arg
       210                 215                 220
   gac tgg atc agg tac aac cag ttc cgc cgc gag ctg acc ctg acc gtg     720
   Asp Trp Ile Arg Tyr Asn Gln Phe Arg Arg Glu Leu Thr Leu Thr Val
   225                 230                 235                 240
   ctg gac atc gtg agc ctg ttc ccc aac tac gac agc cgc acc tac ccc     768
   Leu Asp Ile Val Ser Leu Phe Pro Asn Tyr Asp Ser Arg Thr Tyr Pro
                   245                 250                 255
   atc cgc acc gtg agc cag ctg acc cgc gag att tac acc aac ccc gtg     816
   Ile Arg Thr Val Ser Gln Leu Thr Arg Glu Ile Tyr Thr Asn Pro Val
               260                 265                 270
   ctg gag aac ttc gac ggc agc ttc cgc ggc agc gcc cag ggc atc gag     864
   Leu Glu Asn Phe Asp Gly Ser Phe Arg Gly Ser Ala Gln Gly Ile Glu
           275                 280                 285
   ggc agc atc cgc agc ccc cac ctg atg gac atc ctg aac agc atc acc     912
   Gly Ser Ile Arg Ser Pro His Leu Met Asp Ile Leu Asn Ser Ile Thr
       290                 295                 300
   atc tac acc gac gcc cac cgc ggc gag tac tac tgg agc ggc cac cag     960
   Ile Tyr Thr Asp Ala His Arg Gly Glu Tyr Tyr Trp Ser Gly His Gln
   305                 310                 315                 320
   atc atg gcc agc ccc gtc ggc ttc agc ggc ccc gag ttc acc ttc ccc     1008
   Ile Met Ala Ser Pro Val Gly Phe Ser Gly Pro Glu Phe Thr Phe Pro
                   325                 330                 335
   ctg tac ggc acc atg ggc aac gct gca cct cag cag cgc atc gtg gca     1056
   Leu Tyr Gly Thr Met Gly Asn Ala Ala Pro Gln Gln Arg Ile Val Ala
               340                 345                 350
   cag ctg ggc cag gga gtg tac cgc acc ctg agc agc acc ctg tac cgt     1104
   Gln Leu Gly Gln Gly Val Tyr Arg Thr Leu Ser Ser Thr Leu Tyr Arg
           355                 360                 365
   cga cct ttc aac atc ggc atc aac aac cag cag ctg agc gtg ctg gac     1152
   Arg Pro Phe Asn Ile Gly Ile Asn Asn Gln Gln Leu Ser Val Leu Asp
       370                 375                 380
   ggc acc gag ttc gcc tac ggc acc agc agc aac ctg ccc agc gcc gtg     1200
   Gly Thr Glu Phe Ala Tyr Gly Thr Ser Ser Asn Leu Pro Ser Ala Val
   385                 390                 395                 400
   tac cgc aag agc ggc acc gtg gac agc ctg gac gag atc ccc cct cag     1248
   Tyr Arg Lys Ser Gly Thr Val Asp Ser Leu Asp Glu Ile Pro Pro Gln
                   405                 410                 415
   aac aac aac gtg cca cct cga cag ggc ttc agc cac cgt ctg agc cac     1296
   Asn Asn Asn Val Pro Pro Arg Gln Gly Phe Ser His Arg Leu Ser His
               420                 425                 430
   gtg agc atg ttc cgc agt ggc ttc agc aac agc agc gtg agc atc atc     1344
   Val Ser Met Phe Arg Ser Gly Phe Ser Asn Ser Ser Val Ser Ile Ile
           435                 440                 445
   cgt gca ccc atg ttc agc tgg att cac cgc agc gcc acc ctg acc aac 1392
   Arg Ala Pro Met Phe Ser Trp Ile His Arg Ser Ala Thr Leu Thr Asn
   450 455 460
   acc atc gac ccc gag cgc atc aac cag atc ccc ctg gtg aag ggc ttc 1440
   Thr Ile Asp Pro Glu Arg Ile Asn Gln Ile Pro Leu Val Lys Gly Phe
   465 470 475 480
   cgg gtg tgg ggc ggc acc agc gtg atc acc ggc ccc ggc ttc acc gga 1488
   Arg Val Trp Gly Gly Thr Ser Val Ile Thr Gly Pro Gly Phe Thr Gly
   485 490 495
   ggc gac atc ctg cgc aga aac acc ttc ggc gac ttc gtg agc ctg cag 1536
   Gly Asp Ile Leu Arg Arg Asn Thr Phe Gly Asp Phe Val Ser Leu Gln
   500 505 510
   gtg aac atc aac agc ccc atc acc cag cgt tac cgc ctg cgc ttc cgc 1584
   Val Asn Ile Asn Ser Pro Ile Thr Gln Arg Tyr Arg Leu Arg Phe Arg
   515 520 525
   tac gcc agc agc cgc gac gcc cgt gtg atc gtg ctg act ggc gcc gct 1632
   Tyr Ala Ser Ser Arg Asp Ala Arg Val Ile Val Leu Thr Gly Ala Ala
   530 535 540
   agc acc ggt gtg ggc ggt cag gtg agc gtg aac atg ccc ctg cag aag 1680
   Ser Thr Gly Val Gly Gly Gln Val Ser Val Asn Met Pro Leu Gln Lys
   545 550 555 560
   act atg gag atc ggc gag aac ctg act agt cgc acc ttc cgc tac acc 1728
   Thr Met Glu Ile Gly Glu Asn Leu Thr Ser Arg Thr Phe Arg Tyr Thr
   565 570 575
   gac ttc agc aac ccc ttc agc ttc cgc gcc aac ccc gac atc atc ggc 1776
   Asp Phe Ser Asn Pro Phe Ser Phe Arg Ala Asn Pro Asp Ile Ile Gly
   580 585 590
   atc agc gag cag ccc ctg ttc ggt gcc ggc agc atc agc agc ggc gag 1824
   Ile Ser Glu Gln Pro Leu Phe Gly Ala Gly Ser Ile Ser Ser Gly Glu
   595 600 605
   ctg tac atc gac aag atc gag atc atc ctg gcc gac gcc acc ttc gag 1872
   Leu Tyr Ile Asp Lys Ile Glu Ile Ile Leu Ala Asp Ala Thr Phe Glu
   610 615 620
   gcc gag agc gac ctg gag cgc gcc cag aag gcc gtg aac gcc ctg ttc 1920
   Ala Glu Ser Asp Leu Glu Arg Ala Gln Lys Ala Val Asn Ala Leu Phe
   625 630 635 640
   acc agc agc aac cag atc ggc ctg aag acc gac gtg acc gac tac cac 1968
   Thr Ser Ser Asn Gln Ile Gly Leu Lys Thr Asp Val Thr Asp Tyr His
   645 650 655
   atc gac cag gtg agc aac ctg gtg gac tgc tta agc gac gag ttc tgc 2016
   Ile Asp Gln Val Ser Asn Leu Val Asp Cys Leu Ser Asp Glu Phe Cys
   660 665 670
   ctg gac gag aag aag gag ctg agc gag aag gtg aag cac gcc aag cgc 2064
   Leu Asp Glu Lys Lys Glu Leu Ser Glu Lys Val Lys His Ala Lys Arg
   675 680 685
   ctg agc gac gag cgc aac ctg ctg cag gac ccc aac ttc cgc ggc atc 2112
   Leu Ser Asp Glu Arg Asn Leu Leu Gln Asp Pro Asn Phe Arg Gly Ile
       690                 695                 700
   aac cgc cag ctg gac cgc ggc tgg cga ggc agc acc gat atc acc atc     2160
   Asn Arg Gln Leu Asp Arg Gly Trp Arg Gly Ser Thr Asp Ile Thr Ile
   705                 710                 715                 720
   cag ggc ggc gac gac gtg ttc aag gag aac tac gtg acc ctg cag ggc     2208
   Gln Gly Gly Asp Asp Val Phe Lys Glu Asn Tyr Val Thr Leu Gln Gly
                   725                 730                 735
   acc ttc gac gag tgc tac ccc acc tac ctg tac cag ccg atc gac gag     2256
   Thr Phe Asp Glu Cys Tyr Pro Thr Tyr Leu Tyr Gln Pro Ile Asp Glu
               740                 745                 750
   agc aag ctg aag gcc tac acc cgc tac cag ctg cgc ggc tac atc gag     2304
   Ser Lys Leu Lys Ala Tyr Thr Arg Tyr Gln Leu Arg Gly Tyr Ile Glu
           755                 760                 765
   gac agc cag gac ctg gaa atc tac ctg atc cgc tac aac gcg aag cac     2352
   Asp Ser Gln Asp Leu Glu Ile Tyr Leu Ile Arg Tyr Asn Ala Lys His
       770                 775                 780
   gag acc gtg aac gtg ccc ggc acc ggc agc ctg tgg ccc ccg agc gcc     2400
   Glu Thr Val Asn Val Pro Gly Thr Gly Ser Leu Trp Pro Pro Ser Ala
   785                 790                 795                 800
   ccc agc ccc atc ggc aag tgc ggg gag ccg aat cga tgc gct ccg cac     2448
   Pro Ser Pro Ile Gly Lys Cys Gly Glu Pro Asn Arg Cys Ala Pro His
                   805                 810                 815
   ctg gag tgg aac ccg gac cta gac tgc agc tgc agg gac ggg gag aag     2496
   Leu Glu Trp Asn Pro Asp Leu Asp Cys Ser Cys Arg Asp Gly Glu Lys
               820                 825                 830
   tgc gcc cac cac agc cac cac ttc agc ctg gac atc gac gtg ggc tgc     2544
   Cys Ala His His Ser His His Phe Ser Leu Asp Ile Asp Val Gly Cys
           835                 840                 845
   acc gac ctg aac gag gac ctg ggc gtg tgg gtg atc ttc aag atc aag     2592
   Thr Asp Leu Asn Glu Asp Leu Gly Val Trp Val Ile Phe Lys Ile Lys
       850                 855                 860
   acc cag gac ggc cac gcc cgc ctg ggc aat cta gag ttc ctg gag gag     2640
   Thr Gln Asp Gly His Ala Arg Leu Gly Asn Leu Glu Phe Leu Glu Glu
   865                 870                 875                 880
   aag ccc ctg gtg ggc gag gcc ctg gcc cgc gtg aag cgt gct gag aag     2688
   Lys Pro Leu Val Gly Glu Ala Leu Ala Arg Val Lys Arg Ala Glu Lys
                   885                 890                 895
   aag tgg cgc gac aag cgc gag aag ctg gag tgg gag acc aac atc gtg     2736
   Lys Trp Arg Asp Lys Arg Glu Lys Leu Glu Trp Glu Thr Asn Ile Val
               900                 905                 910
   tac aag gag gcc aag gag agc gtg gac gcc ctg ttc gtg aac agc cag     2784
   Tyr Lys Glu Ala Lys Glu Ser Val Asp Ala Leu Phe Val Asn Ser Gln
           915                 920                 925
   tac gac cgc ctg cag gcc gac acc aac atc gcc atg atc cac gcc gcc     2832
   Tyr Asp Arg Leu Gln Ala Asp Thr Asn Ile Ala Met Ile His Ala Ala
               930                      935                      940
   gac aag cgc gtg cac agc att cgc gag gcc tac ctg ccc gag ctg agc    2880
   Asp Lys Arg Val His Ser Ile Arg Glu Ala Tyr Leu Pro Glu Leu Ser
   945                     950                     955                     960
   gtg atc ccc ggt gtg aac gcc gcc atc ttc gag gaa ctc gag ggc cgc    2928
   Val Ile Pro Gly Val Asn Ala Ala Ile Phe Glu Glu Leu Glu Gly Arg
                   965                     970                     975
   atc ttc acc gcc ttc agc ctg tac gac gcc cgc aac gtg atc aag aac    2976
   Ile Phe Thr Ala Phe Ser Leu Tyr Asp Ala Arg Asn Val Ile Lys Asn
               980                     985                     990
   ggc gac ttc aac aac ggc ctg agc tgc tgg aac gtg aag ggc cac gtg    3024
   Gly Asp Phe Asn Asn Gly Leu Ser Cys Trp Asn Val Lys Gly His Val
           995                    1000                    1005
   gac gtg gag gag cag aac aac cac cgc agc gtg ctg gtg gtg ccc gag    3072
   Asp Val Glu Glu Gln Asn Asn His Arg Ser Val Leu Val Val Pro Glu
       1010                    1015                    1020
   tgg gag gcc gag gtg agc cag gag gtg cgc gtg tgc ccc ggc cgc ggc    3120
   Trp Glu Ala Glu Val Ser Gln Glu Val Arg Val Cys Pro Gly Arg Gly
   1025                    1030                    1035                    1040
   tac atc ctg cgc gtg acc gcc tac aag gag ggc tac ggc gag ggc tgc    3168
   Tyr Ile Leu Arg Val Thr Ala Tyr Lys Glu Gly Tyr Gly Glu Gly Cys
                   1045                    1050                    1055
   gtg acc atc cac gag atc gag aac aac acc gac gag ctc aag ttc agc    3216
   Val Thr Ile His Glu Ile Glu Asn Asn Thr Asp Glu Leu Lys Phe Ser
               1060                    1065                    1070
   aac tgc gtg gag gag gag gtt tac ccc aac aac acc gtg acc tgc aac    3264
   Asn Cys Val Glu Glu Glu Val Tyr Pro Asn Asn Thr Val Thr Cys Asn
           1075                    1080                    1085
   gac tac acc gcg acc cag gag gag tac gaa ggc acc tac acc tct cgc    3312
   Asp Tyr Thr Ala Thr Gln Glu Glu Tyr Glu Gly Thr Tyr Thr Ser Arg
       1090                    1095                    1100
   aac agg ggt tac gac ggc gcc tac gag tcc aac agc tcc gtg cca gct    3360
   Asn Arg Gly Tyr Asp Gly Ala Tyr Glu Ser Asn Ser Ser Val Pro Ala
   1105                    1110                    1115                    1120
   gac tac gcc agc gcc cac gag gag aaa gcc tac acc gac ggt aga cgc    3408
   Asp Tyr Ala Ser Ala His Glu Glu Lys Ala Tyr Thr Asp Gly Arg Arg
                   1125                    1130                    1135
   gac aac cca tgt gag agc aac aga ggc tac ggc gac tac acc ccc ctg    3456
   Asp Asn Pro Cys Glu Ser Asn Arg Gly Tyr Gly Asp Tyr Thr Pro Leu
               1140                    1145                    1150
   ccc gct gga tac gtg acc aag gag ctg gag tac ttc ccc gag acc gac    3504
   Pro Ala Gly Tyr Val Thr Lys Glu Leu Glu Tyr Phe Pro Glu Thr Asp
           1155                    1160                    1165
   aag gtg tgg atc gag att ggc gag acc gag ggc acc ttc atc gtg gac    3552
   Lys Val Trp Ile Glu Ile Gly Glu Thr Glu Gly Thr Phe Ile Val Asp
       1170                    1175                    1180
   agc gtg gag ctg ctg ctg atg gag gag tag                                3582
   Ser Val Glu Leu Leu Leu Met Glu Glu
   1185                1190
<210> 6
   <211> 1193
   <212> PRT
   <213> Artificial Sequence
   <223> Description of Artificial Sequence: synthetic gene encoding H04 with full-length Cry1Ab tail
<400> 6
   Met Asp Asn Asn Pro Asn Ile Asn Glu Cys Ile Pro Tyr Asn Cys Leu
    1               5                  10                  15
   Ser Asn Pro Glu Val Glu Val Leu Gly Gly Glu Arg Ile Glu Thr Gly
               20                  25                  30
   Tyr Thr Pro Ile Asp Ile Ser Leu Ser Leu Thr Gln Phe Leu Leu Ser
           35                  40                  45
   Glu Phe Val Pro Gly Ala Gly Phe Val Leu Gly Leu Val Asp Ile Ile
       50                  55                  60
   Trp Gly Ile Phe Gly Pro Ser Gln Trp Asp Ala Phe Leu Val Gln Ile
   65                  70                  75                  80
   Glu Gln Leu Ile Asn Gln Arg Ile Glu Glu Phe Ala Arg Asn Gln Ala
                   85                  90                  95
   Ile Ser Arg Leu Glu Gly Leu Ser Asn Leu Tyr Gln Ile Tyr Ala Glu
               100                 105                 110
   Ser Phe Arg Glu Trp Glu Ala Asp Pro Thr Asn Pro Ala Leu Arg Glu
           115                 120                 125
   Glu Met Arg Ile Gln Phe Asn Asp Met Asn Ser Ala Leu Thr Thr Ala
       130                 135                 140
   Ile Pro Leu Phe Ala Val Gln Asn Tyr Gln Val Pro Leu Leu Ser Val
   145                 150                 155                 160
   Tyr Val Gln Ala Ala Asn Leu His Leu Ser Val Leu Arg Asp Val Ser
                   165                 170                 175
   Val Phe Gly Gln Arg Trp Gly Phe Asp Ala Ala Thr Ile Asn Ser Arg
               180                 185                 190
   Tyr Asn Asp Leu Thr Arg Leu Ile Gly Asn Tyr Thr Asp His Ala Val
           195                 200                 205
   Arg Trp Tyr Asn Thr Gly Leu Glu Arg Val Trp Gly Pro Asp Ser Arg
       210                 215                 220
   Asp Trp Ile Arg Tyr Asn Gln Phe Arg Arg Glu Leu Thr Leu Thr Val
   225                 230                 235                 240
   Leu Asp Ile Val Ser Leu Phe Pro Asn Tyr Asp Ser Arg Thr Tyr Pro
                   245                 250                 255
   Ile Arg Thr Val Ser Gln Leu Thr Arg Glu Ile Tyr Thr Asn Pro Val
               260                 265                 270
   Leu Glu Asn Phe Asp Gly Ser Phe Arg Gly Ser Ala Gln Gly Ile Glu
           275                 280                 285
   Gly Ser Ile Arg Ser Pro His Leu Met Asp Ile Leu Asn Ser Ile Thr
       290                 295                 300
   Ile Tyr Thr Asp Ala His Arg Gly Glu Tyr Tyr Trp Ser Gly His Gln
   305                 310                 315                 320
   Ile Met Ala Ser Pro Val Gly Phe Ser Gly Pro Glu Phe Thr Phe Pro
                   325                 330                 335
   Leu Tyr Gly Thr Met Gly Asn Ala Ala Pro Gln Gln Arg Ile Val Ala
               340                 345                 350
   Gln Leu Gly Gln Gly Val Tyr Arg Thr Leu Ser Ser Thr Leu Tyr Arg
           355                 360                 365
   Arg Pro Phe Asn Ile Gly Ile Asn Asn Gln Gln Leu Ser Val Leu Asp
   370 375 380
   Gly Thr Glu Phe Ala Tyr Gly Thr Ser Ser Asn Leu Pro Ser Ala Val
   385 390 395 400
   Tyr Arg Lys Ser Gly Thr Val Asp Ser Leu Asp Glu Ile Pro Pro Gln
   405 410 415
   Asn Asn Asn Val Pro Pro Arg Gln Gly Phe Ser His Arg Leu Ser His
   420 425 430
   Val Ser Met Phe Arg Ser Gly Phe Ser Asn Ser Ser Val Ser Ile Ile
   435 440 445
   Arg Ala Pro Met Phe Ser Trp Ile His Arg Ser Ala Thr Leu Thr Asn
   450 455 460
   Thr Ile Asp Pro Glu Arg Ile Asn Gln Ile Pro Leu Val Lys Gly Phe
   465 470 475 480
   Arg Val Trp Gly Gly Thr Ser Val Ile Thr Gly Pro Gly Phe Thr Gly
   485 490 495
   Gly Asp Ile Leu Arg Arg Asn Thr Phe Gly Asp Phe Val Ser Leu Gln
   500 505 510
   Val Asn Ile Asn Ser Pro Ile Thr Gln Arg Tyr Arg Leu Arg Phe Arg
   515 520 525
   Tyr Ala Ser Ser Arg Asp Ala Arg Val Ile Val Leu Thr Gly Ala Ala
   530 535 540
   Ser Thr Gly Val Gly Gly Gln Val Ser Val Asn Met Pro Leu Gln Lys
   545 550 555 560
   Thr Met Glu Ile Gly Glu Asn Leu Thr Ser Arg Thr Phe Arg Tyr Thr
   565 570 575
   Asp Phe Ser Asn Pro Phe Ser Phe Arg Ala Asn Pro Asp Ile Ile Gly
   580 585 590
   Ile Ser Glu Gln Pro Leu Phe Gly Ala Gly Ser Ile Ser Ser Gly Glu
   595 600 605
   Leu Tyr Ile Asp Lys Ile Glu Ile Ile Leu Ala Asp Ala Thr Phe Glu
   610 615 620
   Ala Glu Ser Asp Leu Glu Arg Ala Gln Lys Ala Val Asn Ala Leu Phe
   625 630 635 640
   Thr Ser Ser Asn Gln Ile Gly Leu Lys Thr Asp Val Thr Asp Tyr His
   645 650 655
   Ile Asp Gln Val Ser Asn Leu Val Asp Cys Leu Ser Asp Glu Phe Cys
   660 665 670
   Leu Asp Glu Lys Lys Glu Leu Ser Glu Lys Val Lys His Ala Lys Arg
   675 680 685
   Leu Ser Asp Glu Arg Asn Leu Leu Gln Asp Pro Asn Phe Arg Gly Ile
   690 695 700
   Asn Arg Gln Leu Asp Arg Gly Trp Arg Gly Ser Thr Asp Ile Thr Ile
   705 710 715 720
   Gln Gly Gly Asp Asp Val Phe Lys Glu Asn Tyr Val Thr Leu Gln Gly
   725 730 735
   Thr Phe Asp Glu Cys Tyr Pro Thr Tyr Leu Tyr Gln Pro Ile Asp Glu
   740 745 750
   Ser Lys Leu Lys Ala Tyr Thr Arg Tyr Gln Leu Arg Gly Tyr Ile Glu
   755 760 765
   Asp Ser Gln Asp Leu Glu Ile Tyr Leu Ile Arg Tyr Asn Ala Lys His
   770 775 780
   Glu Thr Val Asn Val Pro Gly Thr Gly Ser Leu Trp Pro Pro Ser Ala
   785 790 795 800
   Pro Ser Pro Ile Gly Lys Cys Gly Glu Pro Asn Arg Cys Ala Pro His
   805 810 815
   Leu Glu Trp Asn Pro Asp Leu Asp Cys Ser Cys Arg Asp Gly Glu Lys
   820 825 830
   Cys Ala His His Ser His His Phe Ser Leu Asp Ile Asp Val Gly Cys
   835 840 845
   Thr Asp Leu Asn Glu Asp Leu Gly Val Trp Val Ile Phe Lys Ile Lys
   850 855 860
   Thr Gln Asp Gly His Ala Arg Leu Gly Asn Leu Glu Phe Leu Glu Glu
   865                 870                 875                 880
   Lys Pro Leu Val Gly Glu Ala Leu Ala Arg Val Lys Arg Ala Glu Lys
                   885                 890                 895
   Lys Trp Arg Asp Lys Arg Glu Lys Leu Glu Trp Glu Thr Asn Ile Val
               900                 905                 910
   Tyr Lys Glu Ala Lys Glu Ser Val Asp Ala Leu Phe Val Asn Ser Gln
           915                 920                 925
   Tyr Asp Arg Leu Gln Ala Asp Thr Asn Ile Ala Met Ile His Ala Ala
       930                 935                 940
   Asp Lys Arg Val His Ser Ile Arg Glu Ala Tyr Leu Pro Glu Leu Ser
   945                 950                 955                 960
   Val Ile Pro Gly Val Asn Ala Ala Ile Phe Glu Glu Leu Glu Gly Arg
                   965                 970                 975
   Ile Phe Thr Ala Phe Ser Leu Tyr Asp Ala Arg Asn Val Ile Lys Asn
               980                 985                 990
   Gly Asp Phe Asn Asn Gly Leu Ser Cys Trp Asn Val Lys Gly His Val
           995                 1000                1005
   Asp Val Glu Glu Gln Asn Asn His Arg Ser Val Leu Val Val Pro Glu
       1010                1015                1020
   Trp Glu Ala Glu Val Ser Gln Glu Val Arg Val Cys Pro Gly Arg Gly
   1025                1030                1035                1040
   Tyr Ile Leu Arg Val Thr Ala Tyr Lys Glu Gly Tyr Gly Glu Gly Cys
                   1045                1050                1055
   Val Thr Ile His Glu Ile Glu Asn Asn Thr Asp Glu Leu Lys Phe Ser
               1060                1065                1070
   Asn Cys Val Glu Glu Glu Val Tyr Pro Asn Asn Thr Val Thr Cys Asn
           1075                1080                1085
   Asp Tyr Thr Ala Thr Gln Glu Glu Tyr Glu Gly Thr Tyr Thr Ser Arg
       1090                1095                1100
   Asn Arg Gly Tyr Asp Gly Ala Tyr Glu Ser Asn Ser Ser Val Pro Ala
   1105                1110                1115                1120
   Asp Tyr Ala Ser Ala His Glu Glu Lys Ala Tyr Thr Asp Gly Arg Arg
                   1125                1130                1135
   Asp Asn Pro Cys Glu Ser Asn Arg Gly Tyr Gly Asp Tyr Thr Pro Leu
               1140                1145                1150
   Pro Ala Gly Tyr Val Thr Lys Glu Leu Glu Tyr Phe Pro Glu Thr Asp
           1155                1160                1165
   Lys Val Trp Ile Glu Ile Gly Glu Thr Glu Gly Thr Phe Ile Val Asp
       1170                1175                1180
   Ser Val Glu Leu Leu Leu Met Glu Glu
   1185                1190
<210> 7
   <211> 3582
   <212> DNA
   <213> Artificial Sequence
<220>
   <223> Description of Artificial Sequence: synthetic gene encoding H04 with full-length Cry1Ab tail
<220>
   <221> CDS
   <222> (1)..(3582)
   <223> H04 with full-length Cry1Ab tail
<400> 7
   atg gac aac aac ccc aac atc aac gag tgc atc ccc tac aac tgc ctg    48
   Met Asp Asn Asn Pro Asn Ile Asn Glu Cys Ile Pro Tyr Asn Cys Leu
   1 5 10 15
   agc aac ccc gag gtg gag gtg ctg ggc ggc gag cgc atc gag acc ggc 96
   Ser Asn Pro Glu Val Glu Val Leu Gly Gly Glu Arg Ile Glu Thr Gly
   20 25 30
   tac acc ccc atc gac atc agc ctg agc ctg acc cag ttc ctg ctg agc 144
   Tyr Thr Pro Ile Asp Ile Ser Leu Ser Leu Thr Gln Phe Leu Leu Ser
   35 40 45
   gag ttc gtg ccc ggc gcc ggc ttc gtg ctg ggc ctg gtg gac atc atc 192
   Glu Phe Val Pro Gly Ala Gly Phe Val Leu Gly Leu Val Asp Ile Ile
   50 55 60
   tgg ggc atc ttc ggc ccc agc cag tgg gac gcc ttc ctg gtg cag atc 240
   Trp Gly Ile Phe Gly Pro Ser Gln Trp Asp Ala Phe Leu Val Gln Ile
   65 70 75 80
   gag cag ttg ata aac caa cgc ata gag gaa ttc gcc cgc aac cag gcc 288
   Glu Gln Leu Ile Asn Gln Arg Ile Glu Glu Phe Ala Arg Asn Gln Ala
   85 90 95
   atc agc cgc ctg gag ggc ctg agc aac ctg tac caa atc tac gcc gag 336
   Ile Ser Arg Leu Glu Gly Leu Ser Asn Leu Tyr Gln Ile Tyr Ala Glu
   100 105 110
   agc ttc cgc gag tgg gag gcc gac ccc acc aac ccc gcc ctg cgc gag 384
   Ser Phe Arg Glu Trp Glu Ala Asp Pro Thr Asn Pro Ala Leu Arg Glu
   115 120 125
   gag atg cgc atc cag ttc aac gac atg aac agc gcc ctg acc acc gcc 432
   Glu Met Arg Ile Gln Phe Asn Asp Met Asn Ser Ala Leu Thr Thr Ala
   130 135 140
   atc ccc ctg ttc gcc gtg cag aac tac cag gtg ccc ctg ctg agc gtg 480
   Ile Pro Leu Phe Ala Val Gln Asn Tyr Gln Val Pro Leu Leu Ser Val
   145 150 155 160
   tac gtg cag gcc gcc aac ctg cac ctg agc gtg ctg cgc gac gtc agc 528
   Tyr Val Gln Ala Ala Asn Leu His Leu Ser Val Leu Arg Asp Val Ser
   165 170 175
   gtg ttc ggc cag cgc tgg ggc ttc gac gcc gcc acc atc aac agc cgc 576
   Val Phe Gly Gln Arg Trp Gly Phe Asp Ala Ala Thr Ile Asn Ser Arg
   180 185 190
   tac aac gac ctg acc cgc ctg atc ggc aac tac acc gac cac gcc gtg 624
   Tyr Asn Asp Leu Thr Arg Leu Ile Gly Asn Tyr Thr Asp His Ala Val
   195 200 205
   cgc tgg tac aac acc ggc ctg gag cgc gtg tgg ggt ccc gac agc cgc 672
   Arg Trp Tyr Asn Thr Gly Leu Glu Arg Val Trp Gly Pro Asp Ser Arg
   210 215 220
   gac tgg atc agg tac aac cag ttc cgc cgc gag ctg acc ctg acc gtg 720
   Asp Trp Ile Arg Tyr Asn Gln Phe Arg Arg Glu Leu Thr Leu Thr Val
   225 230 235 240
   ctg gac atc gtg agc ctg ttc ccc aac tac gac agc cgc acc tac ccc 768
   Leu Asp Ile Val Ser Leu Phe Pro Asn Tyr Asp Ser Arg Thr Tyr Pro
                   245                 250                 255
   atc cgc acc gtg agc cag ctg acc cgc gag att tac acc aac ccc gtg     816
   Ile Arg Thr Val Ser Gln Leu Thr Arg Glu Ile Tyr Thr Asn Pro Val
               260                 265                 270
   ctg gag aac ttc gac ggc agc ttc cgc ggc agc gcc cag ggc atc gag     864
   Leu Glu Asn Phe Asp Gly Ser Phe Arg Gly Ser Ala Gln Gly Ile Glu
           275                 280                 285
   ggc agc atc cgc agc ccc cac ctg atg gac atc ctg aac agc atc acc     912
   Gly Ser Ile Arg Ser Pro His Leu Met Asp Ile Leu Asn Ser Ile Thr
       290                 295                 300
   atc tac acc gac gcc cac cgc ggc gag tac tac tgg agc ggc cac cag     960
   Ile Tyr Thr Asp Ala His Arg Gly Glu Tyr Tyr Trp Ser Gly His Gln
   305                 310                 315                 320
   atc atg gcc agc ccc gtc ggc ttc agc ggc ccc gag ttc acc ttc ccc    1008
   Ile Met Ala Ser Pro Val Gly Phe Ser Gly Pro Glu Phe Thr Phe Pro
                   325                 330                 335
   ctg tac ggc acc atg ggc aac gct gca cct cag cag cgc atc gtg gca    1056
   Leu Tyr Gly Thr Met Gly Asn Ala Ala Pro Gln Gln Arg Ile Val Ala
               340                 345                 350
   cag ctg ggc cag gga gtg tac cgc acc ctg agc agc acc ctg tac cgt    1104
   Gln Leu Gly Gln Gly Val Tyr Arg Thr Leu Ser Ser Thr Leu Tyr Arg
           355                 360                 365
   cga cct ttc aac atc ggc atc aac aac cag cag ctg agc gtg ctg gac    1152
   Arg Pro Phe Asn Ile Gly Ile Asn Asn Gln Gln Leu Ser Val Leu Asp
       370                 375                 380
   ggc acc gag ttc gcc tac ggc acc agc agc aac ctg ccc agc gcc gtg    1200
   Gly Thr Glu Phe Ala Tyr Gly Thr Ser Ser Asn Leu Pro Ser Ala Val
   385                 390                 395                 400
   tac cgc aag agc ggc acc gtg gac agc ctg gac gag atc ccc cct cag    1248
   Tyr Arg Lys Ser Gly Thr Val Asp Ser Leu Asp Glu Ile Pro Pro Gln
                   405                 410                 415
   aac aac aac gtg cca cct cga cag ggc ttc agc cac cgt ctg agc cac    1296
   Asn Asn Asn Val Pro Pro Arg Gln Gly Phe Ser His Arg Leu Ser His
               420                 425                 430
   gtg agc atg ttc cgc agt ggc ttc agc aac agc agc gtg agc atc atc    1344
   Val Ser Met Phe Arg Ser Gly Phe Ser Asn Ser Ser Val Ser Ile Ile
           435                 440                 445
   cgt gca ccc atg ttc agc tgg att cac cgc agc gcc acc ctg acc aac    1392
   Arg Ala Pro Met Phe Ser Trp Ile His Arg Ser Ala Thr Leu Thr Asn
       450                 455                 460
   acc atc gac ccc gag cgc atc aac cag atc ccc ctg gtg aag ggc ttc    1440
   Thr Ile Asp Pro Glu Arg Ile Asn Gln Ile Pro Leu Val Lys Gly Phe
   465                 470                 475                 480
   cgg gtg tgg ggc ggc acc agc gtg atc acc ggc ccc ggc ttc acc gga    1488
   Arg Val Trp Gly Gly Thr Ser Val Ile Thr Gly Pro Gly Phe Thr Gly
                   485                 490                 495
   ggc gac atc ctg cgc aga aac acc ttc ggc gac ttc gtg agc ctg cag   1536
   Gly Asp Ile Leu Arg Arg Asn Thr Phe Gly Asp Phe Val Ser Leu Gln
               500                 505                 510
   gtg aac atc aac agc ccc atc acc cag cgt tac cgc ctg cgc ttc cgc   1584
   Val Asn Ile Asn Ser Pro Ile Thr Gln Arg Tyr Arg Leu Arg Phe Arg
           515                 520                 525
   tac gcc agc agc cgc gac gcc cgt gtg atc gtg ctg act ggc gcc gct   1632
   Tyr Ala Ser Ser Arg Asp Ala Arg Val Ile Val Leu Thr Gly Ala Ala
       530                 535                 540
   agc acc ggt gtg ggc ggt cag gtg agc gtg aac atg ccc ctg cag aag   1680
   Ser Thr Gly Val Gly Gly Gln Val Ser Val Asn Met Pro Leu Gln Lys
   545                 550                 555                 560
   act atg gag atc ggc gag aac ctg act agt cgc acc ttc cgc tac acc   1728
   Thr Met Glu Ile Gly Glu Asn Leu Thr Ser Arg Thr Phe Arg Tyr Thr
                   565                 570                 575
   gac ttc agc aac ccc ttc agc ttc cgc gcc aac ccc gac atc atc ggc   1776
   Asp Phe Ser Asn Pro Phe Ser Phe Arg Ala Asn Pro Asp Ile Ile Gly
               580                 585                 590
   atc agc gag cag ccc ctg ttc ggt gcc ggc agc atc agc agc ggc gag   1824
   Ile Ser Glu Gln Pro Leu Phe Gly Ala Gly Ser Ile Ser Ser Gly Glu
           595                 600                 605
   ctg tac atc gac aag atc gag atc atc ctg gcc gac gcc acc ttc gag   1872
   Leu Tyr Ile Asp Lys Ile Glu Ile Ile Leu Ala Asp Ala Thr Phe Glu
       610                 615                 620
   gcc gag agc gac ctg gag cgc gcc cag aag gcc gtg aac gcc ctg ttc   1920
   Ala Glu Ser Asp Leu Glu Arg Ala Gln Lys Ala Val Asn Ala Leu Phe
   625                 630                 635                 640
   acc agc agc aac cag atc ggc ctg aag acc gac gtg acc gac tac cac   1968
   Thr Ser Ser Asn Gln Ile Gly Leu Lys Thr Asp Val Thr Asp Tyr His
                   645                 650                 655
   atc gac cag gtg agc aac ctg gtg gac tgc tta agc gac gag ttc tgc   2016
   Ile Asp Gln Val Ser Asn Leu Val Asp Cys Leu Ser Asp Glu Phe Cys
               660                 665                 670
   ctg gac gag aag aag gag ctg agc gag aag gtg aag cac gcc aag cgc   2064
   Leu Asp Glu Lys Lys Glu Leu Ser Glu Lys Val Lys His Ala Lys Arg
           675                 680                 685
   ctg agc gac gag cgc aac ctg ctg cag gac ccc aac ttc cgc ggc atc   2112
   Leu Ser Asp Glu Arg Asn Leu Leu Gln Asp Pro Asn Phe Arg Gly Ile
       690                 695                 700
   aac cgc cag ctg gac cgc ggc tgg cga ggc agc acc gat atc acc atc   2160
   Asn Arg Gln Leu Asp Arg Gly Trp Arg Gly Ser Thr Asp Ile Thr Ile
   705                 710                 715                 720
   cag ggc ggc gac gac gtg ttc aag gag aac tac gtg acc ctg cag ggc   2208
   Gln Gly Gly Asp Asp Val Phe Lys Glu Asn Tyr Val Thr Leu Gln Gly
                   725                 730                 735
   acc ttc gac gag tgc tac ccc acc tac ctg tac cag ccg atc gac gag    2256
   Thr Phe Asp Glu Cys Tyr Pro Thr Tyr Leu Tyr Gln Pro Ile Asp Glu
               740                 745                 750
   agc aag ctg aag gcc tac acc cgc tac cag ctg cgc ggc tac atc gag    2304
   Ser Lys Leu Lys Ala Tyr Thr Arg Tyr Gln Leu Arg Gly Tyr Ile Glu
           755                 760                 765
   gac agc cag gac ctg gaa atc tac ctg atc cgc tac aac gcg aag cac    2352
   Asp Ser Gln Asp Leu Glu Ile Tyr Leu Ile Arg Tyr Asn Ala Lys His
       770                 775                 780
   gag acc gtg aac gtg ccc ggc acc ggc agc ctg tgg ccc ctg agc gcc    2400
   Glu Thr Val Asn Val Pro Gly Thr Gly Ser Leu Trp Pro Leu Ser Ala
   785                 790                 795                 800
   ccc agc ccc atc ggc aag tgc ggg gag ccg aat cga tgc gct ccg cac    2448
   Pro Ser Pro Ile Gly Lys Cys Gly Glu Pro Asn Arg Cys Ala Pro His
                   805                 810                 815
   ctg gag tgg aac ccg gac cta gac tgc agc tgc agg gac ggg gag aag    2496
   Leu Glu Trp Asn Pro Asp Leu Asp Cys Ser Cys Arg Asp Gly Glu Lys
               820                 825                 830
   tgc gcc cac cac agc cac cac ttc agc ctg gac atc gac gtg ggc tgc    2544
   Cys Ala His His Ser His His Phe Ser Leu Asp Ile Asp Val Gly Cys
           835                 840                 845
   acc gac ctg aac gag gac ctg ggc gtg tgg gtg atc ttc aag atc aag    2592
   Thr Asp Leu Asn Glu Asp Leu Gly Val Trp Val Ile Phe Lys Ile Lys
       850                 855                 860
   acc cag gac ggc cac gcc cgc ctg ggc aat cta gag ttc ctg gag gag    2640
   Thr Gln Asp Gly His Ala Arg Leu Gly Asn Leu Glu Phe Leu Glu Glu
   865                 870                 875                 880
   aag ccc ctg gtg ggc gag gcc ctg gcc cgc gtg aag cgt gct gag aag    2688
   Lys Pro Leu Val Gly Glu Ala Leu Ala Arg Val Lys Arg Ala Glu Lys
                   885                 890                 895
   aag tgg cgc gac aag cgc gag aag ctg gag tgg gag acc aac atc gtg    2736
   Lys Trp Arg Asp Lys Arg Glu Lys Leu Glu Trp Glu Thr Asn Ile Val
               900                 905                 910
   tac aag gag gcc aag gag agc gtg gac gcc ctg ttc gtg aac agc cag    2784
   Tyr Lys Glu Ala Lys Glu Ser Val Asp Ala Leu Phe Val Asn Ser Gln
           915                 920                 925
   tac gac cgc ctg cag gcc gac acc aac atc gcc atg atc cac gcc gcc    2832
   Tyr Asp Arg Leu Gln Ala Asp Thr Asn Ile Ala Met Ile His Ala Ala
       930                 935                 940
   gac aag cgc gtg cac agc att cgc gag gcc tac ctg ccc gag ctg agc    2880
   Asp Lys Arg Val His Ser Ile Arg Glu Ala Tyr Leu Pro Glu Leu Ser
   945                 950                 955                 960
   gtg atc ccc ggt gtg aac gcc gcc atc ttc gag gaa ctc gag ggc cgc    2928
   Val Ile Pro Gly Val Asn Ala Ala Ile Phe Glu Glu Leu Glu Gly Arg
                   965                 970                 975
   atc ttc acc gcc ttc agc ctg tac gac gcc cgc aac gtg atc aag aac    2976
   Ile Phe Thr Ala Phe Ser Leu Tyr Asp Ala Arg Asn Val Ile Lys Asn
               980                 985                 990
   ggc gac ttc aac aac ggc ctg agc tgc tgg aac gtg aag ggc cac gtg   3024
   Gly Asp Phe Asn Asn Gly Leu Ser Cys Trp Asn Val Lys Gly His Val
           995                 1000                1005
   gac gtg gag gag cag aac aac cac cgc agc gtg ctg gtg gtg ccc gag   3072
   Asp Val Glu Glu Gln Asn Asn His Arg Ser Val Leu Val Val Pro Glu
       1010                1015                1020
   tgg gag gcc gag gtg agc cag gag gtg cgc gtg tgc ccc ggc cgc ggc   3120
   Trp Glu Ala Glu Val Ser Gln Glu Val Arg Val Cys Pro Gly Arg Gly
   1025                1030                1035                1040
   tac atc ctg cgc gtg acc gcc tac aag gag ggc tac ggc gag ggc tgc   3168
   Tyr Ile Leu Arg Val Thr Ala Tyr Lys Glu Gly Tyr Gly Glu Gly Cys
                   1045                1050                1055
   gtg acc atc cac gag atc gag aac aac acc gac gag ctc aag ttc agc   3216
   Val Thr Ile His Glu Ile Glu Asn Asn Thr Asp Glu Leu Lys Phe Ser
               1060                1065                1070
   aac tgc gtg gag gag gag gtt tac ccc aac aac acc gtg acc tgc aac   3264
   Asn Cys Val Glu Glu Glu Val Tyr Pro Asn Asn Thr Val Thr Cys Asn
           1075                1080                1085
   gac tac acc gcg acc cag gag gag tac gaa ggc acc tac acc tct cgc   3312
   Asp Tyr Thr Ala Thr Gln Glu Glu Tyr Glu Gly Thr Tyr Thr Ser Arg
       1090                1095                1100
   aac agg ggt tac gac ggc gcc tac gag tcc aac agc tcc gtg cca gct   3360
   Asn Arg Gly Tyr Asp Gly Ala Tyr Glu Ser Asn Ser Ser Val Pro Ala
   1105                1110                1115                1120
   gac tac gcc agc gcc tac gag gag aaa gcc tac acc gac ggt aga cgc   3408
   Asp Tyr Ala Ser Ala Tyr Glu Glu Lys Ala Tyr Thr Asp Gly Arg Arg
                   1125                1130                1135
   gac aac cca tgt gag agc aac aga ggc tac ggc gac tac acc ccc ctg   3456
   Asp Asn Pro Cys Glu Ser Asn Arg Gly Tyr Gly Asp Tyr Thr Pro Leu
               1140                1145                1150
   ccc gct gga tac gtg acc aag gag ctg gag tac ttc ccc gag acc gac   3504
   Pro Ala Gly Tyr Val Thr Lys Glu Leu Glu Tyr Phe Pro Glu Thr Asp
           1155                1160                1165
   aag gtg tgg atc gag att ggc gag acc gag ggc acc ttc atc gtg gac   3552
   Lys Val Trp Ile Glu Ile Gly Glu Thr Glu Gly Thr Phe Ile Val Asp
       1170                1175                1180
   agc gtg gag ctg ctg ctg atg gag gag tag                           3582
   Ser Val Glu Leu Leu Leu Met Glu Glu
   1185                1190
<210> 8
   <211> 1193
   <212> PRT
   <213> Artificial Sequence
   <223> Description of Artificial Sequence: synthetic gene encoding H04 with full-length Cry1Ab tail
<400> 8
   Met Asp Asn Asn Pro Asn Ile Asn Glu Cys Ile Pro Tyr Asn Cys Leu
   1               5                   10                  15
   Ser Asn Pro Glu Val Glu Val Leu Gly Gly Glu Arg Ile Glu Thr Gly
               20                  25                  30
   Tyr Thr Pro Ile Asp Ile Ser Leu Ser Leu Thr Gln Phe Leu Leu Ser
           35                  40                  45
   Glu Phe Val Pro Gly Ala Gly Phe Val Leu Gly Leu Val Asp Ile Ile
       50                  55                  60
   Trp Gly Ile Phe Gly Pro Ser Gln Trp Asp Ala Phe Leu Val Gln Ile
   65                  70                  75                  80
   Glu Gln Leu Ile Asn Gln Arg Ile Glu Glu Phe Ala Arg Asn Gln Ala
                   85                  90                  95
   Ile Ser Arg Leu Glu Gly Leu Ser Asn Leu Tyr Gln Ile Tyr Ala Glu
               100                 105                 110
   Ser Phe Arg Glu Trp Glu Ala Asp Pro Thr Asn Pro Ala Leu Arg Glu
           115                 120                 125
   Glu Met Arg Ile Gln Phe Asn Asp Met Asn Ser Ala Leu Thr Thr Ala
       130                 135                 140
   Ile Pro Leu Phe Ala Val Gln Asn Tyr Gln Val Pro Leu Leu Ser Val
   145                 150                 155                 160
   Tyr Val Gln Ala Ala Asn Leu His Leu Ser Val Leu Arg Asp Val Ser
                   165                 170                 175
   Val Phe Gly Gln Arg Trp Gly Phe Asp Ala Ala Thr Ile Asn Ser Arg
               180                 185                 190
   Tyr Asn Asp Leu Thr Arg Leu Ile Gly Asn Tyr Thr Asp His Ala Val
           195                 200                 205
   Arg Trp Tyr Asn Thr Gly Leu Glu Arg Val Trp Gly Pro Asp Ser Arg
       210                 215                 220
   Asp Trp Ile Arg Tyr Asn Gln Phe Arg Arg Glu Leu Thr Leu Thr Val
   225                 230                 235                 240
   Leu Asp Ile Val Ser Leu Phe Pro Asn Tyr Asp Ser Arg Thr Tyr Pro
                   245                 250                 255
   Ile Arg Thr Val Ser Gln Leu Thr Arg Glu Ile Tyr Thr Asn Pro Val
               260                 265                 270
   Leu Glu Asn Phe Asp Gly Ser Phe Arg Gly Ser Ala Gln Gly Ile Glu
           275                 280                 285
   Gly Ser Ile Arg Ser Pro His Leu Met Asp Ile Leu Asn Ser Ile Thr
       290                 295                 300
   Ile Tyr Thr Asp Ala His Arg Gly Glu Tyr Tyr Trp Ser Gly His Gln
   305                 310                 315                 320
   Ile Met Ala Ser Pro Val Gly Phe Ser Gly Pro Glu Phe Thr Phe Pro
                   325                 330                 335
   Leu Tyr Gly Thr Met Gly Asn Ala Ala Pro Gln Gln Arg Ile Val Ala
               340                 345                 350
   Gln Leu Gly Gln Gly Val Tyr Arg Thr Leu Ser Ser Thr Leu Tyr Arg
           355                 360                 365
   Arg Pro Phe Asn Ile Gly Ile Asn Asn Gln Gln Leu Ser Val Leu Asp
       370                 375                 380
   Gly Thr Glu Phe Ala Tyr Gly Thr Ser Ser Asn Leu Pro Ser Ala Val
   385                 390                 395                 400
   Tyr Arg Lys Ser Gly Thr Val Asp Ser Leu Asp Glu Ile Pro Pro Gln
                   405                 410                 415
   Asn Asn Asn Val Pro Pro Arg Gln Gly Phe Ser His Arg Leu Ser His
               420                 425                 430
   Val Ser Met Phe Arg Ser Gly Phe Ser Asn Ser Ser Val Ser Ile Ile
           435                 440                 445
   Arg Ala Pro Met Phe Ser Trp Ile His Arg Ser Ala Thr Leu Thr Asn
       450                 455                 460
   Thr Ile Asp Pro Glu Arg Ile Asn Gln Ile Pro Leu Val Lys Gly Phe
   465                 470                 475                 480
   Arg Val Trp Gly Gly Thr Ser Val Ile Thr Gly Pro Gly Phe Thr Gly
                   485                 490                 495
   Gly Asp Ile Leu Arg Arg Asn Thr Phe Gly Asp Phe Val Ser Leu Gln
               500                 505                 510
   Val Asn Ile Asn Ser Pro Ile Thr Gln Arg Tyr Arg Leu Arg Phe Arg
           515                 520                 525
   Tyr Ala Ser Ser Arg Asp Ala Arg Val Ile Val Leu Thr Gly Ala Ala
       530                 535                 540
   Ser Thr Gly Val Gly Gly Gln Val Ser Val Asn Met Pro Leu Gln Lys
   545                 550                 555                 560
   Thr Met Glu Ile Gly Glu Asn Leu Thr Ser Arg Thr Phe Arg Tyr Thr
                   565                 570                 575
   Asp Phe Ser Asn Pro Phe Ser Phe Arg Ala Asn Pro Asp Ile Ile Gly
               580                 585                 590
   Ile Ser Glu Gln Pro Leu Phe Gly Ala Gly Ser Ile Ser Ser Gly Glu
           595                 600                 605
   Leu Tyr Ile Asp Lys Ile Glu Ile Ile Leu Ala Asp Ala Thr Phe Glu
       610                 615                 620
   Ala Glu Ser Asp Leu Glu Arg Ala Gln Lys Ala Val Asn Ala Leu Phe
   625                 630                 635                 640
   Thr Ser Ser Asn Gln Ile Gly Leu Lys Thr Asp Val Thr Asp Tyr His
                   645                 650                 655
   Ile Asp Gln Val Ser Asn Leu Val Asp Cys Leu Ser Asp Glu Phe Cys
               660                 665                 670
   Leu Asp Glu Lys Lys Glu Leu Ser Glu Lys Val Lys His Ala Lys Arg
           675                 680                 685
   Leu Ser Asp Glu Arg Asn Leu Leu Gln Asp Pro Asn Phe Arg Gly Ile
       690                 695                 700
   Asn Arg Gln Leu Asp Arg Gly Trp Arg Gly Ser Thr Asp Ile Thr Ile
   705                 710                 715                 720
   Gln Gly Gly Asp Asp Val Phe Lys Glu Asn Tyr Val Thr Leu Gln Gly
                   725                 730                 735
   Thr Phe Asp Glu Cys Tyr Pro Thr Tyr Leu Tyr Gln Pro Ile Asp Glu
               740                 745                 750
   Ser Lys Leu Lys Ala Tyr Thr Arg Tyr Gln Leu Arg Gly Tyr Ile Glu
           755                 760                 765
   Asp Ser Gln Asp Leu Glu Ile Tyr Leu Ile Arg Tyr Asn Ala Lys His
       770                 775                 780
   Glu Thr Val Asn Val Pro Gly Thr Gly Ser Leu Trp Pro Leu Ser Ala
   785                 790                 795                 800
   Pro Ser Pro Ile Gly Lys Cys Gly Glu Pro Asn Arg Cys Ala Pro His
                   805                 810                 815
   Leu Glu Trp Asn Pro Asp Leu Asp Cys Ser Cys Arg Asp Gly Glu Lys
               820                 825                 830
   Cys Ala His His Ser His His Phe Ser Leu Asp Ile Asp Val Gly Cys
           835                 840                 845
   Thr Asp Leu Asn Glu Asp Leu Gly Val Trp Val Ile Phe Lys Ile Lys
       850                 855                 860
   Thr Gln Asp Gly His Ala Arg Leu Gly Asn Leu Glu Phe Leu Glu Glu
   865                 870                 875                 880
   Lys Pro Leu Val Gly Glu Ala Leu Ala Arg Val Lys Arg Ala Glu Lys
                   885                 890                 895
   Lys Trp Arg Asp Lys Arg Glu Lys Leu Glu Trp Glu Thr Asn Ile Val
               900                 905                 910
   Tyr Lys Glu Ala Lys Glu Ser Val Asp Ala Leu Phe Val Asn Ser Gln
           915                 920                 925
   Tyr Asp Arg Leu Gln Ala Asp Thr Asn Ile Ala Met Ile His Ala Ala
       930                 935                 940
   Asp Lys Arg Val His Ser Ile Arg Glu Ala Tyr Leu Pro Glu Leu Ser
   945                 950                 955                 960
   Val Ile Pro Gly Val Asn Ala Ala Ile Phe Glu Glu Leu Glu Gly Arg
                   965                 970                 975
   Ile Phe Thr Ala Phe Ser Leu Tyr Asp Ala Arg Asn Val Ile Lys Asn
               980                 985                 990
   Gly Asp Phe Asn Asn Gly Leu Ser Cys Trp Asn Val Lys Gly His Val
           995                 1000                1005
   Asp Val Glu Glu Gln Asn Asn His Arg Ser Val Leu Val Val Pro Glu
       1010                1015                1020
   Trp Glu Ala Glu Val Ser Gln Glu Val Arg Val Cys Pro Gly Arg Gly
   1025                1030                1035                1040
   Tyr Ile Leu Arg Val Thr Ala Tyr Lys Glu Gly Tyr Gly Glu Gly Cys
                   1045                1050                1055
   Val Thr Ile His Glu Ile Glu Asn Asn Thr Asp Glu Leu Lys Phe Ser
               1060                1065                1070
   Asn Cys Val Glu Glu Glu Val Tyr Pro Asn Asn Thr Val Thr Cys Asn
           1075                1080                1085
   Asp Tyr Thr Ala Thr Gln Glu Glu Tyr Glu Gly Thr Tyr Thr Ser Arg
       1090                1095                1100
   Asn Arg Gly Tyr Asp Gly Ala Tyr Glu Ser Asn Ser Ser Val Pro Ala
   1105                1110                1115                1120
   Asp Tyr Ala Ser Ala Tyr Glu Glu Lys Ala Tyr Thr Asp Gly Arg Arg
                   1125                1130                1135
   Asp Asn Pro Cys Glu Ser Asn Arg Gly Tyr Gly Asp Tyr Thr Pro Leu
               1140                1145                1150
   Pro Ala Gly Tyr Val Thr Lys Glu Leu Glu Tyr Phe Pro Glu Thr Asp
           1155                1160                1165
   Lys Val Trp Ile Glu Ile Gly Glu Thr Glu Gly Thr Phe Ile Val Asp
       1170                1175                1180
   Ser Val Glu Leu Leu Leu Met Glu Glu
   1185                1190
<210> 9
   <211> 2007
   <212> DNA
   <213> Artificial Sequence
<220>
   <223> Description of Artificial Sequence: synthetic gene encoding H04 plus the first 40 amino acids of the Cry1Ab tail
<220>
   <221> CDS
   <222> (1)..(2007)
   <223> H04 with truncated Cry1Ab tail
<400> 9
   atg gac aac aac ccc aac atc aac gag tgc atc ccc tac aac tgc ctg    48
   Met Asp Asn Asn Pro Asn Ile Asn Glu Cys Ile Pro Tyr Asn Cys Leu
    1               5                  10                  15
   agc aac ccc gag gtg gag gtg ctg ggc ggc gag cgc atc gag acc ggc    96
   Ser Asn Pro Glu Val Glu Val Leu Gly Gly Glu Arg Ile Glu Thr Gly
               20                  25                  30
   tac acc ccc atc gac atc agc ctg agc ctg acc cag ttc ctg ctg agc   144
   Tyr Thr Pro Ile Asp Ile Ser Leu Ser Leu Thr Gln Phe Leu Leu Ser
           35                  40                  45
   gag ttc gtg ccc ggc gcc ggc ttc gtg ctg ggc ctg gtg gac atc atc     192
   Glu Phe Val Pro Gly Ala Gly Phe Val Leu Gly Leu Val Asp Ile Ile
       50                  55                  60
   tgg ggc atc ttc ggc ccc agc cag tgg gac gcc ttc ctg gtg cag atc     240
   Trp Gly Ile Phe Gly Pro Ser Gln Trp Asp Ala Phe Leu Val Gln Ile
   65                  70                  75                  80
   gag cag ttg ata aac caa cgc ata gag gaa ttc gcc cgc aac cag gcc     288
   Glu Gln Leu Ile Asn Gln Arg Ile Glu Glu Phe Ala Arg Asn Gln Ala
                   85                  90                  95
   atc agc cgc ctg gag ggc ctg agc aac ctg tac caa atc tac gcc gag     336
   Ile Ser Arg Leu Glu Gly Leu Ser Asn Leu Tyr Gln Ile Tyr Ala Glu
               100                 105                 110
   agc ttc cgc gag tgg gag gcc gac ccc acc aac ccc gcc ctg cgc gag     384
   Ser Phe Arg Glu Trp Glu Ala Asp Pro Thr Asn Pro Ala Leu Arg Glu
           115                 120                 125
   gag atg cgc atc cag ttc aac gac atg aac agc gcc ctg acc acc gcc     432
   Glu Met Arg Ile Gln Phe Asn Asp Met Asn Ser Ala Leu Thr Thr Ala
       130                 135                 140
   atc ccc ctg ttc gcc gtg cag aac tac cag gtg ccc ctg ctg agc gtg     480
   Ile Pro Leu Phe Ala Val Gln Asn Tyr Gln Val Pro Leu Leu Ser Val
   145                 150                 155                 160
   tac gtg cag gcc gcc aac ctg cac ctg agc gtg ctg cgc gac gtc agc     528
   Tyr Val Gln Ala Ala Asn Leu His Leu Ser Val Leu Arg Asp Val Ser
                   165                 170                 175
   gtg ttc ggc cag cgc tgg ggc ttc gac gcc gcc acc atc aac agc cgc     576
   Val Phe Gly Gln Arg Trp Gly Phe Asp Ala Ala Thr Ile Asn Ser Arg
               180                 185                 190
   tac aac gac ctg acc cgc ctg atc ggc aac tac acc gac cac gcc gtg     624
   Tyr Asn Asp Leu Thr Arg Leu Ile Gly Asn Tyr Thr Asp His Ala Val
           195                 200                 205
   cgc tgg tac aac acc ggc ctg gag cgc gtg tgg ggt ccc gac agc cgc     672
   Arg Trp Tyr Asn Thr Gly Leu Glu Arg Val Trp Gly Pro Asp Ser Arg
       210                 215                 220
   gac tgg atc agg tac aac cag ttc cgc cgc gag ctg acc ctg acc gtg     720
   Asp Trp Ile Arg Tyr Asn Gln Phe Arg Arg Glu Leu Thr Leu Thr Val
   225                 230                 235                 240
   ctg gac atc gtg agc ctg ttc ccc aac tac gac agc cgc acc tac ccc     768
   Leu Asp Ile Val Ser Leu Phe Pro Asn Tyr Asp Ser Arg Thr Tyr Pro
                   245                 250                 255
   atc cgc acc gtg agc cag ctg acc cgc gag att tac acc aac ccc gtg     816
   Ile Arg Thr Val Ser Gln Leu Thr Arg Glu Ile Tyr Thr Asn Pro Val
               260                 265                 270
   ctg gag aac ttc gac ggc agc ttc cgc ggc agc gcc cag ggc atc gag     864
   Leu Glu Asn Phe Asp Gly Ser Phe Arg Gly Ser Ala Gln Gly Ile Glu
           275                 280                 285
   ggc agc atc cgc agc ccc cac ctg atg gac atc ctg aac agc atc acc    912
   Gly Ser Ile Arg Ser Pro His Leu Met Asp Ile Leu Asn Ser Ile Thr
       290                 295                 300
   atc tac acc gac gcc cac cgc ggc gag tac tac tgg agc ggc cac cag    960
   Ile Tyr Thr Asp Ala His Arg Gly Glu Tyr Tyr Trp Ser Gly His Gln
   305                 310                 315                 320
   atc atg gcc agc ccc gtc ggc ttc agc ggc ccc gag ttc acc ttc ccc    1008
   Ile Met Ala Ser Pro Val Gly Phe Ser Gly Pro Glu Phe Thr Phe Pro
                   325                 330                 335
   ctg tac ggc acc atg ggc aac gct gca cct cag cag cgc atc gtg gca    1056
   Leu Tyr Gly Thr Met Gly Asn Ala Ala Pro Gln Gln Arg Ile Val Ala
               340                 345                 350
   cag ctg ggc cag gga gtg tac cgc acc ctg agc agc acc ctg tac cgt    1104
   Gln Leu Gly Gln Gly Val Tyr Arg Thr Leu Ser Ser Thr Leu Tyr Arg
           355                 360                 365
   cga cct ttc aac atc ggc atc aac aac cag cag ctg agc gtg ctg gac    1152
   Arg Pro Phe Asn Ile Gly Ile Asn Asn Gln Gln Leu Ser Val Leu Asp
       370                 375                 380
   ggc acc gag ttc gcc tac ggc acc agc agc aac ctg ccc agc gcc gtg    1200
   Gly Thr Glu Phe Ala Tyr Gly Thr Ser Ser Asn Leu Pro Ser Ala Val
   385                 390                 395                 400
   tac cgc aag agc ggc acc gtg gac agc ctg gac gag atc ccc cct cag    1248
   Tyr Arg Lys Ser Gly Thr Val Asp Ser Leu Asp Glu Ile Pro Pro Gln
                   405                 410                 415
   aac aac aac gtg cca cct cga cag ggc ttc agc cac cgt ctg agc cac    1296
   Asn Asn Asn Val Pro Pro Arg Gln Gly Phe Ser His Arg Leu Ser His
               420                 425                 430
   gtg agc atg ttc cgc agt ggc ttc agc aac agc agc gtg agc atc atc    1344
   Val Ser Met Phe Arg Ser Gly Phe Ser Asn Ser Ser Val Ser Ile Ile
           435                 440                 445
   cgt gca ccc atg ttc agc tgg att cac cgc agc gcc acc ctg acc aac    1392
   Arg Ala Pro Met Phe Ser Trp Ile His Arg Ser Ala Thr Leu Thr Asn
       450                 455                 460
   acc atc gac ccc gag cgc atc aac cag atc ccc ctg gtg aag ggc ttc    1440
   Thr Ile Asp Pro Glu Arg Ile Asn Gln Ile Pro Leu Val Lys Gly Phe
   465                 470                 475                 480
   cgg gtg tgg ggc ggc acc agc gtg atc acc ggc ccc ggc ttc acc gga    1488
   Arg Val Trp Gly Gly Thr Ser Val Ile Thr Gly Pro Gly Phe Thr Gly
                   485                 490                 495
   ggc gac atc ctg cgc aga aac acc ttc ggc gac ttc gtg agc ctg cag    1536
   Gly Asp Ile Leu Arg Arg Asn Thr Phe Gly Asp Phe Val Ser Leu Gln
               500                 505                 510
   gtg aac atc aac agc ccc atc acc cag cgt tac cgc ctg cgc ttc cgc    1584
   Val Asn Ile Asn Ser Pro Ile Thr Gln Arg Tyr Arg Leu Arg Phe Arg
           515                 520                 525
   tac gcc agc agc cgc gac gcc cgt gtg atc gtg ctg act ggc gcc gct    1632
   Tyr Ala Ser Ser Arg Asp Ala Arg Val Ile Val Leu Thr Gly Ala Ala
       530                 535                 540
   agc acc ggt gtg ggc ggt cag gtg agc gtg aac atg ccc ctg cag aag    1680
   Ser Thr Gly Val Gly Gly Gln Val Ser Val Asn Met Pro Leu Gln Lys
   545                 550                 555                 560
   act atg gag atc ggc gag aac ctg act agt cgc acc ttc cgc tac acc    1728
   Thr Met Glu Ile Gly Glu Asn Leu Thr Ser Arg Thr Phe Arg Tyr Thr
                   565                 570                 575
   gac ttc agc aac ccc ttc agc ttc cgc gcc aac ccc gac atc atc ggc    1776
   Asp Phe Ser Asn Pro Phe Ser Phe Arg Ala Asn Pro Asp Ile Ile Gly
               580                 585                 590
   atc agc gag cag ccc ctg ttc ggt gcc ggc agc atc agc agc ggc gag    1824
   Ile Ser Glu Gln Pro Leu Phe Gly Ala Gly Ser Ile Ser Ser Gly Glu
           595                 600                 605
   ctg tac atc gac aag atc gag atc atc ctg gcc gac gcc acc ttc gag    1872
   Leu Tyr Ile Asp Lys Ile Glu Ile Ile Leu Ala Asp Ala Thr Phe Glu
       610                 615                 620
   gcc gag agc gac ctg gag cgc gcc cag aag gcc gtg aac gcc ctg ttc    1920
   Ala Glu Ser Asp Leu Glu Arg Ala Gln Lys Ala Val Asn Ala Leu Phe
   625                 630                 635                 640
   acc agc agc aac cag atc ggc ctg aag acc gac gtg acc gac tac cac    1968
   Thr Ser Ser Asn Gln Ile Gly Leu Lys Thr Asp Val Thr Asp Tyr His
                   645                 650                 655
   atc gac cag gtg agc aac ctg gtg gac tgc tta agc tag                2007
   Ile Asp Gln Val Ser Asn Leu Val Asp Cys Leu Ser
               660                 665
<210> 10
   <211> 668
   <212> PRT
   <213> Artificial Sequence
   <223> Description of Artificial Sequence: synthetic gene encoding H04 plus the first 40 amino acids of the Cry1Ab tail
<400> 10
   Met Asp Asn Asn Pro Asn Ile Asn Glu Cys Ile Pro Tyr Asn Cys Leu
   1               5                   10                  15
   Ser Asn Pro Glu Val Glu Val Leu Gly Gly Glu Arg Ile Glu Thr Gly
               20                  25                  30
   Tyr Thr Pro Ile Asp Ile Ser Leu Ser Leu Thr Gln Phe Leu Leu Ser
           35                  40                  45
   Glu Phe Val Pro Gly Ala Gly Phe Val Leu Gly Leu Val Asp Ile Ile
       50                  55                  60
   Trp Gly Ile Phe Gly Pro Ser Gln Trp Asp Ala Phe Leu Val Gln Ile
   65                  70                  75                  80
   Glu Gln Leu Ile Asn Gln Arg Ile Glu Glu Phe Ala Arg Asn Gln Ala
                   85                  90                  95
   Ile Ser Arg Leu Glu Gly Leu Ser Asn Leu Tyr Gln Ile Tyr Ala Glu
               100                 105                 110
   Ser Phe Arg Glu Trp Glu Ala Asp Pro Thr Asn Pro Ala Leu Arg Glu
           115                 120                 125
   Glu Met Arg Ile Gln Phe Asn Asp Met Asn Ser Ala Leu Thr Thr Ala
       130                 135                 140
   Ile Pro Leu Phe Ala Val Gln Asn Tyr Gln Val Pro Leu Leu Ser Val
   145                 150                 155                 160
   Tyr Val Gln Ala Ala Asn Leu His Leu Ser Val Leu Arg Asp Val Ser
                   165                 170                 175
   Val Phe Gly Gln Arg Trp Gly Phe Asp Ala Ala Thr Ile Asn Ser Arg
               180                 185                 190
   Tyr Asn Asp Leu Thr Arg Leu Ile Gly Asn Tyr Thr Asp His Ala Val
           195                 200                 205
   Arg Trp Tyr Asn Thr Gly Leu Glu Arg Val Trp Gly Pro Asp Ser Arg
       210                 215                 220
   Asp Trp Ile Arg Tyr Asn Gln Phe Arg Arg Glu Leu Thr Leu Thr Val
   225                 230                 235                 240
   Leu Asp Ile Val Ser Leu Phe Pro Asn Tyr Asp Ser Arg Thr Tyr Pro
                   245                 250                 255
   Ile Arg Thr Val Ser Gln Leu Thr Arg Glu Ile Tyr Thr Asn Pro Val
               260                 265                 270
   Leu Glu Asn Phe Asp Gly Ser Phe Arg Gly Ser Ala Gln Gly Ile Glu
           275                 280                 285
   Gly Ser Ile Arg Ser Pro His Leu Met Asp Ile Leu Asn Ser Ile Thr
       290                 295                 300
   Ile Tyr Thr Asp Ala His Arg Gly Glu Tyr Tyr Trp Ser Gly His Gln
   305                 310                 315                 320
   Ile Met Ala Ser Pro Val Gly Phe Ser Gly Pro Glu Phe Thr Phe Pro
                   325                 330                 335
   Leu Tyr Gly Thr Met Gly Asn Ala Ala Pro Gln Gln Arg Ile Val Ala
               340                 345                 350
   Gln Leu Gly Gln Gly Val Tyr Arg Thr Leu Ser Ser Thr Leu Tyr Arg
           355                 360                 365
   Arg Pro Phe Asn Ile Gly Ile Asn Asn Gln Gln Leu Ser Val Leu Asp
       370                 375                 380
   Gly Thr Glu Phe Ala Tyr Gly Thr Ser Ser Asn Leu Pro Ser Ala Val
   385                 390                 395                 400
   Tyr Arg Lys Ser Gly Thr Val Asp Ser Leu Asp Glu Ile Pro Pro Gln
                   405                 410                 415
   Asn Asn Asn Val Pro Pro Arg Gln Gly Phe Ser His Arg Leu Ser His
               420                 425                 430
   Val Ser Met Phe Arg Ser Gly Phe Ser Asn Ser Ser Val Ser Ile Ile
           435                 440                 445
   Arg Ala Pro Met Phe Ser Trp Ile His Arg Ser Ala Thr Leu Thr Asn
       450                 455                 460
   Thr Ile Asp Pro Glu Arg Ile Asn Gln Ile Pro Leu Val Lys Gly Phe
   465                 470                 475                 480
   Arg Val Trp Gly Gly Thr Ser Val Ile Thr Gly Pro Gly Phe Thr Gly
                   485                 490                 495
   Gly Asp Ile Leu Arg Arg Asn Thr Phe Gly Asp Phe Val Ser Leu Gln
               500                 505                 510
   Val Asn Ile Asn Ser Pro Ile Thr Gln Arg Tyr Arg Leu Arg Phe Arg
           515                 520                 525
   Tyr Ala Ser Ser Arg Asp Ala Arg Val Ile Val Leu Thr Gly Ala Ala
       530                 535                 540
   Ser Thr Gly Val Gly Gly Gln Val Ser Val Asn Met Pro Leu Gln Lys
   545                 550                 555                 560
   Thr Met Glu Ile Gly Glu Asn Leu Thr Ser Arg Thr Phe Arg Tyr Thr
                   565                 570                 575
   Asp Phe Ser Asn Pro Phe Ser Phe Arg Ala Asn Pro Asp Ile Ile Gly
               580                 585                 590
   Ile Ser Glu Gln Pro Leu Phe Gly Ala Gly Ser Ile Ser Ser Gly Glu
           595                 600                 605
   Leu Tyr Ile Asp Lys Ile Glu Ile Ile Leu Ala Asp Ala Thr Phe Glu
       610                 615                 620
   Ala Glu Ser Asp Leu Glu Arg Ala Gln Lys Ala Val Asn Ala Leu Phe
   625                 630                 635                 640
   Thr Ser Ser Asn Gln Ile Gly Leu Lys Thr Asp Val Thr Asp Tyr His
                   645                 650                 655
   Ile Asp Gln Val Ser Asn Leu Val Asp Cys Leu Ser
               660                 665
<210> 11
   <211> 13269
   <212> DNA
   <213> Artificial Sequence
<220>
   <223> Description of Artificial Sequence: pNOV1308
<220>
   <221> misc_feature
   <222> (1)..(1896)
   <223> synthetic nucleotide sequence encoding the toxin portion of H04, without a tail
<220>
   <221> misc_feature
   <222> (2102)..(4083)
   <223> Zea mays ubiquitin promoter
<220>
   <221> misc_feature
   <222> (4180)..(5283)
   <223> PMI marker gene
<220>
   <221> misc_feature
   <222> (11247).. (12647)
   <223> Zm Ubi promoter
<400> 11
   atggacaaca accccaacat caacgagtgc atcccctaca actgcctgag caaccccgag 60
   gtggaggtgc tgggcggcga gcgcatcgag accggctaca cccccatcga catcagcctg 120
   agcctgaccc agttcctgct gagcgagttc gtgcccggcg ccggcttcgt gctgggcctg 180
   gtggacatca tctggggcat cttcggcccc agccagtggg acgccttcct ggtgcagatc 240
   gagcagttga taaaccaacg catagaggaa ttcgcccgca accaggccat cagccgcctg 300
   gagggcctga gcaacctgta ccaaatctac gccgagagct tccgcgagtg ggaggccgac 360
   cccaccaacc ccgccctgcg cgaggagatg cgcatccagt tcaacgacat gaacagcgcc 420
   ctgaccaccg ccatccccct gttcgccgtg cagaactacc aggtgcccct gctgagcgtg 480
   tacgtgcagg ccgccaacct gcacctgagc gtgctgcgcg acgtcagcgt gttcggccag 540
   cgctggggct tcgacgccgc caccatcaac agccgctaca acgacctgac ccgcctgatc 600
   ggcaactaca ccgaccacgc cgtgcgctgg tacaacaccg gcctggagcg cgtgtggggt 660
   cccgacagcc gcgactggat caggtacaac cagttccgcc gcgagctgac cctgaccgtg 720
   ctggacatcg tgagcctgtt ccccaactac gacagccgca cctaccccat ccgcaccgtg 780
   agccagctga cccgcgagat ttacaccaac cccgtgctgg agaacttcga cggcagcttc 840
   cgcggcagcg cccagggcat cgagggcagc atccgcagcc cccacctgat ggacatcctg 900
   aacagcatca ccatctacac cgacgcccac cgcggcgagt actactggag cggccaccag 960
   atcatggcca gccccgtcgg cttcagcggc cccgagttca ccttccccct gtacggcacc 1020
   atgggcaacg ctgcacctca gcagcgcatc gtggcacagc tgggccaggg agtgtaccgc 1080
   accctgagca gcaccctgta ccgtcgacct ttcaacatcg gcatcaacaa ccagcagctg 1140
   agcgtgctgg acggcaccga gttcgcctac ggcaccagca gcaacctgcc cagcgccgtg 1200
   taccgcaaga gcggcaccgt ggacagcctg gacgagatcc cccctcagaa caacaacgtg 1260
   ccacctcgac agggcttcag ccaccgtctg agccacgtga gcatgttccg cagtggcttc 1320
   agcaacagca gcgtgagcat catccgtgca cccatgttca gctggattca ccgcagcgcc 1380
   accctgacca acaccatcga ccccgagcgc atcaaccaga tccccctggt gaagggcttc 1440
   cgggtgtggg gcggcaccag cgtgatcacc ggccccggct tcaccggagg cgacatcctg 1500
   cgcagaaaca ccttcggcga cttcgtgagc ctgcaggtga acatcaacag ccccatcacc 1560
   cagcgttacc gcctgcgctt ccgctacgcc agcagccgcg acgcccgtgt gatcgtgctg 1620
   actggcgccg ctagcaccgg tgtgggcggt caggtgagcg tgaacatgcc cctgcagaag 1680
   actatggaga tcggcgagaa cctgactagt cgcaccttcc gctacaccga cttcagcaac 1740
   cccttcagct tccgcgccaa ccccgacatc atcggcatca gcgagcagcc cctgttcggt 1800
   gccggcagca tcagcagcgg cgagctgtac atcgacaaga tcgagatcat cctggccgac 1860
   gccaccttcg aggccgagag cgacctggag cgctaagatc tgttctgcac aaagtggagt 1920
   agtcagtcat cgatcaggaa ccagacacca gacttttatt catacagtga agtgaagtga 1980
   agtgcagtgc agtgagttgc tggtttttgt acaacttagt atgtatttgt atttgtaaaa 2040
   tacttctatc aataaaattt ctaattccta aaaccaaaat ccaggggtac cagcttgcat 2100
   gcctgcagtg cagcgtgacc cggtcgtgcc cctctctaga gataatgagc attgcatgtc 2160
   taagttataa aaaattacca catatttttt ttgtcacact tgtttgaagt gcagtttatc 2220
   tatctttata catatattta aactttactc tacgaataat ataatctata gtactacaat 2280
   aatatcagtg ttttagagaa tcatataaat gaacagttag acatggtcta aaggacaatt 2340
   gagtattttg acaacaggac tctacagttt tatcttttta gtgtgcatgt gttctccttt 2400
   ttttttgcaa atagcttcac ctatataata cttcatccat tttattagta catccattta 2460
   gggtttaggg ttaatggttt ttatagacta atttttttag tacatctatt ttattctatt 2520
   ttagcctcta aattaagaaa actaaaactc tattttagtt tttttattta ataatttaga 2580
   tataaaatag aataaaataa agtgactaaa aattaaacaa atacccttta agaaattaaa 2640
   aaaactaagg aaacattttt cttgtttcga gtagataatg ccagcctgtt aaacgccgtc 2700
   gacgagtcta acggacacca accagcgaac cagcagcgtc gcgtcgggcc aagcgaagca 2760
   gacggcacgg catctctgtc gctgcctctg gacccctctc gagagttccg ctccaccgtt 2820
   ggacttgctc cgctgtcggc atccagaaat tgcgtggcgg agcggcagac gtgagccggc 2880
   acggcaggcg gcctcctcct cctctcacgg caccggcagc tacgggggat tcctttccca 2940
   ccgctccttc gctttccctt cctcgcccgc cgtaataaat agacaccccc tccacaccct 3000
   ctttccccaa cctcgtgttg ttcggagcgc acacacacac aaccagatct cccccaaatc 3060
   cacccgtcgg cacctccgct tcaaggtacg ccgctcgtcc tccccccccc cccctctcta 3120
   ccttctctag atcggcgttc cggtccatgg ttagggcccg gtagttctac ttctgttcat 3180
   gtttgtgtta gatccgtgtt tgtgttagat ccgtgctgct agcgttcgta cacggatgcg 3240
   acctgtacgt cagacacgtt ctgattgcta acttgccagt gtttctcttt ggggaatcct 3300
   gggatggctc tagccgttcc gcagacggga tcgatttcat gatttttttt gtttcgttgc 3360
   atagggtttg gtttgccctt ttcctttatt tcaatatatg ccgtgcactt gtttgtcggg 3420
   tcatcttttc atgctttttt ttgtcttggt tgtgatgatg tggtctggtt gggcggtcgt 3480
   tctagatcgg agtagaattc tgtttcaaac tacctggtgg atttattaat tttggatctg 3540
   tatgtgtgtg ccatacatat tcatagttac gaattgaaga tgatggatgg aaatatcgat 3600
   ctaggatagg tatacatgtt gatgcgggtt ttactgatgc atatacagag atgctttttg 3660
   ttcgcttggt tgtgatgatg tggtgtggtt gggcggtcgt tcattcgttc tagatcggag 3720
   tagaatactg tttcaaacta cctggtgtat ttattaattt tggaactgta tgtgtgtgtc 3780
   atacatcttc atagttacga gtttaagatg gatggaaata tcgatctagg ataggtatac 3840
   atgttgatgt gggttttact gatgcatata catgatggca tatgcagcat ctattcatat 3900
   gctctaacct tgagtaccta tctattataa taaacaagta tgttttataa ttattttgat 3960
   cttgatatac ttggatgatg gcatatgcag cagctatatg tggatttttt tagccctgcc 4020
   ttcatacgct atttatttgc ttggtactgt ttcttttgtc gatgctcacc ctgttgtttg 4080
   gtgttacttc tgcagggatc cccgatcatg caaaaactca ttaactcagt gcaaaactat 4140
   gcctggggca gcaaaacggc gttgactgaa ctttatggta tggaaaatcc gtccagccag 4200
   ccgatggccg agctgtggat gggcgcacat ccgaaaagca gttcacgagt gcagaatgcc 4260
   gccggagata tcgtttcact gcgtgatgtg attgagagtg ataaatcgac tctgctcgga 4320
   gaggccgttg ccaaacgctt tggcgaactg cctttcctgt tcaaagtatt atgcgcagca 4380
   cagccactct ccattcaggt tcatccaaac aaacacaatt ctgaaatcgg ttttgccaaa 4440
   gaaaatgccg caggtatccc gatggatgcc gccgagcgta actataaaga tcctaaccac 4500
   aagccggagc tggtttttgc gctgacgcct ttccttgcga tgaacgcgtt tcgtgaattt 4560
   tccgagattg tctccctact ccagccggtc gcaggtgcac atccggcgat tgctcacttt 4620
   ttacaacagc ctgatgccga acgtttaagc gaactgttcg ccagcctgtt gaatatgcag 4680
   ggtgaagaaa aatcccgcgc gctggcgatt ttaaaatcgg ccctcgatag ccagcagggt 4740
   gaaccgtggc aaacgattcg tttaatttct gaattttacc cggaagacag cggtctgttc 4800
   tccccgctat tgctgaatgt ggtgaaattg aaccctggcg aagcgatgtt cctgttcgct 4860
   gaaacaccgc acgcttacct gcaaggcgtg gcgctggaag tgatggcaaa ctccgataac 4920
   gtgctgcgtg cgggtctgac gcctaaatac attgatattc cggaactggt tgccaatgtg 4980
   aaattcgaag ccaaaccggc taaccagttg ttgacccagc cggtgaaaca aggtgcagaa 5040
   ctggacttcc cgattccagt ggatgatttt gccttctcgc tgcatgacct tagtgataaa 5100
   gaaaccacca ttagccagca gagtgccgcc attttgttct gcgtcgaagg cgatgcaacg 5160
   ttgtggaaag gttctcagca gttacagctt aaaccgggtg aatcagcgtt tattgccgcc 5220
   aacgaatcac cggtgactgt caaaggccac ggccgtttag cgcgtgttta caacaagctg 5280
   taagagctta ctgaaaaaat taacatctct tgctaagctg ggagctcgat ccgtcgacct 5340
   gcagatcgtt caaacatttg gcaataaagt ttcttaagat tgaatcctgt tgccggtctt 5400
   gcgatgatta tcatataatt tctgttgaat tacgttaagc atgtaataat taacatgtaa 5460
   tgcatgacgt tatttatgag atgggttttt atgattagag tcccgcaatt atacatttaa 5520
   tacgcgatag aaaacaaaat atagcgcgca aactaggata aattatcgcg cgcggtgtca 5580
   tctatgttac tagatctgct agccctgcag gaaatttacc ggtgcccggg cggccagcat 5640
   ggccgtatcc gcaatgtgtt attaagttgt ctaagcgtca atttgtttac accacaatat 5700
   atcctgccac cagccagcca acagctcccc gaccggcagc tcggcacaaa atcaccactc 5760
   gatacaggca gcccatcaga attaattctc atgtttgaca gcttatcatc gactgcacgg 5820
   tgcaccaatg cttctggcgt caggcagcca tcggaagctg tggtatggct gtgcaggtcg 5880
   taaatcactg cataattcgt gtcgctcaag gcgcactccc gttctggata atgttttttg 5940
   cgccgacatc ataacggttc tggcaaatat tctgaaatga gctgttgaca attaatcatc 6000
   cggctcgtat aatgtgtgga attgtgagcg gataacaatt tcacacagga aacagaccat 6060
   gagggaagcg ttgatcgccg aagtatcgac tcaactatca gaggtagttg gcgtcatcga 6120
   gcgccatctc gaaccgacgt tgctggccgt acatttgtac ggctccgcag tggatggcgg 6180
   cctgaagcca cacagtgata ttgatttgct ggttacggtg accgtaaggc ttgatgaaac 6240
   aacgcggcga gctttgatca acgacctttt ggaaacttcg gcttcccctg gagagagcga 6300
   gattctccgc gctgtagaag tcaccattgt tgtgcacgac gacatcattc cgtggcgtta 6360
   tccagctaag cgcgaactgc aatttggaga atggcagcgc aatgacattc ttgcaggtat 6420
   cttcgagcca gccacgatcg acattgatct ggctatcttg ctgacaaaag caagagaaca 6480
   tagcgttgcc ttggtaggtc cagcggcgga ggaactcttt gatccggttc ctgaacagga 6540
   tctatttgag gcgctaaatg aaaccttaac gctatggaac tcgccgcccg actgggctgg 6600
   cgatgagcga aatgtagtgc ttacgttgtc ccgcatttgg tacagcgcag taaccggcaa 6660
   aatcgcgccg aaggatgtcg ctgccgactg ggcaatggag cgcctgccgg cccagtatca 6720
   gcccgtcata cttgaagcta ggcaggctta tcttggacaa gaagatcgct tggcctcgcg 6780
   cgcagatcag ttggaagaat ttgttcacta cgtgaaaggc gagatcacca aagtagtcgg 6840
   caaataaagc tctagtggat ctccgtaccc ccgggggatc tggctcgcgg cggacgcacg 6900
   acgccggggc gagaccatag gcgatctcct aaatcaatag tagctgtaac ctcgaagcgt 6960
   ttcacttgta acaacgattg agaatttttg tcataaaatt gaaatacttg gttcgcattt 7020
   ttgtcatccg cggtcagccg caattctgac gaactgccca tttagctgga gatgattgta 7080
   catccttcac gtgaaaattt ctcaagcgct gtgaacaagg gttcagattt tagattgaaa 7140
   ggtgagccgt tgaaacacgt tcttcttgtc gatgacgacg tcgctatgcg gcatcttatt 7200
   attgaatacc ttacgatcca cgccttcaaa gtgaccgcgg tagccgacag cacccagttc 7260
   acaagagtac tctcttccgc gacggtcgat gtcgtggttg ttgatctaaa tttaggtcgt 7320
   gaagatgggc tcgagatcgt tcgtaatctg gcggcaaagt ctgatattcc aatcataatt 7380
   atcagtggcg accgccttga ggagacggat aaagttgttg cactcgagct aggagcaagt 7440
   gattttatcg ctaagccgtt cagtatcaga gagtttctag cacgcattcg ggttgccttg 7500
   cgcgtgcgcc ccaacgttgt ccgctccaaa gaccgacggt ctttttgttt tactgactgg 7560
   acacttaatc tcaggcaacg tcgcttgatg tccgaagctg gcggtgaggt gaaacttacg 7620
   gcaggtgagt tcaatcttct cctcgcgttt ttagagaaac cccgcgacgt tctatcgcgc 7680
   gagcaacttc tcattgccag tcgagtacgc gacgaggagg tttatgacag gagtatagat 7740
   gttctcattt tgaggctgcg ccgcaaactt gaggcagatc cgtcaagccc tcaactgata 7800
   aaaacagcaa gaggtgccgg ttatttcttt gacgcggacg tgcaggtttc gcacgggggg 7860
   acgatggcag cctgagccaa ttcccagatc cccgaggaat cggcgtgagc ggtcgcaaac 7920
   catccggccc ggtacaaatc ggcgcggcgc tgggtgatga cctggtggag aagttgaagg 7980
   ccgcgcaggc cgcccagcgg caacgcatcg aggcagaagc acgccccggt gaatcgtggc 8040
   aagcggccgc tgatcgaatc cgcaaagaat cccggcaacc gccggcagcc ggtgcgccgt 8100
   cgattaggaa gccgcccaag ggcgacgagc aaccagattt tttcgttccg atgctctatg 8160
   acgtgggcac ccgcgatagt cgcagcatca tggacgtggc cgttttccgt ctgtcgaagc 8220
   gtgaccgacg agctggcgag gtgatccgct acgagcttcc agacgggcac gtagaggttt 8280
   ccgcagggcc ggccggcatg gccagtgtgt gggattacga cctggtactg atggcggttt 8340
   cccatctaac cgaatccatg aaccgatacc gggaagggaa gggagacaag cccggccgcg 8400
   tgttccgtcc acacgttgcg gacgtactca agttctgccg gcgagccgat ggcggaaagc 8460
   agaaagacga cctggtagaa acctgcattc ggttaaacac cacgcacgtt gccatgcagc 8520
   gtacgaagaa ggccaagaac ggccgcctgg tgacggtatc cgagggtgaa gccttgatta 8580
   gccgctacaa gatcgtaaag agcgaaaccg ggcggccgga gtacatcgag atcgagctag 8640
   ctgattggat gtaccgcgag atcacagaag gcaagaaccc ggacgtgctg acggttcacc 8700
   ccgattactt tttgatcgat cccggcatcg gccgttttct ctaccgcctg gcacgccgcg 8760
   ccgcaggcaa ggcagaagcc agatggttgt tcaagacgat ctacgaacgc agtggcagcg 8820
   ccggagagtt caagaagttc tgtttcaccg tgcgcaagct gatcgggtca aatgacctgc 8880
   cggagtacga tttgaaggag gaggcggggc aggctggccc gatcctagtc atgcgctacc 8940
   gcaacctgat cgagggcgaa gcatccgccg gttcctaatg tacggagcag atgctagggc 9000
   aaattgccct agcaggggaa aaaggtcgaa aaggtctctt tcctgtggat agcacgtaca 9060
   ttgggaaccc aaagccgtac attgggaacc ggaacccgta cattgggaac ccaaagccgt 9120
   acattgggaa ccggtcacac atgtaagtga ctgatataaa agagaaaaaa ggcgattttt 9180
   ccgcctaaaa ctctttaaaa cttattaaaa ctcttaaaac ccgcctggcc tgtgcataac 9240
   tgtctggcca gcgcacagcc gaagagctgc aaaaagcgcc tacccttcgg tcgctgcgct 9300
   ccctacgccc cgccgcttcg cgtcggccta tcgcggccgc tggccgctca aaaatggctg 9360
   gcctacggcc aggcaatcta ccagggcgcg gacaagccgc gccgtcgcca ctcgaccgcc 9420
   ggcgctgagg tctgcctcgt gaagaaggtg ttgctgactc ataccaggcc tgaatcgccc 9480
   catcatccag ccagaaagtg agggagccac ggttgatgag agctttgttg taggtggacc 9540
   agttggtgat tttgaacttt tgctttgcca cggaacggtc tgcgttgtcg ggaagatgcg 9600
   tgatctgatc cttcaactca gcaaaagttc gatttattca acaaagccgc cgtcccgtca 9660
   agtcagcgta atgctctgcc agtgttacaa ccaattaacc aattctgatt agaaaaactc 9720
   atcgagcatc aaatgaaact gcaatttatt catatcagga ttatcaatac catatttttg 9780
   aaaaagccgt ttctgtaatg aaggagaaaa ctcaccgagg cagttccata ggatggcaag 9840
   atcctggtat cggtctgcga ttccgactcg tccaacatca atacaaccta ttaatttccc 9900
   ctcgtcaaaa ataaggttat caagtgagaa atcaccatga gtgacgactg aatccggtga 9960
   gaatggcaaa agctctgcat taatgaatcg gccaacgcgc ggggagaggc ggtttgcgta 10020
   ttgggcgctc ttccgcttcc tcgctcactg actcgctgcg ctcggtcgtt cggctgcggc 10080
   gagcggtatc agctcactca aaggcggtaa tacggttatc cacagaatca ggggataacg 10140
   caggaaagaa catgtgagca aaaggccagc aaaaggccag gaaccgtaaa aaggccgcgt 10200
   tgctggcgtt tttccatagg ctccgccccc ctgacgagca tcacaaaaat cgacgctcaa 10260
   gtcagaggtg gcgaaacccg acaggactat aaagatacca ggcgtttccc cctggaagct 10320
   ccctcgtgcg ctctcctgtt ccgaccctgc cgcttaccgg atacctgtcc gcctttctcc 10380
   cttcgggaag cgtggcgctt tctcatagct cacgctgtag gtatctcagt tcggtgtagg 10440
   tcgttcgctc caagctgggc tgtgtgcacg aaccccccgt tcagcccgac cgctgcgcct 10500
   tatccggtaa ctatcgtctt gagtccaacc cggtaagaca cgacttatcg ccactggcag 10560
   cagccactgg taacaggatt agcagagcga ggtatgtagg cggtgctaca gagttcttga 10620
   agtggtggcc taactacggc tacactagaa gaacagtatt tggtatctgc gctctgctga 10680
   agccagttac cttcggaaaa agagttggta gctcttgatc cggcaaacaa accaccgctg 10740
   gtagcggtgg tttttttgtt tgcaagcagc agattacgcg cagaaaaaaa ggatctcaag 10800
   aagatccttt gatcttttct acggggtctg acgctcagtg gaacgaaaac tcacgttaag 10860
   ggattttggt catgagatta tcaaaaagga tcttcaccta gatccttttg atccggaatt 10920
   aattcctgtg gttggcatgc acatacaaat ggacgaacgg ataaaccttt tcacgccctt 10980
   ttaaatatcc gattattcta ataaacgctc ttttctctta ggtttacccg ccaatatatc 11040
   ctgtcaaaca ctgatagttt aaactgaagg cgggaaacga caatctgatc atgagcggag 11100
   aattaaggga gtcacgttat gacccccgcc gatgacgcgg gacaagccgt tttacgtttg 11160
   gaactgacag aaccgcaacg ctgcaggaat tggccgcagc ggccatttaa atcaattggg 11220
   cgcgccgaat tcgagctcgg tacaagcttg catgcctgca gtgcagcgtg acccggtcgt 11280
   gcccctctct agagataatg agcattgcat gtctaagtta taaaaaatta ccacatattt 11340
   tttttgtcac acttgtttga agtgcagttt atctatcttt atacatatat ttaaacttta 11400
   ctctacgaat aatataatct atagtactac aataatatca gtgttttaga gaatcatata 11460
   aatgaacagt tagacatggt ctaaaggaca attgagtatt ttgacaacag gactctacag 11520
   ttttatcttt ttagtgtgca tgtgttctcc tttttttttg caaatagctt cacctatata 11580
   atacttcatc cattttatta gtacatccat ttagggttta gggttaatgg tttttataga 11640
   ctaatttttt tagtacatct attttattct attttagcct ctaaattaag aaaactaaaa 11700
   ctctatttta gtttttttat ttaataattt agatataaaa tagaataaaa taaagtgact 11760
   aaaaattaaa caaataccct ttaagaaatt aaaaaaacta aggaaacatt tttcttgttt 11820
   cgagtagata atgccagcct gttaaacgcc gtcgacgagt ctaacggaca ccaaccagcg 11880
   aaccagcagc gtcgcgtcgg gccaagcgaa gcagacggca cggcatctct gtcgctgcct 11940
   ctggacccct ctcgagagtt ccgctccacc gttggacttg ctccgctgtc ggcatccaga 12000
   aattgcgtgg cggagcggca gacgtgagcc ggcacggcag gcggcctcct cctcctctca 12060
   cggcacggca gctacggggg attcctttcc caccgctcct tcgctttccc ttcctcgccc 12120
   gccgtaataa atagacaccc cctccacacc ctctttcccc aacctcgtgt tgttcggagc 12180
   gcacacacac acaaccagat ctcccccaaa tccacccgtc ggcacctccg cttcaaggta 12240
   cgccgctcgt cctccccccc cccccctctc taccttctct agatcggcgt tccggtccat 12300
   ggttagggcc cggtagttct acttctgttc atgtttgtgt tagatccgtg tttgtgttag 12360
   atccgtgctg ctagcgttcg tacacggatg cgacctgtac gtcagacacg ttctgattgc 12420
   taacttgcca gtgtttctct ttggggaatc ctgggatggc tctagccgtt ccgcagacgg 12480
   gatcgatttc atgatttttt ttgtttcgtt gcatagggtt tggtttgccc ttttccttta 12540
   tttcaatata tgccgtgcac ttgtttgtcg ggtcatcttt tcatgctttt ttttgtcttg 12600
   gttgtgatga tgtggtctgg ttgggcggtc gttctagatc ggagtagaat tctgtttcaa 12660
   actacctggt ggatttatta attttggatc tgtatgtgtg tgccatacat attcatagtt 12720
   acgaattgaa gatgatggat ggaaatatcg atctaggata ggtatacatg ttgatgcggg 12780
   ttttactgat gcatatacag agatgctttt tgttcgcttg gttgtgatga tgtggtgtgg 12840
   ttgggcggtc gttcattcgt tctagatcgg agtagaatac tgtttcaaac tacctggtgt 12900
   atttattaat tttggaactg tatgtgtgtg tcatacatct tcatagttac gagtttaaga 12960
   tggatggaaa tatcgatcta ggataggtat acatgttgat gtgggtttta ctgatgcata 13020
   tacatgatgg catatgcagc atctattcat atgctctaac cttgagtacc tatctattat 13080
   aataaacaag tatgttttat aattattttg atcttgatat acttggatga tggcatatgc 13140
   agcagctata tgtggatttt tttagccctg ccttcatacg ctatttattt gcttggtact 13200
   gtttcttttg tcgatgctca ccctgttgtt tggtgttact tctgcaggtc gactctagag 13260
   gatccaaca                                                         13269
<210> 12
   <211> 16179
   <212> DNA
   <213> Artificial Sequence
<220>
   <223> Description of Artificial Sequence: pNOV1436
<220>
   <221> misc_feature
   <222> (1)..(3582)
   <223> synthetic nucleotide sequence encoding the toxin portion of H04 plus a full-length Cry1Ab tail portion
<220>
   <221> misc_feature
   <222> Complement((10390)..(21598))
   <223> PhosphoMannose Isomerase (PMI) marker gene
<220>
   <221> misc_feature
   <222> Complement((12718)..(13608))
   <223> Maize ubiquitin (Zm Ubi) promoter
<220>
   <221> misc_feature
   <222> (13613)..(16170)
   <223> MTL promoter
<400> 12
   atggacaaca accccaacat caacgagtgc atcccctaca actgcctgag caaccccgag 60
   gtggaggtgc tgggcggcga gcgcatcgag accggctaca cccccatcga catcagcctg 120
   agcctgaccc agttcctgct gagcgagttc gtgcccggcg ccggcttcgt gctgggcctg 180
   gtggacatca tctggggcat cttcggcccc agccagtggg acgccttcct ggtgcagatc 240
   gagcagttga taaaccaacg catagaggaa ttcgcccgca accaggccat cagccgcctg 300
   gagggcctga gcaacctgta ccaaatctac gccgagagct tccgcgagtg ggaggccgac 360
   cccaccaacc ccgccctgcg cgaggagatg cgcatccagt tcaacgacat gaacagcgcc 420
   ctgaccaccg ccatccccct gttcgccgtg cagaactacc aggtgcccct gctgagcgtg 480
   tacgtgcagg ccgccaacct gcacctgagc gtgctgcgcg acgtcagcgt gttcggccag 540
   cgctggggct tcgacgccgc caccatcaac agccgctaca acgacctgac ccgcctgatc 600
   ggcaactaca ccgaccacgc cgtgcgctgg tacaacaccg gcctggagcg cgtgtggggt 660
   cccgacagcc gcgactggat caggtacaac cagttccgcc gcgagctgac cctgaccgtg 720
   ctggacatcg tgagcctgtt ccccaactac gacagccgca cctaccccat ccgcaccgtg 780
   agccagctga cccgcgagat ttacaccaac cccgtgctgg agaacttcga cggcagcttc 840
   cgcggcagcg cccagggcat cgagggcagc atccgcagcc cccacctgat ggacatcctg 900
   aacagcatca ccatctacac cgacgcccac cgcggcgagt actactggag cggccaccag 960
   atcatggcca gccccgtcgg cttcagcggc cccgagttca ccttccccct gtacggcacc 1020
   atgggcaacg ctgcacctca gcagcgcatc gtggcacagc tgggccaggg agtgtaccgc 1080
   accctgagca gcaccctgta ccgtcgacct ttcaacatcg gcatcaacaa ccagcagctg 1140
   agcgtgctgg acggcaccga gttcgcctac ggcaccagca gcaacctgcc cagcgccgtg 1200
   taccgcaaga gcggcaccgt ggacagcctg gacgagatcc cccctcagaa caacaacgtg 1260
   ccacctcgac agggcttcag ccaccgtctg agccacgtga gcatgttccg cagtggcttc 1320
   agcaacagca gcgtgagcat catccgtgca cccatgttca gctggattca ccgcagcgcc 1380
   accctgacca acaccatcga ccccgagcgc atcaaccaga tccccctggt gaagggcttc 1440
   cgggtgtggg gcggcaccag cgtgatcacc ggccccggct tcaccggagg cgacatcctg 1500
   cgcagaaaca ccttcggcga cttcgtgagc ctgcaggtga acatcaacag ccccatcacc 1560
   cagcgttacc gcctgcgctt ccgctacgcc agcagccgcg acgcccgtgt gatcgtgctg 1620
   actggcgccg ctagcaccgg tgtgggcggt caggtgagcg tgaacatgcc cctgcagaag 1680
   actatggaga tcggcgagaa cctgactagt cgcaccttcc gctacaccga cttcagcaac 1740
   cccttcagct tccgcgccaa ccccgacatc atcggcatca gcgagcagcc cctgttcggt 1800
   gccggcagca tcagcagcgg cgagctgtac atcgacaaga tcgagatcat cctggccgac 1860
   gccaccttcg aggccgagag cgacctggag cgcgcccaga aggccgtgaa cgccctgttc 1920
   accagcagca accagatcgg cctgaagacc gacgtgaccg actaccacat cgaccaggtg 1980
   agcaacctgg tggactgctt aagcgacgag ttctgcctgg acgagaagaa ggagctgagc 2040
   gagaaggtga agcacgccaa gcgcctgagc gacgagcgca acctgctgca ggaccccaac 2100
   ttccgcggca tcaaccgcca gctggaccgc ggctggcgag gcagcaccga tatcaccatc 2160
   cagggcggcg acgacgtgtt caaggagaac tacgtgaccc tgcagggcac cttcgacgag 2220
   tgctacccca cctacctgta ccagccgatc gacgagagca agctgaaggc ctacacccgc 2280
   taccagctgc gcggctacat cgaggacagc caggacctgg aaatctacct gatccgctac 2340
   aacgcgaagc acgagaccgt gaacgtgccc ggcaccggca gcctgtggcc cccgagcgcc 2400
   cccagcccca tcggcaagtg cggggagccg aatcgatgcg ctccgcacct ggagtggaac 2460
   ccggacctag actgcagctg cagggacggg gagaagtgcg cccaccacag ccaccacttc 2520
   agcctggaca tcgacgtggg ctgcaccgac ctgaacgagg acctgggcgt gtgggtgatc 2580
   ttcaagatca agacccagga cggccacgcc cgcctgggca atctagagtt cctggaggag 2640
   aagcccctgg tgggcgaggc cctggcccgc gtgaagcgtg ctgagaagaa gtggcgcgac 2700
   aagcgcgaga agctggagtg ggagaccaac atcgtgtaca aggaggccaa ggagagcgtg 2760
   gacgccctgt tcgtgaacag ccagtacgac cgcctgcagg ccgacaccaa catcgccatg 2820
   atccacgccg ccgacaagcg cgtgcacagc attcgcgagg cctacctgcc cgagctgagc 2880
   gtgatccccg gtgtgaacgc cgccatcttc gaggaactcg agggccgcat cttcaccgcc 2940
   ttcagcctgt acgacgcccg caacgtgatc aagaacggcg acttcaacaa cggcctgagc 3000
   tgctggaacg tgaagggcca cgtggacgtg gaggagcaga acaaccaccg cagcgtgctg 3060
   gtggtgcccg agtgggaggc cgaggtgagc caggaggtgc gcgtgtgccc cggccgcggc 3120
   tacatcctgc gcgtgaccgc ctacaaggag ggctacggcg agggctgcgt gaccatccac 3180
   gagatcgaga acaacaccga cgagctcaag ttcagcaact gcgtggagga ggaggtttac 3240
   cccaacaaca ccgtgacctg caacgactac accgcgaccc aggaggagta cgaaggcacc 3300
   tacacctctc gcaacagggg ttacgacggc gcctacgagt ccaacagctc cgtgccagct 3360
   gactacgcca gcgcccacga ggagaaagcc tacaccgacg gtagacgcga caacccatgt 3420
   gagagcaaca gaggctacgg cgactacacc cccctgcccg ctggatacgt gaccaaggag 3480
   ctggagtact tccccgagac cgacaaggtg tggatcgaga ttggcgagac cgagggcacc 3540
   ttcatcgtgg acagcgtgga gctgctgctg atggaggagt agtagatctg ttctgcacaa 3600
   agtggagtag tcagtcatcg atcaggaacc agacaccaga cttttattca tacagtgaag 3660
   tgaagtgaag tgcagtgcag tgagttgctg gtttttgtac cacttagtat gtatttgtat 3720
   ttgtaaaata cttctatcaa taaaatttct aattcctaaa accaaaatcc agtgggtacc 3780
   agcttgggct gagtggctcc ttcaacgttg cggttctgtc agttccaaac gtaaaacggc 3840
   ttgtcccgcg tcatcggcgg gggtcataac gtgactccct taattctccg ctcatgatca 3900
   gattgtcgtt tcccgccttc agtttaaact atcagtgttt gacaggatat attggcgggt 3960
   aaacctaaga gaaaagagcg tttattagaa taacggatat ttaaaagggc gtgaaaaggt 4020
   ttatccgttc gtccatttgt atgtgcatgc caaccacagg gttcccctcg ggagtgcttg 4080
   gcattccgta cgataatgac ttctgttcaa ccacccaaac gtcggaaagc ctgacgacgg 4140
   agcagcattc caaaaagatc ccttggctcg tctgggtcgg ctagaaggtc gagtgggctg 4200
   ctgtggcttg atccctcaac gcggtcgcgg acgtagcgca gcgccgaaaa atcctcgatc 4260
   gcaaatccga cgctgtcgaa aagcgtgatc tgcttgtcgc tctttcggcc gacgtcctgg 4320
   ccagtcatca cgcgccaaag ttccgtcaca ggatgatctg gcgcgagttg ctggatctcg 4380
   ccttcaatcc gggtctgtgg cgggaactcc acgaaaatat ccgaacgcag caagatcgtc 4440
   gaccaattct tgaagacgaa agggcctcgt gatacgccta tttttatagg ttaatgtcat 4500
   gataataatg gtttcttaga cgtcaggtgg cacttttcgg ggaaatgtgc gcggaacccc 4560
   tatttgttta tttttctaaa tacattcaaa tatgtatccg ctcatgagac aataaccctg 4620
   ataaatgctt caataatatt gaaaaaggaa gagtatgagt attcaacatt tccgtgtcgc 4680
   ccttattccc ttttttgcgg cattttgcct tcctgttttt gctcacccag aaacgctggt 4740
   gaaagtaaaa gatgctgaag atcagttggg tgcacgagtg ggttacatcg aactggatct 4800
   caacagcggt aagatccttg agagttttcg ccccgaagaa cgttttccaa tgatgagcac 4860
   ttttaaagtt ctgctatgtg gcgcggtatt atcccgtgtt gacgccgggc aagagcaact 4920
   cggtcgccgc atacactatt ctcagaatga cttggttgag tactcaccag tcacagaaaa 4980
   gcatcttacg gatggcatga cagtaagaga attatgcagt gctgccataa ccatgagtga 5040
   taacactgcg gccaacttac ttctgacaac gatcggagga ccgaaggagc taaccgcttt 5100
   tttgcacaac atgggggatc atgtaactcg ccttgatcgt tgggaaccgg agctgaatga 5160
   agccatacca aacgacgagc gtgacaccac gatgcctgca gggggggggg ggggggggac 5220
   atgaggttgc cccgtattca gtgtcgctga tttgtattgt ctgaagttgt ttttacgtta 5280
   agttgatgca gatcaattaa tacgatacct gcgtcataat tgattatttg acgtggtttg 5340
   atggcctcca cgcacgttgt gatatgtaga tgataatcat tatcacttta cgggtccttt 5400
   ccggtgatcc gacaggttac ggggcggcga cctcgcgggt tttcgctatt tatgaaaatt 5460
   ttccggttta aggcgtttcc gttcttcttc gtcataactt aatgttttta tttaaaatac 5520
   cctctgaaaa gaaaggaaac gacaggtgct gaaagcgagg ctttttggcc tctgtcgttt 5580
   cctttctctg tttttgtccg tggaatgaac aatggaagtc cccccccccc cccccccctg 5640
   cagcaatggc aacaacgttg cgcaaactat taactggcga actacttact ctagcttccc 5700
   ggcaacaatt aatagactgg atggaggcgg ataaagttgc aggaccactt ctgcgctcgg 5760
   cccttccggc tggctggttt attgctgata aatctggagc cggtgagcgt gggtctcgcg 5820
   gtatcattgc agcactgggg ccagatggta agccctcccg tatcgtagtt atctacacga 5880
   cggggagtca ggcaactatg gatgaacgaa atagacagat cgctgagata ggtgcctcac 5940
   tgattaagca ttggtaactg tcagaccaag tttactcata tatactttag attgatttaa 6000
   aacttcattt ttaatttaaa aggatctagg tgaagatcct ttttgataat ctcatgacca 6060
   aaatccctta acgtgagttt tcgttccact gagcgtcaga ccccgtagaa aagatcaaag 6120
   gatcttcttg agatcctttt tttctgcgcg taatctgctg cttgcaaaca aaaaaaccac 6180
   cgctaccagc ggtggtttgt ttgccggatc aagagctacc aactcttttt ccgaaggtaa 6240
   ctggcttcag cagagcgcag ataccaaata ctgtccttct agtgtagccg tagttaggcc 6300
   accacttcaa gaactctgta gcaccgccta catacctcgc tctgctaatc ctgttaccag 6360
   tggctgctgc cagtggcgat aagtcgtgtc ttaccgggtt ggactcaaga cgatagttac 6420
   cggataaggc gcagcggtcg ggctgaacgg ggggttcgtg cacacagccc agcttggagc 6480
   gaacgaccta caccgaactg agatacctac agcgtgagct atgagaaagc gccacgcttc 6540
   ccgaagggag aaaggcggac aggtatccgg taagcggcag ggtcggaaca ggagagcgca 6600
   cgagggagct tccaggggga aacgcctggt atctttatag tcctgtcggg tttcgccacc 6660
   tctgacttga gcgtcgattt ttgtgatgct cgtcaggggg gcggagccta tggaaaaacg 6720
   ccagcaacgc ggccttttta cggttcctgg ccttttgctg gccttttgct cacatgttct 6780
   ttcctgcgtt atcccctgat tctgtggata accgtattac cgcctttgag tgagctgata 6840
   ccgctcgccg cagccgaacg accgagcgca gcgagtcagt gagcgaggaa gcggaagagc 6900
   gcctgatgcg gtattttctc cttacgcatc tgtgcggtat ttcacaccgc atatggtgca 6960
   ctctcagtac aatctgctct gatgccgcat agttaagcca gtatacactc cgctatcgct 7020
   acgtgactgg gtcatggctg cgccccgaca cccgccaaca cccgctgacg cgccctgacg 7080
   ggcttgtctg ctcccggcat ccgcttacag acaagctgtg accgtctccg ggagctgcat 7140
   gtgtcagagg ttttcaccgt catcaccgaa acgcgcgagg cagcagatcc cccgatcaag 7200
   tagatacact acatatatct acaatagaca tcgagccgga aggtgatgtt tactttcctg 7260
   aaatccccag caattttagg ccagttttta cccaagactt cgcctctaac ataaattata 7320
   gttaccaaat ctggcaaaag ggttaacaag tggcagcaac ggattcgcaa acctgtcacg 7380
   ccttttgtgc caaaagccgc gccaggtttg cgatccgctg tgccaggcgt taggcgtcat 7440
   atgaagattt cggtgatccc tgagcaggtg gcggaaacat tggatgctga gaaccatttc 7500
   attgttcgtg aagtgttcga tgtgcaccta tccgaccaag gctttgaact atctaccaga 7560
   agtgtgagcc cctaccggaa ggattacatc tcggatgatg actctgatga agactctgct 7620
   tgctatggcg cattcatcga ccaagagctt gtcgggaaga ttgaactcaa ctcaacatgg 7680
   aacgatctag cctctatcga acacattgtt gtgtcgcaca cgcaccgagg caaaggagtc 7740
   gcgcacagtc tcatcgaatt tgcgaaaaag tgggcactaa gcagacagct ccttggcata 7800
   cgattagaga cacaaacgaa caatgtacct gcctgcaatt tgtacgcaaa atgtggcttt 7860
   actctcggcg gcattgacct gttcacgtat aaaactagac ctcaagtctc gaacgaaaca 7920
   gcgatgtact ggtactggtt ctcgggagca caggatgacg cctaacaatt cattcaagcc 7980
   gacaccgctt cgcggcgcgg cttaattcag gagttaaaca tcatgaggga agcggtgatc 8040
   gccgaagtat cgactcaact atcagaggta gttggcgtca tcgagcgcca tctcgaaccg 8100
   acgttgctgg ccgtacattt gtacggctcc gcagtggatg gcggcctgaa gccacacagt 8160
   gatattgatt tgctggttac ggtgaccgta aggcttgatg aaacaacgcg gcgagctttg 8220
   atcaacgacc ttttggaaac ttcggcttcc cctggagaga gcgagattct ccgcgctgta 8280
   gaagtcacca ttgttgtgca cgacgacatc attccgtggc gttatccagc taagcgcgaa 8340
   ctgcaatttg gagaatggca gcgcaatgac attcttgcag gtatcttcga gccagccacg 8400
   atcgacattg atctggctat cttgctgaca aaagcaagag aacatagcgt tgccttggta 8460
   ggtccagcgg cggaggaact ctttgatccg gttcctgaac aggatctatt tgaggcgcta 8520
   aatgaaacct taacgctatg gaactcgccg cccgactggg ctggcgatga gcgaaatgta 8580
   gtgcttacgt tgtcccgcat ttggtacagc gcagtaaccg gcaaaatcgc gccgaaggat 8640
   gtcgctgccg actgggcaat ggagcgcctg ccggcccagt atcagcccgt catacttgaa 8700
   gctaggcagg cttatcttgg acaagaagat cgcttggcct cgcgcgcaga tcagttggaa 8760
   gaatttgttc actacgtgaa aggcgagatc accaaggtag tcggcaaata atgtctaaca 8820
   attcgttcaa gccgacgccg cttcgcggcg cggcttaact caagcgttag agagctgggg 8880
   aagactatgc gcgatctgtt gaaggtggtt ctaagcctcg tacttgcgat ggcatcgggg 8940
   caggcacttg ctgacctgcc aattgtttta gtggatgaag ctcgtcttcc ctatgactac 9000
   tccccatcca actacgacat ttctccaagc aactacgaca actccataag caattacgac 9060
   aatagtccat caaattacga caactctgag agcaactacg ataatagttc atccaattac 9120
   gacaatagtc gcaacggaaa tcgtaggctt atatatagcg caaatgggtc tcgcactttc 9180
   gccggctact acgtcattgc caacaatggg acaacgaact tcttttccac atctggcaaa 9240
   aggatgttct acaccccaaa aggggggcgc ggcgtctatg gcggcaaaga tgggagcttc 9300
   tgcggggcat tggtcgtcat aaatggccaa ttttcgcttg ccctgacaga taacggcctg 9360
   aagatcatgt atctaagcaa ctagcctgct ctctaataaa atgttaggcc tcaacatcta 9420
   gtcgcaagct gaggggaacc actagtgtca tacgaacctc caagagacgg ttacacaaac 9480
   gggtacattg ttgatgtcat gtatgacaat cgcccaagta agtatccagc tgtgttcaga 9540
   acgtacgtcc gaattaattc atcggggtac ggtcgacgat cgtcaacgtt cacttctaaa 9600
   gaaatagcgc cactcagctt cctcagcggc tttatccagc gatttcctat tatgtcggca 9660
   tagttctcaa gatcgacagc ctgtcacggt taagcgagaa atgaataaga aggctgataa 9720
   ttcggatctc tgcgagggag atgatatttg atcacaggca gcaacgctct gtcatcgtta 9780
   caatcaacat gctaccctcc gcgagatcat ccgtgtttca aacccggcag cttagttgcc 9840
   gttcttccga atagcatcgg taacatgagc aaagtctgcc gccttacaac ggctctcccg 9900
   ctgacgccgt cccggactga tgggctgcct gtatcgagtg gtgattttgt gccgagctgc 9960
   cggtcgggga gctgttggct ggctggtggc aggatatatt gtggtgtaaa caaattgacg 10020
   cttagacaac ttaataacac attgcggacg tttttaatgt actgaattgt ctagacccgg 10080
   ggatctcatg tttgacagct tatcatcgga tctagtaaca tagatgacac cgcgcgcgat 10140
   aatttatcct agtttgcgcg ctatattttg ttttctatcg cgtattaaat gtataattgc 10200
   gggactctaa tcataaaaac ccatctcata aataacgtca tgcattacat gttaattatt 10260
   acatgcttaa cgtaattcaa cagaaattag atgataatca tcgcaagacc ggcaacagga 10320
   ttcaatctta agaaacttta ttgccaaatg tttgaacgat ctctgcaggt cgacggatcg 10380
   agctcccagc ttagcaagag atgttaattt tttcagtaag ctcttacagc ttgttgtaaa 10440
   cacgcgctaa acggccgtgg cctttgacag tcaccggtga ttcgttggcg gcaataaacg 10500
   ctgattcacc cggtttaagc tgtaactgct gagaaccttt ccacaacgtt gcatcgcctt 10560
   cgacgcagaa caaaatggcg gcactctgct ggctaatggt ggtttcttta tcactaaggt 10620
   catgcagcga gaaggcaaaa tcatccactg gaatcgggaa gtccagttct gcaccttgtt 10680
   tcaccggctg ggtcaacaac tggttagccg gtttggcttc gaatttcaca ttggcaacca 10740
   gttccggaat atcaatgtat ttaggcgtca gacccgcacg cagcacgtta tcggagtttg 10800
   ccatcacttc cagcgccacg ccttgcaggt aagcgtgcgg tgtttcagcg aacaggaaca 10860
   tcgcttcgcc agggttcaat ttcaccacat tcagcaatag cggggagaac agaccgctgt 10920
   cttccgggta aaattcagaa attaaacgaa tcgtttgcca cggttcaccc tgctggctat 10980
   cgagggccga ttttaaaatc gccagcgcgc gggatttttc ttcaccctgc atattcaaca 11040
   ggctggcgaa cagttcgctt aaacgttcgg catcaggctg ttgtaaaaag tgagcaatcg 11100
   ccggatgtgc acctgcgacc ggctggagta gggagacaat ctcggaaaat tcacgaaacg 11160
   cgttcatcgc aaggaaaggc gtcagcgcaa aaaccagctc cggcttgtgg ttaggatctt 11220
   tatagttacg ctcggcggca tccatcggga tacctgcggc attttctttg gcaaaaccga 11280
   tttcagaatt gtgtttgttt ggatgaacct gaatggagag tggctgtgct gcgcataata 11340
   ctttgaacag gaaaggcagt tcgccaaagc gtttggcaac ggcctctccg agcagagtcg 11400
   atttatcact ctcaatcaca tcacgcagtg aaacgatatc tccggcggca ttctgcactc 11460
   gtgaactgct tttcggatgt gcgcccatcc acagctcggc catcggctgg ctggacggat 11520
   tttccatacc ataaagttca gtcaacgcgt tttgctgccc caggcatagt tttgcactga 11580
   gttaatgagt ttttgcatga tcggggatcc ctgcagaagt aacaccaaac aacagggtga 11640
   gcatcgacaa aagaaacagt accaagcaaa taaatagcgt atgaaggcag ggctaaaaaa 11700
   atccacatat agctgctgca tatgccatca tccaagtata tcaagatcaa aataattata 11760
   aaacatactt gtttattata atagataggt actcaaggtt agagcatatg aatagatgct 11820
   gcatatgcca tcatgtatat gcatcagtaa aacccacatc aacatgtata cctatcctag 11880
   atcgatattt ccatccatct taaactcgta actatgaaga tgtatgacac acacatacag 11940
   ttccaaaatt aataaataca ccaggtagtt tgaaacggcg tctactccga tctagaacga 12000
   atgaacgacc gcccaaccac accacatcat cacaaccaag cgaacaaaaa gcatctctgt 12060
   atatgcatca gtaaaacccg catcaacatg tatacctatc ctagatcgat atttccatcc 12120
   atcatcttca attcgtaact atgaatatgt atggcacaca catacagatc caaaattaat 12180
   aaatccacca ggtagtttga aacagaattc tactccgatc tagaacgacc gcccaaccag 12240
   accacatcat cacaaccaag acaaaaaaaa gcatgaaaag atgacccgac aaacaagtgc 12300
   acggcatata ttgaaataaa ggaaaagggc aaaccaaacc ctatgcaacg aaacaaaaaa 12360
   aatcatgaaa tcgatcccgt ctgcggaacg gctagagcca tcccaggatt ccccaaagag 12420
   aaacactggc aagttagcaa tcagaacgtg tctgacgtac aggtcgcatc cgtgtacgaa 12480
   cgctagcagc acggatctaa cacaaacacg gatctaacac aaacatgaac agaagtagaa 12540
   ctaccgggcc ctaaccatgg accggaacgc cgatctagag aaggtagaga gggggggggg 12600
   gggaggacga gcggcgtacc ttgaagcgga ggtgccgacg ggtggatttg gggagatctt 12660
   ggttgtgtgt gtgtgcgctc cgaacaacac gaggttgggg aaagagggtg tggagggggt 12720
   gtctatttat tacggcgggc gaggaaggga aagcgaagga gcggtgggaa aggaatcccc 12780
   cgtagctgcc gtgccgtgag aggaggagga ggccgcctgc cgtgccggct cacgtctgcc 12840
   gctccgccac gcaatttctg gatgccgaca gcggagcaag tccaacggtg gagcggaact 12900
   ctcgagaggg gtccagaggc agcgacagag atgccgtgcc gtctgcttcg cttggcccga 12960
   cgcgacgctg ctggttcgct ggttggtgtc cgttagactc gtcgacggcg tttaacaggc 13020
   tggcattatc tactcgaaac aagaaaaatg tttccttagt ttttttaatt tcttaaaggg 13080
   tatttgttta atttttagtc actttatttt attctatttt atatctaaat tattaaataa 13140
   aaaaactaaa atagagtttt agttttctta atttagaggc taaaatagaa taaaatagat 13200
   gtactaaaaa aattagtcta taaaaaccat taaccctaaa ccctaaatgg atgtactaat 13260
   aaaatggatg aagtattata taggtgaagc tatttgcaaa aaaaaaggag aacacatgca 13320
   cactaaaaag ataaaactgt agagtcctgt tgtcaaaata ctcaattgtc ctttagacca 13380
   tgtctaactg ttcatttata tgattctcta aaacactgat attattgtag tactatagat 13440
   tatattattc gtagagtaaa gtttaaatat atgtataaag atagataaac tgcacttcaa 13500
   acaagtgtga caaaaaaaat atgtggtaat tttttataac ttagacatgc aatgctcatt 13560
   atctctagag aggggcacga ccgggtcacg ctgcactgca ggcatgcaag cttgcacatg 13620
   acaacaattg taagaggatg gagaccacaa cgatccaaca atacttctgc gacgggctgt 13680
   gaagtataga gaagttaaac gcccaaaagc cattgtgttt ggaattttta gttattctat 13740
   ttttcatgat gtatcttcct ctaacatgcc ttaatttgca aatttggtat aactactgat 13800
   tgaaaatata tgtatgtaaa aaaatactaa gcatatttgt gaagctaaac atgatgttat 13860
   ttaagaaaat atgttgttaa cagaataaga ttaatatcga aatggaaaca tctgtaaatt 13920
   agaatcatct tacaagctaa gagatgttca cgctttgaga aacttcttca gatcatgacc 13980
   gtagaagtag ctctccaaga ctcaacgaag gctgctgcaa ttccacaaat gcatgacatg 14040
   catccttgta accgtcgtcg ccgctataaa cacggataac tcaattccct gctccatcaa 14100
   tttagaaatg agcaagcaag cacccgatcg ctcaccccat atgcaccaat ctgactccca 14160
   agtctctgtt tcgcattagt accgccagca ctccacctat agctaccaat tgagaccttt 14220
   ccagcctaag cagatcgatt gatcgttaga gtcaaagagt tggtggtacg ggtactttaa 14280
   ctaccatgga atgatggggc gtgatgtaga gcggaaagcg cctccctacg cggaacaaca 14340
   ccctcgccat gccgctcgac tacagcctcc tcctcgtcgg ccgcccacaa cgagggagcc 14400
   cgtggtcgca gccaccgacc agcatgtctc tgtgtcctcg tccgacctcg acatgtcatg 14460
   gcaaacagtc ggacgccagc accagactga cgacatgagt ctctgaagag cccgccacct 14520
   agaaagatcc gagccctgct gctggtagtg gtaaccattt tcgtcgcgct gacgcggaga 14580
   gcgagaggcc agaaatttat agcgactgac gctgtggcag gcacgctatc ggaggttacg 14640
   acgtggcggg tcactcgacg cggagttcac aggtcctatc cttgcatcgc tcgggccgga 14700
   gtttacggga cttatcctta cgacgtgctc taaggttgcg ataacgggcg gaggaaggcg 14760
   tgtggcgtgc ggagacggtt tatacacgta gtgtgcggga gtgtgtttcg tagacgcggg 14820
   aaagcacgac gacttacgaa ggttagtgga ggaggaggac acactaaaat caggacgcaa 14880
   gaaactcttc tattatagta gtagagaaga gattatagga gtgtgggttg attctaaaga 14940
   aaatcgacgc aggacaaccg tcaaaacggg tgctttaata tagtagatat atatatatag 15000
   agagagagag aaagtacaaa ggatgcattt gtgtctgcat atgatcggag tattactaac 15060
   ggccgtcgta agaaggtcca tcatgcgtgg agcgagccca tttggttggt tgtcaggccg 15120
   cagttaaggc ctccatatat gattgtcgtc gggcccataa cagcatctcc tccaccagtt 15180
   tattgtaaga ataaattaag tagagatatt tgtcgtcggg cagaagaaac ttggacaaga 15240
   agaagaagca agctaggcca atttcttgcc ggcaagagga agatagtggc ctctagttta 15300
   tatatcggcg tgatgatgat gctcctagct agaaatgaga gaagaaaaac ggacgcgtgt 15360
   ttggtgtgtg tcaatggcgt ccatccttcc atcagatcag aacgatgaaa aagtcaagca 15420
   cggcatgcat agtatatgta tagcttgttt tagtgtggct ttgctgagac gaatgaaagc 15480
   aacggcgggc atatttttca gtggctgtag ctttcaggct gaaagagacg tggcatgcaa 15540
   taattcaggg aattcgtcag ccaattgagg tagctagtca acttgtacat tggtgcgagc 15600
   aattttccgc actcaggagg gctagtttga gagtccaaaa actataggag attaaagagg 15660
   ctaaaatcct ctccttattt aattttaaat aagtagtgta tttgtatttt aactcctcca 15720
   acccttccga ttttatggct ctcaaactag cattcagtct aatgcatgca tgcttggcta 15780
   gaggtcgtat ggggttgtta atagcatagc tagctacaag ttaaccgggt cttttatatt 15840
   taataaggac aggcaaagta ttacttacaa ataaagaata aagctaggac gaactcgtgg 15900
   attattacta aatcgaaatg gacgtaatat tccaggcaag aataattgtt cgatcaggag 15960
   acaagtgggg cattggaccg gttcttgcaa gcaagagcct atggcgtggt gacacggcgc 16020
   gttgcccata catcatgcct ccatcgatga tccatcctca cttgctataa aaagaggtgt 16080
   ccatggtgct caagctcagc caagcaaata agacgacttg tttcattgat tcttcaagag 16140
   atcgagcttc ttttgcacca caaggtcgag gatccaaca                        16179
<210> 13
   <211> 15643
   <212> DNA
   <213> Artificial Sequence
<220>
   <223> Description of Artificial Sequence: pNOV1441
<220>
   <221> misc_feature
   <222> (14)..(1414)
   <223> Maize ubiquitin (Mz Ubi) promoter
<220>
   <221> misc_feature
   <222> (2037)..(5618)
   <223> synthetic nucleotide sequence encoding the toxin portion of H04 plus a full-length Cry1Ab tail portion
<220>
   <221> misc_feature
   <222> (5821)..(6711)
   <223> Mz Ubi promoter
<220>
   <221> misc_feature
   <222> (7831)..(9039)
   <223> PMI
<400> 13
   aagctggtac aagcttgcat gcctgcagtg cagcgtgacc cggtcgtgcc cctctctaga 60
   gataatgagc attgcatgtc taagttataa aaaattacca catatttttt ttgtcacact 120
   tgtttgaagt gcagtttatc tatctttata catatattta aactttactc tacgaataat 180
   ataatctata gtactacaat aatatcagtg ttttagagaa tcatataaat gaacagttag 240
   acatggtcta aaggacaatt gagtattttg acaacaggac tctacagttt tatcttttta 300
   gtgtgcatgt gttctccttt ttttttgcaa atagcttcac ctatataata cttcatccat 360
   tttattagta catccattta gggtttaggg ttaatggttt ttatagacta atttttttag 420
   tacatctatt ttattctatt ttagcctcta aattaagaaa actaaaactc tattttagtt 480
   tttttattta ataatttaga tataaaatag aataaaataa agtgactaaa aattaaacaa 540
   atacccttta agaaattaaa aaaactaagg aaacattttt cttgtttcga gtagataatg 600
   ccagcctgtt aaacgccgtc gacgagtcta acggacacca accagcgaac cagcagcgtc 660
   gcgtcgggcc aagcgaagca gacggcacgg catctctgtc gctgcctctg gacccctctc 720
   gagagttccg ctccaccgtt ggacttgctc cgctgtcggc atccagaaat tgcgtggcgg 780
   agcggcagac gtgagccggc acggcaggcg gcctcctcct cctctcacgg cacggcagct 840
   acgggggatt cctttcccac cgctccttcg ctttcccttc ctcgcccgcc gtaataaata 900
   gacaccccct ccacaccctc tttccccaac ctcgtgttgt tcggagcgca cacacacaca 960
   accagatctc ccccaaatcc acccgtcggc acctccgctt caaggtacgc cgctcgtcct 1020
   cccccccccc ccctctctac cttctctaga tcggcgttcc ggtccatggt tagggcccgg 1080
   tagttctact tctgttcatg tttgtgttag atccgtgttt gtgttagatc cgtgctgcta 1140
   gcgttcgtac acggatgcga cctgtacgtc agacacgttc tgattgctaa cttgccagtg 1200
   tttctctttg gggaatcctg ggatggctct agccgttccg cagacgggat cgatttcatg 1260
   attttttttg tttcgttgca tagggtttgg tttgcccttt tcctttattt caatatatgc 1320
   cgtgcacttg tttgtcgggt catcttttca tgcttttttt tgtcttggtt gtgatgatgt 1380
   ggtctggttg ggcggtcgtt ctagatcgga gtagaattct gtttcaaact acctggtgga 1440
   tttattaatt ttggatctgt atgtgtgtgc catacatatt catagttacg aattgaagat 1500
   gatggatgga aatatcgatc taggataggt atacatgttg atgcgggttt tactgatgca 1560
   tatacagaga tgctttttgt tcgcttggtt gtgatgatgt ggtgtggttg ggcggtcgtt 1620
   cattcgttct agatcggagt agaatactgt ttcaaactac ctggtgtatt tattaatttt 1680
   ggaactgtat gtgtgtgtca tacatcttca tagttacgag tttaagatgg atggaaatat 1740
   cgatctagga taggtataca tgttgatgtg ggttttactg atgcatatac atgatggcat 1800
   atgcagcatc tattcatatg ctctaacctt gagtacctat ctattataat aaacaagtat 1860
   gttttataat tattttgatc ttgatatact tggatgatgg catatgcagc agctatatgt 1920
   ggattttttt agccctgcct tcatacgcta tttatttgct tggtactgtt tcttttgtcg 1980
   atgctcaccc tgttgtttgg tgttacttct gcaggtcgac tctagaggat ccaacaatgg 2040
   acaacaaccc caacatcaac gagtgcatcc cctacaactg cctgagcaac cccgaggtgg 2100
   aggtgctggg cggcgagcgc atcgagaccg gctacacccc catcgacatc agcctgagcc 2160
   tgacccagtt cctgctgagc gagttcgtgc ccggcgccgg cttcgtgctg ggcctggtgg 2220
   acatcatctg gggcatcttc ggccccagcc agtgggacgc cttcctggtg cagatcgagc 2280
   agttgataaa ccaacgcata gaggaattcg cccgcaacca ggccatcagc cgcctggagg 2340
   gcctgagcaa cctgtaccaa atctacgccg agagcttccg cgagtgggag gccgacccca 2400
   ccaaccccgc cctgcgcgag gagatgcgca tccagttcaa cgacatgaac agcgccctga 2460
   ccaccgccat ccccctgttc gccgtgcaga actaccaggt gcccctgctg agcgtgtacg 2520
   tgcaggccgc caacctgcac ctgagcgtgc tgcgcgacgt cagcgtgttc ggccagcgct 2580
   ggggcttcga cgccgccacc atcaacagcc gctacaacga cctgacccgc ctgatcggca 2640
   actacaccga ccacgccgtg cgctggtaca acaccggcct ggagcgcgtg tggggtcccg 2700
   acagccgcga ctggatcagg tacaaccagt tccgccgcga gctgaccctg accgtgctgg 2760
   acatcgtgag cctgttcccc aactacgaca gccgcaccta ccccatccgc accgtgagcc 2820
   agctgacccg cgagatttac accaaccccg tgctggagaa cttcgacggc agcttccgcg 2880
   gcagcgccca gggcatcgag ggcagcatcc gcagccccca cctgatggac atcctgaaca 2940
   gcatcaccat ctacaccgac gcccaccgcg gcgagtacta ctggagcggc caccagatca 3000
   tggccagccc cgtcggcttc agcggccccg agttcacctt ccccctgtac ggcaccatgg 3060
   gcaacgctgc acctcagcag cgcatcgtgg cacagctggg ccagggagtg taccgcaccc 3120
   tgagcagcac cctgtaccgt cgacctttca acatcggcat caacaaccag cagctgagcg 3180
   tgctggacgg caccgagttc gcctacggca ccagcagcaa cctgcccagc gccgtgtacc 3240
   gcaagagcgg caccgtggac agcctggacg agatcccccc tcagaacaac aacgtgccac 3300
   ctcgacaggg cttcagccac cgtctgagcc acgtgagcat gttccgcagt ggcttcagca 3360
   acagcagcgt gagcatcatc cgtgcaccca tgttcagctg gattcaccgc agcgccaccc 3420
   tgaccaacac catcgacccc gagcgcatca accagatccc cctggtgaag ggcttccggg 3480
   tgtggggcgg caccagcgtg atcaccggcc ccggcttcac cggaggcgac atcctgcgca 3540
   gaaacacctt cggcgacttc gtgagcctgc aggtgaacat caacagcccc atcacccagc 3600
   gttaccgcct gcgcttccgc tacgccagca gccgcgacgc ccgtgtgatc gtgctgactg 3660
   gcgccgctag caccggtgtg ggcggtcagg tgagcgtgaa catgcccctg cagaagacta 3720
   tggagatcgg cgagaacctg actagtcgca ccttccgcta caccgacttc agcaacccct 3780
   tcagcttccg cgccaacccc gacatcatcg gcatcagcga gcagcccctg ttcggtgccg 3840
   gcagcatcag cagcggcgag ctgtacatcg acaagatcga gatcatcctg gccgacgcca 3900
   ccttcgaggc cgagagcgac ctggagcgcg cccagaaggc cgtgaacgcc ctgttcacca 3960
   gcagcaacca gatcggcctg aagaccgacg tgaccgacta ccacatcgac caggtgagca 4020
   acctggtgga ctgcttaagc gacgagttct gcctggacga gaagaaggag ctgagcgaga 4080
   aggtgaagca cgccaagcgc ctgagcgacg agcgcaacct gctgcaggac cccaacttcc 4140
   gcggcatcaa ccgccagctg gaccgcggct ggcgaggcag caccgatatc accatccagg 4200
   gcggcgacga cgtgttcaag gagaactacg tgaccctgca gggcaccttc gacgagtgct 4260
   accccaccta cctgtaccag ccgatcgacg agagcaagct gaaggcctac acccgctacc 4320
   agctgcgcgg ctacatcgag gacagccagg acctggaaat ctacctgatc cgctacaacg 4380
   cgaagcacga gaccgtgaac gtgcccggca ccggcagcct gtggcccccg agcgccccca 4440
   gccccatcgg caagtgcggg gagccgaatc gatgcgctcc gcacctggag tggaacccgg 4500
   acctagactg cagctgcagg gacggggaga agtgcgccca ccacagccac cacttcagcc 4560
   tggacatcga cgtgggctgc accgacctga acgaggacct gggcgtgtgg gtgatcttca 4620
   agatcaagac ccaggacggc cacgcccgcc tgggcaatct agagttcctg gaggagaagc 4680
   ccctggtggg cgaggccctg gcccgcgtga agcgtgctga gaagaagtgg cgcgacaagc 4740
   gcgagaagct ggagtgggag accaacatcg tgtacaagga ggccaaggag agcgtggacg 4800
   ccctgttcgt gaacagccag tacgaccgcc tgcaggccga caccaacatc gccatgatcc 4860
   acgccgccga caagcgcgtg cacagcattc gcgaggccta cctgcccgag ctgagcgtga 4920
   tccccggtgt gaacgccgcc atcttcgagg aactcgaggg ccgcatcttc accgccttca 4980
   gcctgtacga cgcccgcaac gtgatcaaga acggcgactt caacaacggc ctgagctgct 5040
   ggaacgtgaa gggccacgtg gacgtggagg agcagaacaa ccaccgcagc gtgctggtgg 5100
   tgcccgagtg ggaggccgag gtgagccagg aggtgcgcgt gtgccccggc cgcggctaca 5160
   tcctgcgcgt gaccgcctac aaggagggct acggcgaggg ctgcgtgacc atccacgaga 5220
   tcgagaacaa caccgacgag ctcaagttca gcaactgcgt ggaggaggag gtttacccca 5280
   acaacaccgt gacctgcaac gactacaccg cgacccagga ggagtacgaa ggcacctaca 5340
   cctctcgcaa caggggttac gacggcgcct acgagtccaa cagctccgtg ccagctgact 5400
   acgccagcgc ccacgaggag aaagcctaca ccgacggtag acgcgacaac ccatgtgaga 5460
   gcaacagagg ctacggcgac tacacccccc tgcccgctgg atacgtgacc aaggagctgg 5520
   agtacttccc cgagaccgac aaggtgtgga tcgagattgg cgagaccgag ggcaccttca 5580
   tcgtggacag cgtggagctg ctgctgatgg aggagtagta gatctgttct gcacaaagtg 5640
   gagtagtcag tcatcgatca ggaaccagac accagacttt tattcataca gtgaagtgaa 5700
   gtgaagtgca gtgcagtgag ttgctggttt ttgtaccact tagtatgtat ttgtatttgt 5760
   aaaatacttc tatcaataaa atttctaatt cctaaaacca aaatccagtg ggtaccagct 5820
   tgcatgcctg cagtgcagcg tgacccggtc gtgcccctct ctagagataa tgagcattgc 5880
   atgtctaagt tataaaaaat taccacatat tttttttgtc acacttgttt gaagtgcagt 5940
   ttatctatct ttatacatat atttaaactt tactctacga ataatataat ctatagtact 6000
   acaataatat cagtgtttta gagaatcata taaatgaaca gttagacatg gtctaaagga 6060
   caattgagta ttttgacaac aggactctac agttttatct ttttagtgtg catgtgttct 6120
   cctttttttt tgcaaatagc ttcacctata taatacttca tccattttat tagtacatcc 6180
   atttagggtt tagggttaat ggtttttata gactaatttt tttagtacat ctattttatt 6240
   ctattttagc ctctaaatta agaaaactaa aactctattt tagttttttt atttaataat 6300
   ttagatataa aatagaataa aataaagtga ctaaaaatta aacaaatacc ctttaagaaa 6360
   ttaaaaaaac taaggaaaca tttttcttgt ttcgagtaga taatgccagc ctgttaaacg 6420
   ccgtcgacga gtctaacgga caccaaccag cgaaccagca gcgtcgcgtc gggccaagcg 6480
   aagcagacgg cacggcatct ctgtcgctgc ctctggaccc ctctcgagag ttccgctcca 6540
   ccgttggact tgctccgctg tcggcatcca gaaattgcgt ggcggagcgg cagacgtgag 6600
   ccggcacggc aggcggcctc ctcctcctct cacggcacgg cagctacggg ggattccttt 6660
   cccaccgctc cttcgctttc ccttcctcgc ccgccgtaat aaatagacac cccctccaca 6720
   ccctctttcc ccaacctcgt gttgttcgga gcgcacacac acacaaccag atctccccca 6780
   aatccacccg tcggcacctc cgcttcaagg tacgccgctc gtcctccccc cccccccctc 6840
   tctaccttct ctagatcggc gttccggtcc atggttaggg cccggtagtt ctacttctgt 6900
   tcatgtttgt gttagatccg tgtttgtgtt agatccgtgc tgctagcgtt cgtacacgga 6960
   tgcgacctgt acgtcagaca cgttctgatt gctaacttgc cagtgtttct ctttggggaa 7020
   tcctgggatg gctctagccg ttccgcagac gggatcgatt tcatgatttt ttttgtttcg 7080
   ttgcataggg tttggtttgc ccttttcctt tatttcaata tatgccgtgc acttgtttgt 7140
   cgggtcatct tttcatgctt ttttttgtct tggttgtgat gatgtggtct ggttgggcgg 7200
   tcgttctaga tcggagtaga attctgtttc aaactacctg gtggatttat taattttgga 7260
   tctgtatgtg tgtgccatac atattcatag ttacgaattg aagatgatgg atggaaatat 7320
   cgatctagga taggtataca tgttgatgcg ggttttactg atgcatatac agagatgctt 7380
   tttgttcgct tggttgtgat gatgtggtgt ggttgggcgg tcgttcattc gttctagatc 7440
   ggagtagacg ccgtttcaaa ctacctggtg tatttattaa ttttggaact gtatgtgtgt 7500
   gtcatacatc ttcatagtta cgagtttaag atggatggaa atatcgatct aggataggta 7560
   tacatgttga tgtgggtttt actgatgcat atacatgatg gcatatgcag catctattca 7620
   tatgctctaa ccttgagtac ctatctatta taataaacaa gtatgtttta taattatttt 7680
   gatcttgata tacttggatg atggcatatg cagcagctat atgtggattt ttttagccct 7740
   gccttcatac gctatttatt tgcttggtac tgtttctttt gtcgatgctc accctgttgt 7800
   ttggtgttac ttctgcaggg atccccgatc atgcaaaaac tcattaactc agtgcaaaac 7860
   tatgcctggg gcagcaaaac gcgttgactg aactttatgg tatggaaaat ccgtccagcc 7920
   agccgatggc cgagctgtgg atgggcgcac atccgaaaag cagttcacga gtgcagaatg 7980
   ccgccggaga tatcgtttca ctgcgtgatg tgattgagag tgataaatcg actctgctcg 8040
   gagaggccgt tgccaaacgc tttggcgaac tgcctttcct gttcaaagta ttatgcgcag 8100
   cacagccact ctccattcag gttcatccaa acaaacacaa ttctgaaatc ggttttgcca 8160
   aagaaaatgc cgcaggtatc ccgatggatg ccgccgagcg taactataaa gatcctaacc 8220
   acaagccgga gctggttttt gcgctgacgc ctttccttgc gatgaacgcg tttcgtgaat 8280
   tttccgagat tgtctcccta ctccagccgg tcgcaggtgc acatccggcg attgctcact 8340
   ttttacaaca gcctgatgcc gaacgtttaa gcgaactgtt cgccagcctg ttgaatatgc 8400
   agggtgaaga aaaatcccgc gcgctggcga ttttaaaatc ggccctcgat agccagcagg 8460
   gtgaaccgtg gcaaacgatt cgtttaattt ctgaatttta cccggaagac agcggtctgt 8520
   tctccccgct attgctgaat gtggtgaaat tgaaccctgg cgaagcgatg ttcctgttcg 8580
   ctgaaacacc gcacgcttac ctgcaaggcg tggcgctgga agtgatggca aactccgata 8640
   acgtgctgcg tgcgggtctg acgcctaaat acattgatat tccggaactg gttgccaatg 8700
   tgaaattcga agccaaaccg gctaaccagt tgttgaccca gccggtgaaa caaggtgcag 8760
   aactggactt cccgattcca gtggatgatt ttgccttctc gctgcatgac cttagtgata 8820
   aagaaaccac cattagccag cagagtgccg ccattttgtt ctgcgtcgaa ggcgatgcaa 8880
   cgttgtggaa aggttctcag cagttacagc ttaaaccggg tgaatcagcg tttattgccg 8940
   ccaacgaatc accggtgact gtcaaaggcc acggccgttt agcgcgtgtt tacaacaagc 9000
   tgtaagagct tactgaaaaa attaacatct cttgctaagc tgggagctcg atccgtcgac 9060
   ctgcagagat cgttcaaaca tttggcaata aagtttctta agattgaatc ctgttgccgg 9120
   tcttgcgatg attatcatct aatttctgtt gaattacgtt aagcatgtaa taattaacat 9180
   gtaatgcatg acgttattta tgagatgggt ttttatgatt agagtcccgc aattatacat 9240
   ttaatacgcg atagaaaaca aaatatagcg cgcaaactag gataaattat cgcgcgcggt 9300
   gtcatctatg ttactagatc cgatgataag ctgtcaaaca tgagatcccc gggtctagac 9360
   aattcagtac attaaaaacg tccgcaatgt gttattaagt tgtctaagcg tcaatttgtt 9420
   tacaccacaa tatatcctgc caccagccag ccaacagctc cccgaccggc agctcggcac 9480
   aaaatcacca ctcgatacag gcagcccatc agtccgggac ggcgtcagcg ggagagccgt 9540
   tgtaaggcgg cagactttgc tcatgttacc gatgctattc ggaagaacgg caactaagct 9600
   gccgggtttg aaacacggat gatctcgcgg agggtagcat gttgattgta acgatgacag 9660
   agcgttgctg cctgtgatca aatatcatct ccctcgcaga gatccgaatt atcagccttc 9720
   ttattcattt ctcgcttaac cgtgacaggc tgtcgatctt gagaactatg ccgacataat 9780
   aggaaatcgc tggataaagc cgctgaggaa gctgagtggc gctatttctt tagaagtgaa 9840
   cgttgacgat cgtcgaccgt accccgatga attaattcgg acgtacgttc tgaacacagc 9900
   tggatactta cttgggcgat tgtcatacat gacatcaaca atgtacccgt ttgtgtaacc 9960
   gtctcttgga ggttcgtatg acactagtgg ttcccctcag cttgcgacta gatgttgagg 10020
   cctaacattt tattagagag caggctagtt gcttagatac atgatcttca ggccgttatc 10080
   tgtcagggca agcgaaaatt ggccatttat gacgaccaat gccccgcaga agctcccatc 10140
   tttgccgcca tagacgccgc gccccccttt tggggtgtag aacatccttt tgccagatgt 10200
   ggaaaagaag ttcgttgtcc cattgttggc aatgacgtag tagccggcga aagtgcgaga 10260
   cccatttgcg ctatatataa gcctacgatt tccgttgcga ctattgtcgt aattggatga 10320
   actattatcg tagttgctct cagagttgtc gtaatttgat ggactattgt cgtaattgct 10380
   tatggagttg tcgtagttgc ttggagaaat gtcgtagttg gatggggagt agtcataggg 10440
   aagacgagct tcatccacta aaacaattgg caggtcagca agtgcctgcc ccgatgccat 10500
   cgcaagtacg aggcttagaa ccaccttcaa cagatcgcgc atagtcttcc ccagctctct 10560
   aacgcttgag ttaagccgcg ccgcgaagcg gcgtcggctt gaacgaattg ttagacatta 10620
   tttgccgact accttggtga tctcgccttt cacgtagtga acaaattctt ccaactgatc 10680
   tgcgcgcgag gccaagcgat cttcttgtcc aagataagcc tgcctagctt caagtatgac 10740
   gggctgatac tgggccggca ggcgctccat tgcccagtcg gcagcgacat ccttcggcgc 10800
   gattttgccg gttactgcgc tgtaccaaat gcgggacaac gtaagcacta catttcgctc 10860
   atcgccagcc cagtcgggcg gcgagttcca tagcgttaag gtttcattta gcgcctcaaa 10920
   tagatcctgt tcaggaaccg gatcaaagag ttcctccgcc gctggaccta ccaaggcaac 10980
   gctatgttct cttgcttttg tcagcaagat agccagatca atgtcgatcg tggctggctc 11040
   gaagatacct gcaagaatgt cattgcgctg ccattctcca aattgcagtt cgcgcttagc 11100
   tggataacgc cacggaatga tgtcgtcgtg cacaacaatg gtgacttcta cagcgcggag 11160
   aatctcgctc tctccagggg aagccgaagt ttccaaaagg tcgttgatca aagctcgccg 11220
   cgttgtttca tcaagcctta cggtcaccgt aaccagcaaa tcaatatcac tgtgtggctt 11280
   caggccgcca tccactgcgg agccgtacaa atgtacggcc agcaacgtcg gttcgagatg 11340
   gcgctcgatg acgccaacta cctctgatag ttgagtcgat acttcggcga tcaccgcttc 11400
   cctcatgatg tttaactcct gaattaagcc gcgccgcgaa gcggtgtcgg cttgaatgaa 11460
   ttgttaggcg tcatcctgtg ctcccgagaa ccagtaccag tacatcgctg tttcgttcga 11520
   gacttgaggt ctagttttat acgtgaacag gtcaatgccg ccgagagtaa agccacattt 11580
   tgcgtacaaa ttgcaggcag gtacattgtt cgtttgtgtc tctaatcgta tgccaaggag 11640
   ctgtctgctt agtgcccact ttttcgcaaa ttcgatgaga ctgtgcgcga ctcctttgcc 11700
   tcggtgcgtg tgcgacacaa caatgtgttc gatagaggct agatcgttcc atgttgagtt 11760
   gagttcaatc ttcccgacaa gctcttggtc gatgaatgcg ccatagcaag cagagtcttc 11820
   atcagagtca tcatccgaga tgtaatcctt ccggtagggg ctcacacttc tggtagatag 11880
   ttcaaagcct tggtcggata ggtgcacatc gaacacttca cgaacaatga aatggttctc 11940
   agcatccaat gtttccgcca cctgctcagg gatcaccgaa atcttcatat gacgcctaac 12000
   gcctggcaca gcggatcgca aacctggcgc ggcttttggc acaaaaggcg tgacaggttt 12060
   gcgaatccgt tgctgccact tgttaaccct tttgccagat ttggtaacta taatttatgt 12120
   tagaggcgaa gtcttgggta aaaactggcc taaaattgct ggggatttca ggaaagtaaa 12180
   catcaccttc cggctcgatg tctattgtag atatatgtag tgtatctact tgatcggggg 12240
   atctgctgcc tcgcgcgttt cggtgatgac ggtgaaaacc tctgacacat gcagctcccg 12300
   gagacggtca cagcttgtct gtaagcggat gccgggagca gacaagcccg tcagggcgcg 12360
   tcagcgggtg ttggcgggtg tcggggcgca gccatgaccc agtcacgtag cgatagcgga 12420
   gtgtatactg gcttaactat gcggcatcag agcagattgt actgagagtg caccatatgc 12480
   ggtgtgaaat accgcacaga tgcgtaagga gaaaataccg catcaggcgc tcttccgctt 12540
   cctcgctcac tgactcgctg cgctcggtcg ttcggctgcg gcgagcggta tcagctcact 12600
   caaaggcggt aatacggtta tccacagaat caggggataa cgcaggaaag aacatgtgag 12660
   caaaaggcca gcaaaaggcc aggaaccgta aaaaggccgc gttgctggcg tttttccata 12720
   ggctccgccc ccctgacgag catcacaaaa atcgacgctc aagtcagagg tggcgaaacc 12780
   cgacaggact ataaagatac caggcgtttc cccctggaag ctccctcgtg cgctctcctg 12840
   ttccgaccct gccgcttacc ggatacctgt ccgcctttct cccttcggga agcgtggcgc 12900
   tttctcatag ctcacgctgt aggtatctca gttcggtgta ggtcgttcgc tccaagctgg 12960
   gctgtgtgca cgaacccccc gttcagcccg accgctgcgc cttatccggt aactatcgtc 13020
   ttgagtccaa cccggtaaga cacgacttat cgccactggc agcagccact ggtaacagga 13080
   ttagcagagc gaggtatgta ggcggtgcta cagagttctt gaagtggtgg cctaactacg 13140
   gctacactag aaggacagta tttggtatct gcgctctgct gaagccagtt accttcggaa 13200
   aaagagttgg tagctcttga tccggcaaac aaaccaccgc tggtagcggt ggtttttttg 13260
   tttgcaagca gcagattacg cgcagaaaaa aaggatctca agaagatcct ttgatctttt 13320
   ctacggggtc tgacgctcag tggaacgaaa actcacgtta agggattttg gtcatgagat 13380
   tatcaaaaag gatcttcacc tagatccttt taaattaaaa atgaagtttt aaatcaatct 13440
   aaagtatata tgagtaaact tggtctgaca gttaccaatg cttaatcagt gaggcaccta 13500
   tctcagcgat ctgtctattt cgttcatcca tagttgcctg actccccgtc gtgtagataa 13560
   ctacgatacg ggagggctta ccatctggcc ccagtgctgc aatgataccg cgagacccac 13620
   gctcaccggc tccagattta tcagcaataa accagccagc cggaagggcc gagcgcagaa 13680
   gtggtcctgc aactttatcc gcctccatcc agtctattaa ttgttgccgg gaagctagag 13740
   taagtagttc gccagttaat agtttgcgca acgttgttgc cattgctgca gggggggggg 13800
   ggggggggga cttccattgt tcattccacg gacaaaaaca gagaaaggaa acgacagagg 13860
   ccaaaaagcc tcgctttcag cacctgtcgt ttcctttctt ttcagagggt attttaaata 13920
   aaaacattaa gttatgacga agaagaacgg aaacgcctta aaccggaaaa ttttcataaa 13980
   tagcgaaaac ccgcgaggtc gccgccccgt aacctgtcgg atcaccggaa aggacccgta 14040
   aagtgataat gattatcatc tacatatcac aacgtgcgtg gaggccatca aaccacgtca 14100
   aataatcaat tatgacgcag gtatcgtatt aattgatctg catcaactta acgtaaaaac 14160
   aacttcagac aatacaaatc agcgacactg aatacggggc aacctcatgt cccccccccc 14220
   cccccccctg caggcatcgt ggtgtcacgc tcgtcgtttg gtatggcttc attcagctcc 14280
   ggttcccaac gatcaaggcg agttacatga tcccccatgt tgtgcaaaaa agcggttagc 14340
   tccttcggtc ctccgatcgt tgtcagaagt aagttggccg cagtgttatc actcatggtt 14400
   atggcagcac tgcataattc tcttactgtc atgccatccg taagatgctt ttctgtgact 14460
   ggtgagtact caaccaagtc attctgagaa tagtgtatgc ggcgaccgag ttgctcttgc 14520
   ccggcgtcaa cacgggataa taccgcgcca catagcagaa ctttaaaagt gctcatcatt 14580
   ggaaaacgtt cttcggggcg aaaactctca aggatcttac cgctgttgag atccagttcg 14640
   atgtaaccca ctcgtgcacc caactgatct tcagcatctt ttactttcac cagcgtttct 14700
   gggtgagcaa aaacaggaag gcaaaatgcc gcaaaaaagg gaataagggc gacacggaaa 14760
   tgttgaatac tcatactctt cctttttcaa tattattgaa gcatttatca gggttattgt 14820
   ctcatgagcg gatacatatt tgaatgtatt tagaaaaata aacaaatagg ggttccgcgc 14880
   acatttcccc gaaaagtgcc acctgacgtc taagaaacca ttattatcat gacattaacc 14940
   tataaaaata ggcgtatcac gaggcccttt cgtcttcaag aattggtcga cgatcttgct 15000
   gcgttcggat attttcgtgg agttcccgcc acagacccgg attgaaggcg agatccagca 15060
   actcgcgcca gatcatcctg tgacggaact ttggcgcgtg atgactggcc aggacgtcgg 15120
   ccgaaagagc gacaagcaga tcacgctttt cgacagcgtc ggatttgcga tcgaggattt 15180
   ttcggcgctg cgctacgtcc gcgaccgcgt tgagggatca agccacagca gcccactcga 15240
   ccttctagcc gacccagacg agccaaggga tctttttgga atgctgctcc gtcgtcaggc 15300
   tttccgacgt ttgggtggtt gaacagaagt cattatcgta cggaatgcca agcactcccg 15360
   aggggaaccc tgtggttggc atgcacatac aaatggacga acggataaac cttttcacgc 15420
   ccttttaaat atccgttatt ctaataaacg ctcttttctc ttaggtttac ccgccaatat 15480
   atcctgtcaa acactgatag tttaaactga aggcgggaaa cgacaatctg atcatgagcg 15540
   gagaattaag ggagtcacgt tatgaccccc gccgatgacg cgggacaagc cgttttacgt 15600
   ttggaactga cagaaccgca acgttgaagg agccactcag ccc                   15643
<210> 14
   <211> 15503
   <212> DNA
   <213> Artificial Sequence
<220>
   <223> Description of Artificial Sequence: pNOV1305
<220>
   <221> misc_feature
   <222> (1)..(3582)
   <223> synthetic nucleotide sequence encoding the toxin portion of H04 plus a full-length Cry1Ab tail portion
<220>
   <221> misc_feature
   <222> (3790)..(5771)
   <223> Zm Ubi promoter
<220>
   <221> misc_feature
   <222> (5868)..(6971)
   <223> PMI
<220>
   <221> misc_feature
   <222> (12934)..(15494)
   <223> MTL promoter
<400> 14
   atggacaaca accccaacat caacgagtgc atcccctaca actgcctgag caaccccgag 60
   gtggaggtgc tgggcggcga gcgcatcgag accggctaca cccccatcga catcagcctg 120
   agcctgaccc agttcctgct gagcgagttc gtgcccggcg ccggcttcgt gctgggcctg 180
   gtggacatca tctggggcat cttcggcccc agccagtggg acgccttcct ggtgcagatc 240
   gagcagttga taaaccaacg catagaggaa ttcgcccgca accaggccat cagccgcctg 300
   gagggcctga gcaacctgta ccaaatctac gccgagagct tccgcgagtg ggaggccgac 360
   cccaccaacc ccgccctgcg cgaggagatg cgcatccagt tcaacgacat gaacagcgcc 420
   ctgaccaccg ccatccccct gttcgccgtg cagaactacc aggtgcccct gctgagcgtg 480
   tacgtgcagg ccgccaacct gcacctgagc gtgctgcgcg acgtcagcgt gttcggccag 540
   cgctggggct tcgacgccgc caccatcaac agccgctaca acgacctgac ccgcctgatc 600
   ggcaactaca ccgaccacgc cgtgcgctgg tacaacaccg gcctggagcg cgtgtggggt 660
   cccgacagcc gcgactggat caggtacaac cagttccgcc gcgagctgac cctgaccgtg 720
   ctggacatcg tgagcctgtt ccccaactac gacagccgca cctaccccat ccgcaccgtg 780
   agccagctga cccgcgagat ttacaccaac cccgtgctgg agaacttcga cggcagcttc 840
   cgcggcagcg cccagggcat cgagggcagc atccgcagcc cccacctgat ggacatcctg 900
   aacagcatca ccatctacac cgacgcccac cgcggcgagt actactggag cggccaccag 960
   atcatggcca gccccgtcgg cttcagcggc cccgagttca ccttccccct gtacggcacc 1020
   atgggcaacg ctgcacctca gcagcgcatc gtggcacagc tgggccaggg agtgtaccgc 1080
   accctgagca gcaccctgta ccgtcgacct ttcaacatcg gcatcaacaa ccagcagctg 1140
   agcgtgctgg acggcaccga gttcgcctac ggcaccagca gcaacctgcc cagcgccgtg 1200
   taccgcaaga gcggcaccgt ggacagcctg gacgagatcc cccctcagaa caacaacgtg 1260
   ccacctcgac agggcttcag ccaccgtctg agccacgtga gcatgttccg cagtggcttc 1320
   agcaacagca gcgtgagcat catccgtgca cccatgttca gctggattca ccgcagcgcc 1380
   accctgacca acaccatcga ccccgagcgc atcaaccaga tccccctggt gaagggcttc 1440
   cgggtgtggg gcggcaccag cgtgatcacc ggccccggct tcaccggagg cgacatcctg 1500
   cgcagaaaca ccttcggcga cttcgtgagc ctgcaggtga acatcaacag ccccatcacc 1560
   cagcgttacc gcctgcgctt ccgctacgcc agcagccgcg acgcccgtgt gatcgtgctg 1620
   actggcgccg ctagcaccgg tgtgggcggt caggtgagcg tgaacatgcc cctgcagaag 1680
   actatggaga tcggcgagaa cctgactagt cgcaccttcc gctacaccga cttcagcaac 1740
   cccttcagct tccgcgccaa ccccgacatc atcggcatca gcgagcagcc cctgttcggt 1800
   gccggcagca tcagcagcgg cgagctgtac atcgacaaga tcgagatcat cctggccgac 1860
   gccaccttcg aggccgagag cgacctggag cgcgcccaga aggccgtgaa cgccctgttc 1920
   accagcagca accagatcgg cctgaagacc gacgtgaccg actaccacat cgaccaggtg 1980
   agcaacctgg tggactgctt aagcgacgag ttctgcctgg acgagaagaa ggagctgagc 2040
   gagaaggtga agcacgccaa gcgcctgagc gacgagcgca acctgctgca ggaccccaac 2100
   ttccgcggca tcaaccgcca gctggaccgc ggctggcgag gcagcaccga tatcaccatc 2160
   cagggcggcg acgacgtgtt caaggagaac tacgtgaccc tgcagggcac cttcgacgag 2220
   tgctacccca cctacctgta ccagccgatc gacgagagca agctgaaggc ctacacccgc 2280
   taccagctgc gcggctacat cgaggacagc caggacctgg aaatctacct gatccgctac 2340
   aacgcgaagc acgagaccgt gaacgtgccc ggcaccggca gcctgtggcc cctgagcgcc 2400
   cccagcccca tcggcaagtg cggggagccg aatcgatgcg ctccgcacct ggagtggaac 2460
   ccggacctag actgcagctg cagggacggg gagaagtgcg cccaccacag ccaccacttc 2520
   agcctggaca tcgacgtggg ctgcaccgac ctgaacgagg acctgggcgt gtgggtgatc 2580
   ttcaagatca agacccagga cggccacgcc cgcctgggca atctagagtt cctggaggag 2640
   aagcccctgg tgggcgaggc cctggcccgc gtgaagcgtg ctgagaagaa gtggcgcgac 2700
   aagcgcgaga agctggagtg ggagaccaac atcgtgtaca aggaggccaa ggagagcgtg 2760
   gacgccctgt tcgtgaacag ccagtacgac cgcctgcagg ccgacaccaa catcgccatg 2820
   atccacgccg ccgacaagcg cgtgcacagc attcgcgagg cctacctgcc cgagctgagc 2880
   gtgatccccg gtgtgaacgc cgccatcttc gaggaactcg agggccgcat cttcaccgcc 2940
   ttcagcctgt acgacgcccg caacgtgatc aagaacggcg acttcaacaa cggcctgagc 3000
   tgctggaacg tgaagggcca cgtggacgtg gaggagcaga acaaccaccg cagcgtgctg 3060
   gtggtgcccg agtgggaggc cgaggtgagc caggaggtgc gcgtgtgccc cggccgcggc 3120
   tacatcctgc gcgtgaccgc ctacaaggag ggctacggcg agggctgcgt gaccatccac 3180
   gagatcgaga acaacaccga cgagctcaag ttcagcaact gcgtggagga ggaggtttac 3240
   cccaacaaca ccgtgacctg caacgactac accgcgaccc aggaggagta cgaaggcacc 3300
   tacacctctc gcaacagggg ttacgacggc gcctacgagt ccaacagctc cgtgccagct 3360
   gactacgcca gcgcctacga ggagaaagcc tacaccgacg gtagacgcga caacccatgt 3420
   gagagcaaca gaggctacgg cgactacacc cccctgcccg ctggatacgt gaccaaggag 3480
   ctggagtact tccccgagac cgacaaggtg tggatcgaga ttggcgagac cgagggcacc 3540
   ttcatcgtgg acagcgtgga gctgctgctg atggaggagt agtagatctg ttctgcacaa 3600
   agtggagtag tcagtcatcg atcaggaacc agacaccaga cttttattca tacagtgaag 3660
   tgaagtgaag tgcagtgcag tgagttgctg gtttttgtac aacttagtat gtatttgtat 3720
   ttgtaaaata cttctatcaa taaaatttct aattcctaaa accaaaatcc aggggtacca 3780
   gcttgcatgc ctgcagtgca gcgtgacccg gtcgtgcccc tctctagaga taatgagcat 3840
   tgcatgtcta agttataaaa aattaccaca tatttttttt gtcacacttg tttgaagtgc 3900
   agtttatcta tctttataca tatatttaaa ctttactcta cgaataatat aatctatagt 3960
   actacaataa tatcagtgtt ttagagaatc atataaatga acagttagac atggtctaaa 4020
   ggacaattga gtattttgac aacaggactc tacagtttta tctttttagt gtgcatgtgt 4080
   tctccttttt ttttgcaaat agcttcacct atataatact tcatccattt tattagtaca 4140
   tccatttagg gtttagggtt aatggttttt atagactaat ttttttagta catctatttt 4200
   attctatttt agcctctaaa ttaagaaaac taaaactcta ttttagtttt tttatttaat 4260
   aatttagata taaaatagaa taaaataaag tgactaaaaa ttaaacaaat accctttaag 4320
   aaattaaaaa aactaaggaa acatttttct tgtttcgagt agataatgcc agcctgttaa 4380
   acgccgtcga cgagtctaac ggacaccaac cagcgaacca gcagcgtcgc gtcgggccaa 4440
   gcgaagcaga cggcacggca tctctgtcgc tgcctctgga cccctctcga gagttccgct 4500
   ccaccgttgg acttgctccg ctgtcggcat ccagaaattg cgtggcggag cggcagacgt 4560
   gagccggcac ggcaggcggc ctcctcctcc tctcacggca ccggcagcta cgggggattc 4620
   ctttcccacc gctccttcgc tttcccttcc tcgcccgccg taataaatag acaccccctc 4680
   cacaccctct ttccccaacc tcgtgttgtt cggagcgcac acacacacaa ccagatctcc 4740
   cccaaatcca cccgtcggca cctccgcttc aaggtacgcc gctcgtcctc cccccccccc 4800
   cctctctacc ttctctagat cggcgttccg gtccatggtt agggcccggt agttctactt 4860
   ctgttcatgt ttgtgttaga tccgtgtttg tgttagatcc gtgctgctag cgttcgtaca 4920
   cggatgcgac ctgtacgtca gacacgttct gattgctaac ttgccagtgt ttctctttgg 4980
   ggaatcctgg gatggctcta gccgttccgc agacgggatc gatttcatga ttttttttgt 5040
   ttcgttgcat agggtttggt ttgccctttt cctttatttc aatatatgcc gtgcacttgt 5100
   ttgtcgggtc atcttttcat gctttttttt gtcttggttg tgatgatgtg gtctggttgg 5160
   gcggtcgttc tagatcggag tagaattctg tttcaaacta cctggtggat ttattaattt 5220
   tggatctgta tgtgtgtgcc atacatattc atagttacga attgaagatg atggatggaa 5280
   atatcgatct aggataggta tacatgttga tgcgggtttt actgatgcat atacagagat 5340
   gctttttgtt cgcttggttg tgatgatgtg gtgtggttgg gcggtcgttc attcgttcta 5400
   gatcggagta gaatactgtt tcaaactacc tggtgtattt attaattttg gaactgtatg 5460
   tgtgtgtcat acatcttcat agttacgagt ttaagatgga tggaaatatc gatctaggat 5520
   aggtatacat gttgatgtgg gttttactga tgcatataca tgatggcata tgcagcatct 5580
   attcatatgc tctaaccttg agtacctatc tattataata aacaagtatg ttttataatt 5640
   attttgatct tgatatactt ggatgatggc atatgcagca gctatatgtg gattttttta 5700
   gccctgcctt catacgctat ttatttgctt ggtactgttt cttttgtcga tgctcaccct 5760
   gttgtttggt gttacttctg cagggatccc cgatcatgca aaaactcatt aactcagtgc 5820
   aaaactatgc ctggggcagc aaaacggcgt tgactgaact ttatggtatg gaaaatccgt 5880
   ccagccagcc gatggccgag ctgtggatgg gcgcacatcc gaaaagcagt tcacgagtgc 5940
   agaatgccgc cggagatatc gtttcactgc gtgatgtgat tgagagtgat aaatcgactc 6000
   tgctcggaga ggccgttgcc aaacgctttg gcgaactgcc tttcctgttc aaagtattat 6060
   gcgcagcaca gccactctcc attcaggttc atccaaacaa acacaattct gaaatcggtt 6120
   ttgccaaaga aaatgccgca ggtatcccga tggatgccgc cgagcgtaac tataaagatc 6180
   ctaaccacaa gccggagctg gtttttgcgc tgacgccttt ccttgcgatg aacgcgtttc 6240
   gtgaattttc cgagattgtc tccctactcc agccggtcgc aggtgcacat ccggcgattg 6300
   ctcacttttt acaacagcct gatgccgaac gtttaagcga actgttcgcc agcctgttga 6360
   atatgcaggg tgaagaaaaa tcccgcgcgc tggcgatttt aaaatcggcc ctcgatagcc 6420
   agcagggtga accgtggcaa acgattcgtt taatttctga attttacccg gaagacagcg 6480
   gtctgttctc cccgctattg ctgaatgtgg tgaaattgaa ccctggcgaa gcgatgttcc 6540
   tgttcgctga aacaccgcac gcttacctgc aaggcgtggc gctggaagtg atggcaaact 6600
   ccgataacgt gctgcgtgcg ggtctgacgc ctaaatacat tgatattccg gaactggttg 6660
   ccaatgtgaa attcgaagcc aaaccggcta accagttgtt gacccagccg gtgaaacaag 6720
   gtgcagaact ggacttcccg attccagtgg atgattttgc cttctcgctg catgacctta 6780
   gtgataaaga aaccaccatt agccagcaga gtgccgccat tttgttctgc gtcgaaggcg 6840
   atgcaacgtt gtggaaaggt tctcagcagt tacagcttaa accgggtgaa tcagcgttta 6900
   ttgccgccaa cgaatcaccg gtgactgtca aaggccacgg ccgtttagcg cgtgtttaca 6960
   acaagctgta agagcttact gaaaaaatta acatctcttg ctaagctggg agctcgatcc 7020
   gtcgacctgc agatcgttca aacatttggc aataaagttt cttaagattg aatcctgttg 7080
   ccggtcttgc gatgattatc atataatttc tgttgaatta cgttaagcat gtaataatta 7140
   acatgtaatg catgacgtta tttatgagat gggtttttat gattagagtc ccgcaattat 7200
   acatttaata cgcgatagaa aacaaaatat agcgcgcaaa ctaggataaa ttatcgcgcg 7260
   cggtgtcatc tatgttacta gatctgctag ccctgcagga aatttaccgg tgcccgggcg 7320
   gccagcatgg ccgtatccgc aatgtgttat taagttgtct aagcgtcaat ttgtttacac 7380
   cacaatatat cctgccacca gccagccaac agctccccga ccggcagctc ggcacaaaat 7440
   caccactcga tacaggcagc ccatcagaat taattctcat gtttgacagc ttatcatcga 7500
   ctgcacggtg caccaatgct tctggcgtca ggcagccatc ggaagctgtg gtatggctgt 7560
   gcaggtcgta aatcactgca taattcgtgt cgctcaaggc gcactcccgt tctggataat 7620
   gttttttgcg ccgacatcat aacggttctg gcaaatattc tgaaatgagc tgttgacaat 7680
   taatcatccg gctcgtataa tgtgtggaat tgtgagcgga taacaatttc acacaggaaa 7740
   cagaccatga gggaagcgtt gatcgccgaa gtatcgactc aactatcaga ggtagttggc 7800
   gtcatcgagc gccatctcga accgacgttg ctggccgtac atttgtacgg ctccgcagtg 7860
   gatggcggcc tgaagccaca cagtgatatt gatttgctgg ttacggtgac cgtaaggctt 7920
   gatgaaacaa cgcggcgagc tttgatcaac gaccttttgg aaacttcggc ttcccctgga 7980
   gagagcgaga ttctccgcgc tgtagaagtc accattgttg tgcacgacga catcattccg 8040
   tggcgttatc cagctaagcg cgaactgcaa tttggagaat ggcagcgcaa tgacattctt 8100
   gcaggtatct tcgagccagc cacgatcgac attgatctgg ctatcttgct gacaaaagca 8160
   agagaacata gcgttgcctt ggtaggtcca gcggcggagg aactctttga tccggttcct 8220
   gaacaggatc tatttgaggc gctaaatgaa accttaacgc tatggaactc gccgcccgac 8280
   tgggctggcg atgagcgaaa tgtagtgctt acgttgtccc gcatttggta cagcgcagta 8340
   accggcaaaa tcgcgccgaa ggatgtcgct gccgactggg caatggagcg cctgccggcc 8400
   cagtatcagc ccgtcatact tgaagctagg caggcttatc ttggacaaga agatcgcttg 8460
   gcctcgcgcg cagatcagtt ggaagaattt gttcactacg tgaaaggcga gatcaccaaa 8520
   gtagtcggca aataaagctc tagtggatct ccgtaccccc gggggatctg gctcgcggcg 8580
   gacgcacgac gccggggcga gaccataggc gatctcctaa atcaatagta gctgtaacct 8640
   cgaagcgttt cacttgtaac aacgattgag aatttttgtc ataaaattga aatacttggt 8700
   tcgcattttt gtcatccgcg gtcagccgca attctgacga actgcccatt tagctggaga 8760
   tgattgtaca tccttcacgt gaaaatttct caagcgctgt gaacaagggt tcagatttta 8820
   gattgaaagg tgagccgttg aaacacgttc ttcttgtcga tgacgacgtc gctatgcggc 8880
   atcttattat tgaatacctt acgatccacg ccttcaaagt gaccgcggta gccgacagca 8940
   cccagttcac aagagtactc tcttccgcga cggtcgatgt cgtggttgtt gatctaaatt 9000
   taggtcgtga agatgggctc gagatcgttc gtaatctggc ggcaaagtct gatattccaa 9060
   tcataattat cagtggcgac cgccttgagg agacggataa agttgttgca ctcgagctag 9120
   gagcaagtga ttttatcgct aagccgttca gtatcagaga gtttctagca cgcattcggg 9180
   ttgccttgcg cgtgcgcccc aacgttgtcc gctccaaaga ccgacggtct ttttgtttta 9240
   ctgactggac acttaatctc aggcaacgtc gcttgatgtc cgaagctggc ggtgaggtga 9300
   aacttacggc aggtgagttc aatcttctcc tcgcgttttt agagaaaccc cgcgacgttc 9360
   tatcgcgcga gcaacttctc attgccagtc gagtacgcga cgaggaggtt tatgacagga 9420
   gtatagatgt tctcattttg aggctgcgcc gcaaacttga ggcagatccg tcaagccctc 9480
   aactgataaa aacagcaaga ggtgccggtt atttctttga cgcggacgtg caggtttcgc 9540
   acggggggac gatggcagcc tgagccaatt cccagatccc cgaggaatcg gcgtgagcgg 9600
   tcgcaaacca tccggcccgg tacaaatcgg cgcggcgctg ggtgatgacc tggtggagaa 9660
   gttgaaggcc gcgcaggccg cccagcggca acgcatcgag gcagaagcac gccccggtga 9720
   atcgtggcaa gcggccgctg atcgaatccg caaagaatcc cggcaaccgc cggcagccgg 9780
   tgcgccgtcg attaggaagc cgcccaaggg cgacgagcaa ccagattttt tcgttccgat 9840
   gctctatgac gtgggcaccc gcgatagtcg cagcatcatg gacgtggccg ttttccgtct 9900
   gtcgaagcgt gaccgacgag ctggcgaggt gatccgctac gagcttccag acgggcacgt 9960
   agaggtttcc gcagggccgg ccggcatggc cagtgtgtgg gattacgacc tggtactgat 10020
   ggcggtttcc catctaaccg aatccatgaa ccgataccgg gaagggaagg gagacaagcc 10080
   cggccgcgtg ttccgtccac acgttgcgga cgtactcaag ttctgccggc gagccgatgg 10140
   cggaaagcag aaagacgacc tggtagaaac ctgcattcgg ttaaacacca cgcacgttgc 10200
   catgcagcgt acgaagaagg ccaagaacgg ccgcctggtg acggtatccg agggtgaagc 10260
   cttgattagc cgctacaaga tcgtaaagag cgaaaccggg cggccggagt acatcgagat 10320
   cgagctagct gattggatgt accgcgagat cacagaaggc aagaacccgg acgtgctgac 10380
   ggttcacccc gattactttt tgatcgatcc cggcatcggc cgttttctct accgcctggc 10440
   acgccgcgcc gcaggcaagg cagaagccag atggttgttc aagacgatct acgaacgcag 10500
   tggcagcgcc ggagagttca agaagttctg tttcaccgtg cgcaagctga tcgggtcaaa 10560
   tgacctgccg gagtacgatt tgaaggagga ggcggggcag gctggcccga tcctagtcat 10620
   gcgctaccgc aacctgatcg agggcgaagc atccgccggt tcctaatgta cggagcagat 10680
   gctagggcaa attgccctag caggggaaaa aggtcgaaaa ggtctctttc ctgtggatag 10740
   cacgtacatt gggaacccaa agccgtacat tgggaaccgg aacccgtaca ttgggaaccc 10800
   aaagccgtac attgggaacc ggtcacacat gtaagtgact gatataaaag agaaaaaagg 10860
   cgatttttcc gcctaaaact ctttaaaact tattaaaact cttaaaaccc gcctggcctg 10920
   tgcataactg tctggccagc gcacagccga agagctgcaa aaagcgccta cccttcggtc 10980
   gctgcgctcc ctacgccccg ccgcttcgcg tcggcctatc gcggccgctg gccgctcaaa 11040
   aatggctggc ctacggccag gcaatctacc agggcgcgga caagccgcgc cgtcgccact 11100
   cgaccgccgg cgctgaggtc tgcctcgtga agaaggtgtt gctgactcat accaggcctg 11160
   aatcgcccca tcatccagcc agaaagtgag ggagccacgg ttgatgagag ctttgttgta 11220
   ggtggaccag ttggtgattt tgaacttttg ctttgccacg gaacggtctg cgttgtcggg 11280
   aagatgcgtg atctgatcct tcaactcagc aaaagttcga tttattcaac aaagccgccg 11340
   tcccgtcaag tcagcgtaat gctctgccag tgttacaacc aattaaccaa ttctgattag 11400
   aaaaactcat cgagcatcaa atgaaactgc aatttattca tatcaggatt atcaatacca 11460
   tatttttgaa aaagccgttt ctgtaatgaa ggagaaaact caccgaggca gttccatagg 11520
   atggcaagat cctggtatcg gtctgcgatt ccgactcgtc caacatcaat acaacctatt 11580
   aatttcccct cgtcaaaaat aaggttatca agtgagaaat caccatgagt gacgactgaa 11640
   tccggtgaga atggcaaaag ctctgcatta atgaatcggc caacgcgcgg ggagaggcgg 11700
   tttgcgtatt gggcgctctt ccgcttcctc gctcactgac tcgctgcgct cggtcgttcg 11760
   gctgcggcga gcggtatcag ctcactcaaa ggcggtaata cggttatcca cagaatcagg 11820
   ggataacgca ggaaagaaca tgtgagcaaa aggccagcaa aaggccagga accgtaaaaa 11880
   ggccgcgttg ctggcgtttt tccataggct ccgcccccct gacgagcatc acaaaaatcg 11940
   acgctcaagt cagaggtggc gaaacccgac aggactataa agataccagg cgtttccccc 12000
   tggaagctcc ctcgtgcgct ctcctgttcc gaccctgccg cttaccggat acctgtccgc 12060
   ctttctccct tcgggaagcg tggcgctttc tcatagctca cgctgtaggt atctcagttc 12120
   ggtgtaggtc gttcgctcca agctgggctg tgtgcacgaa ccccccgttc agcccgaccg 12180
   ctgcgcctta tccggtaact atcgtcttga gtccaacccg gtaagacacg acttatcgcc 12240
   actggcagca gccactggta acaggattag cagagcgagg tatgtaggcg gtgctacaga 12300
   gttcttgaag tggtggccta actacggcta cactagaaga acagtatttg gtatctgcgc 12360
   tctgctgaag ccagttacct tcggaaaaag agttggtagc tcttgatccg gcaaacaaac 12420
   caccgctggt agcggtggtt tttttgtttg caagcagcag attacgcgca gaaaaaaagg 12480
   atctcaagaa gatcctttga tcttttctac ggggtctgac gctcagtgga acgaaaactc 12540
   acgttaaggg attttggtca tgagattatc aaaaaggatc ttcacctaga tccttttgat 12600
   ccggaattaa ttcctgtggt tggcatgcac atacaaatgg acgaacggat aaaccttttc 12660
   acgccctttt aaatatccga ttattctaat aaacgctctt ttctcttagg tttacccgcc 12720
   aatatatcct gtcaaacact gatagtttaa actgaaggcg ggaaacgaca atctgatcat 12780
   gagcggagaa ttaagggagt cacgttatga cccccgccga tgacgcggga caagccgttt 12840
   tacgtttgga actgacagaa ccgcaacgct gcaggaattg gccgcagcgg ccatttaaat 12900
   caattgggcg cgccgaattc gagctcggta caagcttgca catgacaaca attgtaagag 12960
   gatggagacc acaacgatcc aacaatactt ctgcgacggg ctgtgaagta tagagaagtt 13020
   aaacgcccaa aagccattgt gtttggaatt tttagttatt ctatttttca tgatgtatct 13080
   tcctctaaca tgccttaatt tgcaaatttg gtataactac tgattgaaaa tatatgtatg 13140
   taaaaaaata ctaagcatat ttgtgaagct aaacatgatg ttatttaaga aaatatgttg 13200
   ttaacagaat aagattaata tcgaaatgga aacatctgta aattagaatc atcttacaag 13260
   ctaagagatg ttcacgcttt gagaaacttc ttcagatcat gaccgtagaa gtagctctcc 13320
   aagactcaac gaaggctgct gcaattccac aaatgcatga catgcatcct tgtaaccgtc 13380
   gtcgccgcta taaacacgga taactcaatt ccctgctcca tcaatttaga aatgagcaag 13440
   caagcacccg atcgctcacc ccatatgcac caatctgact cccaagtctc tgtttcgcat 13500
   tagtaccgcc agcactccac ctatagctac caattgagac ctttccagcc taagcagatc 13560
   gattgatcgt tagagtcaaa gagttggtgg tacgggtact ttaactacca tggaatgatg 13620
   gggcgtgatg tagagcggaa agcgcctccc tacgcggaac aacaccctcg ccatgccgct 13680
   cgactacagc ctcctcctcg tcggccgccc acaacgaggg agcccgtggt cgcagccacc 13740
   gaccagcatg tctctgtgtc ctcgtccgac ctcgacatgt catggcaaac agtcggacgc 13800
   cagcaccaga ctgacgacat gagtctctga agagcccgcc acctagaaag atccgagccc 13860
   tgctgctggt agtggtaacc attttcgtcg cgctgacgcg gagagcgaga ggccagaaat 13920
   ttatagcgac tgacgctgtg gcaggcacgc tatcggaggt tacgacgtgg cgggtcactc 13980
   gacgcggagt tcacaggtcc tatccttgca tcgctcgggc cggagtttac gggacttatc 14040
   cttacgacgt gctctaaggt tgcgataacg ggcggaggaa ggcgtgtggc gtgcggagac 14100
   ggtttataca cgtagtgtgc gggagtgtgt ttcgtagacg cgggaaagca cgacgactta 14160
   cgaaggttag tggaggagga ggacacacta aaatcaggac gcaagaaact cttctattat 14220
   agtagtagag aagagattat aggagtgtgg gttgattcta aagaaaatcg acgcaggaca 14280
   accgtcaaaa cgggtgcttt aatatagtag atatatatat atagagagag agagaaagta 14340
   caaaggatgc atttgtgtct gcatatgatc ggagtattac taacggccgt cgtaagaagg 14400
   tccatcatgc gtggagcgag cccatttggt tggttgtcag gccgcagtta aggcctccat 14460
   atatgattgt cgtcgggccc ataacagcat ctcctccacc agtttattgt aagaataaat 14520
   taagtagaga tatttgtcgt cgggcagaag aaacttggac aagaagaaga agcaagctag 14580
   gccaatttct tgccggcaag aggaagatag tggcctctag tttatatatc ggcgtgatga 14640
   tgatgctcct agctagaaat gagagaagaa aaacggacgc gtgtttggtg tgtgtcaatg 14700
   gcgtccatcc ttccatcaga tcagaacgat gaaaaagtca agcacggcat gcatagtata 14760
   tgtatagctt gttttagtgt ggctttgctg agacgaatga aagcaacggc gggcatattt 14820
   ttcagtggct gtagctttca ggctgaaaga gacgtggcat gcaataattc agggaattcg 14880
   tcagccaatt gaggtagcta gtcaacttgt acattggtgc gagcaatttt ccgcactcag 14940
   gagggctagt ttgagagtcc aaaaactata ggagattaaa gaggctaaaa tcctctcctt 15000
   atttaatttt aaataagtag tgtatttgta ttttaactcc tccaaccctt ccgattttat 15060
   ggctctcaaa ctagcattca gtctaatgca tgcatgcttg gctagaggtc gtatggggtt 15120
   gttaatagca tagctagcta caagttaacc gggtctttta tatttaataa ggacaggcaa 15180
   agtattactt acaaataaag aataaagcta ggacgaactc gtggattatt actaaatcga 15240
   aatggacgta atattccagg caagaataat tgttcgatca ggagacaagt ggggcattgg 15300
   accggttctt gcaagcaaga gcctatggcg tggtgacacg gcgcgttgcc catacatcat 15360
   gcctccatcg atgatccatc ctcacttgct ataaaaagag gtgtccatgg tgctcaagct 15420
   cagccaagca aataagacga cttgtttcat tgattcttca agagatcgag cttcttttgc 15480
   accacaaggt cgaggatcca aca                                         15503
<210> 15
   <211> 14946
   <212> DNA
   <213> Artificial Sequence
<220>
   <223> Description of Artificial Sequence: pNOV1313
<220>
   <221> misc_feature
   <222> (12)..(1993)
   <223> Zm Ubi promoter
<220>
   <221> misc_feature
   <222> (2016)..(5597)
   <223> synthetic nucleotide sequence encoding the toxin portion of H04 plus a full-length Cry1Ab tail portion
<220>
   <221> misc_feature
   <222> (5805)..(7786)
   <223> Zm Ubi promoter
<220>
   <221> misc_feature
   <222> (7883)..(8986)
   <223> PMI
<400> 15
   aagcttgcat gcctgcagtg cagcgtgacc cggtcgtgcc cctctctaga gataatgagc 60
   attgcatgtc taagttataa aaaattacca catatttttt ttgtcacact tgtttgaagt 120
   gcagtttatc tatctttata catatattta aactttactc tacgaataat ataatctata 180
   gtactacaat aatatcagtg ttttagagaa tcatataaat gaacagttag acatggtcta 240
   aaggacaatt gagtattttg acaacaggac tctacagttt tatcttttta gtgtgcatgt 300
   gttctccttt ttttttgcaa atagcttcac ctatataata cttcatccat tttattagta 360
   catccattta gggtttaggg ttaatggttt ttatagacta atttttttag tacatctatt 420
   ttattctatt ttagcctcta aattaagaaa actaaaactc tattttagtt tttttattta 480
   ataatttaga tataaaatag aataaaataa agtgactaaa aattaaacaa atacccttta 540
   agaaattaaa aaaactaagg aaacattttt cttgtttcga gtagataatg ccagcctgtt 600
   aaacgccgtc gacgagtcta acggacacca accagcgaac cagcagcgtc gcgtcgggcc 660
   aagcgaagca gacggcacgg catctctgtc gctgcctctg gacccctctc gagagttccg 720
   ctccaccgtt ggacttgctc cgctgtcggc atccagaaat tgcgtggcgg agcggcagac 780
   gtgagccggc acggcaggcg gcctcctcct cctctcacgg caccggcagc tacgggggat 840
   tcctttccca ccgctccttc gctttccctt cctcgcccgc cgtaataaat agacaccccc 900
   tccacaccct ctttccccaa cctcgtgttg ttcggagcgc acacacacac aaccagatct 960
   cccccaaatc cacccgtcgg cacctccgct tcaaggtacg ccgctcgtcc tccccccccc 1020
   cccctctcta ccttctctag atcggcgttc cggtccatgg ttagggcccg gtagttctac 1080
   ttctgttcat gtttgtgtta gatccgtgtt tgtgttagat ccgtgctgct agcgttcgta 1140
   cacggatgcg acctgtacgt cagacacgtt ctgattgcta acttgccagt gtttctcttt 1200
   ggggaatcct gggatggctc tagccgttcc gcagacggga tcgatttcat gatttttttt 1260
   gtttcgttgc atagggtttg gtttgccctt ttcctttatt tcaatatatg ccgtgcactt 1320
   gtttgtcggg tcatcttttc atgctttttt ttgtcttggt tgtgatgatg tggtctggtt 1380
   gggcggtcgt tctagatcgg agtagaattc tgtttcaaac tacctggtgg atttattaat 1440
   tttggatctg tatgtgtgtg ccatacatat tcatagttac gaattgaaga tgatggatgg 1500
   aaatatcgat ctaggatagg tatacatgtt gatgcgggtt ttactgatgc atatacagag 1560
   atgctttttg ttcgcttggt tgtgatgatg tggtgtggtt gggcggtcgt tcattcgttc 1620
   tagatcggag tagaatactg tttcaaacta cctggtgtat ttattaattt tggaactgta 1680
   tgtgtgtgtc atacatcttc atagttacga gtttaagatg gatggaaata tcgatctagg 1740
   ataggtatac atgttgatgt gggttttact gatgcatata catgatggca tatgcagcat 1800
   ctattcatat gctctaacct tgagtaccta tctattataa taaacaagta tgttttataa 1860
   ttattttgat cttgatatac ttggatgatg gcatatgcag cagctatatg tggatttttt 1920
   tagccctgcc ttcatacgct atttatttgc ttggtactgt ttcttttgtc gatgctcacc 1980
   ctgttgtttg gtgttacttc tgcagggatc caacaatgga caacaacccc aacatcaacg 2040
   agtgcatccc ctacaactgc ctgagcaacc ccgaggtgga ggtgctgggc ggcgagcgca 2100
   tcgagaccgg ctacaccccc atcgacatca gcctgagcct gacccagttc ctgctgagcg 2160
   agttcgtgcc cggcgccggc ttcgtgctgg gcctggtgga catcatctgg ggcatcttcg 2220
   gccccagcca gtgggacgcc ttcctggtgc agatcgagca gttgataaac caacgcatag 2280
   aggaattcgc ccgcaaccag gccatcagcc gcctggaggg cctgagcaac ctgtaccaaa 2340
   tctacgccga gagcttccgc gagtgggagg ccgaccccac caaccccgcc ctgcgcgagg 2400
   agatgcgcat ccagttcaac gacatgaaca gcgccctgac caccgccatc cccctgttcg 2460
   ccgtgcagaa ctaccaggtg cccctgctga gcgtgtacgt gcaggccgcc aacctgcacc 2520
   tgagcgtgct gcgcgacgtc agcgtgttcg gccagcgctg gggcttcgac gccgccacca 2580
   tcaacagccg ctacaacgac ctgacccgcc tgatcggcaa ctacaccgac cacgccgtgc 2640
   gctggtacaa caccggcctg gagcgcgtgt ggggtcccga cagccgcgac tggatcaggt 2700
   acaaccagtt ccgccgcgag ctgaccctga ccgtgctgga catcgtgagc ctgttcccca 2760
   actacgacag ccgcacctac cccatccgca ccgtgagcca gctgacccgc gagatttaca 2820
   ccaaccccgt gctggagaac ttcgacggca gcttccgcgg cagcgcccag ggcatcgagg 2880
   gcagcatccg cagcccccac ctgatggaca tcctgaacag catcaccatc tacaccgacg 2940
   cccaccgcgg cgagtactac tggagcggcc accagatcat ggccagcccc gtcggcttca 3000
   gcggccccga gttcaccttc cccctgtacg gcaccatggg caacgctgca cctcagcagc 3060
   gcatcgtggc acagctgggc cagggagtgt accgcaccct gagcagcacc ctgtaccgtc 3120
   gacctttcaa catcggcatc aacaaccagc agctgagcgt gctggacggc accgagttcg 3180
   cctacggcac cagcagcaac ctgcccagcg ccgtgtaccg caagagcggc accgtggaca 3240
   gcctggacga gatcccccct cagaacaaca acgtgccacc tcgacagggc ttcagccacc 3300
   gtctgagcca cgtgagcatg ttccgcagtg gcttcagcaa cagcagcgtg agcatcatcc 3360
   gtgcacccat gttcagctgg attcaccgca gcgccaccct gaccaacacc atcgaccccg 3420
   agcgcatcaa ccagatcccc ctggtgaagg gcttccgggt gtggggcggc accagcgtga 3480
   tcaccggccc cggcttcacc ggaggcgaca tcctgcgcag aaacaccttc ggcgacttcg 3540
   tgagcctgca ggtgaacatc aacagcccca tcacccagcg ttaccgcctg cgcttccgct 3600
   acgccagcag ccgcgacgcc cgtgtgatcg tgctgactgg cgccgctagc accggtgtgg 3660
   gcggtcaggt gagcgtgaac atgcccctgc agaagactat ggagatcggc gagaacctga 3720
   ctagtcgcac cttccgctac accgacttca gcaacccctt cagcttccgc gccaaccccg 3780
   acatcatcgg catcagcgag cagcccctgt tcggtgccgg cagcatcagc agcggcgagc 3840
   tgtacatcga caagatcgag atcatcctgg ccgacgccac cttcgaggcc gagagcgacc 3900
   tggagcgcgc ccagaaggcc gtgaacgccc tgttcaccag cagcaaccag atcggcctga 3960
   agaccgacgt gaccgactac cacatcgacc aggtgagcaa cctggtggac tgcttaagcg 4020
   acgagttctg cctggacgag aagaaggagc tgagcgagaa ggtgaagcac gccaagcgcc 4080
   tgagcgacga gcgcaacctg ctgcaggacc ccaacttccg cggcatcaac cgccagctgg 4140
   accgcggctg gcgaggcagc accgatatca ccatccaggg cggcgacgac gtgttcaagg 4200
   agaactacgt gaccctgcag ggcaccttcg acgagtgcta ccccacctac ctgtaccagc 4260
   cgatcgacga gagcaagctg aaggcctaca cccgctacca gctgcgcggc tacatcgagg 4320
   acagccagga cctggaaatc tacctgatcc gctacaacgc gaagcacgag accgtgaacg 4380
   tgcccggcac cggcagcctg tggcccctga gcgcccccag ccccatcggc aagtgcgggg 4440
   agccgaatcg atgcgctccg cacctggagt ggaacccgga cctagactgc agctgcaggg 4500
   acggggagaa gtgcgcccac cacagccacc acttcagcct ggacatcgac gtgggctgca 4560
   ccgacctgaa cgaggacctg ggcgtgtggg tgatcttcaa gatcaagacc caggacggcc 4620
   acgcccgcct gggcaatcta gagttcctgg aggagaagcc cctggtgggc gaggccctgg 4680
   cccgcgtgaa gcgtgctgag aagaagtggc gcgacaagcg cgagaagctg gagtgggaga 4740
   ccaacatcgt gtacaaggag gccaaggaga gcgtggacgc cctgttcgtg aacagccagt 4800
   acgaccgcct gcaggccgac accaacatcg ccatgatcca cgccgccgac aagcgcgtgc 4860
   acagcattcg cgaggcctac ctgcccgagc tgagcgtgat ccccggtgtg aacgccgcca 4920
   tcttcgagga actcgagggc cgcatcttca ccgccttcag cctgtacgac gcccgcaacg 4980
   tgatcaagaa cggcgacttc aacaacggcc tgagctgctg gaacgtgaag ggccacgtgg 5040
   acgtggagga gcagaacaac caccgcagcg tgctggtggt gcccgagtgg gaggccgagg 5100
   tgagccagga ggtgcgcgtg tgccccggcc gcggctacat cctgcgcgtg accgcctaca 5160
   aggagggcta cggcgagggc tgcgtgacca tccacgagat cgagaacaac accgacgagc 5220
   tcaagttcag caactgcgtg gaggaggagg tttaccccaa caacaccgtg acctgcaacg 5280
   actacaccgc gacccaggag gagtacgaag gcacctacac ctctcgcaac aggggttacg 5340
   acggcgccta cgagtccaac agctccgtgc cagctgacta cgccagcgcc tacgaggaga 5400
   aagcctacac cgacggtaga cgcgacaacc catgtgagag caacagaggc tacggcgact 5460
   acacccccct gcccgctgga tacgtgacca aggagctgga gtacttcccc gagaccgaca 5520
   aggtgtggat cgagattggc gagaccgagg gcaccttcat cgtggacagc gtggagctgc 5580
   tgctgatgga ggagtagtag atctgttctg cacaaagtgg agtagtcagt catcgatcag 5640
   gaaccagaca ccagactttt attcatacag tgaagtgaag tgaagtgcag tgcagtgagt 5700
   tgctggtttt tgtacaactt agtatgtatt tgtatttgta aaatacttct atcaataaaa 5760
   tttctaattc ctaaaaccaa aatccagggg taccagcttg catgcctgca gtgcagcgtg 5820
   acccggtcgt gcccctctct agagataatg agcattgcat gtctaagtta taaaaaatta 5880
   ccacatattt tttttgtcac acttgtttga agtgcagttt atctatcttt atacatatat 5940
   ttaaacttta ctctacgaat aatataatct atagtactac aataatatca gtgttttaga 6000
   gaatcatata aatgaacagt tagacatggt ctaaaggaca attgagtatt ttgacaacag 6060
   gactctacag ttttatcttt ttagtgtgca tgtgttctcc tttttttttg caaatagctt 6120
   cacctatata atacttcatc cattttatta gtacatccat ttagggttta gggttaatgg 6180
   tttttataga ctaatttttt tagtacatct attttattct attttagcct ctaaattaag 6240
   aaaactaaaa ctctatttta gtttttttat ttaataattt agatataaaa tagaataaaa 6300
   taaagtgact aaaaattaaa caaataccct ttaagaaatt aaaaaaacta aggaaacatt 6360
   tttcttgttt cgagtagata atgccagcct gttaaacgcc gtcgacgagt ctaacggaca 6420
   ccaaccagcg aaccagcagc gtcgcgtcgg gccaagcgaa gcagacggca cggcatctct 6480
   gtcgctgcct ctggacccct ctcgagagtt ccgctccacc gttggacttg ctccgctgtc 6540
   ggcatccaga aattgcgtgg cggagcggca gacgtgagcc ggcacggcag gcggcctcct 6600
   cctcctctca cggcaccggc agctacgggg gattcctttc ccaccgctcc ttcgctttcc 6660
   cttcctcgcc cgccgtaata aatagacacc ccctccacac cctctttccc caacctcgtg 6720
   ttgttcggag cgcacacaca cacaaccaga tctcccccaa atccacccgt cggcacctcc 6780
   gcttcaaggt acgccgctcg tcctcccccc ccccccctct ctaccttctc tagatcggcg 6840
   ttccggtcca tggttagggc ccggtagttc tacttctgtt catgtttgtg ttagatccgt 6900
   gtttgtgtta gatccgtgct gctagcgttc gtacacggat gcgacctgta cgtcagacac 6960
   gttctgattg ctaacttgcc agtgtttctc tttggggaat cctgggatgg ctctagccgt 7020
   tccgcagacg ggatcgattt catgattttt tttgtttcgt tgcatagggt ttggtttgcc 7080
   cttttccttt atttcaatat atgccgtgca cttgtttgtc gggtcatctt ttcatgcttt 7140
   tttttgtctt ggttgtgatg atgtggtctg gttgggcggt cgttctagat cggagtagaa 7200
   ttctgtttca aactacctgg tggatttatt aattttggat ctgtatgtgt gtgccataca 7260
   tattcatagt tacgaattga agatgatgga tggaaatatc gatctaggat aggtatacat 7320
   gttgatgcgg gttttactga tgcatataca gagatgcttt ttgttcgctt ggttgtgatg 7380
   atgtggtgtg gttgggcggt cgttcattcg ttctagatcg gagtagaata ctgtttcaaa 7440
   ctacctggtg tatttattaa ttttggaact gtatgtgtgt gtcatacatc ttcatagtta 7500
   cgagtttaag atggatggaa atatcgatct aggataggta tacatgttga tgtgggtttt 7560
   actgatgcat atacatgatg gcatatgcag catctattca tatgctctaa ccttgagtac 7620
   ctatctatta taataaacaa gtatgtttta taattatttt gatcttgata tacttggatg 7680
   atggcatatg cagcagctat atgtggattt ttttagccct gccttcatac gctatttatt 7740
   tgcttggtac tgtttctttt gtcgatgctc accctgttgt ttggtgttac ttctgcaggg 7800
   atccccgatc atgcaaaaac tcattaactc agtgcaaaac tatgcctggg gcagcaaaac 7860
   ggcgttgact gaactttatg gtatggaaaa tccgtccagc cagccgatgg ccgagctgtg 7920
   gatgggcgca catccgaaaa gcagttcacg agtgcagaat gccgccggag atatcgtttc 7980
   actgcgtgat gtgattgaga gtgataaatc gactctgctc ggagaggccg ttgccaaacg 8040
   ctttggcgaa ctgcctttcc tgttcaaagt attatgcgca gcacagccac tctccattca 8100
   ggttcatcca aacaaacaca attctgaaat cggttttgcc aaagaaaatg ccgcaggtat 8160
   cccgatggat gccgccgagc gtaactataa agatcctaac cacaagccgg agctggtttt 8220
   tgcgctgacg cctttccttg cgatgaacgc gtttcgtgaa ttttccgaga ttgtctccct 8280
   actccagccg gtcgcaggtg cacatccggc gattgctcac tttttacaac agcctgatgc 8340
   cgaacgttta agcgaactgt tcgccagcct gttgaatatg cagggtgaag aaaaatcccg 8400
   cgcgctggcg attttaaaat cggccctcga tagccagcag ggtgaaccgt ggcaaacgat 8460
   tcgtttaatt tctgaatttt acccggaaga cagcggtctg ttctccccgc tattgctgaa 8520
   tgtggtgaaa ttgaaccctg gcgaagcgat gttcctgttc gctgaaacac cgcacgctta 8580
   cctgcaaggc gtggcgctgg aagtgatggc aaactccgat aacgtgctgc gtgcgggtct 8640
   gacgcctaaa tacattgata ttccggaact ggttgccaat gtgaaattcg aagccaaacc 8700
   ggctaaccag ttgttgaccc agccggtgaa acaaggtgca gaactggact tcccgattcc 8760
   agtggatgat tttgccttct cgctgcatga ccttagtgat aaagaaacca ccattagcca 8820
   gcagagtgcc gccattttgt tctgcgtcga aggcgatgca acgttgtgga aaggttctca 8880
   gcagttacag cttaaaccgg gtgaatcagc gtttattgcc gccaacgaat caccggtgac 8940
   tgtcaaaggc cacggccgtt tagcgcgtgt ttacaacaag ctgtaagagc ttactgaaaa 9000
   aattaacatc tcttgctaag ctgggagctc gatccgtcga cctgcagatc gttcaaacat 9060
   ttggcaataa agtttcttaa gattgaatcc tgttgccggt cttgcgatga ttatcatata 9120
   atttctgttg aattacgtta agcatgtaat aattaacatg taatgcatga cgttatttat 9180
   gagatgggtt tttatgatta gagtcccgca attatacatt taatacgcga tagaaaacaa 9240
   aatatagcgc gcaaactagg ataaattatc gcgcgcggtg tcatctatgt tactagatct 9300
   gctagccctg caggaaattt accggtgccc gggcggccag catggccgta tccgcaatgt 9360
   gttattaagt tgtctaagcg tcaatttgtt tacaccacaa tatatcctgc caccagccag 9420
   ccaacagctc cccgaccggc agctcggcac aaaatcacca ctcgatacag gcagcccatc 9480
   agaattaatt ctcatgtttg acagcttatc atcgactgca cggtgcacca atgcttctgg 9540
   cgtcaggcag ccatcggaag ctgtggtatg gctgtgcagg tcgtaaatca ctgcataatt 9600
   cgtgtcgctc aaggcgcact cccgttctgg ataatgtttt ttgcgccgac atcataacgg 9660
   ttctggcaaa tattctgaaa tgagctgttg acaattaatc atccggctcg tataatgtgt 9720
   ggaattgtga gcggataaca atttcacaca ggaaacagac catgagggaa gcgttgatcg 9780
   ccgaagtatc gactcaacta tcagaggtag ttggcgtcat cgagcgccat ctcgaaccga 9840
   cgttgctggc cgtacatttg tacggctccg cagtggatgg cggcctgaag ccacacagtg 9900
   atattgattt gctggttacg gtgaccgtaa ggcttgatga aacaacgcgg cgagctttga 9960
   tcaacgacct tttggaaact tcggcttccc ctggagagag cgagattctc cgcgctgtag 10020
   aagtcaccat tgttgtgcac gacgacatca ttccgtggcg ttatccagct aagcgcgaac 10080
   tgcaatttgg agaatggcag cgcaatgaca ttcttgcagg tatcttcgag ccagccacga 10140
   tcgacattga tctggctatc ttgctgacaa aagcaagaga acatagcgtt gccttggtag 10200
   gtccagcggc ggaggaactc tttgatccgg ttcctgaaca ggatctattt gaggcgctaa 10260
   atgaaacctt aacgctatgg aactcgccgc ccgactgggc tggcgatgag cgaaatgtag 10320
   tgcttacgtt gtcccgcatt tggtacagcg cagtaaccgg caaaatcgcg ccgaaggatg 10380
   tcgctgccga ctgggcaatg gagcgcctgc cggcccagta tcagcccgtc atacttgaag 10440
   ctaggcaggc ttatcttgga caagaagatc gcttggcctc gcgcgcagat cagttggaag 10500
   aatttgttca ctacgtgaaa ggcgagatca ccaaagtagt cggcaaataa agctctagtg 10560
   gatctccgta cccccggggg atctggctcg cggcggacgc acgacgccgg ggcgagacca 10620
   taggcgatct cctaaatcaa tagtagctgt aacctcgaag cgtttcactt gtaacaacga 10680
   ttgagaattt ttgtcataaa attgaaatac ttggttcgca tttttgtcat ccgcggtcag 10740
   ccgcaattct gacgaactgc ccatttagct ggagatgatt gtacatcctt cacgtgaaaa 10800
   tttctcaagc gctgtgaaca agggttcaga ttttagattg aaaggtgagc cgttgaaaca 10860
   cgttcttctt gtcgatgacg acgtcgctat gcggcatctt attattgaat accttacgat 10920
   ccacgccttc aaagtgaccg cggtagccga cagcacccag ttcacaagag tactctcttc 10980
   cgcgacggtc gatgtcgtgg ttgttgatct aaatttaggt cgtgaagatg ggctcgagat 11040
   cgttcgtaat ctggcggcaa agtctgatat tccaatcata attatcagtg gcgaccgcct 11100
   tgaggagacg gataaagttg ttgcactcga gctaggagca agtgatttta tcgctaagcc 11160
   gttcagtatc agagagtttc tagcacgcat tcgggttgcc ttgcgcgtgc gccccaacgt 11220
   tgtccgctcc aaagaccgac ggtctttttg ttttactgac tggacactta atctcaggca 11280
   acgtcgcttg atgtccgaag ctggcggtga ggtgaaactt acggcaggtg agttcaatct 11340
   tctcctcgcg tttttagaga aaccccgcga cgttctatcg cgcgagcaac ttctcattgc 11400
   cagtcgagta cgcgacgagg aggtttatga caggagtata gatgttctca ttttgaggct 11460
   gcgccgcaaa cttgaggcag atccgtcaag ccctcaactg ataaaaacag caagaggtgc 11520
   cggttatttc tttgacgcgg acgtgcaggt ttcgcacggg gggacgatgg cagcctgagc 11580
   caattcccag atccccgagg aatcggcgtg agcggtcgca aaccatccgg cccggtacaa 11640
   atcggcgcgg cgctgggtga tgacctggtg gagaagttga aggccgcgca ggccgcccag 11700
   cggcaacgca tcgaggcaga agcacgcccc ggtgaatcgt ggcaagcggc cgctgatcga 11760
   atccgcaaag aatcccggca accgccggca gccggtgcgc cgtcgattag gaagccgccc 11820
   aagggcgacg agcaaccaga ttttttcgtt ccgatgctct atgacgtggg cacccgcgat 11880
   agtcgcagca tcatggacgt ggccgttttc cgtctgtcga agcgtgaccg acgagctggc 11940
   gaggtgatcc gctacgagct tccagacggg cacgtagagg tttccgcagg gccggccggc 12000
   atggccagtg tgtgggatta cgacctggta ctgatggcgg tttcccatct aaccgaatcc 12060
   atgaaccgat accgggaagg gaagggagac aagcccggcc gcgtgttccg tccacacgtt 12120
   gcggacgtac tcaagttctg ccggcgagcc gatggcggaa agcagaaaga cgacctggta 12180
   gaaacctgca ttcggttaaa caccacgcac gttgccatgc agcgtacgaa gaaggccaag 12240
   aacggccgcc tggtgacggt atccgagggt gaagccttga ttagccgcta caagatcgta 12300
   aagagcgaaa ccgggcggcc ggagtacatc gagatcgagc tagctgattg gatgtaccgc 12360
   gagatcacag aaggcaagaa cccggacgtg ctgacggttc accccgatta ctttttgatc 12420
   gatcccggca tcggccgttt tctctaccgc ctggcacgcc gcgccgcagg caaggcagaa 12480
   gccagatggt tgttcaagac gatctacgaa cgcagtggca gcgccggaga gttcaagaag 12540
   ttctgtttca ccgtgcgcaa gctgatcggg tcaaatgacc tgccggagta cgatttgaag 12600
   gaggaggcgg ggcaggctgg cccgatccta gtcatgcgct accgcaacct gatcgagggc 12660
   gaagcatccg ccggttccta atgtacggag cagatgctag ggcaaattgc cctagcaggg 12720
   gaaaaaggtc gaaaaggtct ctttcctgtg gatagcacgt acattgggaa cccaaagccg 12780
   tacattggga accggaaccc gtacattggg aacccaaagc cgtacattgg gaaccggtca 12840
   cacatgtaag tgactgatat aaaagagaaa aaaggcgatt tttccgccta aaactcttta 12900
   aaacttatta aaactcttaa aacccgcctg gcctgtgcat aactgtctgg ccagcgcaca 12960
   gccgaagagc tgcaaaaagc gcctaccctt cggtcgctgc gctccctacg ccccgccgct 13020
   tcgcgtcggc ctatcgcggc cgctggccgc tcaaaaatgg ctggcctacg gccaggcaat 13080
   ctaccagggc gcggacaagc cgcgccgtcg ccactcgacc gccggcgctg aggtctgcct 13140
   cgtgaagaag gtgttgctga ctcataccag gcctgaatcg ccccatcatc cagccagaaa 13200
   gtgagggagc cacggttgat gagagctttg ttgtaggtgg accagttggt gattttgaac 13260
   ttttgctttg ccacggaacg gtctgcgttg tcgggaagat gcgtgatctg atccttcaac 13320
   tcagcaaaag ttcgatttat tcaacaaagc cgccgtcccg tcaagtcagc gtaatgctct 13380
   gccagtgtta caaccaatta accaattctg attagaaaaa ctcatcgagc atcaaatgaa 13440
   actgcaattt attcatatca ggattatcaa taccatattt ttgaaaaagc cgtttctgta 13500
   atgaaggaga aaactcaccg aggcagttcc ataggatggc aagatcctgg tatcggtctg 13560
   cgattccgac tcgtccaaca tcaatacaac ctattaattt cccctcgtca aaaataaggt 13620
   tatcaagtga gaaatcacca tgagtgacga ctgaatccgg tgagaatggc aaaagctctg 13680
   cattaatgaa tcggccaacg cgcggggaga ggcggtttgc gtattgggcg ctcttccgct 13740
   tcctcgctca ctgactcgct gcgctcggtc gttcggctgc ggcgagcggt atcagctcac 13800
   tcaaaggcgg taatacggtt atccacagaa tcaggggata acgcaggaaa gaacatgtga 13860
   gcaaaaggcc agcaaaaggc caggaaccgt aaaaaggccg cgttgctggc gtttttccat 13920
   aggctccgcc cccctgacga gcatcacaaa aatcgacgct caagtcagag gtggcgaaac 13980
   ccgacaggac tataaagata ccaggcgttt ccccctggaa gctccctcgt gcgctctcct 14040
   gttccgaccc tgccgcttac cggatacctg tccgcctttc tcccttcggg aagcgtggcg 14100
   ctttctcata gctcacgctg taggtatctc agttcggtgt aggtcgttcg ctccaagctg 14160
   ggctgtgtgc acgaaccccc cgttcagccc gaccgctgcg ccttatccgg taactatcgt 14220
   cttgagtcca acccggtaag acacgactta tcgccactgg cagcagccac tggtaacagg 14280
   attagcagag cgaggtatgt aggcggtgct acagagttct tgaagtggtg gcctaactac 14340
   ggctacacta gaagaacagt atttggtatc tgcgctctgc tgaagccagt taccttcgga 14400
   aaaagagttg gtagctcttg atccggcaaa caaaccaccg ctggtagcgg tggttttttt 14460
   gtttgcaagc agcagattac gcgcagaaaa aaaggatctc aagaagatcc tttgatcttt 14520
   tctacggggt ctgacgctca gtggaacgaa aactcacgtt aagggatttt ggtcatgaga 14580
   ttatcaaaaa ggatcttcac ctagatcctt ttgatccgga attaattcct gtggttggca 14640
   tgcacataca aatggacgaa cggataaacc ttttcacgcc cttttaaata tccgattatt 14700
   ctaataaacg ctcttttctc ttaggtttac ccgccaatat atcctgtcaa acactgatag 14760
   tttaaactga aggcgggaaa cgacaatctg atcatgagcg gagaattaag ggagtcacgt 14820
   tatgaccccc gccgatgacg cgggacaagc cgttttacgt ttggaactga cagaaccgca 14880
   acgctgcagg aattggccgc agcggccatt taaatcaatt gggcgcgccg aattcgagct 14940
   cggtac                                                            14946
<210> 16
   <211> 14603
   <212> DNA
   <213> Artificial Sequence
<220>
   <223> Description of Artificial Sequence: pNOV1435
<220>
   <221> misc_feature
   <222> (1)..(2007)
   <223> synthetic nucleotide sequence encoding the toxin portion of H04 plus the first 40 amino acids of the Cry1Ab tail
<220>
   <221> misc_feature
   <222> Complement((8814)..(10022))
   <223> PMI
<220>
   <221> misc_feature
   <222> (11142)..(12032)
   <223> Maize ubiquitin promoter
<220>
   <221> misc_feature
   <222> (12037)..(14594)
   <223> MTL promoter
<400> 16
   atggacaaca accccaacat caacgagtgc atcccctaca actgcctgag caaccccgag 60
   gtggaggtgc tgggcggcga gcgcatcgag accggctaca cccccatcga catcagcctg 120
   agcctgaccc agttcctgct gagcgagttc gtgcccggcg ccggcttcgt gctgggcctg 180
   gtggacatca tctggggcat cttcggcccc agccagtggg acgccttcct ggtgcagatc 240
   gagcagttga taaaccaacg catagaggaa ttcgcccgca accaggccat cagccgcctg 300
   gagggcctga gcaacctgta ccaaatctac gccgagagct tccgcgagtg ggaggccgac 360
   cccaccaacc ccgccctgcg cgaggagatg cgcatccagt tcaacgacat gaacagcgcc 420
   ctgaccaccg ccatccccct gttcgccgtg cagaactacc aggtgcccct gctgagcgtg 480
   tacgtgcagg ccgccaacct gcacctgagc gtgctgcgcg acgtcagcgt gttcggccag 540
   cgctggggct tcgacgccgc caccatcaac agccgctaca acgacctgac ccgcctgatc 600
   ggcaactaca ccgaccacgc cgtgcgctgg tacaacaccg gcctggagcg cgtgtggggt 660
   cccgacagcc gcgactggat caggtacaac cagttccgcc gcgagctgac cctgaccgtg 720
   ctggacatcg tgagcctgtt ccccaactac gacagccgca cctaccccat ccgcaccgtg 780
   agccagctga cccgcgagat ttacaccaac cccgtgctgg agaacttcga cggcagcttc 840
   cgcggcagcg cccagggcat cgagggcagc atccgcagcc cccacctgat ggacatcctg 900
   aacagcatca ccatctacac cgacgcccac cgcggcgagt actactggag cggccaccag 960
   atcatggcca gccccgtcgg cttcagcggc cccgagttca ccttccccct gtacggcacc 1020
   atgggcaacg ctgcacctca gcagcgcatc gtggcacagc tgggccaggg agtgtaccgc 1080
   accctgagca gcaccctgta ccgtcgacct ttcaacatcg gcatcaacaa ccagcagctg 1140
   agcgtgctgg acggcaccga gttcgcctac ggcaccagca gcaacctgcc cagcgccgtg 1200
   taccgcaaga gcggcaccgt ggacagcctg gacgagatcc cccctcagaa caacaacgtg 1260
   ccacctcgac agggcttcag ccaccgtctg agccacgtga gcatgttccg cagtggcttc 1320
   agcaacagca gcgtgagcat catccgtgca cccatgttca gctggattca ccgcagcgcc 1380
   accctgacca acaccatcga ccccgagcgc atcaaccaga tccccctggt gaagggcttc 1440
   cgggtgtggg gcggcaccag cgtgatcacc ggccccggct tcaccggagg cgacatcctg 1500
   cgcagaaaca ccttcggcga cttcgtgagc ctgcaggtga acatcaacag ccccatcacc 1560
   cagcgttacc gcctgcgctt ccgctacgcc agcagccgcg acgcccgtgt gatcgtgctg 1620
   actggcgccg ctagcaccgg tgtgggcggt caggtgagcg tgaacatgcc cctgcagaag 1680
   actatggaga tcggcgagaa cctgactagt cgcaccttcc gctacaccga cttcagcaac 1740
   cccttcagct tccgcgccaa ccccgacatc atcggcatca gcgagcagcc cctgttcggt 1800
   gccggcagca tcagcagcgg cgagctgtac atcgacaaga tcgagatcat cctggccgac 1860
   gccaccttcg aggccgagag cgacctggag cgcgcccaga aggccgtgaa cgccctgttc 1920
   accagcagca accagatcgg cctgaagacc gacgtgaccg actaccacat cgaccaggtg 1980
   agcaacctgg tggactgctt aagctagaga tctgttctgc acaaagtgga gtagtcagtc 2040
   atcgatcagg aaccagacac cagactttta ttcatacagt gaagtgaagt gaagtgcagt 2100
   gcagtgagtt gctggttttt gtaccactta gtatgtattt gtatttgtaa aatacttcta 2160
   tcaataaaat ttctaattcc taaaaccaaa atccagtggg taccagcttg ggctgagtgg 2220
   ctccttcaac gttgcggttc tgtcagttcc aaacgtaaaa cggcttgtcc cgcgtcatcg 2280
   gcgggggtca taacgtgact cccttaattc tccgctcatg atcagattgt cgtttcccgc 2340
   cttcagttta aactatcagt gtttgacagg atatattggc gggtaaacct aagagaaaag 2400
   agcgtttatt agaataacgg atatttaaaa gggcgtgaaa aggtttatcc gttcgtccat 2460
   ttgtatgtgc atgccaacca cagggttccc ctcgggagtg cttggcattc cgtacgataa 2520
   tgacttctgt tcaaccaccc aaacgtcgga aagcctgacg acggagcagc attccaaaaa 2580
   gatcccttgg ctcgtctggg tcggctagaa ggtcgagtgg gctgctgtgg cttgatccct 2640
   caacgcggtc gcggacgtag cgcagcgccg aaaaatcctc gatcgcaaat ccgacgctgt 2700
   cgaaaagcgt gatctgcttg tcgctctttc ggccgacgtc ctggccagtc atcacgcgcc 2760
   aaagttccgt cacaggatga tctggcgcga gttgctggat ctcgccttca atccgggtct 2820
   gtggcgggaa ctccacgaaa atatccgaac gcagcaagat cgtcgaccaa ttcttgaaga 2880
   cgaaagggcc tcgtgatacg cctattttta taggttaatg tcatgataat aatggtttct 2940
   tagacgtcag gtggcacttt tcggggaaat gtgcgcggaa cccctatttg tttatttttc 3000
   taaatacatt caaatatgta tccgctcatg agacaataac cctgataaat gcttcaataa 3060
   tattgaaaaa ggaagagtat gagtattcaa catttccgtg tcgcccttat tccctttttt 3120
   gcggcatttt gccttcctgt ttttgctcac ccagaaacgc tggtgaaagt aaaagatgct 3180
   gaagatcagt tgggtgcacg agtgggttac atcgaactgg atctcaacag cggtaagatc 3240
   cttgagagtt ttcgccccga agaacgtttt ccaatgatga gcacttttaa agttctgcta 3300
   tgtggcgcgg tattatcccg tgttgacgcc gggcaagagc aactcggtcg ccgcatacac 3360
   tattctcaga atgacttggt tgagtactca ccagtcacag aaaagcatct tacggatggc 3420
   atgacagtaa gagaattatg cagtgctgcc ataaccatga gtgataacac tgcggccaac 3480
   ttacttctga caacgatcgg aggaccgaag gagctaaccg cttttttgca caacatgggg 3540
   gatcatgtaa ctcgccttga tcgttgggaa ccggagctga atgaagccat accaaacgac 3600
   gagcgtgaca ccacgatgcc tgcagggggg gggggggggg ggacatgagg ttgccccgta 3660
   ttcagtgtcg ctgatttgta ttgtctgaag ttgtttttac gttaagttga tgcagatcaa 3720
   ttaatacgat acctgcgtca taattgatta tttgacgtgg tttgatggcc tccacgcacg 3780
   ttgtgatatg tagatgataa tcattatcac tttacgggtc ctttccggtg atccgacagg 3840
   ttacggggcg gcgacctcgc gggttttcgc tatttatgaa aattttccgg tttaaggcgt 3900
   ttccgttctt cttcgtcata acttaatgtt tttatttaaa ataccctctg aaaagaaagg 3960
   aaacgacagg tgctgaaagc gaggcttttt ggcctctgtc gtttcctttc tctgtttttg 4020
   tccgtggaat gaacaatgga agtccccccc cccccccccc cctgcagcaa tggcaacaac 4080
   gttgcgcaaa ctattaactg gcgaactact tactctagct tcccggcaac aattaataga 4140
   ctggatggag gcggataaag ttgcaggacc acttctgcgc tcggcccttc cggctggctg 4200
   gtttattgct gataaatctg gagccggtga gcgtgggtct cgcggtatca ttgcagcact 4260
   ggggccagat ggtaagccct cccgtatcgt agttatctac acgacgggga gtcaggcaac 4320
   tatggatgaa cgaaatagac agatcgctga gataggtgcc tcactgatta agcattggta 4380
   actgtcagac caagtttact catatatact ttagattgat ttaaaacttc atttttaatt 4440
   taaaaggatc taggtgaaga tcctttttga taatctcatg accaaaatcc cttaacgtga 4500
   gttttcgttc cactgagcgt cagaccccgt agaaaagatc aaaggatctt cttgagatcc 4560
   tttttttctg cgcgtaatct gctgcttgca aacaaaaaaa ccaccgctac cagcggtggt 4620
   ttgtttgccg gatcaagagc taccaactct ttttccgaag gtaactggct tcagcagagc 4680
   gcagatacca aatactgtcc ttctagtgta gccgtagtta ggccaccact tcaagaactc 4740
   tgtagcaccg cctacatacc tcgctctgct aatcctgtta ccagtggctg ctgccagtgg 4800
   cgataagtcg tgtcttaccg ggttggactc aagacgatag ttaccggata aggcgcagcg 4860
   gtcgggctga acggggggtt cgtgcacaca gcccagcttg gagcgaacga cctacaccga 4920
   actgagatac ctacagcgtg agctatgaga aagcgccacg cttcccgaag ggagaaaggc 4980
   ggacaggtat ccggtaagcg gcagggtcgg aacaggagag cgcacgaggg agcttccagg 5040
   gggaaacgcc tggtatcttt atagtcctgt cgggtttcgc cacctctgac ttgagcgtcg 5100
   atttttgtga tgctcgtcag gggggcggag cctatggaaa aacgccagca acgcggcctt 5160
   tttacggttc ctggcctttt gctggccttt tgctcacatg ttctttcctg cgttatcccc 5220
   tgattctgtg gataaccgta ttaccgcctt tgagtgagct gataccgctc gccgcagccg 5280
   aacgaccgag cgcagcgagt cagtgagcga ggaagcggaa gagcgcctga tgcggtattt 5340
   tctccttacg catctgtgcg gtatttcaca ccgcatatgg tgcactctca gtacaatctg 5400
   ctctgatgcc gcatagttaa gccagtatac actccgctat cgctacgtga ctgggtcatg 5460
   gctgcgcccc gacacccgcc aacacccgct gacgcgccct gacgggcttg tctgctcccg 5520
   gcatccgctt acagacaagc tgtgaccgtc tccgggagct gcatgtgtca gaggttttca 5580
   ccgtcatcac cgaaacgcgc gaggcagcag atcccccgat caagtagata cactacatat 5640
   atctacaata gacatcgagc cggaaggtga tgtttacttt cctgaaatcc ccagcaattt 5700
   taggccagtt tttacccaag acttcgcctc taacataaat tatagttacc aaatctggca 5760
   aaagggttaa caagtggcag caacggattc gcaaacctgt cacgcctttt gtgccaaaag 5820
   ccgcgccagg tttgcgatcc gctgtgccag gcgttaggcg tcatatgaag atttcggtga 5880
   tccctgagca ggtggcggaa acattggatg ctgagaacca tttcattgtt cgtgaagtgt 5940
   tcgatgtgca cctatccgac caaggctttg aactatctac cagaagtgtg agcccctacc 6000
   ggaaggatta catctcggat gatgactctg atgaagactc tgcttgctat ggcgcattca 6060
   tcgaccaaga gcttgtcggg aagattgaac tcaactcaac atggaacgat ctagcctcta 6120
   tcgaacacat tgttgtgtcg cacacgcacc gaggcaaagg agtcgcgcac agtctcatcg 6180
   aatttgcgaa aaagtgggca ctaagcagac agctccttgg catacgatta gagacacaaa 6240
   cgaacaatgt acctgcctgc aatttgtacg caaaatgtgg ctttactctc ggcggcattg 6300
   acctgttcac gtataaaact agacctcaag tctcgaacga aacagcgatg tactggtact 6360
   ggttctcggg agcacaggat gacgcctaac aattcattca agccgacacc gcttcgcggc 6420
   gcggcttaat tcaggagtta aacatcatga gggaagcggt gatcgccgaa gtatcgactc 6480
   aactatcaga ggtagttggc gtcatcgagc gccatctcga accgacgttg ctggccgtac 6540
   atttgtacgg ctccgcagtg gatggcggcc tgaagccaca cagtgatatt gatttgctgg 6600
   ttacggtgac cgtaaggctt gatgaaacaa cgcggcgagc tttgatcaac gaccttttgg 6660
   aaacttcggc ttcccctgga gagagcgaga ttctccgcgc tgtagaagtc accattgttg 6720
   tgcacgacga catcattccg tggcgttatc cagctaagcg cgaactgcaa tttggagaat 6780
   ggcagcgcaa tgacattctt gcaggtatct tcgagccagc cacgatcgac attgatctgg 6840
   ctatcttgct gacaaaagca agagaacata gcgttgcctt ggtaggtcca gcggcggagg 6900
   aactctttga tccggttcct gaacaggatc tatttgaggc gctaaatgaa accttaacgc 6960
   tatggaactc gccgcccgac tgggctggcg atgagcgaaa tgtagtgctt acgttgtccc 7020
   gcatttggta cagcgcagta accggcaaaa tcgcgccgaa ggatgtcgct gccgactggg 7080
   caatggagcg cctgccggcc cagtatcagc ccgtcatact tgaagctagg caggcttatc 7140
   ttggacaaga agatcgcttg gcctcgcgcg cagatcagtt ggaagaattt gttcactacg 7200
   tgaaaggcga gatcaccaag gtagtcggca aataatgtct aacaattcgt tcaagccgac 7260
   gccgcttcgc ggcgcggctt aactcaagcg ttagagagct ggggaagact atgcgcgatc 7320
   tgttgaaggt ggttctaagc ctcgtacttg cgatggcatc ggggcaggca cttgctgacc 7380
   tgccaattgt tttagtggat gaagctcgtc ttccctatga ctactcccca tccaactacg 7440
   acatttctcc aagcaactac gacaactcca taagcaatta cgacaatagt ccatcaaatt 7500
   acgacaactc tgagagcaac tacgataata gttcatccaa ttacgacaat agtcgcaacg 7560
   gaaatcgtag gcttatatat agcgcaaatg ggtctcgcac tttcgccggc tactacgtca 7620
   ttgccaacaa tgggacaacg aacttctttt ccacatctgg caaaaggatg ttctacaccc 7680
   caaaaggggg gcgcggcgtc tatggcggca aagatgggag cttctgcggg gcattggtcg 7740
   tcataaatgg ccaattttcg cttgccctga cagataacgg cctgaagatc atgtatctaa 7800
   gcaactagcc tgctctctaa taaaatgtta ggcctcaaca tctagtcgca agctgagggg 7860
   aaccactagt gtcatacgaa cctccaagag acggttacac aaacgggtac attgttgatg 7920
   tcatgtatga caatcgccca agtaagtatc cagctgtgtt cagaacgtac gtccgaatta 7980
   attcatcggg gtacggtcga cgatcgtcaa cgttcacttc taaagaaata gcgccactca 8040
   gcttcctcag cggctttatc cagcgatttc ctattatgtc ggcatagttc tcaagatcga 8100
   cagcctgtca cggttaagcg agaaatgaat aagaaggctg ataattcgga tctctgcgag 8160
   ggagatgata tttgatcaca ggcagcaacg ctctgtcatc gttacaatca acatgctacc 8220
   ctccgcgaga tcatccgtgt ttcaaacccg gcagcttagt tgccgttctt ccgaatagca 8280
   tcggtaacat gagcaaagtc tgccgcctta caacggctct cccgctgacg ccgtcccgga 8340
   ctgatgggct gcctgtatcg agtggtgatt ttgtgccgag ctgccggtcg gggagctgtt 8400
   ggctggctgg tggcaggata tattgtggtg taaacaaatt gacgcttaga caacttaata 8460
   acacattgcg gacgttttta atgtactgaa ttgtctagac ccggggatct catgtttgac 8520
   agcttatcat cggatctagt aacatagatg acaccgcgcg cgataattta tcctagtttg 8580
   cgcgctatat tttgttttct atcgcgtatt aaatgtataa ttgcgggact ctaatcataa 8640
   aaacccatct cataaataac gtcatgcatt acatgttaat tattacatgc ttaacgtaat 8700
   tcaacagaaa ttagatgata atcatcgcaa gaccggcaac aggattcaat cttaagaaac 8760
   tttattgcca aatgtttgaa cgatctctgc aggtcgacgg atcgagctcc cagcttagca 8820
   agagatgtta attttttcag taagctctta cagcttgttg taaacacgcg ctaaacggcc 8880
   gtggcctttg acagtcaccg gtgattcgtt ggcggcaata aacgctgatt cacccggttt 8940
   aagctgtaac tgctgagaac ctttccacaa cgttgcatcg ccttcgacgc agaacaaaat 9000
   ggcggcactc tgctggctaa tggtggtttc tttatcacta aggtcatgca gcgagaaggc 9060
   aaaatcatcc actggaatcg ggaagtccag ttctgcacct tgtttcaccg gctgggtcaa 9120
   caactggtta gccggtttgg cttcgaattt cacattggca accagttccg gaatatcaat 9180
   gtatttaggc gtcagacccg cacgcagcac gttatcggag tttgccatca cttccagcgc 9240
   cacgccttgc aggtaagcgt gcggtgtttc agcgaacagg aacatcgctt cgccagggtt 9300
   caatttcacc acattcagca atagcgggga gaacagaccg ctgtcttccg ggtaaaattc 9360
   agaaattaaa cgaatcgttt gccacggttc accctgctgg ctatcgaggg ccgattttaa 9420
   aatcgccagc gcgcgggatt tttcttcacc ctgcatattc aacaggctgg cgaacagttc 9480
   gcttaaacgt tcggcatcag gctgttgtaa aaagtgagca atcgccggat gtgcacctgc 9540
   gaccggctgg agtagggaga caatctcgga aaattcacga aacgcgttca tcgcaaggaa 9600
   aggcgtcagc gcaaaaacca gctccggctt gtggttagga tctttatagt tacgctcggc 9660
   ggcatccatc gggatacctg cggcattttc tttggcaaaa ccgatttcag aattgtgttt 9720
   gtttggatga acctgaatgg agagtggctg tgctgcgcat aatactttga acaggaaagg 9780
   cagttcgcca aagcgtttgg caacggcctc tccgagcaga gtcgatttat cactctcaat 9840
   cacatcacgc agtgaaacga tatctccggc ggcattctgc actcgtgaac tgcttttcgg 9900
   atgtgcgccc atccacagct cggccatcgg ctggctggac ggattttcca taccataaag 9960
   ttcagtcaac gcgttttgct gccccaggca tagttttgca ctgagttaat gagtttttgc 10020
   atgatcgggg atccctgcag aagtaacacc aaacaacagg gtgagcatcg acaaaagaaa 10080
   cagtaccaag caaataaata gcgtatgaag gcagggctaa aaaaatccac atatagctgc 10140
   tgcatatgcc atcatccaag tatatcaaga tcaaaataat tataaaacat acttgtttat 10200
   tataatagat aggtactcaa ggttagagca tatgaataga tgctgcatat gccatcatgt 10260
   atatgcatca gtaaaaccca catcaacatg tatacctatc ctagatcgat atttccatcc 10320
   atcttaaact cgtaactatg aagatgtatg acacacacat acagttccaa aattaataaa 10380
   tacaccaggt agtttgaaac ggcgtctact ccgatctaga acgaatgaac gaccgcccaa 10440
   ccacaccaca tcatcacaac caagcgaaca aaaagcatct ctgtatatgc atcagtaaaa 10500
   cccgcatcaa catgtatacc tatcctagat cgatatttcc atccatcatc ttcaattcgt 10560
   aactatgaat atgtatggca cacacataca gatccaaaat taataaatcc accaggtagt 10620
   ttgaaacaga attctactcc gatctagaac gaccgcccaa ccagaccaca tcatcacaac 10680
   caagacaaaa aaaagcatga aaagatgacc cgacaaacaa gtgcacggca tatattgaaa 10740
   taaaggaaaa gggcaaacca aaccctatgc aacgaaacaa aaaaaatcat gaaatcgatc 10800
   ccgtctgcgg aacggctaga gccatcccag gattccccaa agagaaacac tggcaagtta 10860
   gcaatcagaa cgtgtctgac gtacaggtcg catccgtgta cgaacgctag cagcacggat 10920
   ctaacacaaa cacggatcta acacaaacat gaacagaagt agaactaccg ggccctaacc 10980
   atggaccgga acgccgatct agagaaggta gagagggggg gggggggagg acgagcggcg 11040
   taccttgaag cggaggtgcc gacgggtgga tttgggggag atctggttgt gtgtgtgtgc 11100
   gctccgaaca acacgaggtt ggggaaagag ggtgtggagg gggtgtctat ttattacggc 11160
   gggcgaggaa gggaaagcga aggagcggtg ggaaaggaat cccccgtagc tgccgtgccg 11220
   tgagaggagg aggaggccgc ctgccgtgcc ggctcacgtc tgccgctccg ccacgcaatt 11280
   tctggatgcc gacagcggag caagtccaac ggtggagcgg aactctcgag aggggtccag 11340
   aggcagcgac agagatgccg tgccgtctgc ttcgcttggc ccgacgcgac gctgctggtt 11400
   cgctggttgg tgtccgttag actcgtcgac ggcgtttaac aggctggcat tatctactcg 11460
   aaacaagaaa aatgtttcct tagttttttt aatttcttaa agggtatttg tttaattttt 11520
   agtcacttta ttttattcta ttttatatct aaattattaa ataaaaaaac taaaatagag 11580
   ttttagtttt cttaatttag aggctaaaat agaataaaat agatgtacta aaaaaattag 11640
   tctataaaaa ccattaaccc taaaccctaa atggatgtac taataaaatg gatgaagtat 11700
   tatataggtg aagctatttg caaaaaaaaa ggagaacaca tgcacactaa aaagataaaa 11760
   ctgtagagtc ctgttgtcaa aatactcaat tgtcctttag accatgtcta actgttcatt 11820
   tatatgattc tctaaaacac tgatattatt gtagtactat agattatatt attcgtagag 11880
   taaagtttaa atatatgtat aaagatagat aaactgcact tcaaacaagt gtgacaaaaa 11940
   aaatatgtgg taatttttta taacttagac atgcaatgct cattatctct agagaggggc 12000
   acgaccgggt cacgctgcac tgcaggcatg caagcttgca catgacaaca attgtaagag 12060
   gatggagacc acaacgatcc aacaatactt ctgcgacggg ctgtgaagta tagagaagtt 12120
   aaacgcccaa aagccattgt gtttggaatt tttagttatt ctatttttca tgatgtatct 12180
   tcctctaaca tgccttaatt tgcaaatttg gtataactac tgattgaaaa tatatgtatg 12240
   taaaaaaata ctaagcatat ttgtgaagct aaacatgatg ttatttaaga aaatatgttg 12300
   ttaacagaat aagattaata tcgaaatgga aacatctgta aattagaatc atcttacaag 12360
   ctaagagatg ttcacgcttt gagaaacttc ttcagatcat gaccgtagaa gtagctctcc 12420
   aagactcaac gaaggctgct gcaattccac aaatgcatga catgcatcct tgtaaccgtc 12480
   gtcgccgcta taaacacgga taactcaatt ccctgctcca tcaatttaga aatgagcaag 12540
   caagcacccg atcgctcacc ccatatgcac caatctgact cccaagtctc tgtttcgcat 12600
   tagtaccgcc agcactccac ctatagctac caattgagac ctttccagcc taagcagatc 12660
   gattgatcgt tagagtcaaa gagttggtgg tacgggtact ttaactacca tggaatgatg 12720
   gggcgtgatg tagagcggaa agcgcctccc tacgcggaac aacaccctcg ccatgccgct 12780
   cgactacagc ctcctcctcg tcggccgccc acaacgaggg agcccgtggt cgcagccacc 12840
   gaccagcatg tctctgtgtc ctcgtccgac ctcgacatgt catggcaaac agtcggacgc 12900
   cagcaccaga ctgacgacat gagtctctga agagcccgcc acctagaaag atccgagccc 12960
   tgctgctggt agtggtaacc attttcgtcg cgctgacgcg gagagcgaga ggccagaaat 13020
   ttatagcgac tgacgctgtg gcaggcacgc tatcggaggt tacgacgtgg cgggtcactc 13080
   gacgcggagt tcacaggtcc tatccttgca tcgctcgggc cggagtttac gggacttatc 13140
   cttacgacgt gctctaaggt tgcgataacg ggcggaggaa ggcgtgtggc gtgcggagac 13200
   ggtttataca cgtagtgtgc gggagtgtgt ttcgtagacg cgggaaagca cgacgactta 13260
   cgaaggttag tggaggagga ggacacacta aaatcaggac gcaagaaact cttctattat 13320
   agtagtagag aagagattat aggagtgtgg gttgattcta aagaaaatcg acgcaggaca 13380
   accgtcaaaa cgggtgcttt aatatagtag atatatatat atagagagag agagaaagta 13440
   caaaggatgc atttgtgtct gcatatgatc ggagtattac taacggccgt cgtaagaagg 13500
   tccatcatgc gtggagcgag cccatttggt tggttgtcag gccgcagtta aggcctccat 13560
   atatgattgt cgtcgggccc ataacagcat ctcctccacc agtttattgt aagaataaat 13620
   taagtagaga tatttgtcgt cgggcagaag aaacttggac aagaagaaga agcaagctag 13680
   gccaatttct tgccggcaag aggaagatag tggcctctag tttatatatc ggcgtgatga 13740
   tgatgctcct agctagaaat gagagaagaa aaacggacgc gtgtttggtg tgtgtcaatg 13800
   gcgtccatcc ttccatcaga tcagaacgat gaaaaagtca agcacggcat gcatagtata 13860
   tgtatagctt gttttagtgt ggctttgctg agacgaatga aagcaacggc gggcatattt 13920
   ttcagtggct gtagctttca ggctgaaaga gacgtggcat gcaataattc agggaattcg 13980
   tcagccaatt gaggtagcta gtcaacttgt acattggtgc gagcaatttt ccgcactcag 14040
   gagggctagt ttgagagtcc aaaaactata ggagattaaa gaggctaaaa tcctctcctt 14100
   atttaatttt aaataagtag tgtatttgta ttttaactcc tccaaccctt ccgattttat 14160
   ggctctcaaa ctagcattca gtctaatgca tgcatgcttg gctagaggtc gtatggggtt 14220
   gttaatagca tagctagcta caagttaacc gggtctttta tatttaataa ggacaggcaa 14280
   agtattactt acaaataaag aataaagcta ggacgaactc gtggattatt actaaatcga 14340
   aatggacgta atattccagg caagaataat tgttcgatca ggagacaagt ggggcattgg 14400
   accggttctt gcaagcaaga gcctatggcg tggtgacacg gcgcgttgcc catacatcat 14460
   gcctccatcg atgatccatc ctcacttgct ataaaaagag gtgtccatgg tgctcaagct 14520
   cagccaagca aataagacga cttgtttcat tgattcttca agagatcgag cttcttttgc 14580
   accacaaggt cgaggatcca aca                                         14603
<210> 17
   <211> 11127
   <212> DNA
   <213> Artificial Sequence
<220>
   <223> Description of Artificial Sequence: pZU578
<220>
   <221> misc_feature
   <222> (1485)..(3491)
   <223> synthetic nucleotide sequence encoding the toxin portion of H04 plus the first 40 amino acids of the Cry1Ab tail
<220>
   <221> misc_feature
   <222> (5052)..(6271)
   <223> PMI
<220>
   <221> misc_feature
   <222> (3859)..(5030)
   <223> SMAS promoter
<220>
   <221> misc_feature
   <222> (56)..(1475)
   <223> Actin 2 promoter U41998
<400> 17
   ggccgcagcg gccatttaaa tcaattgggc gcgccgaatt cgagctcggt accctgcatg     60
   cctgcaggtc gacaaaattt agaacgaact taattatgat ctcaaataca ttgatacata    120
   tctcatctag atctaggtta tcattatgta agaaagtttt gacgaatatg gcacgacaaa    180
   atggctagac tcgatgtaat tggtatctca actcaacatt atacttatac caaacattag    240
   ttagacaaaa tttaaacaac tattttttat gtatgcaaga gtcagcatat gtataattga    300
   ttcagaatcg ttttgacgag ttcggatgta gtagtagcca ttatttaatg tacatactaa    360
   tcgtgaatag tgaatatgat gaagcattgt atcttattgt ataaatatcc ataaacacat    420
   catgaaagac actttctttc acggtctgaa ttaattatga cacaattcta atagaaaacg    480
   aattaaatta cgttgaattg tatgaaatct aattgaacaa gccaaccacg acgacgacta    540
   acgttgcctg gattgactcg gtttaagtta accactaaaa aaacggagct gtcatgtaac    600
   acgcggatcg agcaggtcac agtcatgaag ccatcaaagc aaaagaacta atccaagggc    660
   tgagatgatt aattagttta aaaattagtt aacacgaggg aaaaggctgt ctgacagcca    720
   ggtcacgtta tctttacctg tggtcgaaat gattcgtgtc tgtcgatttt aattattttt    780
   ttgaaaggcc gaaaataaag ttgtaagaga taaacccgcc tatataaatt catatatttt    840
   cctctccgct ttgaattgtc tcgttgtcct cctcactttc atcagccgtt ttgaatctcc    900
   ggcgacttga cagagaagaa caaggaagaa gactaagaga gaaagtaaga gataatccag    960
   gagattcatt ctccgttttg aatcttcctc aatctcatct tcttccgctc tttctttcca   1020
   aggtaatagg aactttctgg atctacttta tttgctggat ctcgatcttg ttttctcaat   1080
   ttccttgaga tctggaattc gtttaatttg gatctgtgaa cctccactaa atcttttggt   1140
   tttactagaa tcgatctaag ttgaccgatc agttagctcg attatagcta ccagaatttg   1200
   gcttgacctt gatggagaga tccatgttca tgttacctgg gaaatgattt gtatatgtga   1260
   attgaaatct gaactgttga agttagattg aatctgaaca ctgtcaatgt tagattgaat   1320
   ctgaacactg tttaagttag atgaagtttg tgtatagatt cttcgaaact ttaggatttg   1380
   tagtgtcgta cgttgaacag aaagctattt ctgattcaat cagggtttat ttgactgtat   1440
   tgaactcttt ttgtgtgttt gcagctcata aaaaggatcc aacaatggac aacaacccca   1500
   acatcaacga gtgcatcccc tacaactgcc tgagcaaccc cgaggtggag gtgctgggcg   1560
   gcgagcgcat cgagaccggc tacaccccca tcgacatcag cctgagcctg acccagttcc   1620
   tgctgagcga gttcgtgccc ggcgccggct tcgtgctggg cctggtggac atcatctggg   1680
   gcatcttcgg ccccagccag tgggacgcct tcctggtgca gatcgagcag ttgataaacc   1740
   aacgcataga ggaattcgcc cgcaaccagg ccatcagccg cctggagggc ctgagcaacc   1800
   tgtaccaaat ctacgccgag agcttccgcg agtgggaggc cgaccccacc aaccccgccc   1860
   tgcgcgagga gatgcgcatc cagttcaacg acatgaacag cgccctgacc accgccatcc   1920
   ccctgttcgc cgtgcagaac taccaggtgc ccctgctgag cgtgtacgtg caggccgcca   1980
   acctgcacct gagcgtgctg cgcgacgtca gcgtgttcgg ccagcgctgg ggcttcgacg   2040
   ccgccaccat caacagccgc tacaacgacc tgacccgcct gatcggcaac tacaccgacc   2100
   acgccgtgcg ctggtacaac accggcctgg agcgcgtgtg gggtcccgac agccgcgact   2160
   ggatcaggta caaccagttc cgccgcgagc tgaccctgac cgtgctggac atcgtgagcc   2220
   tgttccccaa ctacgacagc cgcacctacc ccatccgcac cgtgagccag ctgacccgcg   2280
   agatttacac caaccccgtg ctggagaact tcgacggcag cttccgcggc agcgcccagg   2340
   gcatcgaggg cagcatccgc agcccccacc tgatggacat cctgaacagc atcaccatct   2400
   acaccgacgc ccaccgcggc gagtactact ggagcggcca ccagatcatg gccagccccg   2460
   tcggcttcag cggccccgag ttcaccttcc ccctgtacgg caccatgggc aacgctgcac   2520
   ctcagcagcg catcgtggca cagctgggcc agggagtgta ccgcaccctg agcagcaccc   2580
   tgtaccgtcg acctttcaac atcggcatca acaaccagca gctgagcgtg ctggacggca   2640
   ccgagttcgc ctacggcacc agcagcaacc tgcccagcgc cgtgtaccgc aagagcggca   2700
   ccgtggacag cctggacgag atcccccctc agaacaacaa cgtgccacct cgacagggct   2760
   tcagccaccg tctgagccac gtgagcatgt tccgcagtgg cttcagcaac agcagcgtga   2820
   gcatcatccg tgcacccatg ttcagctgga ttcaccgcag cgccaccctg accaacacca   2880
   tcgaccccga gcgcatcaac cagatccccc tggtgaaggg cttccgggtg tggggcggca   2940
   ccagcgtgat caccggcccc ggcttcaccg gaggcgacat cctgcgcaga aacaccttcg   3000
   gcgacttcgt gagcctgcag gtgaacatca acagccccat cacccagcgt taccgcctgc   3060
   gcttccgcta cgccagcagc cgcgacgccc gtgtgatcgt gctgactggc gccgctagca   3120
   ccggtgtggg cggtcaggtg agcgtgaaca tgcccctgca gaagactatg gagatcggcg   3180
   agaacctgac tagtcgcacc ttccgctaca ccgacttcag caaccccttc agcttccgcg   3240
   ccaaccccga catcatcggc atcagcgagc agcccctgtt cggtgccggc agcatcagca   3300
   gcggcgagct gtacatcgac aagatcgaga tcatcctggc cgacgccacc ttcgaggccg   3360
   agagcgacct ggagcgcgcc cagaaggccg tgaacgccct gttcaccagc agcaaccaga   3420
   tcggcctgaa gaccgacgtg accgactacc acatcgacca ggtgagcaac ctggtggact   3480
   gcttaagcta gagatcctct agagtcgacc atggtgatca ctgcagatcg ttcaaacatt   3540
   tggcaataaa gtttcttaag attgaatcct gttgccggtc ttgcgatgat tatcatataa   3600
   tttctgttga attacgttaa gcatgtaata attaacatgt aatgcatgac gttatttatg   3660
   agatgggttt ttatgattag agtcccgcaa ttatacattt aatacgcgat agaaaacaaa   3720
   atatagcgcg caacctagga taaattatcg cgcgcggtgt catctatgtt actagatctc   3780
   tagaaagctt cgtacgttaa ttaattcgaa tccggagcgg ccgcagggct agcatcgatg   3840
   gtaccgagct cgagactata caggccaaat tcgctcttag ccgtacaata ttactcaccg   3900
   gtgcgatgcc ccccatcgta ggtgaaggtg gaaattaatg atccatcttg agaccacagg   3960
   cccacaacag ctaccagttt cctcaagggt ccaccaaaaa cgtaagcgct tacgtacatg   4020
   gtcgataaga aaaggcaatt tgtagatgtt aacatccaac gtcgctttca gggatcccga   4080
   attccaagct tggaattcgg gatcctacag gccaaattcg ctcttagccg tacaatatta   4140
   ctcaccggtg cgatgccccc catcgtaggt gaaggtggaa attaatgatc catcttgaga   4200
   ccacaggccc acaacagcta ccagtttcct caagggtcca ccaaaaacgt aagcgcttac   4260
   gtacatggtc gataagaaaa ggcaatttgt agatgttaac atccaacgtc gctttcaggg   4320
   atcccgaatt ccaagcttgg aattcgggat cctacaggcc aaattcgctc ttagccgtac   4380
   aatattactc accggtgcga tccccccatc gtaggtgaag gtggaaatta atgatccatc   4440
   ttgagaccac aggcccacaa cagctaccag tttcctcaag ggtccaccaa aaacgtaagc   4500
   gcttacgtac atggtcgata agaaaaggca atttgtagat gttaacatcc aacgtcgctt   4560
   tcagggatcc cgaattccaa gcttgggctg caggtcaatc ccattgcttt tgaagcagct   4620
   caacattgat ctctttctcg agggagattt ttcaaatcag tgcgcaagac gtgacgtaag   4680
   tatccgagtc agtttttatt tttctactaa tttggtcgtt tatttcggcg tgtaggacat   4740
   ggcaaccggg cctgaatttc gcgggtattc tgtttctatt ccaacttttt cttgatccgc   4800
   agccattaac gacttttgaa tagatacgct gacacgccaa gcctcgctag tcaaaagtgt   4860
   accaaacaac gctttacagc aagaacggaa tgcgcgtgac gctcgcggtg acgccatttc   4920
   gccttttcag aaatggataa atagccttgc ttcctattat atcttcccaa attaccaata   4980
   cattacacta gcatctgaat ttcataacca atctcgatac accaaatcga gatctgcagg   5040
   gatccccgat catgcaaaaa ctcattaact cagtgcaaaa ctatgcctgg ggcagcaaaa   5100
   cggcgttgac tgaactttat ggtatggaaa atccgtccag ccagccgatg gccgagctgt   5160
   ggatgggcgc acatccgaaa agcagttcac gagtgcagaa tgccgccgga gatatcgttt 5220
   cactgcgtga tgtgattgag agtgataaat cgactctgct cggagaggcc gttgccaaac 5280
   gctttggcga actgcctttc ctgttcaaag tattatgcgc agcacagcca ctctccattc 5340
   aggttcatcc aaacaaacac aattctgaaa tcggttttgc caaagaaaat gccgcaggta 5400
   tcccgatgga tgccgccgag cgtaactata aagatcctaa ccacaagccg gagctggttt 5460
   ttgcgctgac gcctttcctt gcgatgaacg cgtttcgtga attttccgag attgtctccc 5520
   tactccagcc ggtcgcaggt gcacatccgg cgattgctca ctttttacaa cagcctgatg 5580
   ccgaacgttt aagcgaactg ttcgccagcc tgttgaatat gcagggtgaa gaaaaatccc 5640
   gcgcgctggc gattttaaaa tcggccctcg atagccagca gggtgaaccg tggcaaacga 5700
   ttcgtttaat ttctgaattt tacccggaag acagcggtct gttctccccg ctattgctga 5760
   atgtggtgaa attgaaccct ggcgaagcga tgttcctgtt cgctgaaaca ccgcacgctt 5820
   acctgcaagg cgtggcgctg gaagtgatgg caaactccga taacgtgctg cgtgcgggtc 5880
   tgacgcctaa atacattgat attccggaac tggttgccaa tgtgaaattc gaagccaaac 5940
   cggctaacca gttgttgacc cagccggtga aacaaggtgc agaactggac ttcccgattc 6000
   cagtggatga ttttgccttc tcgctgcatg accttagtga taaagaaacc accattagcc 6060
   agcagagtgc cgccattttg ttctgcgtcg aaggcgatgc aacgttgtgg aaaggttctc 6120
   agcagttaca gcttaaaccg ggtgaatcag cgtttattgc cgccaacgaa tcaccggtga 6180
   ctgtcaaagg ccacggccgt ttagcgcgtg tttacaacaa gctgtaagag cttactgaaa 6240
   aaattaacat ctcttgctaa gctgggagct cgtcgacgga tcgaattcct gcagatcgtt 6300
   caaacatttg gcaataaagt ttcttaagat tgaatcctgt tgccggtctt gcgatgatta 6360
   tcatataatt tctgttgaat tacgttaagc atgtaataat taacatgtaa tgcatgacgt 6420
   tatttatgag atgggttttt atgattagag tcccgcaatt atacatttaa tacgcgatag 6480
   aaaacaaaat atagcgcgca aactaggata aattatcgcg cgcggtgtca tctatgttac 6540
   tagatctcta gaactagtgg atctgctagc cctgcaggaa atttaccggt gcccgggcgg 6600
   ccagcatggc cgtatccgca atgtgttatt aagttgtcta agcgtcaatt tgtttacacc 6660
   acaatatatc ctgccaccag ccagccaaca gctccccgac cggcagctcg gcacaaaatc 6720
   accactcgat acaggcagcc catcagaatt aattctcatg tttgacagct tatcatcgac 6780
   tgcacggtgc accaatgctt ctggcgtcag gcagccatcg gaagctgtgg tatggctgtg 6840
   caggtcgtaa atcactgcat aattcgtgtc gctcaaggcg cactcccgtt ctggataatg 6900
   ttttttgcgc cgacatcata acggttctgg caaatattct gaaatgagct gttgacaatt 6960
   aatcatcggc tcgtataatg tgtggaattg tgagcggata acaatttcac acaggaaaca 7020
   gaccatgagg gaagcggtga tcgccgaagt atcgactcaa ctatcagagg tagttggcgt 7080
   catcgagcgc catctcgaac cgacgttgct ggccgtacat ttgtacggct ccgcagtgga 7140
   tggcggcctg aagccacaca gtgatattga tttgctggtt acggtgaccg taaggcttga 7200
   tgaaacaacg cggcgagctt tgatcaacga ccttttggaa acttcggctt cccctggaga 7260
   gagcgagatt ctccgcgctg tagaagtcac cattgttgtg cacgacgaca tcattccgtg 7320
   gcgttatcca gctaagcgcg aactgcaatt tggagaatgg cagcgcaatg acattcttgc 7380
   aggtatcttc gagccagcca cgatcgacat tgatctggct atcttgctga caaaagcaag 7440
   agaacatagc gttgccttgg taggtccagc ggcggaggaa ctctttgatc cggttcctga 7500
   acaggatcta tttgaggcgc taaatgaaac cttaacgcta tggaactcgc cgcccgactg 7560
   ggctggcgat gagcgaaatg tagtgcttac gttgtcccgc atttggtaca gcgcagtaac 7620
   cggcaaaatc gcgccgaagg atgtcgctgc cgactgggca atggagcgcc tgccggccca 7680
   gtatcagccc gtcatacttg aagctaggca ggcttatctt ggacaagaag atcgcttggc 7740
   ctcgcgcgca gatcagttgg aagaatttgt tcactacgtg aaaggcgaga tcaccaaggt 7800
   agtcggcaaa taaagctcta gtggatcccc gaggaatcgg cgtgacggtc gcaaaccatc 7860
   cggcccggta caaatcggcg cggcgctggg tgatgacctg gtggagaagt tgaaggccgc 7920
   gcaggccgcc cagcggcaac gcatcgaggc agaagcacgc cccggtgaat cgtggcaagc 7980
   ggccgctgat cgaatccgca aagaatcccg gcaaccgccg gcagccggtg cgccgtcgat 8040
   taggaagccg cccaagggcg acgagcaacc agattttttc gttccgatgc tctatgacgt 8100
   gggcacccgc gatagtcgca gcatcatgga cgtggccgtt ttccgtctgt cgaagcgtga 8160
   ccgacgagct ggcgaggtga tccgctacga gcttccagac gggcacgtag aggtttcagc 8220
   agggccggcc ggcatggcca gtgtgtggga ttacgacctg gtactgatgg cggtttccca 8280
   tctaaccgaa tccatgaacc gataccggga agggaaggga gacaagcccg gccgcgtgtt 8340
   ccgtccacac gttgcggacg tactcaagtt ctgccggcga gccgatggcg gaaagcagaa 8400
   agacgacctg gtagaaacct gcattcggtt aaacaccacg cacgttgcca tgcagcgtac 8460
   gaagaaggcc aagaacggcc gcctggtgac ggtatccgag ggtgaagcct tgattagccg 8520
   ctacaagatc gtaaagagcg aaaccgggcg gccggagtac atcgagatcg agctagctga 8580
   ttggatgtac cgcgagatca cagaaggcaa gaacccggac gtgctgacgg ttcaccccga 8640
   ttactttttg atcgatcccg gcatcggccg ttttctctac cgcctggcac gccgcgccgc 8700
   aggcaaggca gaagccagat ggttgttcaa gacgatctac gaacgcagtg gcagcgccgg 8760
   agagttcaag aagttctgtt tcaccgtgcg caagctgatc gggtcaaatg acctgccgga 8820
   gtacgatttg aaggaggagg cggggcaggc tggcccgatc ctagtcatgc gctaccgcaa   8880
   cctgatcgag ggcgaagcat ccgccggttc ctaatgtacg gagcagatgc tagggcaaat   8940
   tgccctagca ggggaaaaag gtcgaaaagg tctctttcct gtggatagca cgtacattgg   9000
   gaacccaaag ccgtacattg ggaaccggaa cccgtacatt gggaacccaa agccgtacat   9060
   tgggaaccgg tcacacatgt aagtgactga tataaaagag aaaaaaggcg atttttccgc   9120
   ctaaaactct ttaaaactta ttaaaactct taaaacccgc ctggcctgtg cataactgtc   9180
   tggccagcgc acagccgaag agctgcaaaa agcgcctacc cttcggtcgc tgcgctccct   9240
   acgccccgcc gcttcgcgtc ggcctatcgc ggccgctggc cgctcaaaaa tggctggcct   9300
   acggccaggc aatctaccag ggcgcggaca agccgcgccg tcgccactcg accgccggcg   9360
   ctgaggtctg cctcgtgaag aaggtgttgc tgactcatac caggcctgaa tcgccccatc   9420
   atccagccag aaagtgaggg agccacggtt gatgagagct ttgttgtagg tggaccagtt   9480
   ggtgattttg aacttttgct ttgccacgga acggtctgcg ttgtcgggaa gatgcgtgat   9540
   ctgatccttc aactcagcaa aagttcgatt tattcaacaa agccgccgtc ccgtcaagtc   9600
   agcgtaatgc tctgccagtg ttacaaccaa ttaaccaatt ctgattagaa aaactcatcg   9660
   agcatcaaat gaaactgcaa tttattcata tcaggattat caataccata tttttgaaaa   9720
   agccgtttct gtaatgaagg agaaaactca ccgaggcagt tccataggat ggcaagatcc   9780
   tggtatcggt ctgcgattcc gactcgtcca acatcaatac aacctattaa tttcccctcg   9840
   tcaaaaataa ggttatcaag tgagaaatca ccatgagtga cgactgaatc cggtgagaat   9900
   ggcaaaagct ctgcattaat gaatcggcca acgcgcgggg agaggcggtt tgcgtattgg   9960
   gcgctcttcc gcttcctcgc tcactgactc gctgcgctcg gtcgttcggc tgcggcgagc  10020
   ggtatcagct cactcaaagg cggtaatacg gttatccaca gaatcagggg ataacgcagg  10080
   aaagaacatg tgagcaaaag gccagcaaaa ggccaggaac cgtaaaaagg ccgcgttgct  10140
   ggcgtttttc cataggctcc gcccccctga cgagcatcac aaaaatcgac gctcaagtca  10200
   gaggtggcga aacccgacag gactataaag ataccaggcg tttccccctg gaagctccct  10260
   cgtgcgctct cctgttccga ccctgccgct taccggatac ctgtccgcct ttctcccttc  10320
   gggaagcgtg gcgctttctc aatgctcacg ctgtaggtat ctcagttcgg tgtaggtcgt  10380
   tcgctccaag ctgggctgtg tgcacgaacc ccccgttcag cccgaccgct gcgccttatc  10440
   cggtaactat cgtcttgagt ccaacccggt aagacacgac ttatcgccac tggcagcagc  10500
   cactggtaac aggattagca gagcgaggta tgtaggcggt gctacagagt tcttgaagtg  10560
   gtggcctaac tacggctaca ctagaaggac agtatttggt atctgcgctc tgctgaagcc  10620
   agttaccttc ggaaaaagag ttggtagctc ttgatccggc aaacaaacca ccgctggtag  10680
   cggtggtttt tttgtttgca agcagcagat tacgcgcaga aaaaaaggat ctcaagaaga  10740
   tcctttgatc ttttctacgg ggtctgacgc tcagtggaac gaaaactcac gttaagggat  10800
   tttggtcatg agattatcaa aaaggatctt cacctagatc cttttgatcc ggaattaatt  10860
   cctgtggttg gcatgcacat acaaatggac gaacggataa accttttcac gcccttttaa  10920
   atatccgatt attctaataa acgctctttt ctcttaggtt tacccgccaa tatatcctgt  10980
   caaacactga tagtttaaac tgaaggcggg aaacgacaat ctgatcatga gcggagaatt  11040
   aagggagtca cgttatgacc cccgccgatg acgcgggaca agccgtttta cgtttggaac  11100
   tgacagaacc gcaacgctgc aggaatt                                     11127